# EUROPEAN PATENT SPECIFICATION

(11) **EP 4 644 573 B1**
(45) Date of publication and mention of the grant of the patent: **28.10.2026**
(21) Application number: 25185379.2
(22) Date of filing: 04.05.2021
(51) Int. Cl.: C12N 9/64, C12Q 1/68, G01N 33/53, G01N 33/567, C12Q 1/6897

(54) **COMPOSITIONS, SYSTEMS, AND METHODS FOR THE GENERATION, IDENTIFICATION, AND CHARACTERIZATION OF EFFECTOR DOMAINS FOR ACTIVATING AND SILENCING GENE EXPRESSION**
ZUSAMMENSETZUNGEN, SYSTEME UND VERFAHREN ZUR ERZEUGUNG, IDENTIFIZIERUNG UND CHARAKTERISIERUNG VON EFFEKTORDOMÄNEN ZUR AKTIVIERUNG UND DÄMPFUNG VON GENEXPRESSION
COMPOSITIONS, SYSTÈMES ET PROCÉDÉS DE GÉNÉRATION, D'IDENTIFICATION ET DE CARACTÉRISATION DE DOMAINES EFFECTEURS POUR L'ACTIVATION ET LE SILENÇAGE DE L'EXPRESSION GÉNIQUE

(30) Priority: 04.05.2020 US 202063019706 P; 04.09.2020 US 202063074793 P
(43) Date of publication of application: 05.11.2025
(62) Document number(s) of the earlier application(s) in accordance with Art. 76 EPC: 21800824.1 / 4 146 801
(73) Proprietor: The Board of Trustees of the Leland Stanford Junior University, Stanford, CA 94305-2038 (US)
(72) Inventor: Bassik, Michael C., Stanford, 94305-2038 (US); Tycko, Josh, Stanford, 94305-2038 (US); Hess, Gaelen T., Stanford, 94305-2038 (US); Bintu, Lacramioara, Stanford, 94305-2038 (US)
(74) Representative: GLAWE DELFS MOLL

(56) References cited:
- WO-A1-2018/204764
- WO-A1-2019/079462
- WO-A1-2019/173636
- WO-A2-2006/055786
- US-A1- 2017 198 308
- STALLER MAX V. ET AL: "A High-Throughput Mutational Scan of an Intrinsically Disordered Acidic Transcriptional Activation Domain", CELL SYSTEMS, vol. 6, no. 4, 25 April 2018 (2018-04-25), US, pages 444 - 455.e6, XP055803792, ISSN: 2405-4712, DOI: 10.1016/j.cels.2018.01.015
- ARNOLD COSMAS D. ET AL: "A high-throughput method to identify trans-activation domains within transcription factor sequences", THE EMBO JOURNAL, vol. 37, no. 16, 15 August 2018 (2018-08-15), pages e98896, XP093027463, ISSN: 1460-2075, DOI: 10.15252/embj.201798896
- DATABASE Geneseq [online] 10 January 2019 (2019-01-10), "Human-derived TF/CR signaling protein, SEQ:31337.", XP002821478, retrieved from EBI accession no. GSP:BFV31374 Database accession no. BFV31374
- DATABASE Geneseq [online] 10 January 2019 (2019-01-10), "Human-derived TF/CR signaling protein, SEQ:31338.", XP002821479, retrieved from EBI accession no. GSP:BFV31375 Database accession no. BFV31375
- DATABASE Geneseq [online] 10 January 2019 (2019-01-10), "Human-derived TF/CR signaling protein, SEQ:31339.", XP002821480, retrieved from EBI accession no. GSP:BFV31376 Database accession no. BFV31376
- DATABASE Geneseq [online] 10 January 2019 (2019-01-10), "Human-derived TF/CR signaling protein, SEQ:31342.", XP002821481, retrieved from EBI accession no. GSP:BFV31379 Database accession no. BFV31379
- DATABASE Geneseq [online] 10 January 2019 (2019-01-10), "Human-derived TF/CR signaling protein, SEQ:10299.", XP002821482, retrieved from EBI accession no. GSP:BFV10336 Database accession no. BFV10336
- DATABASE Geneseq [online] 31 October 2019 (2019-10-31), "Human exemplary transcription factor, SEQ 16186.", XP002821483, retrieved from EBI accession no. GSP:BGT47678 Database accession no. BGT47678

## Description

### FIELD

The invention is set out in the appended claims. The embodiments of the description which do not fall within the scope of said claims are provided for illustrative purposes only and do not form part of the present invention. Provided herein are compositions, systems, and methods for the generation, identification, and characterization of effector domains for activating and silencing gene expression. In particular, high throughput systems are provided to discover and characterize effector domains.

### STATEMENT REGARDING FEDERALLY-SPONSORED RESEARCH

This invention was made with Government support under contract GM128947 awarded by the National Institutes of Health. The Government has certain rights in the invention.

### BACKGROUND

Previous efforts to engineer synthetic transcription factors have pulled activation and repressor domains from a small toolbox of previously-discovered effector domains. New methods are needed to expand this toolbox.

CRISPR/Cas-based modulation of gene expression using a catalytically inactive Cas fused to a transcriptional effector (VPR) is known from US 2017/0198308 A1. Said VPR is different from the KRAB domain of ZNF473. WO 2018/204764 A1 refers to KRAB as a generic family designation, among a list of example functional domains, and provides neither a specific KRAB sequence nor any pointer to ZNF473.

### SUMMARY

The invention is set out in the appended claims. The embodiments of the description which do not fall within the scope of said claims are provided for illustrative purposes only and do not form part of the present invention. Provided herein are compositions, systems, and method for the generation, identification, and characterization of effector domains for activating and silencing gene expression. In particular, high throughput systems are provided to discover and characterize effector domains. Provided herein is a high throughput approach to discover and characterize effector domains that greatly expands the toolbox. These domains satisfy a critical need to engineer enhanced synthetic transcription factors for applications in gene and cell therapy, synthetic biology, and functional genomics.

Provided herein are methods for identification of effector domains comprise: a) preparing a domain library comprising a plurality of nucleic acid sequences each configured to express a fusion protein comprising a protein domain linked to an inducible DNA binding domain; b) transforming reporter cells with the domain library, wherein a reporter cell comprises a two-part reporter gene comprising a surface marker and a fluorescent protein under the control of a strong promoter, wherein the two-part reporter gene is capable of being silenced by a putative transcriptional repressor domain following treatment with an agent configured to induce the inducible DNA binding domain; c) treating the reporter cells with the agent for a length of time necessary for protein and mRNA degradation in the cell; d) separating reporter cells based on presence or absence of the surface marker, the fluorescent protein, or a combination thereof; e) sequencing the protein domains from the separated reporter cells; f) calculating for each protein domain sequence a ratio of sequencing counts from reporter cells not having the surface marker, the fluorescent protein, or a combination thereof to sequencing counts from reporter cells having the surface marker, the fluorescent protein, or a combination thereof; and g) identifying protein domains as transcriptional repressor.

Provided herein are methods for identification of effector domains comprise: a) preparing a domain library comprising a plurality of nucleic acid sequences each configured to express a fusion protein comprising a protein domain linked to an inducible DNA binding domain; b) transforming reporter cells with the domain library, wherein the reporter cells comprises a two-part reporter gene comprising a surface marker and a fluorescent protein under the control of a weak promoter, wherein the two-part reporter gene is capable of being activated by a putative transcriptional activator domain following treatment with an agent configured to induce the inducible DNA binding domain; c) treating the reporter cells with the agent for a length of time necessary for protein and mRNA production in the cell; d) separating reporter cells based on presence or absence of the surface marker, the fluorescent protein, or a combination thereof; e) sequencing the protein domains from the separated reporter cells; f) calculating for each protein domain sequence a ratio of sequencing counts from reporter cells not having the surface marker, the fluorescent protein, or a combination thereof to sequencing counts from reporter cells having the surface marker, the fluorescent protein, or a combination thereof; and g) identifying protein domains as transcriptional activator.

The methods may further comprise stopping treatment of the reporter cells with the agent and repeating steps d-g one or more times. **In** some embodiments, steps d-g are repeated at least 48 hours after stopping treatment of the reported cells with the agent.

Each protein domain may be less than or equal to 80 amino acids. The protein domain may be from a nuclear-localized protein. The protein domain may comprise amino acid sequences of the wild-type protein domains from nuclear-localized proteins. The protein domain may comprises mutated amino acid sequences of protein domains from nuclear-localized proteins.

The inducible DNA binding domain may comprise a tag.

The methods may further comprise measuring expression level of protein domains. The expression level may be determined by measuring a relative presence or absence of the tag on the DNA binding domain.

The reporter cells may be treated with the agent for at least 3 days. The reporter cells may be treated with the agent for at least 5 days. The reporter cells may be treated with the agent for at least 24 hours. The reporter cells may be treated with the agent for at least 48 hours.

The protein domain may be identified as a transcription repressor when log2 of the ratio is at least two standard deviations from (e.g., higher than) the mean of a poorly expressed negative control.

The protein domain may be identified as a transcription activator when log2 of the ratio is at least two standard deviations from (e.g., lower than) the mean of weakly expressing negative control.

Also provided herein are synthetic transcription factor comprising one or more transcriptional activator domains, one or more transcriptional repressor domains, or a combination thereof fused to a heterologous DNA binding domain. At least one of the one or more transcriptional activator domains or at least one of the one or more transcriptional repressor domains may comprise an amino acid sequence having at least 70% identity to any of SEQ ID NOs: 1-896.

The synthetic transcription factor may comprise two or more transcriptional activator domains or two or more transcriptional repressors domains fused to a heterologous DNA binding domain.

At least one of the one or more transcriptional activator domain may comprise an amino acid sequence having at least 70% identity to any of SEQ ID NOs: 563-664. At least one of the one or more transcriptional activator domain may be selected from those found in Table 2.

The at least one of the one or more transcriptional repressor domain may comprise an amino acid sequence having at least 70% identity to any of SEQ ID NOs: from 1-562 and 665-896. The at least one of the one or more transcriptional repressor domain may be selected from those found in any of Tables 1, 3, or 4.

The one or more transcriptional activator domain or the one or more transcriptional repressor domain may be identified by the methods disclosed herein.

The heterologous DNA binding domain may comprise a programmable DNA binding domain. The DNA binding domain may be derived from a Clustered Regularly Interspaced Short Palindromic Repeats associated (Cas) protein. The DNA binding domain may be derived from Transcription activator-like effectors (TALEs) domains.

Also provided herein are nucleic acids encoding a synthetic transcription factor or an effector domain, as disclosed herein. The nucleic acid may be under control of an inducible promoter. The nucleic acid may be under control of a tissue specific promoter. The nucleic acid may encode at least one additional transcription factor or effector domain.

Further provided herein is a composition or system comprising a synthetic transcription factor, a nucleic acid, a vector, or a cell as disclosed herein. The composition may comprise two or more synthetic transcription factors, nucleic acids, vectors, or cells. The composition may further comprises a guide RNA or a nucleic acid encoding a guide RNA.

Additionally, provided herein are methods of modulating the expression of at least one target gene in a cell. The methods may comprise introducing into the cell at least one synthetic transcription factor, nucleic acid, vector, or composition or system, as described herein. The gene expression of the at least one target gene may be modulated when gene expression levels of the at least one target gene are increased or decreased compared to normal gene expression levels for the at least one target gene. The synthetic transcription factor may comprise a Cas protein DNA binding domain and the method further comprises contacting the cell with at least one guide RNA.

The cell may be in vitro (e.g., ex vivo) or in a subject, which is not covered by the invention as defined in the claims.

In some embodiments, the gene expression of at least two genes are modulated.

### BRIEF DESCRIPTION OF THE DRAWINGS

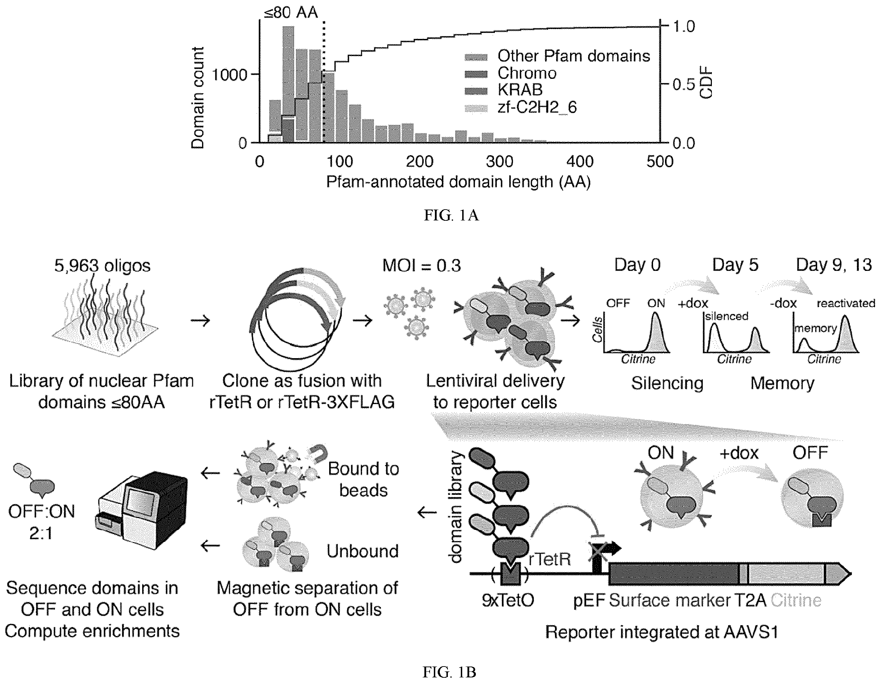

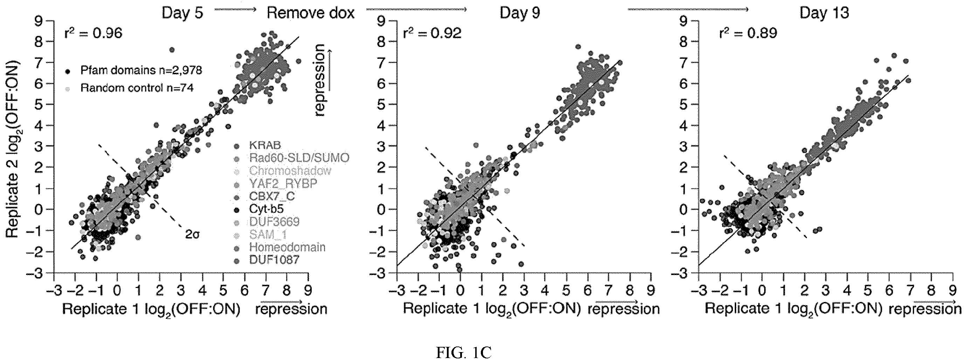

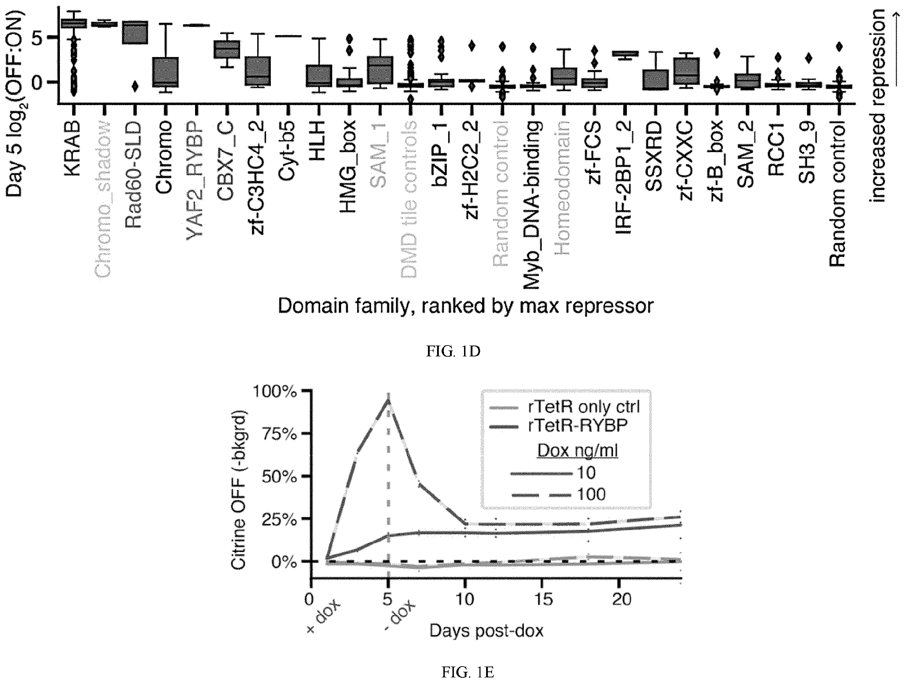

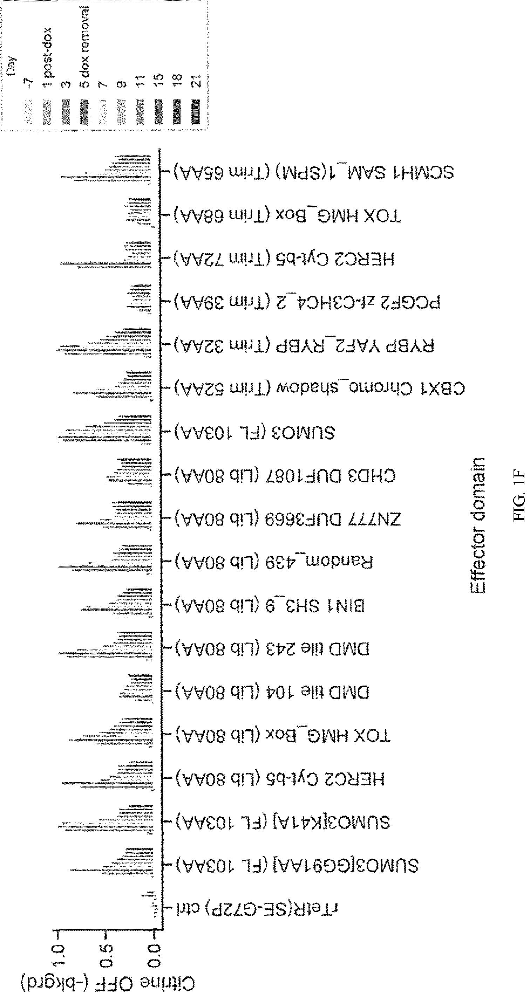

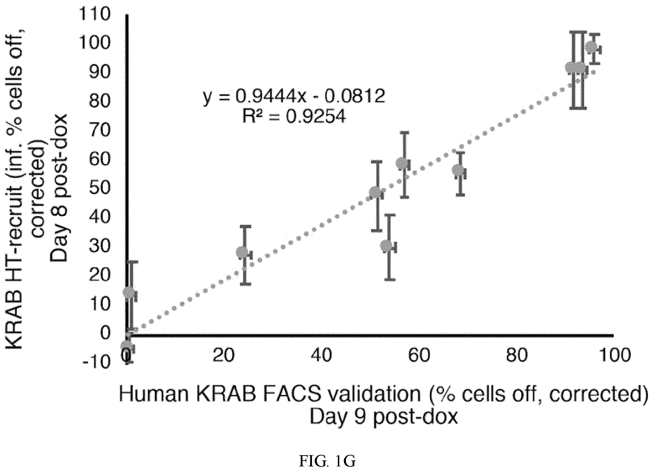

FIGS. 1A-1G show high-throughput recruitment measures transcriptional repressor activity of thousands of Pfam-annotated domains from nuclear-localized proteins. FIG. 1A - Length of Pfam-annotated domains in human proteins that localize to the nucleus. Domains ≤80 amino acids were selected for inclusion in the library. FIG. 1B - Schematic of screen to identify transcriptional repressors. The repression reporter uses a strong pEF promoter that can be silenced by dox-mediated recruitment of repressor domains. The cells were treated with doxycycline for 5 days, ON and OFF cells were magnetically separated and the domains were sequenced. Dox was removed and additional time points were taken on Days 9 and 13. FIG. 1C - The reproducibility of log2(OFF:ON) ratios from independently transduced biological replicates is shown and selected domain families are colored. FIG. 1D - Boxplots of top repressor domain families, ranked by the maximum repressor strength at day 5 of a domain within the family. FIG. 1E - Individual validation time course for hit RYBP domain, measured by flow cytometry. FIG. 1F - Additional validation time courses for a panel of repressor domains. Domain length is listed in parentheses, because some domains were tested as the exact 80 AA sequence from the library and some were tested as a shorter sequence trimmed to the region annotated as a domain by Pfam. 1000 ng/ml dox was added on day 0 and removed on day 5. FIG. 1G - Correlation of screen measurements with individual validation flow cytometry measurements for a collection of KRAB effector domains.

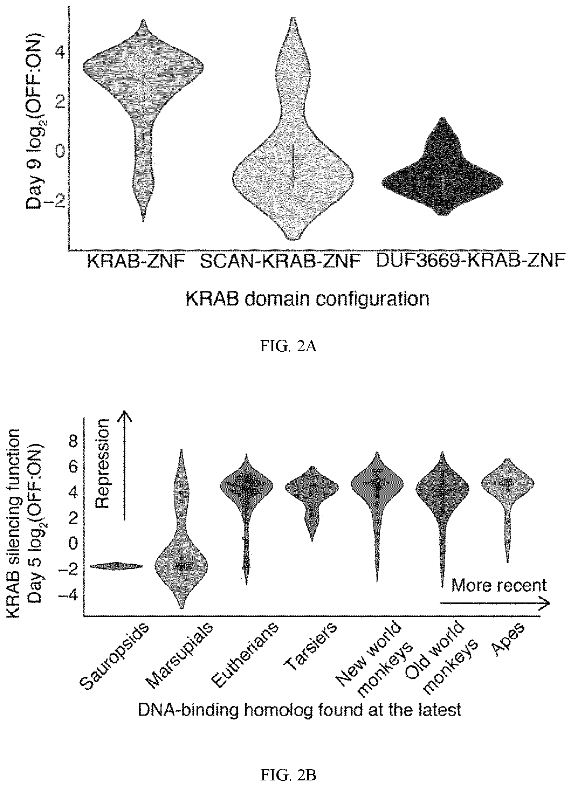

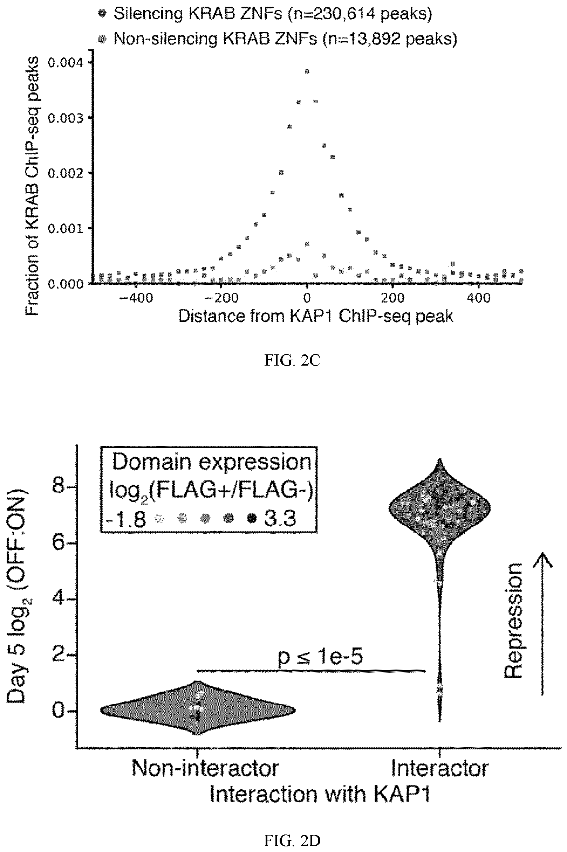

FIGS. 2A-2D show repressive KRAB domains are in younger KRAB-Zinc finger proteins that co-localize and bind to the KAP1 co-repressor. FIG. 2A - KRAB silencing function was compared with the KRAB Zinc Finger protein architecture that the domain is natively found in. FIG. 2B -KRAB silencing function was compared with the KRAB Zinc Finger gene evolutionary age, as determined by finding the most recent ortholog for the gene using its full DNA-binding zinc finger array sequence (ages published in Trono 2017). FIG. 2C - KRAB domains were categorized as silencers or non-silencers and their genomic localization in ChIP-seq datasets was compared with the localization of the co-repressor KRAB-associated Protein 1 (KAP1). FIG. 2D -Repression strength distributions of KRAB domains categorized by whether their KRAB Zinc Finger gene interacts significantly with co-repressor KAP1 in a mass spec dataset (Helleboid 2019). Dot colors are the quintile of the KRAB domain expression level.

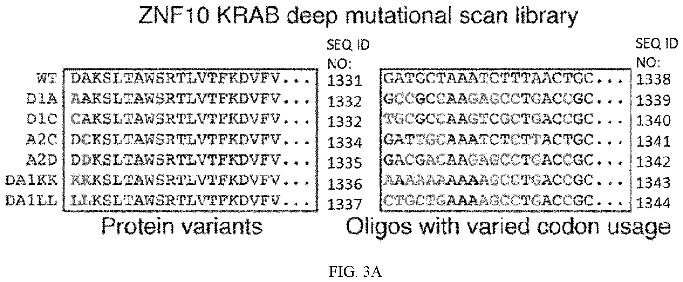

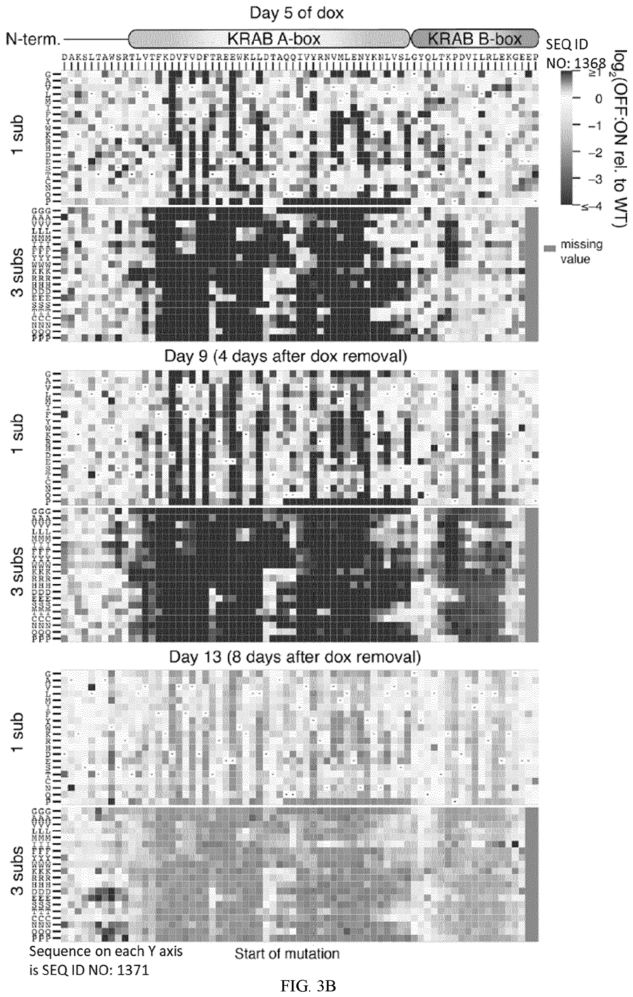

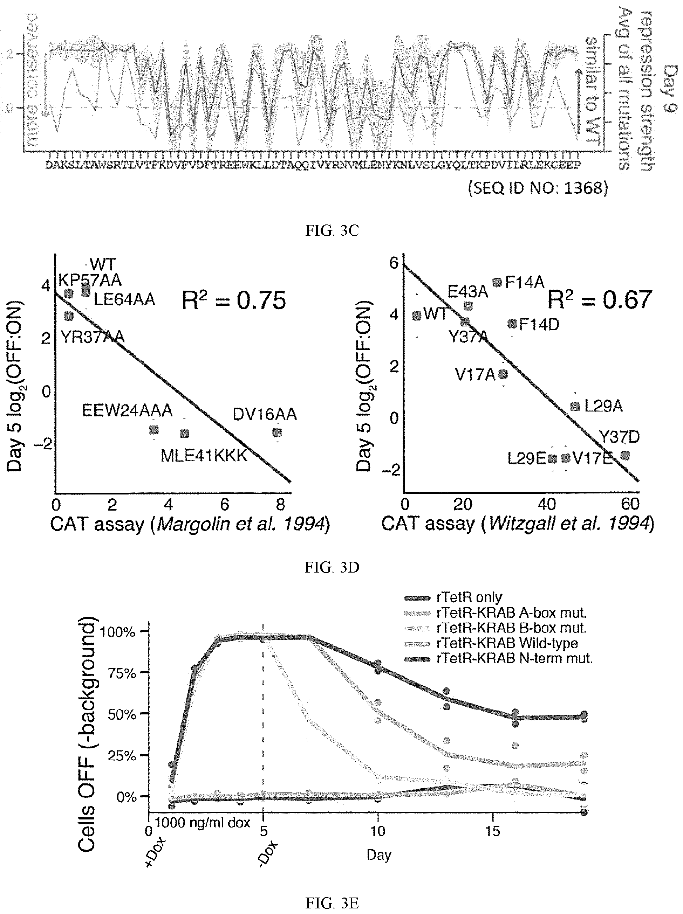

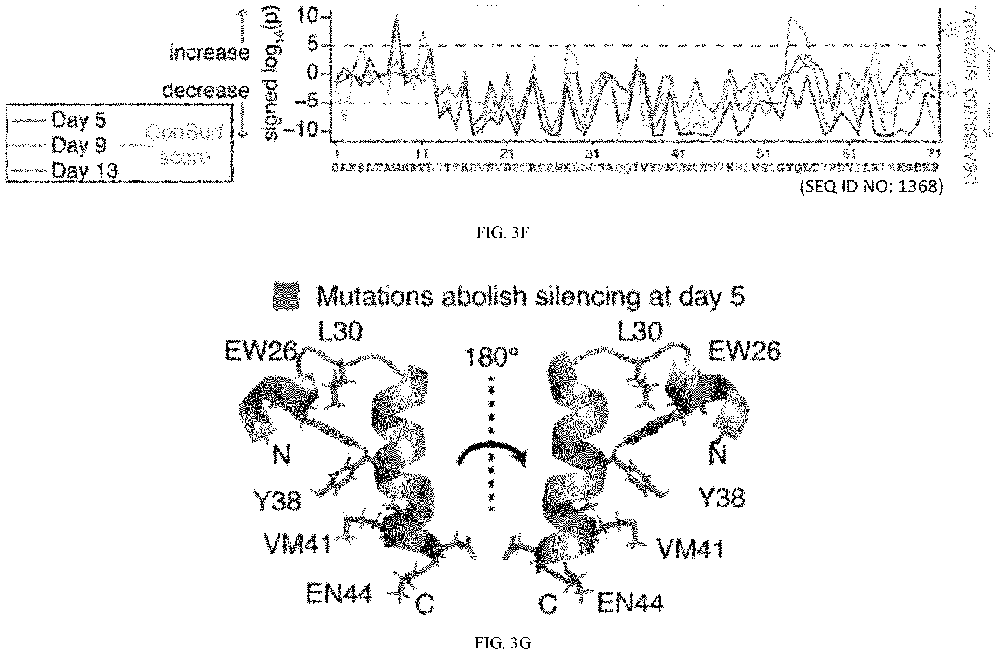

FIGS. 3A-3G show a deep mutational scan of the ZNF10 KRAB domain identifies substitutions that reduce or enhance repressor activity. FIG. 3A - Deep mutational scanning library includes all single and consecutive double and triple substitutions in the KRAB domain from ZNF10. The DNA oligos are designed to be more distinct than the protein sequences by varying codon usage. Red residues differ from the WT sequence. FIG. 3B - All single and triple substitution variants' repressor measurements relative to the WT are shown underneath a schematic of the KRAB domain. FIG. 3C - Average mutation effects on repression at Day 9, compared to sequence conservation from a multiple sequence alignment of all human KRAB domains (computed with ConSurf). FIG. 3D - Correlation of high-throughput measurements with previously published low-throughput data using the CAT assay in a different cell type. FIG. 3E - Individual time-courses of KRAB mutants validate the effects of substitutions in the A/B-boxes and N-terminus. FIG. 3F - For each position at each timepoint in FIG. 3B, the distribution of all single substitutions was compared to the distribution of wild-type effects (Wilcoxon rank sum test). Positions with signed log10(p)<-5 at day 5 are colored in red (highly significantly decrease in silencing), with signed log10(p)<-5 at day 9 but not day 5 are colored in green, and the position W8 with log10(p)>5 at day 13 is colored in blue (highly significant increase). Dashed horizontal lines show the hit thresholds. The sequence conservation ConSurf score is shown in orange. FIG. 3G - Residues that abolish silencing at day 5 when mutated are mapped onto the ordered region of the NMR structure of mouse KRAB A-box (PDB: 1v65).

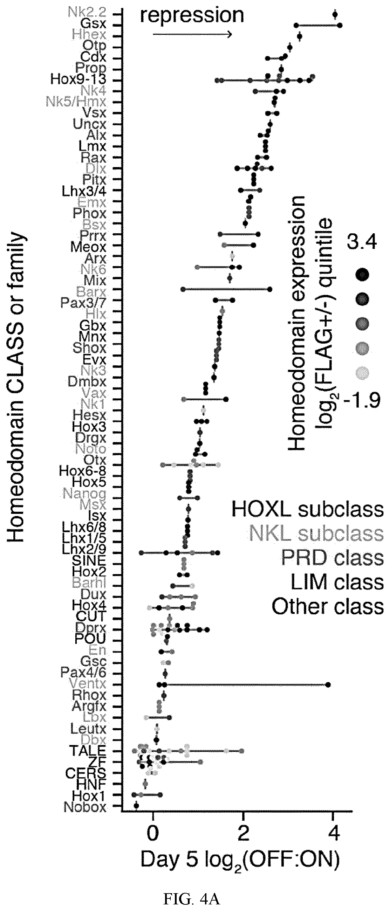

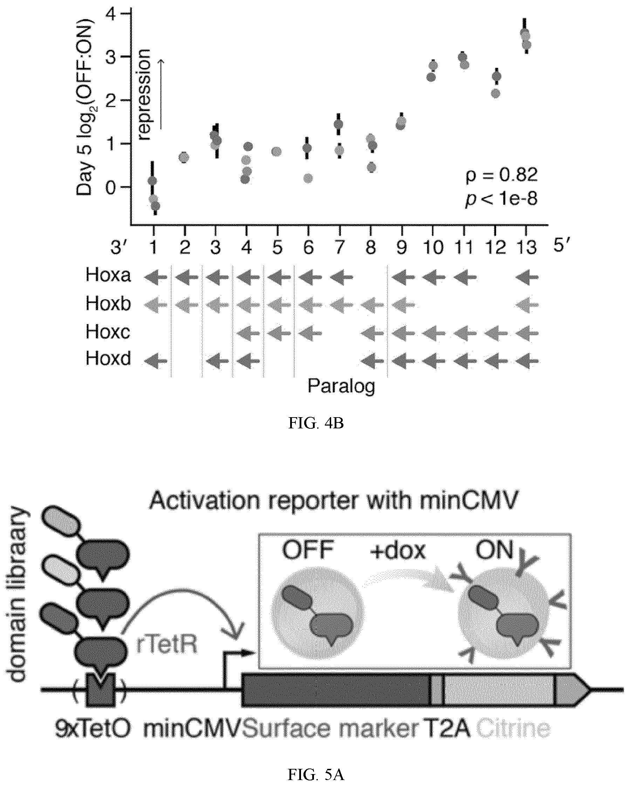

FIGS. 4A and 4B show that homeodomain repression strength is colinear with Hox gene organization. FIG. 4A - Ranking of homeobox gene families or classes by median repression strength at Day 5. The HOXL and NKL subclasses of the ANTP class homeodomains and the PRD and LIM classes, which contain the strongest homeodomain repressors, are split into individual gene families (Holland BMC 2007) while the remaining classes are aggregated. Dot colors are the quintile of the Homeodomain expression level as measured in the HT-expression assay. FIG. 4B - Repressor strength at day 5 of the homeodomains from the Hox gene families. Arrows represent the genes found in the four human Hox loci and point in the direction of Hox gene transcription. Grey bars separate the gene families. Spearman's rho and the p-value were computed for the relationship between the gene number and repressor strength across all Hox genes. Data was filtered to remove any domains that had fewer than 10 counts in any of the Day 5 sequencing samples.

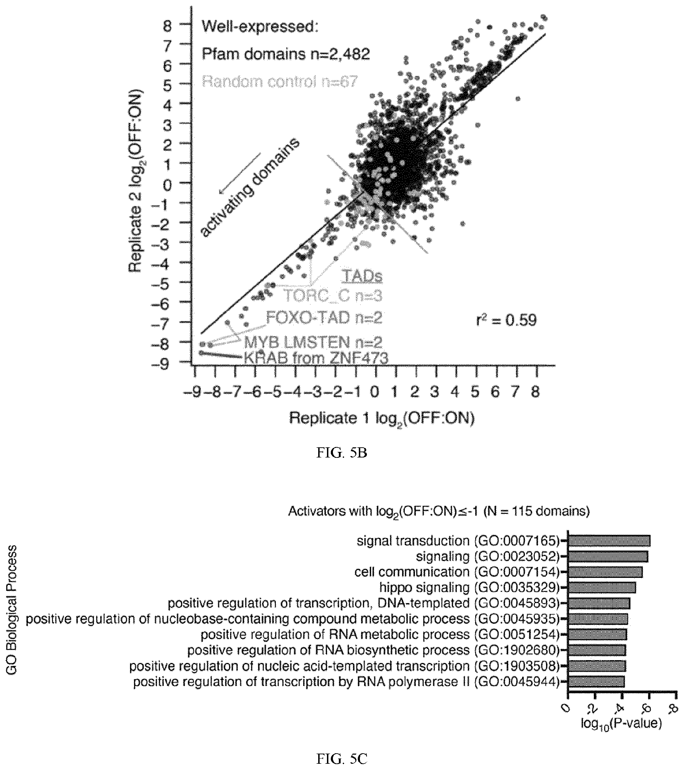

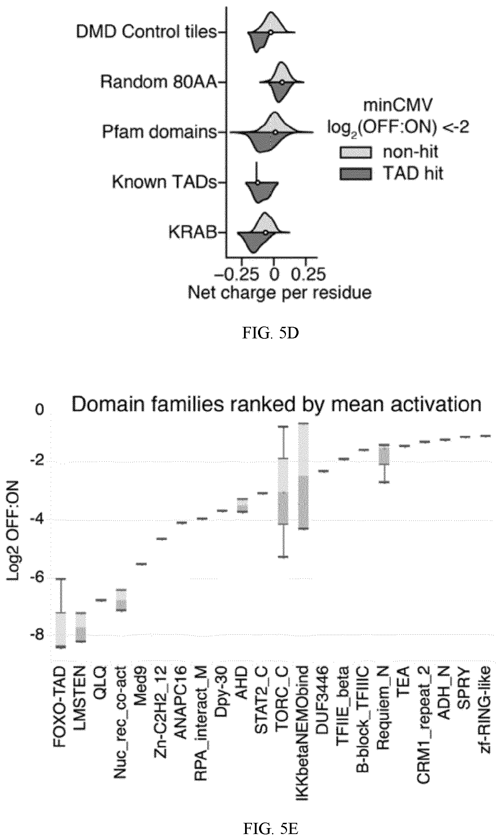

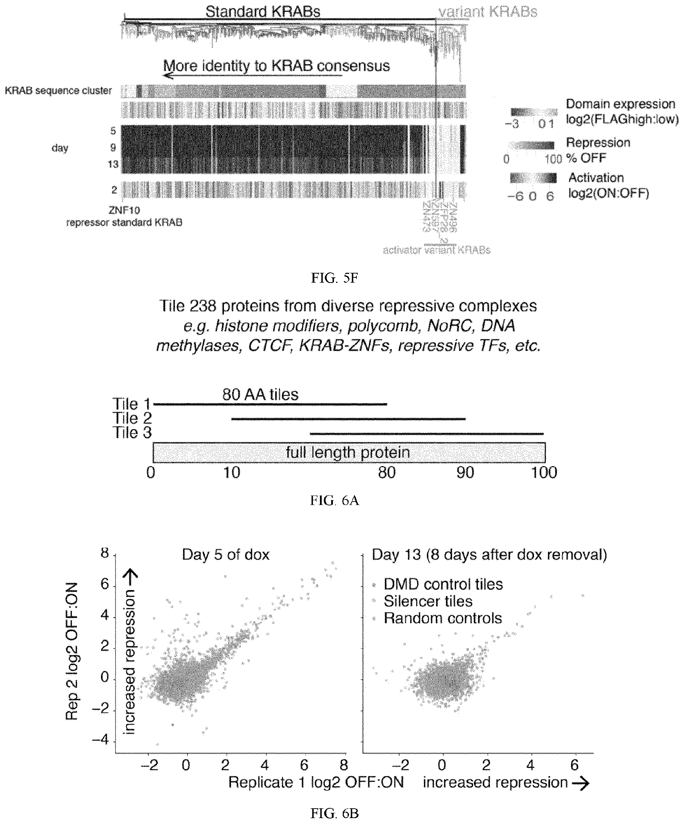

FIGS. 5A-5F show that high-throughput recruitment discovers activator domains, including a potent, acidic, and divergent KRAB domain variant in ZNF473. FIG. 5A - Schematic of the activation reporter which uses a weak minCMV promoter that can be activated by dox-mediated recruitment of activating domains, and a schematic of the activation screen. The pool of cells was treated with doxycycline for 48 hours, ON and OFF cells were magnetically separated with ProG Dynabeads and the domains were sequenced. FIG. 5B - The reproducibility of log2(OFF:ON) ratios from independently transduced biological replicates is shown with known activator domain families (FOXO-TAD, Myb LMSTEN, TORC_C) colored. FIG. 5C - GO term enrichments of genes containing a domain with activation strength below a threshold. FIG. 5D - Activator domains (red) are more acidic than non-hits (grey). FIG. 5E - List of domain families, ranked by mean activation strength. FIG. 5F - KRAB domains were aligned and clustered by sequence, providing similar results to the classification in Helleboid 2019. The cluster of most divergent KRAB sequences is labeled variant KRABs in green. Results from screens are shown below in heatmaps. Standard KRABs function as repressors, if they are well-expressed. The variant KRABs show mixed effects as repressors, activators, and no transcriptional effect in the screens.

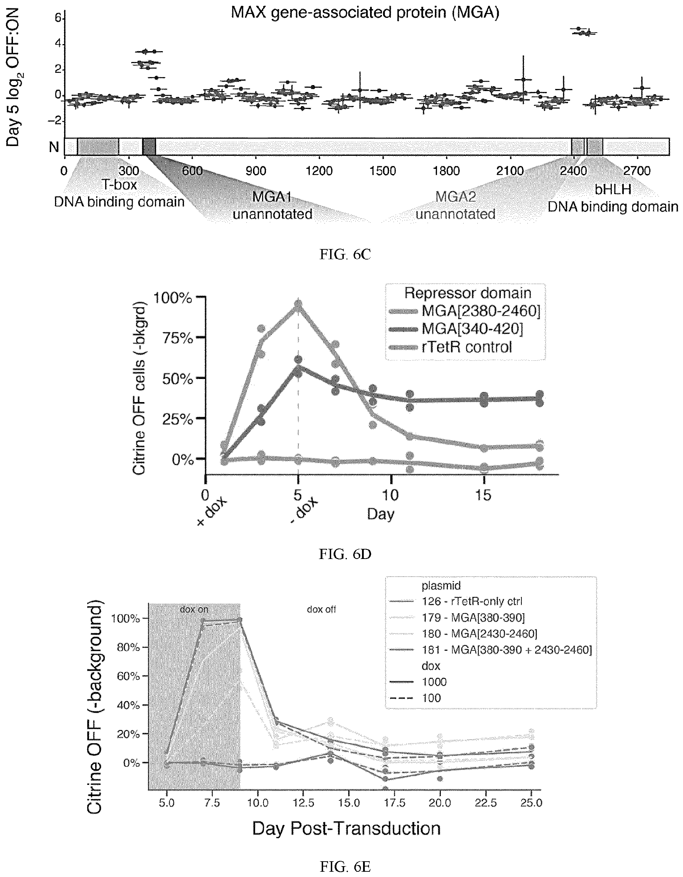

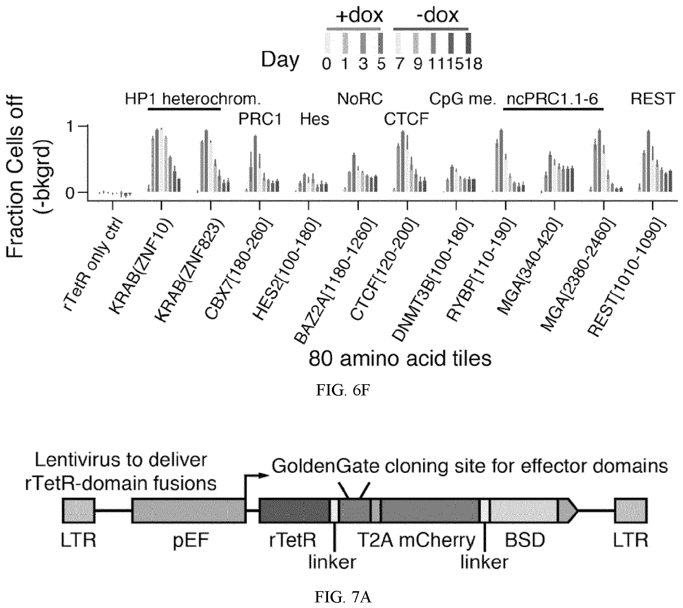

FIGS. 6A-6F show that a tiling library uncovers new autonomous repressor domains within large chromatin regulator proteins. FIG. 6A - Graphical depiction of library in which 80 AA tiles cover the protein sequence, with a 10 AA sliding window. FIG. 6B - The reproducibility of log2(OFF:ON) ratios from independently transduced biological replicates is shown. FIG. 6C - Repression at Day 5 is compared with known domain architecture for the MGA protein. Two repressor domains are found outside the previously annotated regions. FIG. 6D - Flow cytometry time courses validate the individual MGA effectors as 80 AA tiles. FIG. 6E - The effectors were minimized to 10 and 30 AA subtiles by selecting the sequence shared in common among the tiles that show repressor activity in the screen. These minimized sequences were validated individually with flow cytometry time courses. FIG. 6F - Individual validation of additional 80 AA repressor hits from the tiling screen. rTetR-tile fusions were delivered to K562 reporter cells by lentivirus and cells were treated with 100 ng/ml dox for 5 days, then dox was removed. Cells were analyzed by flow cytometry and the fraction of cells OFF was measured by gating the cells by their citrine expression level.

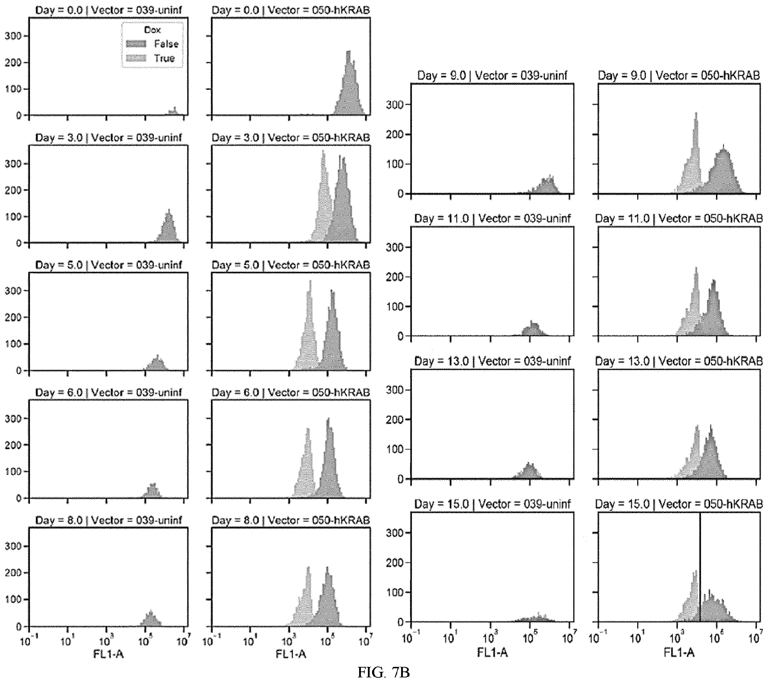

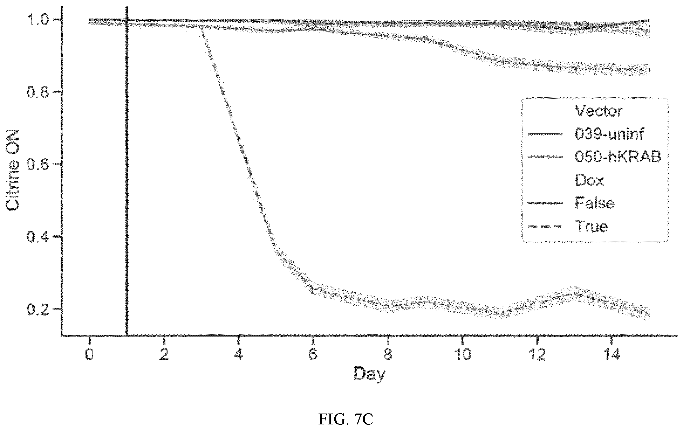

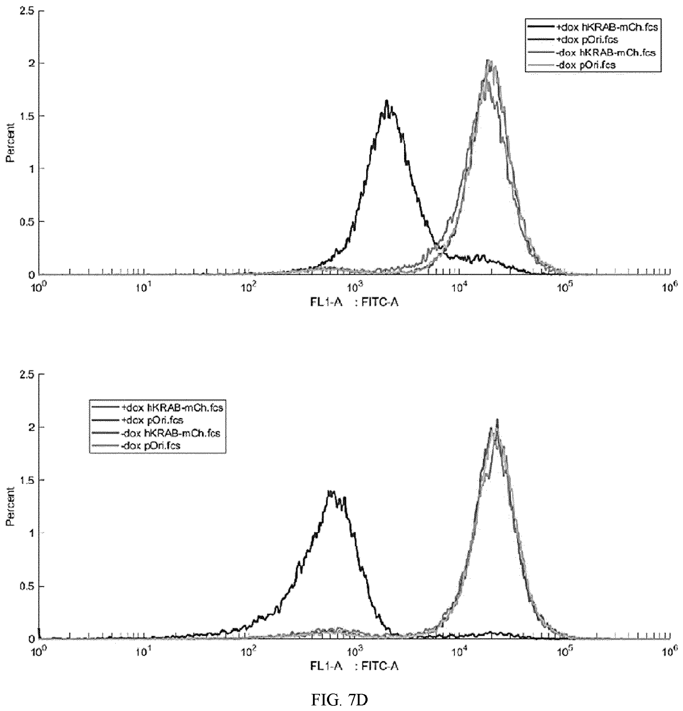

FIGS. 7A-7D show that a recruitment assay measures gene silencing by lentiviral rTetR-domain fusions with a fluorescent reporter. FIG. 7A - Schematic of lentiviral vector. FIG. 7B - Pilot test in K562 reporter cells, showing citrine OFF: ON FACS histograms over time for ZNF10KRAB cloned onto pJT050. 1000 ng/ml dox was added on day 0 and removed on day 5. FIG. 7C - Fraction of cells ON over time FIG. 7D - The reporter system was also established in HEK293T cells. Cells were transfected with plasmid encoding rTetR-KRAB or pOri control and treated with or without 1000 ng/ml dox for 2 (top) and 4 (bottom) days before being analyzed with flow cytometry.

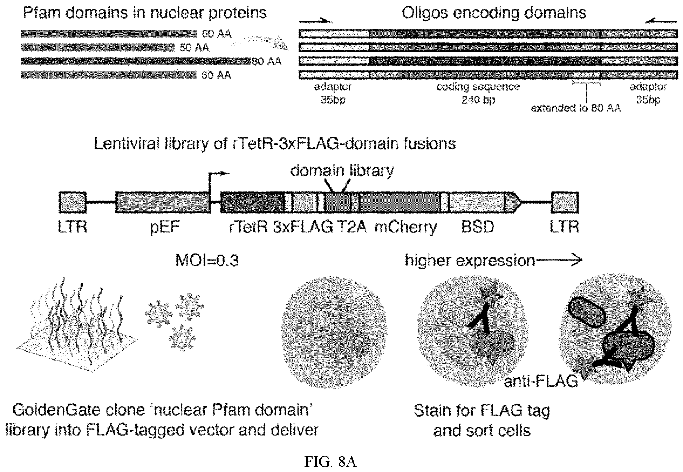

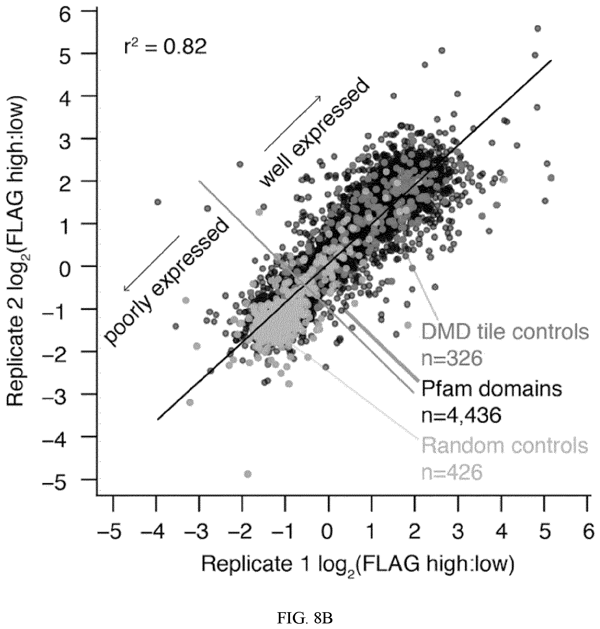

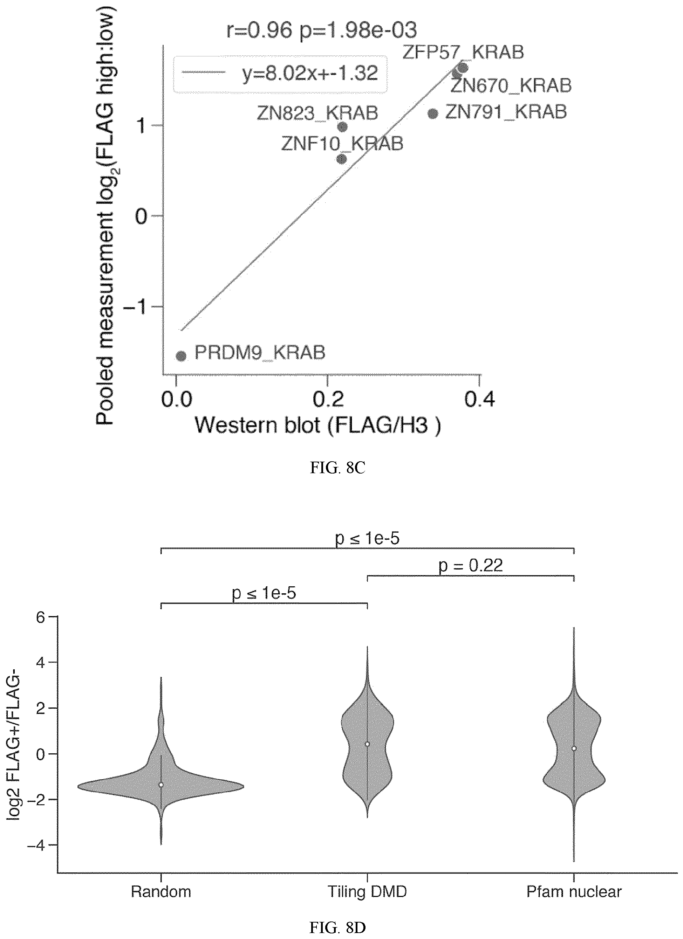

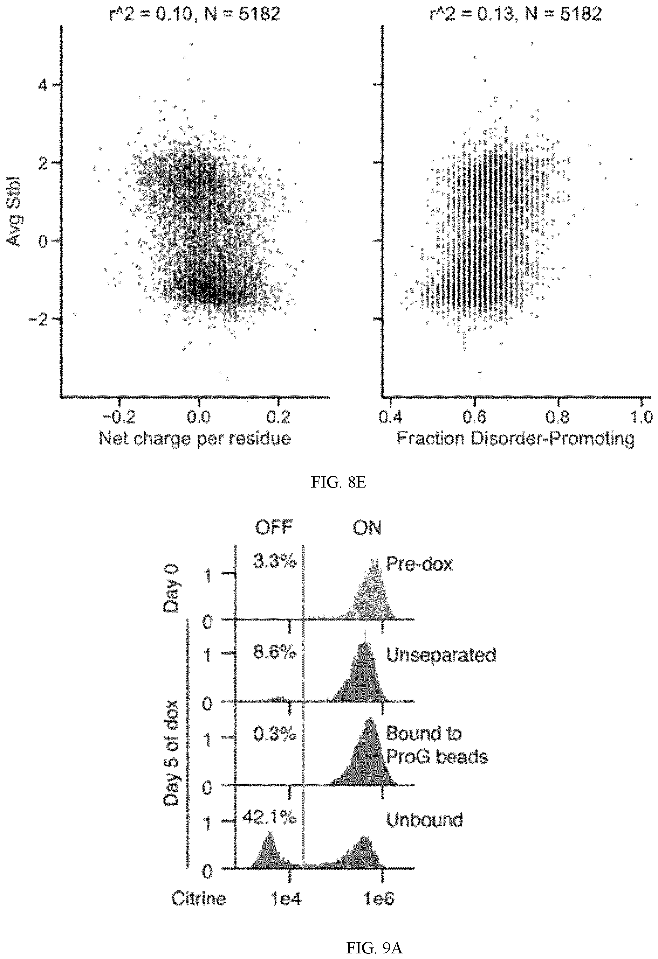

FIGS. 8A-8E show high-throughput measurements of domain expression by FLAG staining, sorting, and sequencing. FIG. 8A - Schematic of a high-throughput approach to measure the expression level for each domain fusion in the library. FIG. 8B - Reproducibility of domain expression measurements. FIG. 8C - Validation with Western Blot. FIG. 8D - Stability of sub-libraries - random are destabilized, tiles are similar to Pfam domains. FIG. 8E - Stability related to net charge of residue and residues which are classified as disorder promoting.

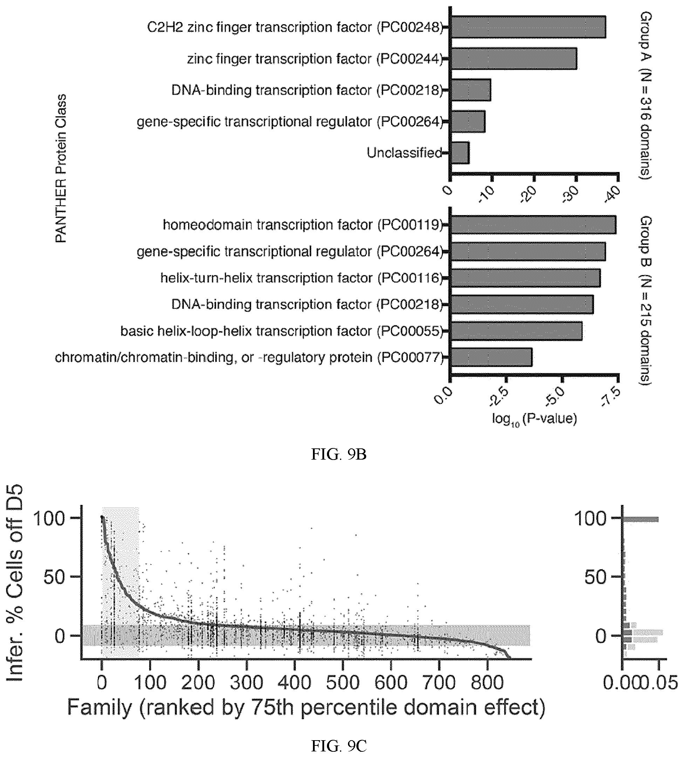

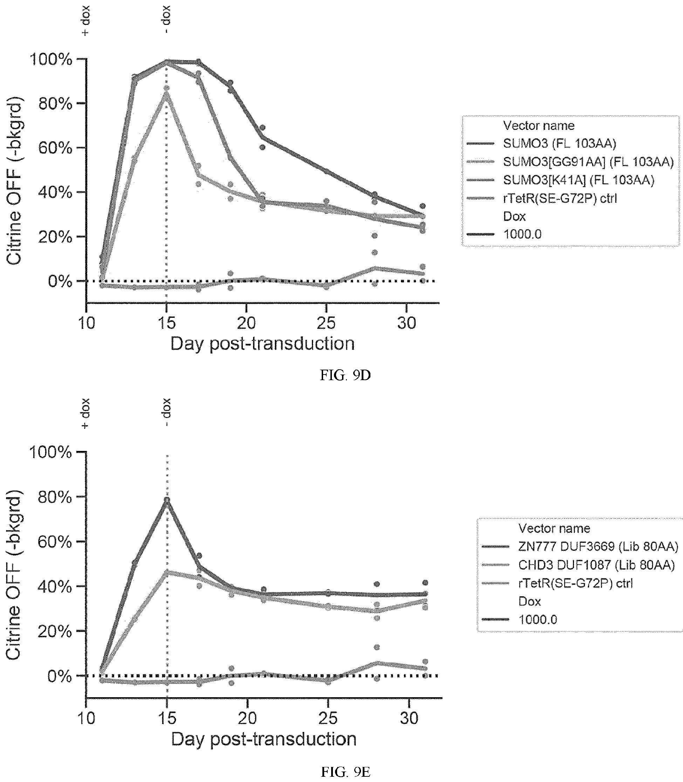

FIGS. 9A-9E show a screen of Pfam domains for repressor function. FIG. 9A - Flow cytometry of library of cells before and after magnetic separation. FIG. 9B - PANTHER protein class enrichments for stable vs transient repressors top 10, log P. FIG. 9C - Full list of domain families, ranked by repressor strength at day 5. FIG. 9D - rTetR-SUMO fusions silence the reporter. Mutation in SUMO conjugation site (GG91AA) reduce silencing speed and mutation in SUMO-interacting non-covalent binding site reduces silencing memory. FIG. 9E - Validation of Domains of Unknown Function (DUFs) with repressor activity.

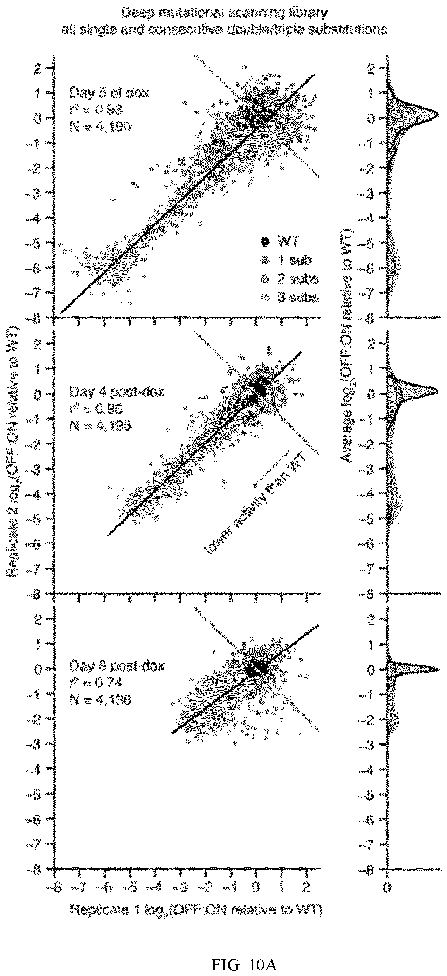

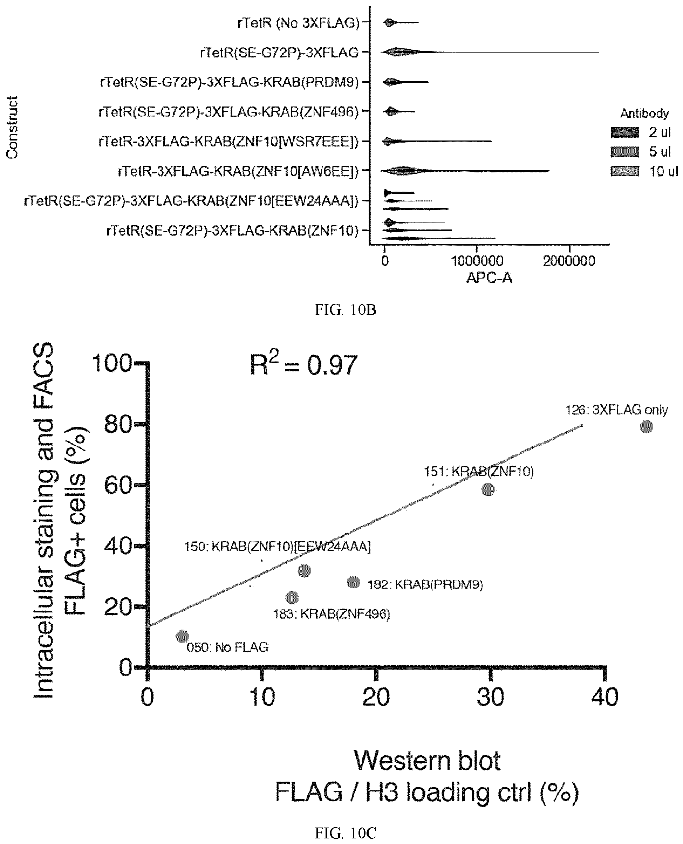

FIGS. 10A-10C show a KRAB deep mutational scan. FIG. 10A - OFF:ON scores from two biological replicates of a deep mutational library of the KRAB domains from ZNF10 at Day 5, 9 and 13. FIG. 10B - FLAG-tag stain for KRAB variant expression level: non-silencing one gets degraded. B-box mutants are stable. FIG. 10C - FLAG-tag stain correlates with FLAG-tag Western Blot.

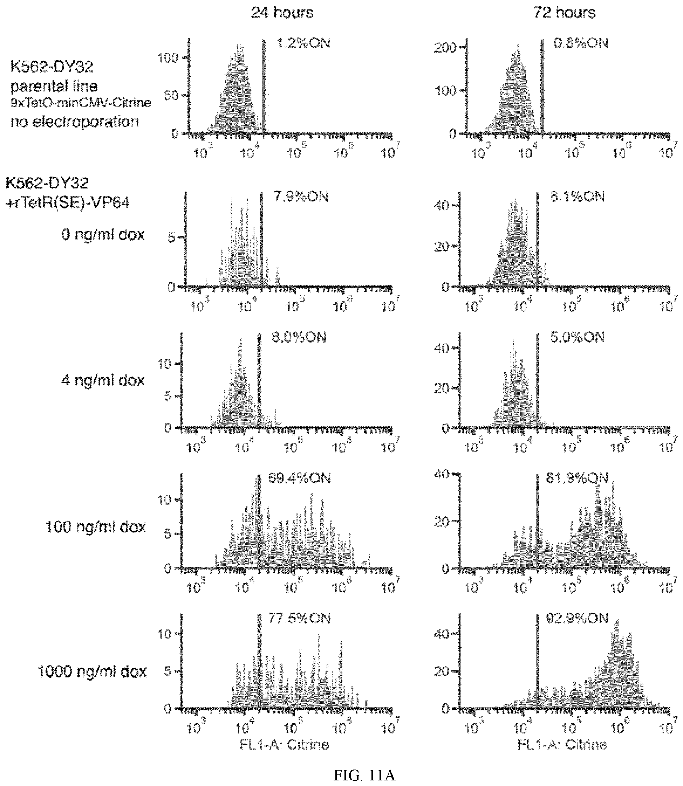

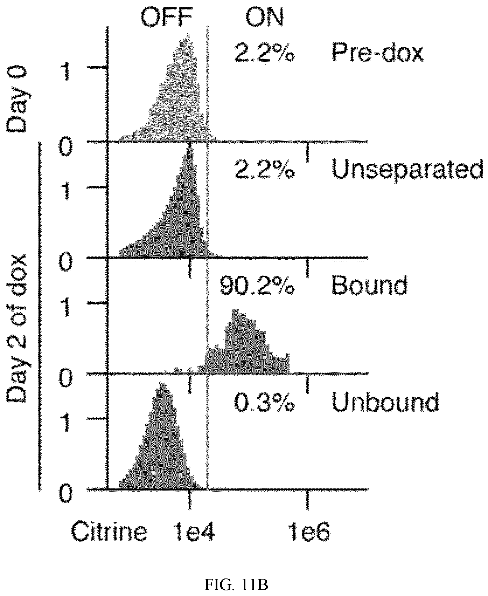

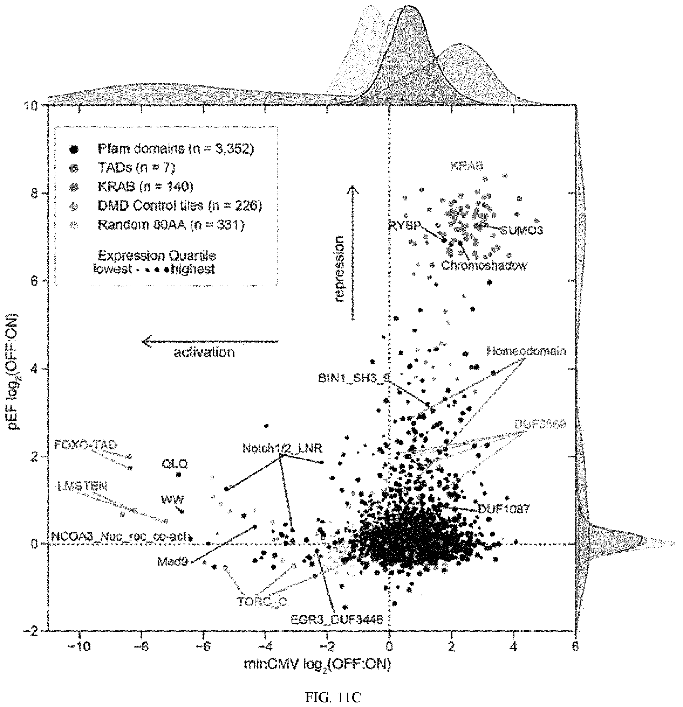

FIGS. 11A-11C show activator screen data. FIG. 11A - Pilot test, electroporating rTetR-VP64 to K562 minCMV reporter cells. After doxycycline is added, the reporter cells turn ON as measured by flow cytometry for citrine expression. FIG. 11B - Magnetic separation of pooled library during activator screen, analyzed by flow cytometry. FIG. 11C - Comparison of HT-recruit transcriptional regulation measurements, using the Pfam domain library with two different reporter promoters. Each domain is a dot and the dot's size is the expression quartile as measured in the FLAG screen.

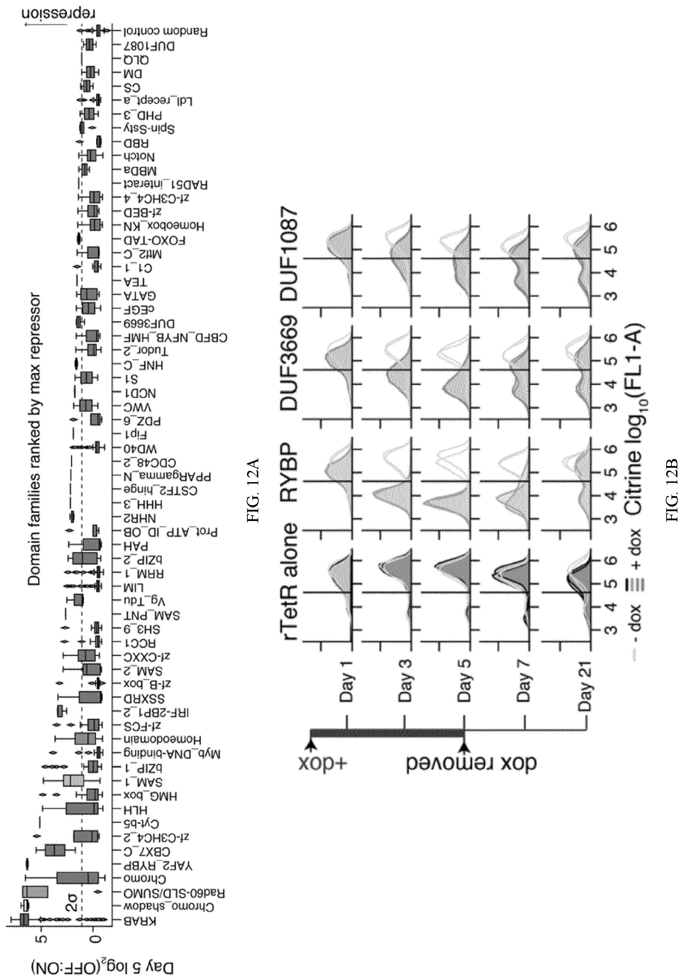

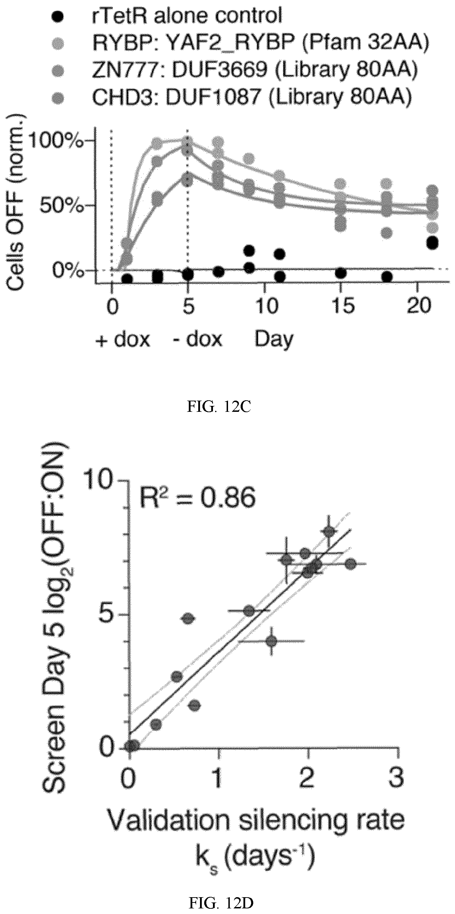

FIGS. 12A-12D show hundreds of repressors discovered in a screen of thousands of Pfam domains. FIG. 12A - Boxplots of top repressor domain families, ranked by the maximum repressor strength at day 5 of any domain within the family. Line shows the median, whiskers extend beyond the high- and low-quartile by 1.5 times the interquartile range, and outliers are shown with diamonds. Dashed line shows the hit threshold. Boxes colored for domain families highlighted in the text. FIG. 12B - Individual validations for RYBP domain and two Domains of Unknown Function (DUF) with repressor activity, measured by flow cytometry. Untreated cell distributions are shown in light grey and doxycycline-treated cells are shown in colors, with two independently-transduced biological replicates in each condition. The vertical line shows the citrine gate used to determine the fraction of cells OFF. FIG. 12C - Validation time courses fit with the gene silencing model: exponential silencing with rate ks, followed by exponential reactivation. Doxycycline (1000 ng/ml) was added on day 0 and removed on day 5 (N = 2 biological replicates). The fraction of mCherry positive cells with the citrine reporter OFF was determined by flow cytometry, as in FIG. 12B, and normalized for background silencing using the untreated, time-matched controls. FIG. 12D - Correlation of high-throughput measurements at day 5 with the silencing rate ks (R² = 0.86, n = 15 domains, N = 2-3 biological replicates). Horizontal error bars are the standard deviation for the fitted rates, vertical error bars are the range of screen biological replicates, and dashed lines are the 95% confidence interval of the linear regression.

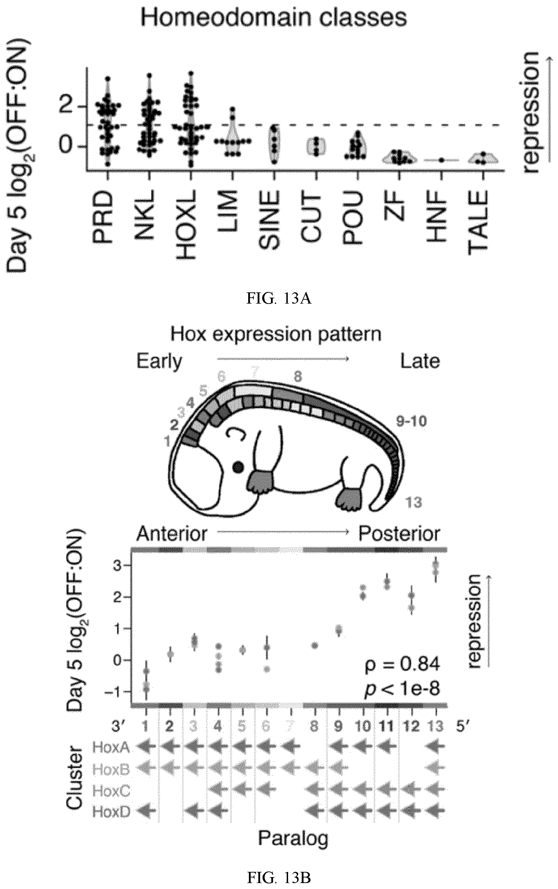

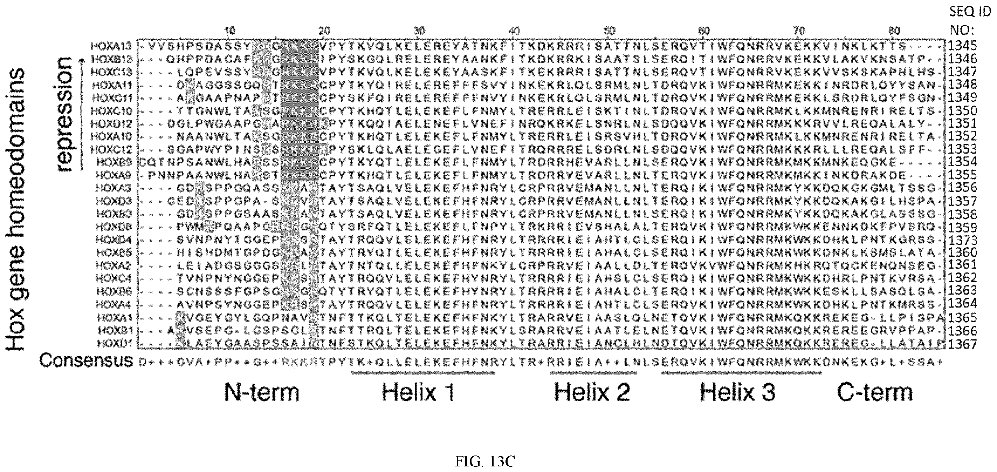

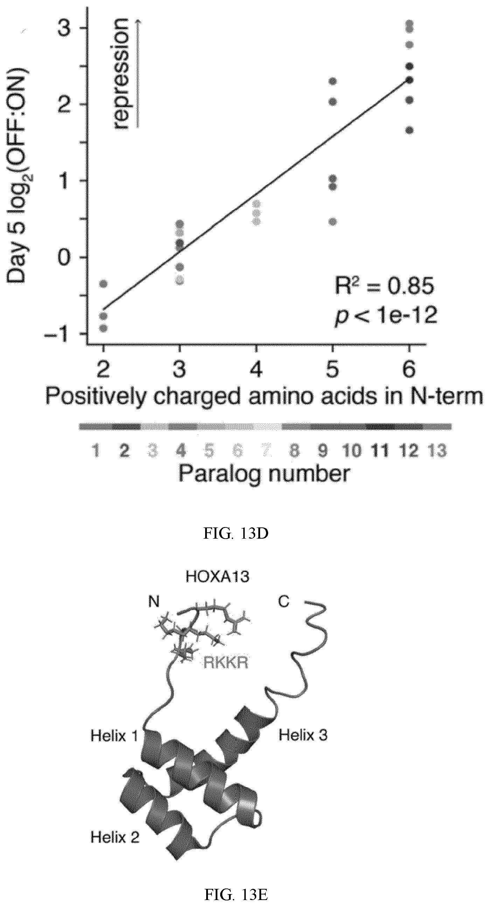

FIGS. 13A-13E show Hox homeodomain repression strength is colinear with Hox gene organization and associated with positive charge. FIG. 13A - Ranking of homeobox gene classes by median repression strength of their homeodomain at day 5. Horizontal line shows the hit threshold. None of the 5 homeodomains from the CERS class were well-expressed. FIG. 13B - Homeodomains from the *Hox* gene families. (Top) Hox gene expression pattern along the anterior-posterior axis is colored by Hox paralog number on an adapted embryo image (Hueber et al., 2010). Hox 11 and 12 are expressed both at the posterior end and along the proximal-distal axis of limbs (Wellik and Capecchi, 2003). (Middle) Repression strength after 5 days of dox. Dots are colored by the Hox cluster and the paralog number is colored as in the embryo diagram. Spearman's rho and p-value were computed for the relationship between the paralog number and repressor strength across all Hox genes. (Bottom) Colored arrows represent the genes found in the four human Hox clusters and point in the direction of Hox gene transcription from 5' to 3'. Grey bars separate gene sequence similarity groups as previously classified (Hueber et al., 2010). FIG. 13C - Multiple sequence alignment of Hox homeodomains, with stronger repressors at the top (as ranked by OFF:ON ratio at day 5), showing the RKKR motif highlighted in red. Other basic residues within the N-terminal arm are colored in lavender. FIG. 13D - Correlation between the number of positively charged residues in the N-terminal arm upstream of Helix 1 of each Hox homeodomain and the average repression at day 5. Dot color shows paralog number. FIG. 13E - NMR structure of the HOXA13 homeodomain retrieved from PDB ID: 2L7Z, with RKKR motif highlighted in red. The sequence from G15 to S81, using the coordinates from the multiple sequence alignment, is shown.

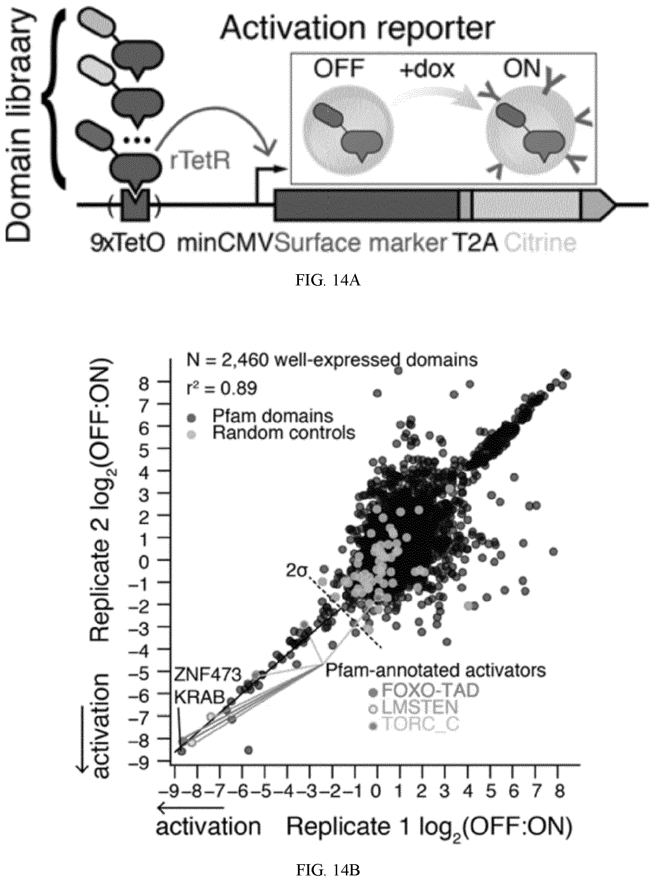

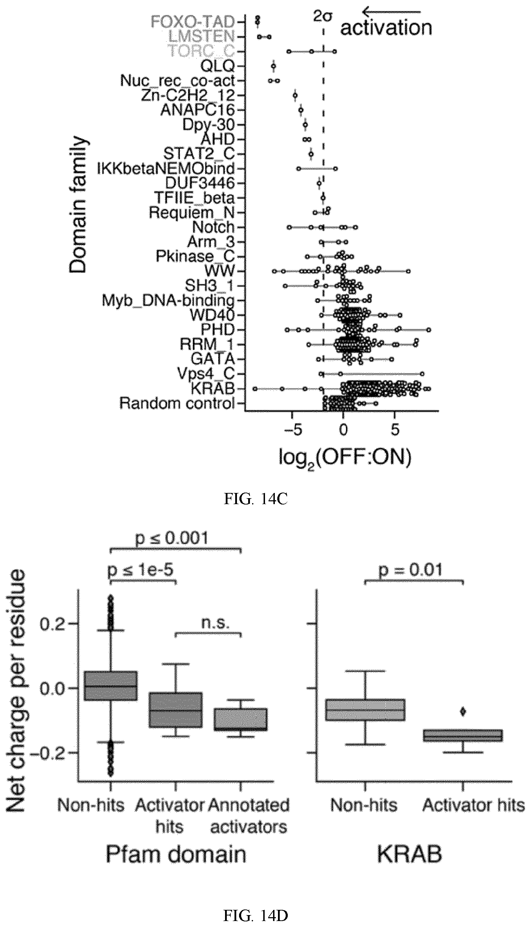

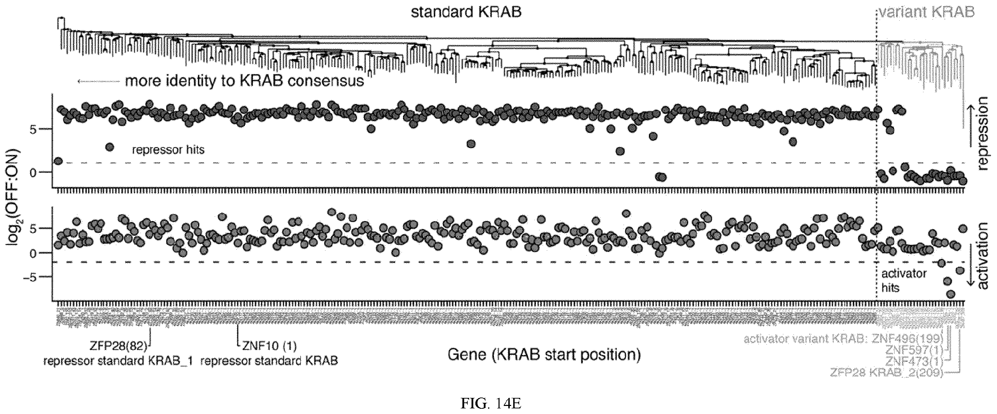

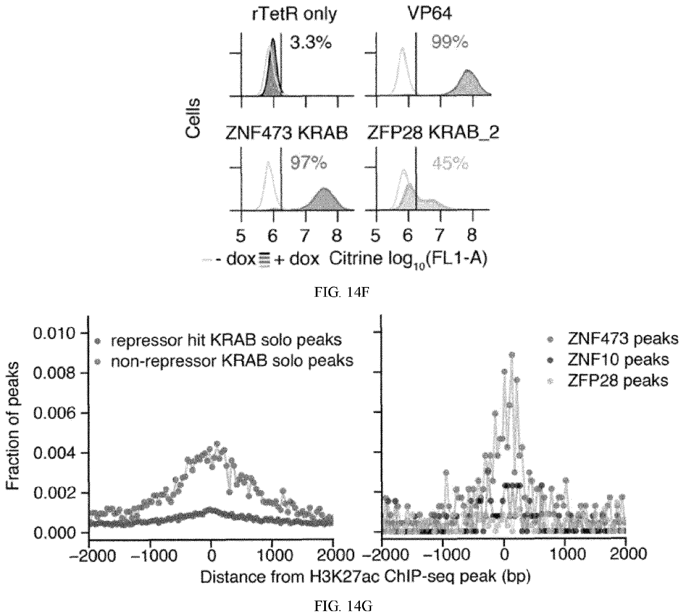

FIGS. 14A-14G show discovery of activator domains. FIG. 14A - Schematic of the activation reporter which uses a weak minCMV promoter that can be activated by doxycycline-mediated recruitment of activating effector domains fused to rTetR. FIG. 14B - Reproducibility of high-throughput activator measurements from two independently transduced biological replicates. The pool of cells containing the activation reporter in (FIG. 14A) were transduced with the nuclear domain library and treated with doxycycline for 48 hours; ON and OFF cells were magnetically separated, and the domains were sequenced. The ratios of sequencing reads from the OFF vs. ON cells are shown for domains that were well-expressed. Pfam-annotated activator domain families (FOXO-TAD, Myb LMSTEN, TORC_C) are colored in shades of red. A line is drawn to the strongest hit, the KRAB domain from ZNF473. The hit threshold is a dashed line drawn two standard deviations below the mean of the poorly expressed domain distribution. FIG. 14C - Rank list of domain families with at least one activator hit. Families previously annotated as activators in Pfam are in red. The dashed line represents the hit threshold, as in FIG. 14B. Only well-expressed domains are shown. FIG. 14D - Acidity of effector domains from the Pfam library, calculated as net charge per amino acid. (Left) Comparison of the nonhit, well-expressed Pfam domains (except KRAB and annotated activators) with the activator hits. The Pfam-annotated activator domain families are shown as a group as a positive control (orange). (Right) Comparison of the activator hits and non-hits from the KRAB domain family. P-values from Mann-Whitney test shown with bars between compared groups. n.s. = not significant (p>0.05). FIG. 14E - Phylogenetic tree of all well-expressed KRAB domains with the sequence-divergent variant KRAB cluster shown in green (top). High-throughput recruitment measurements for repression at Day 5 are shown in blue (middle) and measurements for activation are shown in red (bottom). Dashed horizontal lines show hit thresholds. An example repressor KRAB from ZNF10, the repressor KRAB_1 from ZFP28, and all of the activator KRAB domains are called out with larger labels. The KRAB domain start position is written in parentheses. FIG. 14F - Individual validation of variant KRAB activator domains. rTetR(SE-G72P)-domain fusions were delivered to K562 reporter cells by lentivirus and selected for with blasticidin, cells were treated with 1000 ng/ml doxycycline for 2 days, and then citrine reporter levels were measured by flow cytometry. Untreated cell distributions are shown in light grey and doxycycline-treated cells are shown in colors, with two independently-transduced biological replicates in each condition. The vertical line shows the citrine gate used to determine the fraction of cells ON and the average fraction ON for the doxycycline-treated cells is shown. FIG. 14G - Distance of ChIP peak locations of KRAB Zinc Finger proteins away from the nearest peaks of the active chromatin mark H3K27ac. KRAB proteins are classified by their status as hits (blue) or non-hits (green) in the repressor screen at day 5 (left). In addition, data is shown individually for ZNF10 which contains a repressor hit KRAB (black), ZNF473 which contains an activator hit KRAB (red), and ZFP28 which contains both an activator hit and a repressor hit KRAB (yellow) (right). Each dot shows the fraction of peaks in a 40 basepair bin. ChIP-seq and ChiP-exo data retrieved from (ENCODE Project Consortium et al., 2020; Imbeault et al., 2017; Najafabadi et al., 2015; Schmitges et al., 2016). Only solo peaks, where a single KRAB Zinc Finger binds, are included for the aggregated data (blue and green dots, left), but all peaks are included for the individual proteins because the number of solo peaks is low for each individual protein (red, black, and yellow dots, right).

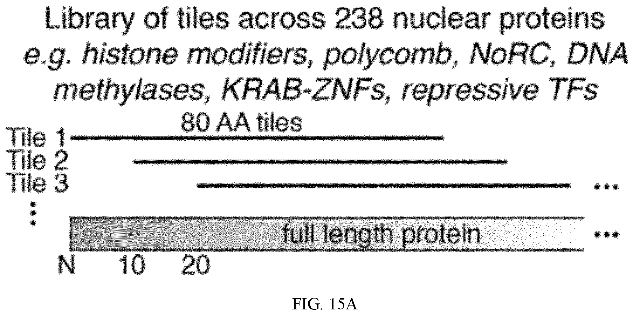

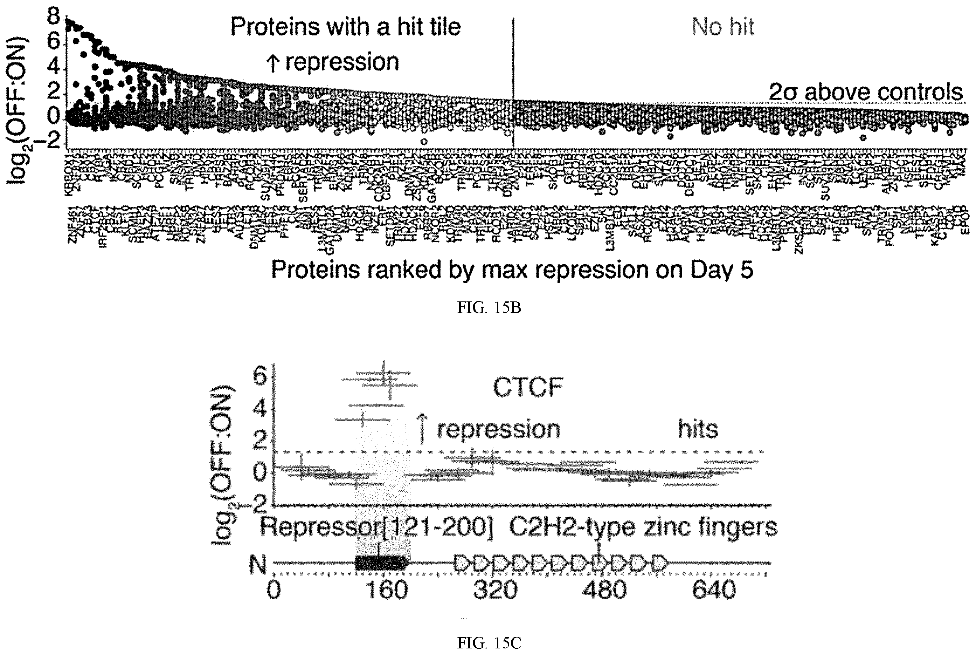

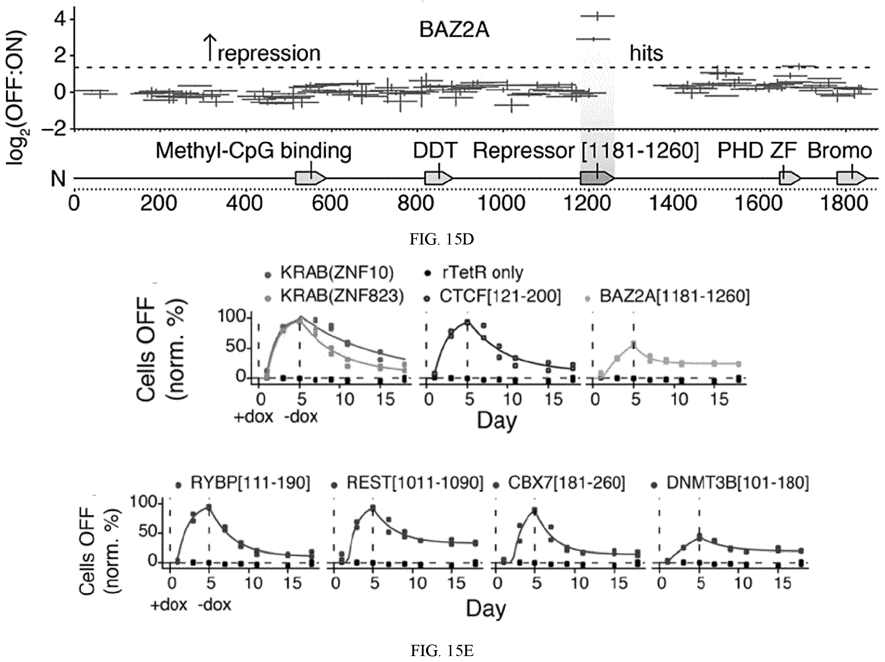

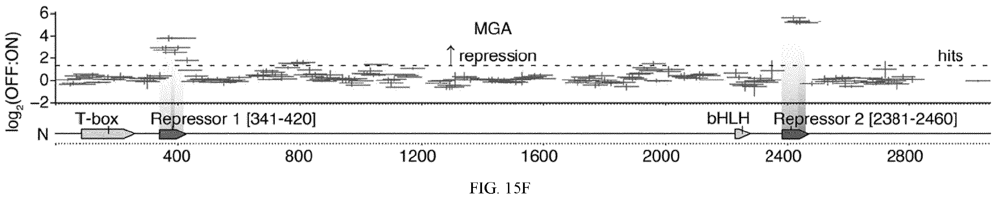

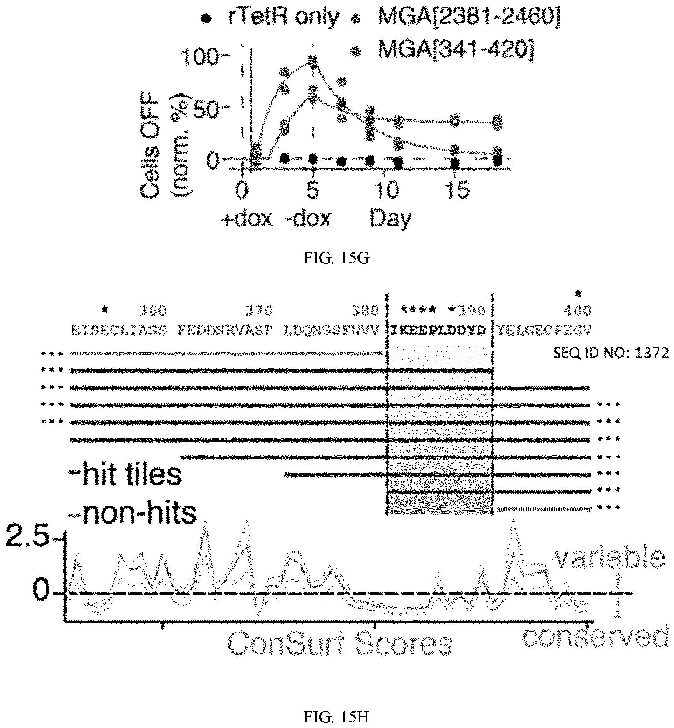

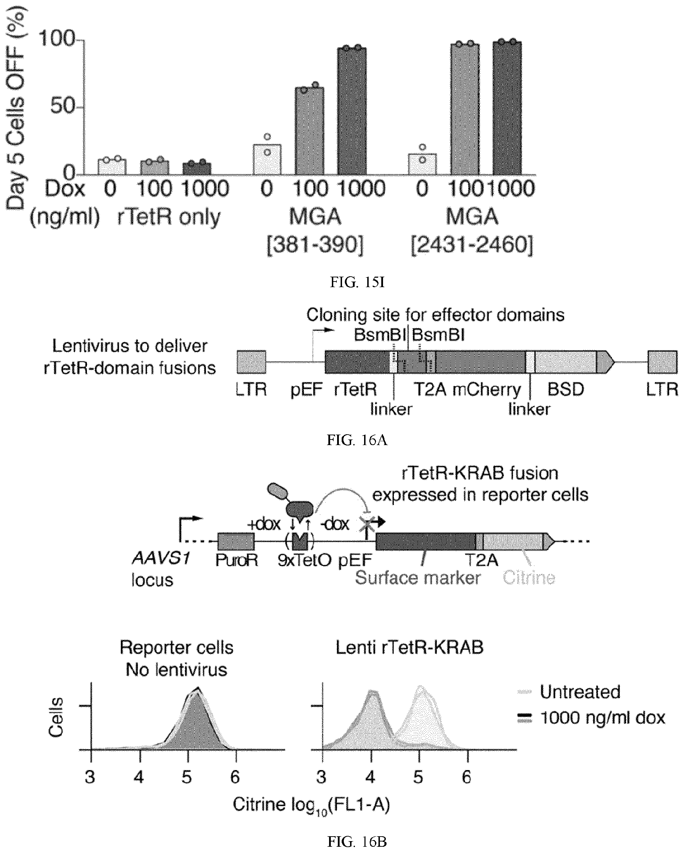

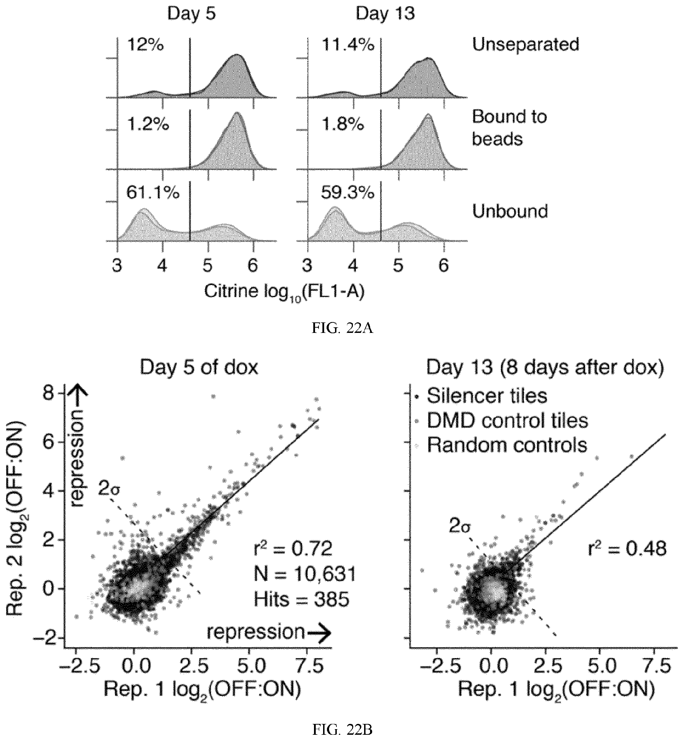

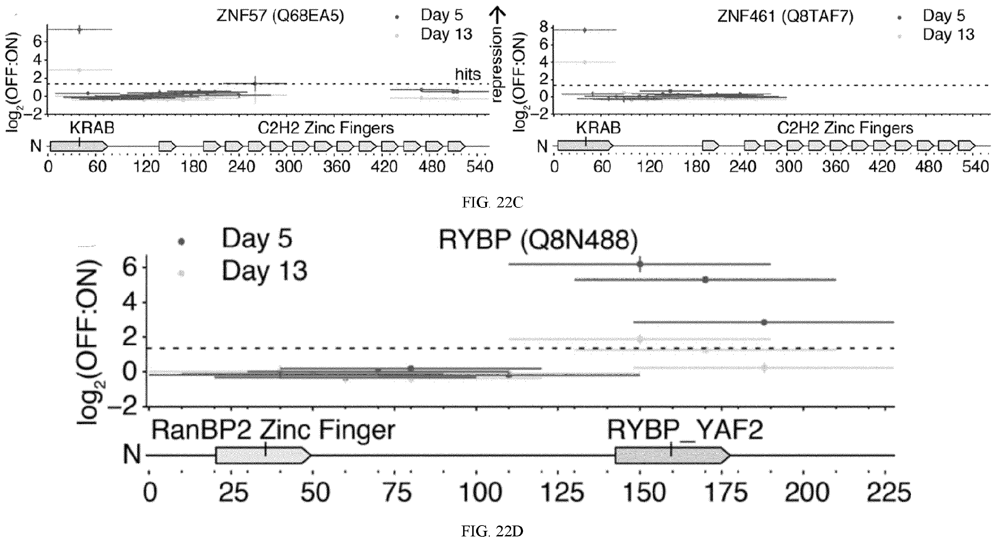

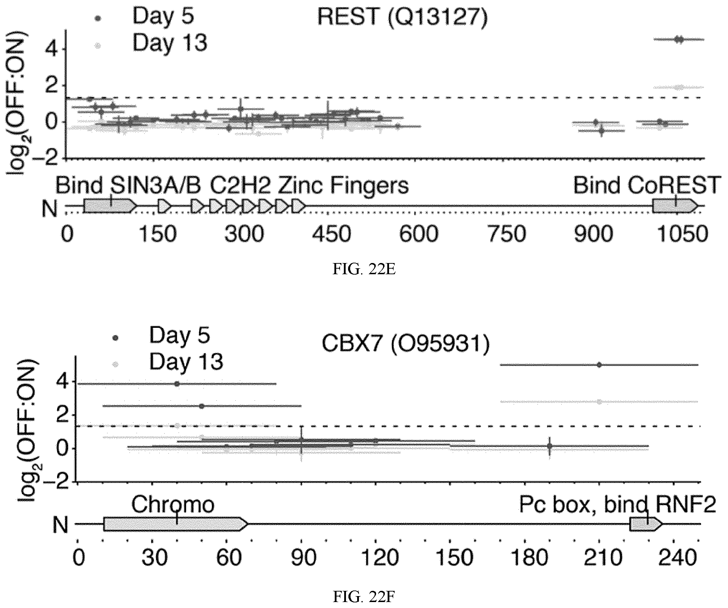

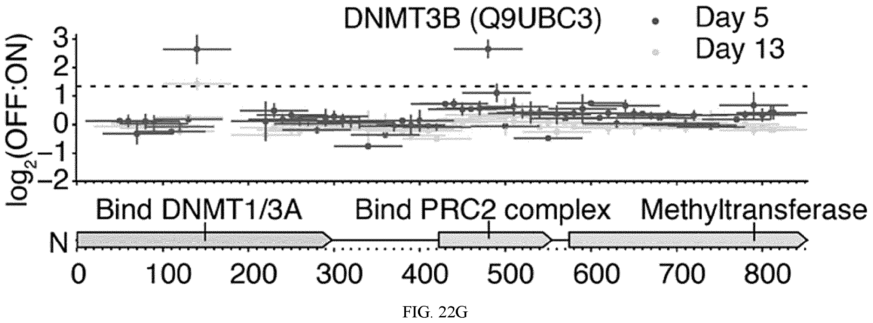

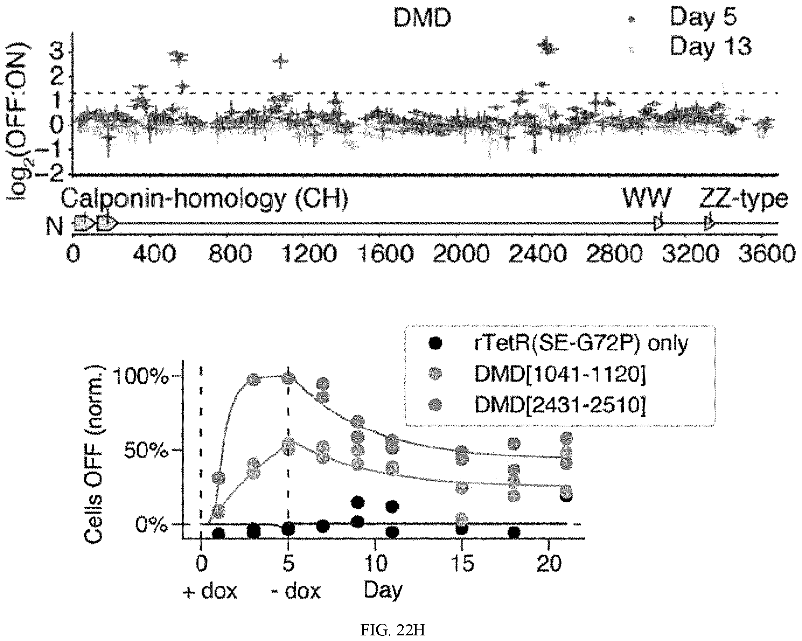

FIGS. 15A-15I show compact repressor domains discovered within nuclear proteins. FIG. 15A - Schematic of 80 AA tiling library covering a curated set of 238 nuclear-localized proteins. These tiles were fused to rTetR and recruited to the reporter, using the same workflow as in **FIG.** 1 to measure repression strength. FIG. 15B - Tiled genes ranked by maximum repressor function at day 5 shown with a dot for each tile. Hits are tiles with a log2(OFF:ON) ≥ 2 standard deviations above the mean of the negative controls. Genes with a hit tile are colored in a gradient and genes without any hit tiles are colored in grey. FIG. 15C - Tiling CTCF. Diagram shows protein annotations retrieved from UniProt. Horizontal bars show the region spanned by each tile and vertical error bars show the standard error from two biological replicates of the screen. The strongest hit tile is highlighted with a vertical gradient and annotated as a repressor domain (orange). FIG. 15D - Tiling BAZ2A (also known as TIP5). FIG. 15E - Individual validations. Lentiviral rTetR(SE-G72P)-tile fusions were delivered to K562 reporter cells, cells were treated with 100 ng/ml doxycycline for 5 days (between dashed vertical lines), and then doxycycline was removed. Cells were analyzed by flow cytometry, the fraction of cells with citrine reporter OFF was determined and the data fit with the gene silencing model (N = 2 biological replicates). Two KRAB repressor domains are shown as positive controls. The tiling screen data that corresponds to the validations shown on the bottom (blue curves) is shown in FIG. 22. FIG. 15F - Tiling MGA. Two repressor domains are found outside the previously annotated regions and labeled as Repressor 1 and 2 (dark red, purple). The minimized repressor regions at the overlap of hit tiles are highlighted with narrow red vertical gradients. FIG. 15G - The maximal strength repressor tiles from two peaks in MGA were individually validated with the method described in FIG. 15E (N = 2 biological replicates). FIG. 15H - The MGA repressor 1 sequence was minimized by selecting the region shared in common between all hit tiles in the peak, shown between dashed vertical lines and shaded in red. The protein sequence conservation ConSurf score is shown below with an orange line and the confidence interval (the 25th and 75th percentiles of the inferred evolutionary rate distribution) is shown in grey. The asterisks mark residues that are predicted to be functional (highly conserved and exposed) by ConSurf. The repressor 2 sequence was minimized with the same approach and also overlaps a region with predicted functional residues (data not shown). FIG. 15I - The MGA effectors were minimized to 10 and 30 AA sub-tiles, as shown in FIG. 15H, cloned as lentiviral rTetR(SE-G72P)-tile fusions, and were delivered to K562 reporter cells. After selection, cells were treated with 100 or 1000 ng/ml doxycycline for 5 days and the percentages of cells with the Citrine reporter silenced were measured by flow cytometry (N = 2 biological replicates).

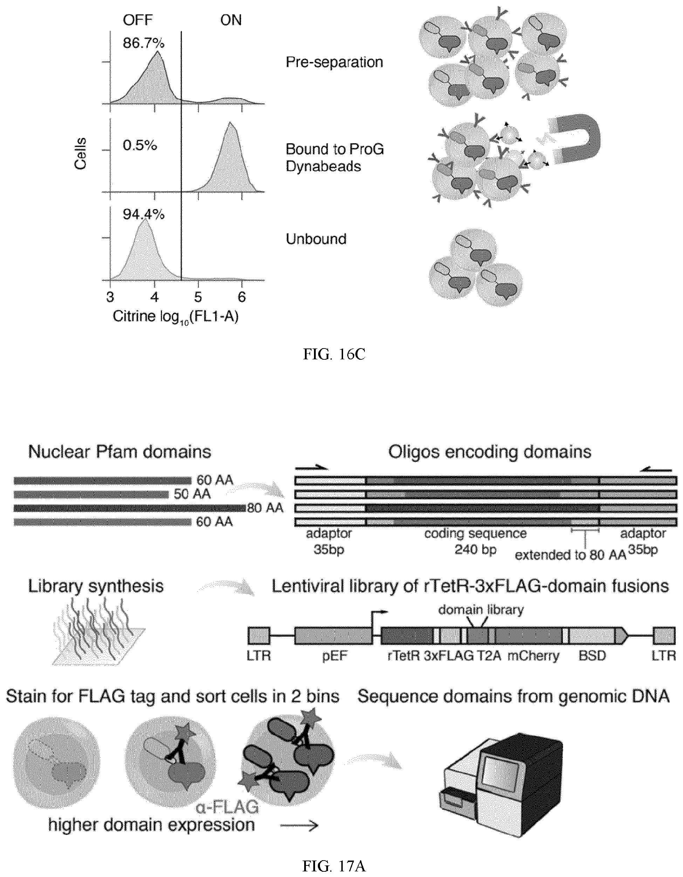

FIGS. 16A-16C show validation of lentiviral recruitment assay and dual reporter for gene silencing. FIG. 16A - Schematic of lentiviral recruitment vector with Golden Gate cloning site for creating fusions of effector domains to the dox-inducible DNA-binding domain rTetR. The constitutive pEF promoter drives expression of the rTetR-effector fusion and mCherry-BSD (Blasticidin S deaminase resistance gene), separated by a T2A self-cleaving peptide. FIG. 16B - (Top) Schematic of rTetR-KRAB fusion recruitment to the dual reporter gene. The reporter is integrated in the *AAVS1* locus by TALEN-mediated homology-directed repair and the PuroR resistance gene is driven by the endogenous *AAVS1* promoter. The dual reporter consists of a synthetic surface marker (Igκ-hIgG1-Fc-PDGFRβ) and a citrine fluorescent protein. (Bottom) Pilot test in K562 reporter cells. Reporter cells were generated by TALEN-mediated homology-directed repair to integrate the reporter into the *AAVS1* locus and then selected with puromycin. Cells were then spinfected with lentivirus to deliver rTetR-KRAB, and then either left untreated or treated with 1000 ng/ml doxycycline to induce rTetR binding to DNA at the TetO sites. Untreated cell distributions are shown in light grey and doxycycline-treated cells are shown in black or orange, with two independently-transduced biological replicates in each condition. The lentivirus-treated cells are gated on mCherry as the delivery marker. The KRAB domain from human ZNF10 was used. FIG. 16C - Demonstration of magnetic separation of OFF from ON cells using ProG Dynabeads that bind to the synthetic surface marker. Ten million cells were subjected to magnetic separation using 30 µl of beads, and the citrine reporter expression was measured before and after by flow cytometry. Illustration of mixed ON and OFF cells being subjected to magnetic separation is shown on the right.

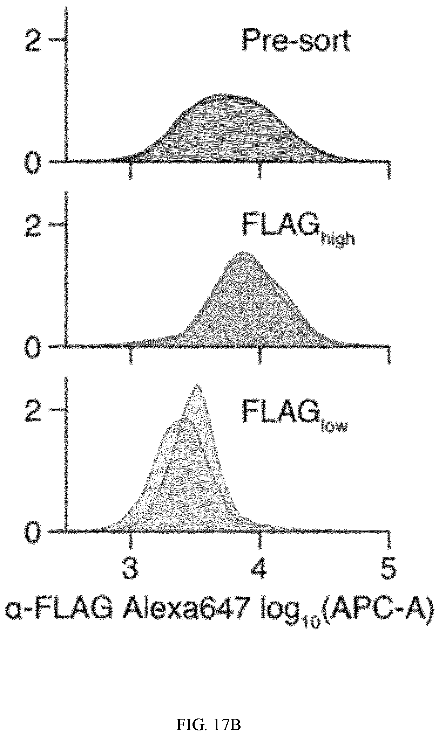

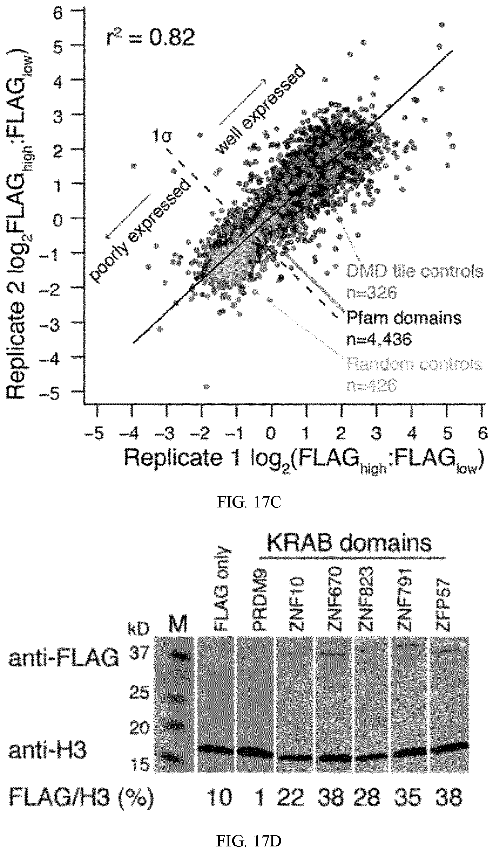

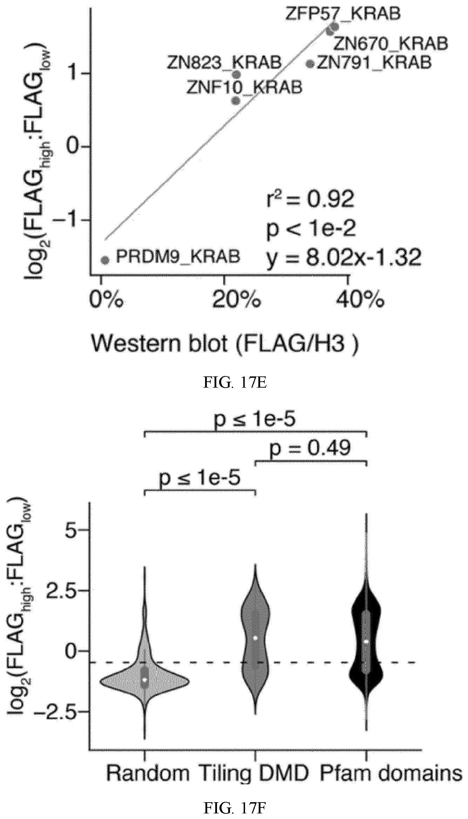

FIGS. 17A-17F show high-throughput measurements of domain expression by FLAG staining, sorting, and sequencing. FIG. 17A - (Top) Schematic of high-throughput strategy for measuring the expression level of each domain in the library. Domains under 80AA long are extended on both sides, using their native protein sequence, to reach 80AA so all synthesized library elements are the same length. (Middle) The library is cloned into a FLAG-tagged construct and delivered to K562 cells by lentivirus at low multiplicity of infection, such that the majority of cells express a single library member. The mCherry-BSD fusion protein enables blasticidin selection and a fluorescent marker for delivery and selection efficiency, without the use of a second 2A component. (Bottom) Expression is measured by staining the cells with anti-FLAG, sorting high and low expression populations, sequencing the domains, and computing the log2(FLAGhigh:FLAGlow) ratio. FIG. 17B - Distribution of FLAG staining levels measured by flow cytometry before and after sorting into two bins (N = 2 biological replicates of the cell library shown with overlapping shaded areas). FIG. 17C - Reproducibility of biological replicates from the domain expression screen (r² = 0.82). Well-expressed domains, above the threshold (dashed line one standard deviation above the median of the random controls), were selected for further analysis in the transcriptional regulation screens. FIG. 17D - Validation of expression level for a panel of KRAB domains. Individual rTetR-3XFLAG-KRAB constructs were delivered to K562 cells by lentivirus. Cells were selected with blasticidin and confirmed to be >80% mCherry positive by flow cytometry. Expression level was measured by Western blot with anti-FLAG antibody. Anti-histone H3 was used as a loading control for normalization. Levels were quantified using ImageJ. FIG. 17E - Comparison of high-throughput measurements of expression with Western blots protein levels. These 6 KRAB domains were cloned individually using the exact 80 AA sequence from the Pfam domain library. FIG. 17F - Distribution of expression levels for different categories of library members. Random controls are poorly expressed compared to tiles across the DMD protein or Pfam domains (p<1e-5, Mann Whitney test). Dashed line shows the threshold for expression level, as in FIG. 17C.

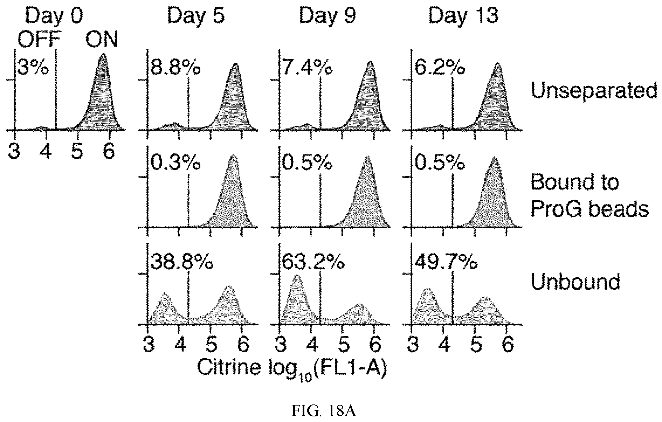

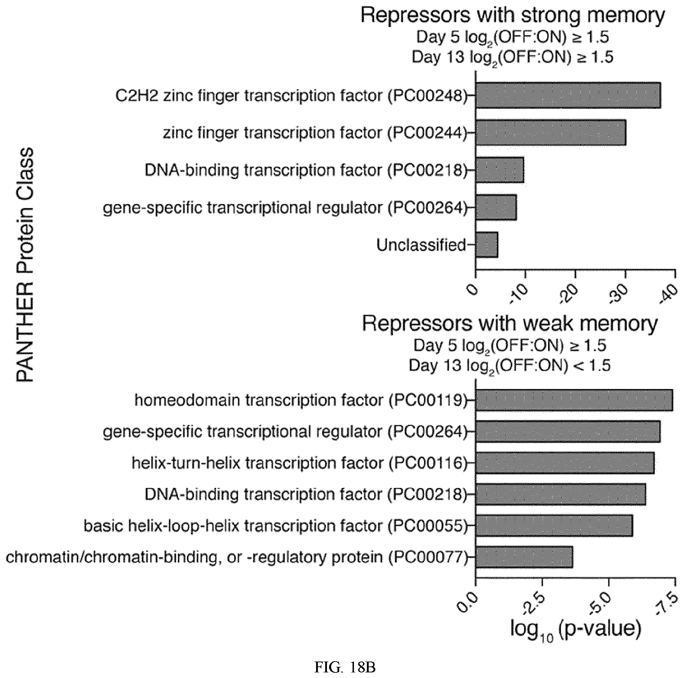

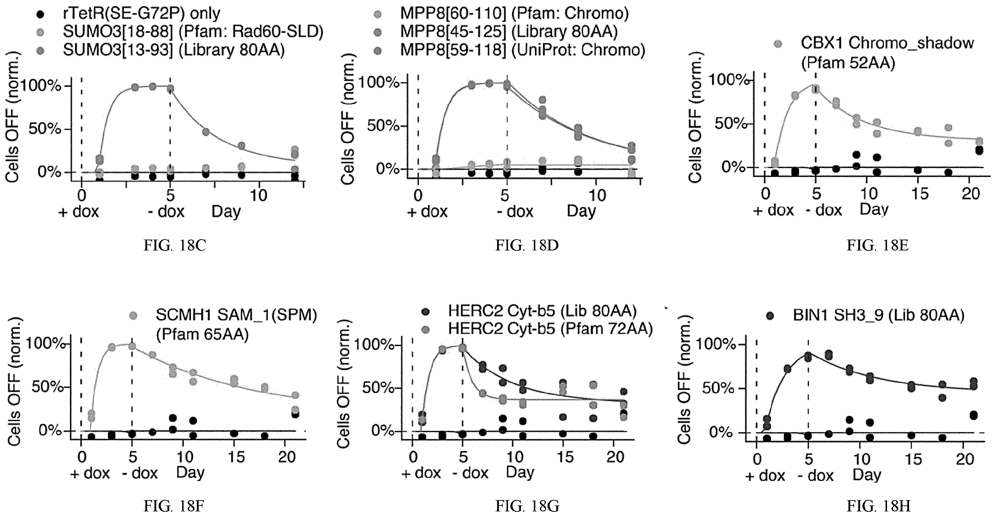

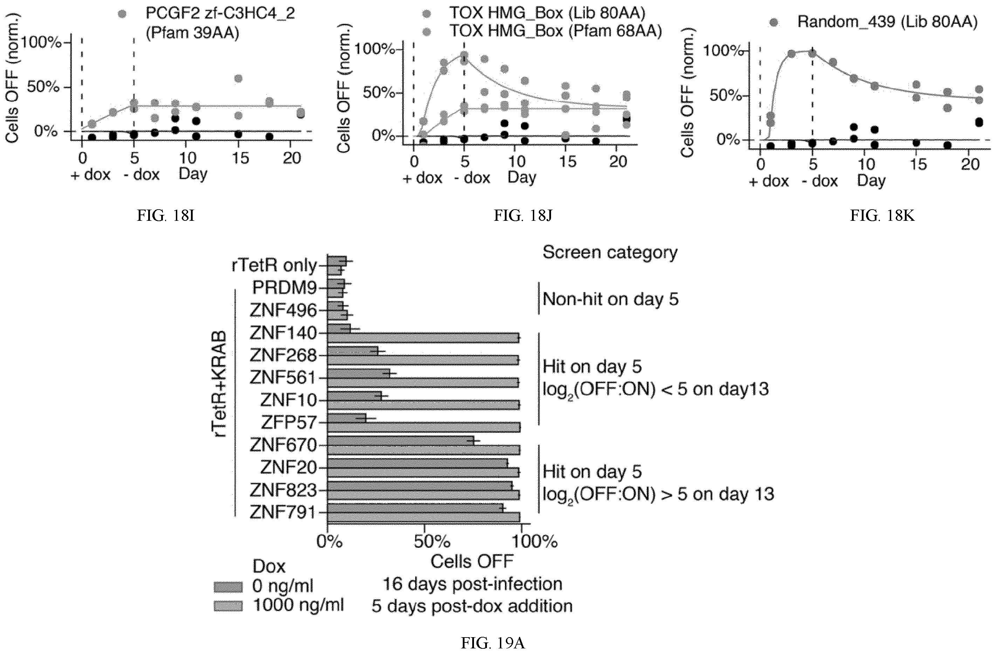

FIGS. 18A-18K show identification of domains with repressor function. FIG. 18A - Flow cytometry shows citrine reporter level distributions in the pool of cells expressing the Pfam domain library, before and after magnetic separation using ProG DynaBeads that bind to the synthetic surface marker. Overlapping histograms are shown for two biological replicates. The average percentage of cells OFF is shown to the left of the vertical line showing the citrine level gate. 1000 ng/ml doxycycline was added on Day 0 and removed on Day 5. FIG. 18B - PANTHER protein class enrichments for nuclear proteins that contain repressor domains with stronger or weaker memory, when compared to the background set of all nuclear proteins with domains included in the library. FIG. 18C - rTetR-SUMO validation time courses fit with gene silencing model. The 80 AA sequence centered around the Rad60-SLD domain of SUMO3 and the trimmed domain were individually cloned into lentivirus and delivered to the reporter cells. 1000 ng/ml doxycycline was added on day 0 and removed on day 5 (N = 2 biological replicates). The fraction of mCherry-positive cells with the citrine reporter OFF was determined by flow cytometry and normalized for background silencing using the untreated, time-matched controls. FIG. 18D - HUSH complex member MPP8 Chromo domain validation with the full 80 AA sequence used in the screen and sequences trimmed to match the Pfam and UniProt annotations. FIG. 18E - CBX1 Chromoshadow domain validation with 52 AA sequence trimmed to match the Pfam annotation. FIG. 18F - Polycomb 1 component SCMH1 SAM1 domain (also known as SPM) validation with 65 AA sequence trimmed to match the Pfam annotation. FIG. 18G - HERC2 Cyt-b5 domain validation with the full 80 AA sequence used in the screen and a 72 AA sequence trimmed to match the Pfam annotation. FIG. 18H - BIN1 SH3_9 domain validation. FIG. 18I - Polycomb 1 component PCGF2 zf-C3HC4_2 domain validation with 39 AA sequence trimmed to match the Pfam annotation. FIG. 18J - TOX HMG box domain validation with the full 80 AA sequence used in the screen and a 68 AA sequence trimmed to match the Pfam annotation. FIG. 18K - Validation of a random 80 AA sequence that functions as a repressor.

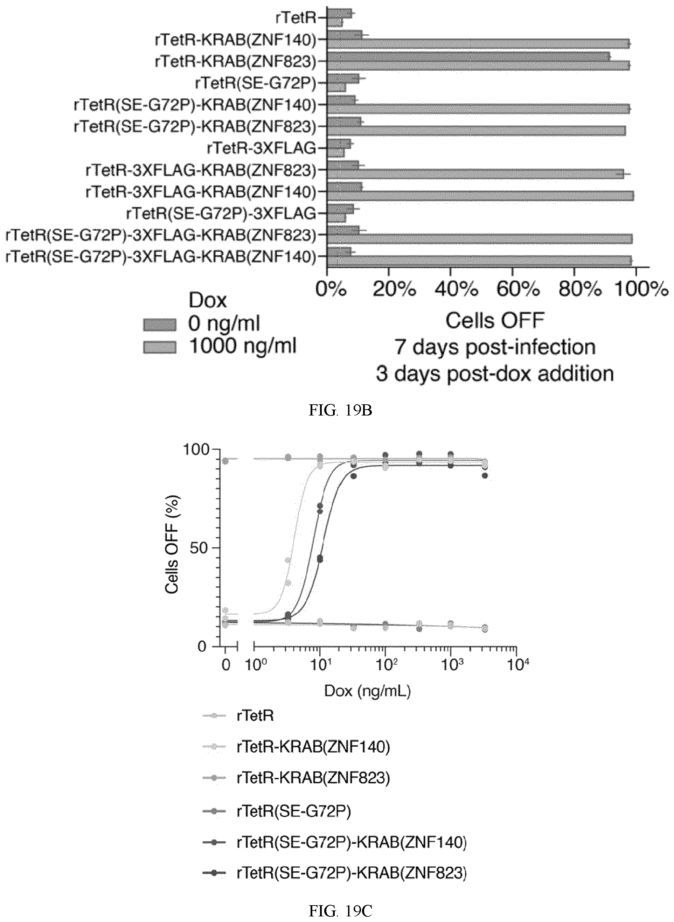

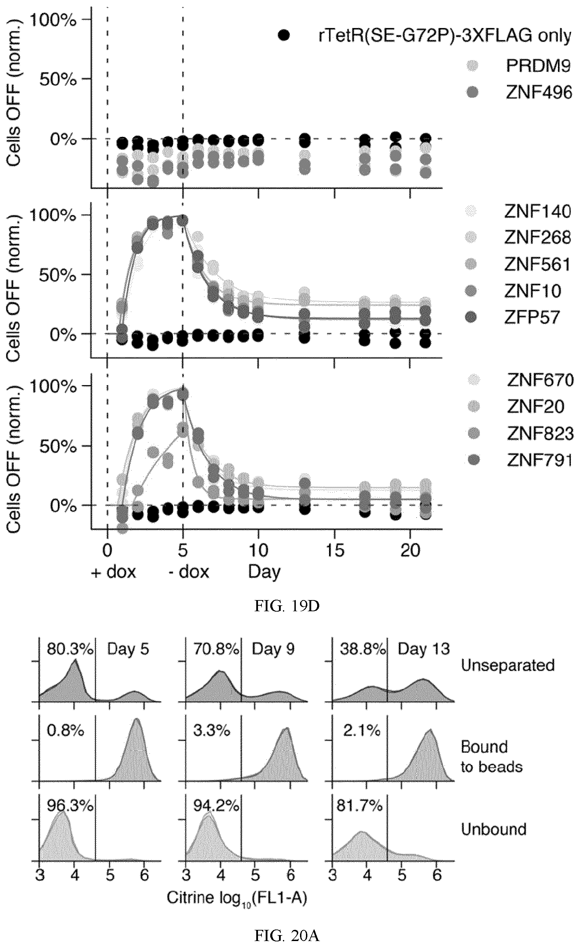

FIGS. 19A-19D show rTetR(SE-G72P) mitigates leaky KRAB silencing in human cells. FIG. 19A - Silencing by rTetR-KRAB fusions, showing leaky silencing without doxycycline treatment for a subset of KRAB domains (high gray bars). Constructs were delivered to reporter cells by lentivirus at day 0, cells were selected with blasticidin between days 3 and 11, cells were split into a doxycycline-treated or untreated condition at day 11, and reporter levels were measured by flow cytometry at day 16. Results are shown after gating for the mCherry positive cells. The KRAB domains were selected from three categories based on their measurements in the screen, labeled on the right. The bar shows the average and the error bar shows the standard deviation (N = 3 independently transduced biological replicates). FIG. 19B - Leakiness can be mitigated by using rTetR(SE-G72P) or introducing 3XFLAG between rTetR and the KRAB domain from ZNF823. Constructs were delivered to reporter cells by lentivirus at day 0, cells were split into a doxycycline-treated or untreated condition at day 4, and reporter levels were measured by flow cytometry at day 7. Results are shown after gating for the mCherry positive cells. A non-leaky KRAB domain from ZNF140 was used as a control. The bar shows the average and the error bar shows the standard deviation (N = 2 independently transduced biological replicates). FIG. 19C - The K562 reporter cell lines with stable lentiviral expression of either a leaky KRAB domain from ZNF823 or a non-leaky repressor KRAB domain from ZNF140 cloned as fusions with either rTetR or rTetR(SE-G72P) were treated with varied doses of doxycycline. Reporter levels were measured by flow cytometry four days later, and the percentage of mCherry positive cells with the citrine reporter OFF is shown (N = 2 independently transduced biological replicates). The dose response was fit by least squares with a non-linear variable slope sigmoidal curve using PRISM statistical analysis software. FIG. 19D - Silencing and memory dynamics for all individual validations of KRAB domains, fit with the gene silencing model. rTetR(SE-G72P)-KRAB fusions were delivered to K562 reporter cells by lentivirus, selected with blasticidin, and then 10 ng/ml doxycycline was added on day 0 and removed on day 5 (N = 2 biological replicates). The fraction of mCherry positive cells with the citrine reporter OFF was determined by flow cytometry and normalized for background silencing using the untreated, time-matched controls. 10 ng/ml dox was used to work in a dynamic range where it is easier to measure differences in silencing and memory capabilities between fast KRAB silencing domains. With 1000 ng/ml doxycycline, all of the repressor hit KRAB domains (greens and oranges) fully silence the reporter within 5 days with indistinguishable dynamics (data not shown). Notably, the KRABs that were leaky on rTetR (oranges), do not show significantly different memory dynamics from the KRABs that were not leaky (greens) when fused to the rTetR(SE-G72P). Importantly, none of the rTetR(SE-G72P)-KRAB fusions showed significant leaky silencing in the untreated condition.

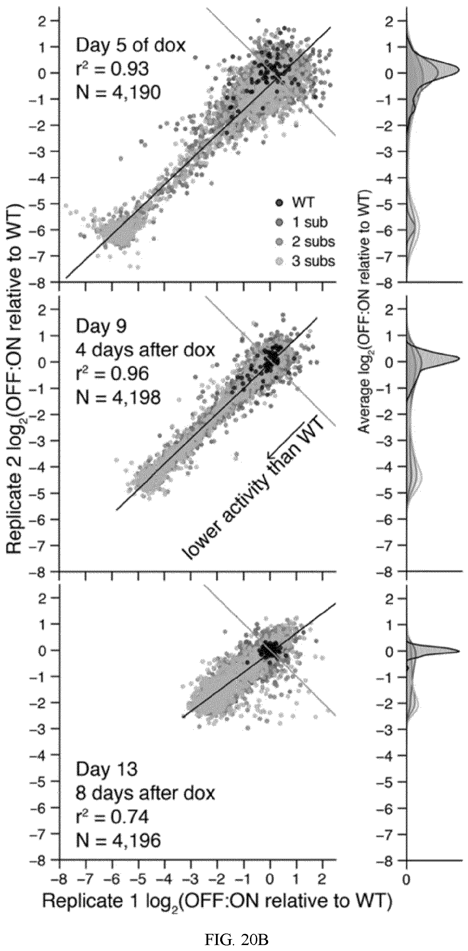

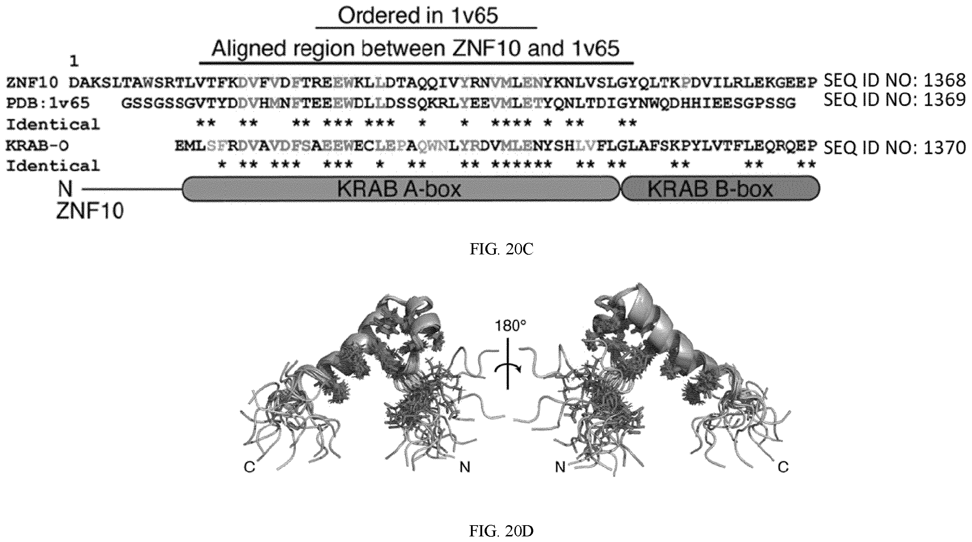

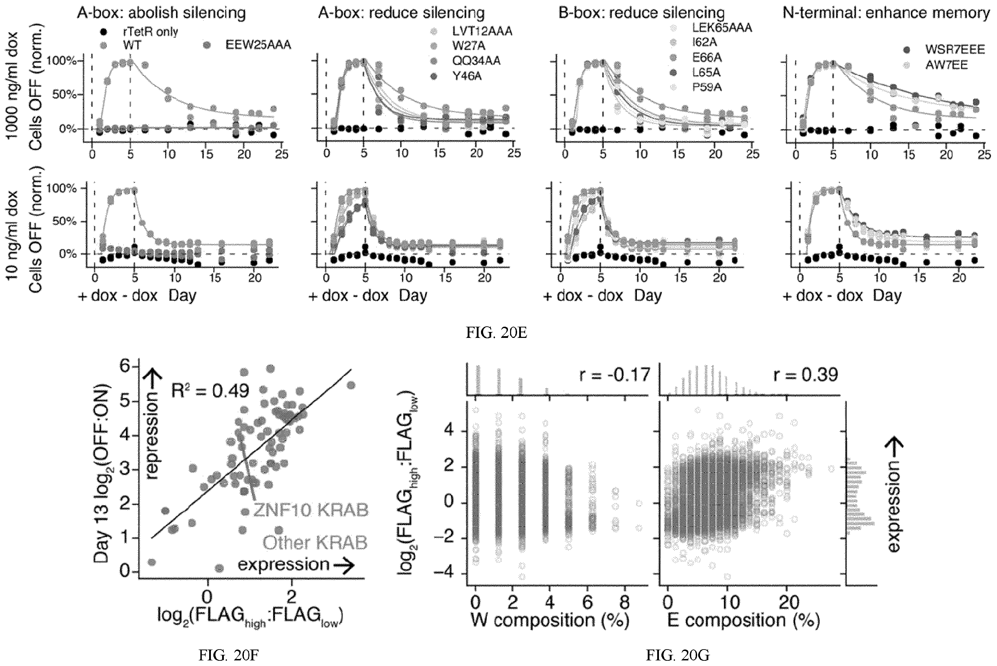

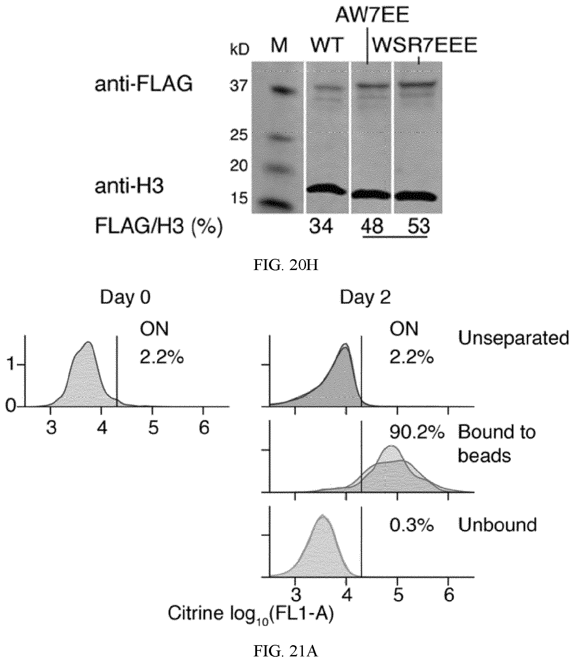

FIGS. 20A-20H show deep mutational scan of ZNF10 KRAB used in CRISPRi. FIG. 20A - Flow cytometry shows citrine reporter levels in the cells with the pooled KRAB library, before and after magnetic separation using ProG DynaBeads that bind to the synthetic surface marker. Overlapping histograms are shown for two biological replicates. The average percentage of cells OFF is shown to the left of the vertical line showing the citrine level gate. FIG. 20B - OFF: ON enrichments from two biological replicates of the deep mutational library of the ZNF10 KRAB domain at days 5, 9 and 13. Cells were treated with 1000 ng/ml doxycycline for the first 5 days. Grey diagonal lines show where the average log2(OFF:ON) is the median of the WT domains (black dots). The black diagonal lines show the fit linear model. FIG. 20C - Alignment of human ZNF10 KRAB with mouse KRAB used in the NMR structure (PDB:1v65) and KRAB-O used in the recombinant protein binding assays (Peng et al., 2009). The ordered region is used in FIG. 3 and the aligned region containing all 12 necessary residues is used in (FIG. 20D). The residues necessary for silencing at day 5 are colored in red in the ZNF10 and PDB: 1v65 sequences. The residues necessary for binding recombinant KAP1 are colored in red and the residues unnecessary for binding KAP1 are colored in grey in the KRAB-O sequence, summarizing previously published results (Peng et al., 2009). FIG. 20D - Ensemble of 20 states of the KRAB NMR structure (PDB:1v65). The residues necessary for silencing at day 5 are colored in red. FIG. 20E - Silencing and memory dynamics for all individual validations of KRAB ZNF10 mutants, fit with the gene silencing model. (Top) rTetR-KRAB fusions were delivered to K562 reporter cells by lentivirus, selected with blasticidin, and then 1000 ng/ml doxycycline was added on day 0 and removed on day 5 (N = 2 biological replicates. (Bottom) rTetR(SE-G72P)-KRAB fusions were delivered to K562 reporter cells by lentivirus, selected with blasticidin, and then 10 ng/ml doxycycline was added on day 0 and removed on day 5 (N = 2 biological replicates). The column labels describe the variant location within the KRAB domain and impact on effector function. The fraction of mCherry positive cells with the citrine reporter OFF was determined by flow cytometry and normalized for background silencing using the untreated, time-matched controls. All of the rTetR(SE-G72P)-KRAB fusions were also measured over 5 days of treatment with 1000 ng/ml doxycycline and the results were indistinguishable from those with rTetR, with all KRAB variants completely silencing the reporter except the EEW25AAA variant that does not silence (data not shown). FIG. 20F - Correlation of rTetR-KRAB fusion expression level and day 13 silencing score, from the Pfam domain library. Only KRAB domains that were shown to interact with co-repressor KAP1 by IP/MS (Helleboid et al., 2019) are included. FIG. 20G - Correlations of amino acid frequency with domain expression level, across the library of Pfam domains and controls (Pearson's r value is shown). FIG. 20H - Western blot for FLAG-tagged rTetR-KRAB fusions after lentiviral delivery to K562. Cells were selected for delivery with blasticidin and were confirmed to be >80% mCherry positive by flow cytometry. Expression level relative to the H3 loading control was quantified using ImageJ.

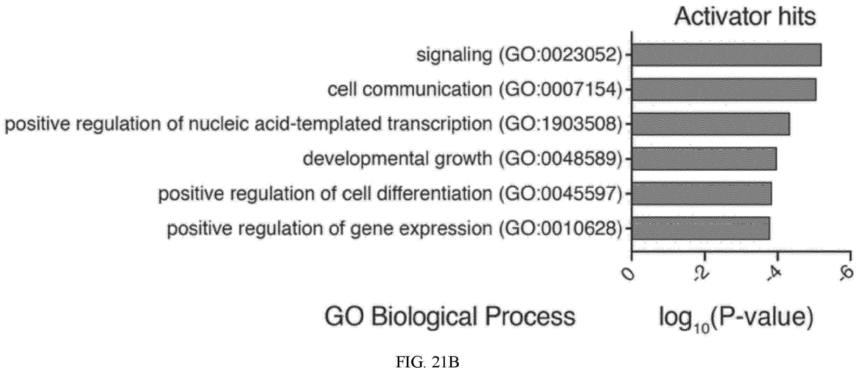

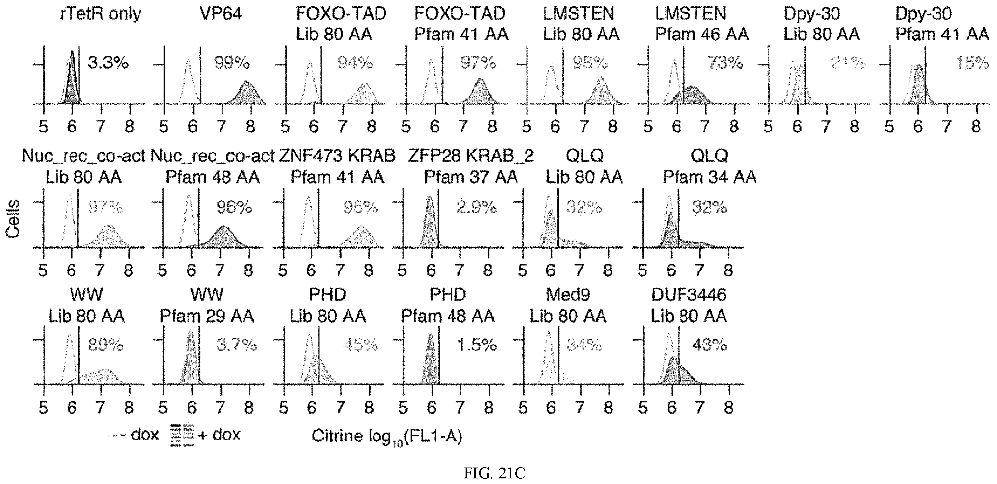

FIGS. 21A-21C show HT-recruit to a minimal promoter discovers activator domains. FIG. 21A - Flow cytometry for pooled library of Pfam domains in activation reporter cells, before and after magnetic separation. The percentage of cells ON is shown to the right of the citrine level gate, drawn with a vertical line. 1-2 biological replicates are shown with overlapping shaded areas. FIG. 21B - GO term enrichment of genes containing a hit activation domain, compared to the background set of all proteins containing a well-expressed domain in the library after counts filtering. Raw p-values are shown, and all shown terms are below a 10% false discovery rate. FIG. 21C - Individual validations of activator domains. rTetR(SE-G72P)-domain fusions were delivered to K562 reporter cells by lentivirus and selected with blasticidin. Cells were treated with 1000 ng/ml doxycycline for 2 days, and then citrine reporter levels were measured by flow cytometry. Untreated cell distributions are shown in light grey and doxycycline-treated cells are shown in colors, with two independently-transduced biological replicates in each condition. The vertical line shows the citrine gate used to determine the fraction of cells ON, and the average fraction ON for the doxycycline-treated cells is shown. VP64 is a positive control. Each domain was tested as both the extended 80 AA sequence from the library or the trimmed Pfam-annotated domain sequence, with the exceptions of Med9 and DUF3446 which had minimal extensions because the Pfam annotated regions are 75 and 69 AA, respectively. The corresponding results for the 80 AA library sequence for the KRAB domains are shown in FIG. 14.
FIGS. 22A-22H show identification of compact repressor domains in nuclear proteins with tiling screen. FIG. 22A - Flow cytometry shows citrine reporter level distributions in the pool of cells expressing the tiling library, before and after magnetic separation using ProG DynaBeads that bind to the synthetic surface marker. Overlapping histograms are shown for two biological replicates. The average percentage of cells OFF is shown to the left of the vertical line showing the citrine level gate. 1000 ng/ml doxycycline was added on Day 0 and removed on Day 5. FIG. 22B - High-throughput recruitment measurements from two biological replicates of a nuclear protein tiling library at Day 5 of doxycycline treatment and Day 13, 8 days after doxycycline removal. The hit calling threshold is 2 standard deviations above the mean of the random and DMD tiling controls. FIG. 22C - Tiling results for KRAB Zinc finger proteins ZNF57 and ZNF461. Each bar is an 80 AA tile and the vertical error bars are the range from 2 biological replicates. Protein annotations are sourced from UniProt. FIG. 22D - Tiling RYBP. Diagram shows protein annotations, retrieved using the UniProt ID written at top. Vertical error bars show the standard error from two biological replicates. FIG. 22E - Tiling REST. FIG. 22F - Tiling CBX7. FIG. 22G - Tiling DNMT3B. FIG. 22H (Top) Tiling DMD. (Bottom) Dynamics of silencing and memory after recruitment of DMD hit tiles. Cells were treated with 1000 ng/ml doxycycline for the first 5 days and citrine reporter levels were measured by flow cytometry. The percentage of cells OFF was normalized to account for background silencing and the data (dots) were fit with a gene silencing model (curves) (N = 2 biological replicates).

### DETAILED DESCRIPTION

The invention is set out in the appended claims. The embodiments of the description which do not fall within the scope of said claims are provided for illustrative purposes only and do not form part of the present invention. Systems and methods to generate a catalog of compact transcriptional effector domains is provided. Further, his catalog of domains is fused onto DNA binding domains to engineer synthetic transcription factors. These find use to perform targeted and tunable regulation of gene expression in eukaryotic (or other) cells. This technology leverages a high-throughput platform to screen and characterize tens of thousands of synthetic transcription factors in cells. These synthetic transcription factors are fusions between a DNA binding domain and a transcriptional effector domain. The system has been used to generate hundreds of short effector domains (e.g., 80 amino acids) and a high-throughput process for shortening them further to the minimally sufficient sequences (e.g., 10 amino acids), which is an advantage for delivery (e.g., packaging in viral vectors). The targeting of these fusions generates local regulation of mRNA transcription, either negatively or positively depending on the effector domain. Some of these synthetic transcription factors mediate long-term epigenetic regulation that persists after the factor itself has been released from the target.

Previously, a limited number of transcriptional effector domains were available for the engineering of synthetic transcription factors. To address this limitation, provided herein is a high-throughput approach to screening and quantifying the function of transcriptional effectors domains. This approach enabled the discovery of hundreds of effector domains that can upregulate or downregulate transcription in a targeted manner when fused onto a DNA binding domain. This process also finds use to identify mutants of effector domains with enhanced activity. These effector domains find use to engineer synthetic transcription factors for applications in gene and cell therapy, synthetic biology, and functional genomics.

Exemplary applications include, but are not limited to:
Targeted repression/activation of endogenous genes with fusions of programmable DNA binding domains (e.g., dCas9, dCas12a, zinc finger, TALE) to transcriptional effector domains.

Gene and cell therapy (e.g., to silence a pathogenic transcript in a patient) or in research.

Synthetic transcription factors find use to perturb the expression of multiple genes simultaneously (e.g., to perform high-throughput genetic interaction mapping with CRISPRi/a screens using multiple guide RNAs).

Use in synthetic transcription factors in genetic circuits, e.g., inducible gene expression or more complex circuits. These circuits find use in gene therapy (e.g., AAV delivery of antibodies) and cell therapy (e.g., ex vivo engineering of CAR-T cells) to achieve therapeutic gene expression outputs in response to environmental and small molecule inputs.

The new transcriptional effector domains provided herein have several advantages for applications that rely on synthetic transcription factors. Short domains were identified (e.g., 80 amino acids or less) and a high-throughput process was generated for shortening them further to the minimally sufficient sequence, which is an advantage for delivery (e.g., packaging in viral vectors). In some cases, potent effector domains were identified that were as short as 10 amino acids. In some embodiments, the domains are extracted from human proteins, which provides the advantage of reducing immunogenicity in comparison to viral effector domains. Most of the domains generated have not been reported as transcriptional effectors previously. In addition, a high-throughput process is provided for testing mutations in these domains in order to identify enhanced variants. The high-throughput approach is more readily aided by the development of an artificial cell surface marker that provides more efficient, inexpensive, and rapid screening of these libraries using magnetic separation. This is an advantage over the more conventional approach of sorting libraries based on fluorescent reporter gene expression.

The collection of domains identified is large and diverse, and the platform readily enables new combinations of domains to be tested as fusions in high-throughput to create synthetic transcription factors with new properties (e.g., compositions of two repressor domains to achieve a combination of fast silencing and permanent silencing).

Hundreds of previously uncharacterized or unknown effector domains that can silence or active transcription and can be fused onto DNA binding domains. For example, a high-throughput approach for screening single domains and pairs of domains using lentiviral screens in human cells is provided. The high-throughput approach is more readily enabled by the development of an artificial cell surface marker that provides more efficient, inexpensive, and rapid screening of these libraries using a magnetic separation.

### 1. Definitions

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

Unless otherwise defined herein, scientific, and technical terms used in connection with the present disclosure shall have the meanings that are commonly understood by those of ordinary skill in the art. For example, any nomenclature used in connection with, and techniques of, cell and tissue culture, molecular biology, immunology, genetics and protein and nucleic acid chemistry and hybridization described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

The term "antibody," as used herein, refers to a protein that is endogenously used by the immune system to identify and neutralize foreign objects, such as bacteria and viruses. Typically, an antibody is a protein that comprises at least one complementarity determining region (CDR). The CDRs form the "hypervariable region" of an antibody, which is responsible for antigen binding (discussed further below). A whole antibody typically consists of four polypeptides: two identical copies of a heavy (H) chain polypeptide and two identical copies of a light (L) chain polypeptide. Each of the heavy chains contains one N-terminal variable (V_{H}) region and three C-terminal constant (C_{H1}, C_{H2}, and C_{H3}) regions, and each light chain contains one N-terminal variable (V_{L}) region and one C-terminal constant (C_{L}) region. The light chains of antibodies can be assigned to one of two distinct types, either kappa (κ) or lambda (λ), based upon the amino acid sequences of their constant domains. In a typical antibody, each light chain is linked to a heavy chain by disulfide bonds, and the two heavy chains are linked to each other by disulfide bonds. The light chain variable region is aligned with the variable region of the heavy chain, and the light chain constant region is aligned with the first constant region of the heavy chain. The remaining constant regions of the heavy chains are aligned with each other. The variable regions of each pair of light and heavy chains form the antigen binding site of an antibody. The V_{H} and V_{L} regions have the same general structure, with each region comprising four framework (FW or FR) regions. The term "framework region," as used herein, refers to the relatively conserved amino acid sequences within the variable region which are located between the CDRs. There are four framework regions in each variable domain, which are designated FR1, FR2, FR3, and FR4. The framework regions form the β sheets that provide the structural framework of the variable region (see, e.g., C. A. Janeway et al. (eds.), Immunobiology, 5th Ed., Garland Publishing, New York, N.Y. (2001)). The framework regions are connected by three CDRs. As discussed above, the three CDRs, known as CDR1, CDR2, and CDR3, form the "hypervariable region" of an antibody, which is responsible for antigen binding. The CDRs form loops connecting, and in some cases comprising part of, the beta-sheet structure formed by the framework regions. While the constant regions of the light and heavy chains are not directly involved in binding of the antibody to an antigen, the constant regions can influence the orientation of the variable regions. The constant regions also exhibit various effector functions, such as participation in antibody-dependent complement-mediated lysis or antibody-dependent cellular toxicity via interactions with effector molecules and cells.

The terms "fragment of an antibody," "antibody fragment," and "antigen-binding fragment" of an antibody are used interchangeably herein to refer to one or more fragments of an antibody that retain the ability to specifically bind to an antigen (see, generally, Holliger et al., Nat. Biotech., 23(9): 1126-1129 (2005)). The antibody fragment desirably comprises, for example, one or more CDRs, the variable region (or portions thereof), the constant region (or portions thereof), or combinations thereof. Examples of antibody fragments include, but are not limited to, (i) a Fab fragment, which is a monovalent fragment consisting of the V_{L}, V_{H}, C_{L}, and C_{H1} domains, (ii) a F(ab')₂ fragment, which is a bivalent fragment comprising two Fab fragments linked by a disulfide bridge at the hinge region, (iii) a Fv fragment consisting of the V_{L} and V_{H} domains of a single arm of an antibody, (iv) a Fab' fragment, which results from breaking the disulfide bridge of an F(ab')₂ fragment using mild reducing conditions, (v) a disulfide-stabilized Fv fragment (dsFv), and (vi) a domain antibody (dAb), which is an antibody single variable region domain (V_{H} or V_{L}) polypeptide that specifically binds antigen.

As used herein, a "nucleic acid" or a "nucleic acid sequence" refers to a polymer or oligomer of pyrimidine and/or purine bases, preferably cytosine, thymine, and uracil, and adenine and guanine, respectively (See Albert L. Lehninger, Principles of Biochemistry, at 793-800 (Worth Pub. 1982)). The present technology contemplates any deoxyribonucleotide, ribonucleotide, or peptide nucleic acid component, and any chemical variants thereof, such as methylated, hydroxymethylated, or glycosylated forms of these bases, and the like. The polymers or oligomers may be heterogenous or homogenous in composition and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states. A nucleic acid or nucleic acid sequence may comprise other kinds of nucleic acid structures such as, for instance, a DNA/RNA helix, peptide nucleic acid (PNA), morpholino nucleic acid (see, e.g., Braasch and Corey, Biochemistry, 41(14): 4503-4510 (2002)) and U.S. Pat. No. 5,034,506), locked nucleic acid (LNA; see Wahlestedt et al., Proc. Natl. Acad. Sci. U.S.A., 97: 5633-5638 (2000)), cyclohexenyl nucleic acids (see Wang, J. Am. Chem. Soc., 122: 8595-8602 (2000)), and/or a ribozyme. Hence, the term "nucleic acid" or "nucleic acid sequence" may also encompass a chain comprising non-natural nucleotides, modified nucleotides, and/or non- nucleotide building blocks that can exhibit the same function as natural nucleotides (e.g., "nucleotide analogs"); further, the term "nucleic acid sequence" as used herein refers to an oligonucleotide, nucleotide or polynucleotide, and fragments or portions thereof, and to DNA or RNA of genomic or synthetic origin, which may be single or double-stranded, and represent the sense or antisense strand. The terms "nucleic acid," "polynucleotide," "nucleotide sequence," and "oligonucleotide" are used interchangeably. They refer to a polymeric form of nucleotides of any length, either deoxyribonucleotides or ribonucleotides, or analogs thereof.

A "peptide" or "polypeptide" is a linked sequence of two or more amino acids linked by peptide bonds. The peptide or polypeptide can be natural, synthetic, or a modification or combination of natural and synthetic. Polypeptides include proteins such as binding proteins, receptors, and antibodies. The proteins may be modified by the addition of sugars, lipids or other moieties not included in the amino acid chain. The terms "polypeptide" and "protein," are used interchangeably herein.

As used herein, the term "percent sequence identity" refers to the percentage of nucleotides or nucleotide analogs in a nucleic acid sequence, or amino acids in an amino acid sequence, that is identical with the corresponding nucleotides or amino acids in a reference sequence after aligning the two sequences and introducing gaps, if necessary, to achieve the maximum percent identity. Hence, in case a nucleic acid according to the technology is longer than a reference sequence, additional nucleotides in the nucleic acid, that do not align with the reference sequence, are not taken into account for determining sequence identity. A number of mathematical algorithms for obtaining the optimal alignment and calculating identity between two or more sequences are known and incorporated into a number of available software programs. Examples of such programs include CLUSTAL-W, T-Coffee, and ALIGN (for alignment of nucleic acid and amino acid sequences), BLAST programs (e.g., BLAST 2.1, BL2SEQ, and later versions thereof) and FASTA programs (e.g., FASTA3x, FAS^{™}, and SSEARCH) (for sequence alignment and sequence similarity searches). Sequence alignment algorithms also are disclosed in, for example, Altschul et al., J. Molecular Biol., 215(3): 403-410 (1990), Beigert et al., Proc. Natl. Acad. Sci. USA, 106(10): 3770-3775 (2009), Durbin et al., eds., Biological Sequence Analysis: Probabilistic Models of Proteins and Nucleic Acids, Cambridge University Press, Cambridge, UK (2009), Soding, Bioinformatics, 21(7): 951-960 (2005), Altschul et al., Nucleic Acids Res., 25(17): 3389-3402 (1997), and Gusfield, Algorithms on Strings, Trees and Sequences, Cambridge University Press, Cambridge UK (1997)).

A "vector" or "expression vector" is a replicon, such as plasmid, phage, virus, or cosmid, to which another DNA segment, e.g., an "insert," may be attached or incorporated so as to bring about the replication of the attached segment in a cell.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified," "mutant," or "polymorphic" refers to a gene or gene product that displays modifications in sequence and or functional properties (e.g., altered characteristics) when compared to the wild-type gene or gene product. It is noted that naturally occurring mutants can be isolated; these are identified by the fact that they have altered characteristics when compared to the wild-type gene or gene product.

### 2. Method for identifying transcriptional modifying domains

Disclosed herein are methods for identifying transcriptional effector (e.g., activator and repressor) domains. The methods may comprise: preparing a domain library comprising a plurality of nucleic acid sequences each configured to express a fusion protein comprising a protein domain from nuclear-localized proteins linked to an inducible DNA binding domain; transforming reporter cells with the domain library, wherein the reporter cells comprises a two-part reporter gene comprising a surface marker and a fluorescent protein under the control of a promoter, wherein the two-part reporter gene is capable of being modulated by a putative transcriptional effector domain following treatment with an agent configured to induce the inducible DNA binding domain; treating the reporter cells with the agent for a length of time necessary for protein and mRNA levels to be altered in the cell (e.g., increased due to production or decreased due to degradation); sequencing the protein domains from the separated reporter cells; calculating for each protein domain sequence a ratio of sequencing counts from reporter cells not having the surface marker, the fluorescent protein, or a combination thereof to sequencing counts from reporter cells having the surface marker, the fluorescent protein, or a combination thereof; and identifying protein domains as transcriptional repressors or activators.

The methods may comprise preparing a domain library comprising a plurality of nucleic acid sequences each configured to express a fusion protein comprising a protein domain from nuclear-localized proteins linked to an inducible DNA binding domain. The protein domain may be less than or equal to 80 amino acids. The protein domain may be about 75 amino acids, about 70 amino acids, about 65 amino acids, about 60 amino acids, about 55 amino acids, about 50 amino acids, about 45 amino acids, about 40 amino acids, about 35 amino acids, about 30 amino acids, about 25 amino acids, about 20 amino acids, about 15 amino acids, about 10 amino acids, or about 5 amino acids.

The protein domain may be derived from any known protein. The protein domain may be from a nuclear-localized protein. A nuclear-localized protein includes those proteins which are or can localize to the nucleus fully or partially during the life-cycle of the protein. The protein domain may comprise amino acid sequences of the wild-type protein domain from nuclear-localized proteins. The protein domain may comprise mutated amino acid sequences of protein domains from nuclear-localized proteins.

The inducible DNA binding domain may use any system for induction of DNA binding, including, but not limited to, tetracycline Tet,/DOX inducible systems, light inducible systems, Abscisic acid (ABA) inducible systems, cumate systems, 40HT/estrogen inducible systems, ecdysone-based inducible systems, and FKBP12/FRAP (FKBP12-rapamycin complex) inducible systems.

The inducible DNA binding domain may comprise a tag. The tag may include any tag known in the art, including tags removable by chemical or enzymatic means. Suitable tags for use in the present method include chitin binding protein (CBP), maltose binding protein (MBP), Strep-tag, glutathione-S-transferase (GST), a polyhistidine (PolyHis) tag, an ALFA-tag, a V5-tag, a Myc-tag, a hemagglutinin(HA)-tag, a Spot-tag, a T7-tag, an NE-tag, a Calmodulin-tag, a polyglutamate tag, a polyarginine tag, a FLAG tag, and the like.

The methods may comprise transforming reporter cells with the domain library, wherein the reporter cell comprises a two-part reporter gene comprising a surface marker and a fluorescent protein under the control of a promoter, wherein the two-part reporter gene is capable of being modulated by a putative transcriptional effector domain following treatment with an agent configured to induce the inducible DNA binding domain.

The promoter may confer a high rate of transcription (a strong promoter) or confer a low rate of transcription (weak promoter). Many promoter libraries have been established experimentally and choice of promoter and promoter strength is dependent on cell type. When identifying transcriptional activator domains, a weak promoter may be used. When identifying transcriptional repressor domains, a strong promoter may be used.

Cell surface markers include proteins and carbohydrates which are attached to the cellular membrane. Cell surface markers are generally known in the art for a variety of cell types and can be expressed in a reporter cell of choice based on known molecular biology methods. The surface marker may be a synthetic surface marker comprising marker polypeptide attached to a transmembrane domain. For example, the marker polypeptide may include an antibody or a fragment thereof (e.g., Fc region) attached to a transmembrane domain. The marker polypeptide may be human IgG1 Fc region and the synthetic surface marker may comprise human IgG1 Fc region attached to a transmembrane domain.

Fluorescent proteins are well known in the art and include proteins adapted to fluoresce in various cellular compartments and as a result of varying wavelengths of incoming light. Examples of fluorescent proteins include phycobiliproteins, cyan fluorescent protein (CFP), green fluorescent protein (GFP), yellow fluorescent protein (YFP), enhanced orange fluorescent protein (OFP), enhanced green fluorescent protein (eGFP), modified green fluorescent protein (emGFP), enhanced yellow fluorescent protein (eYFP) and/or monomeric red fluorescent protein (mRFP) and derivatives and variants thereof.

The methods may comprise separating reporter cells based on presence or absence of the surface marker, the fluorescent protein, or a combination thereof. A number of cell separation techniques are known in the art are suitable for use with the methods disclosed herein, including, for example, immunomagnetic cell separation, fluorescent-activated cell sorting (FACS), and microfluidic cell sorting. Cell separation may comprise immunomagnetic cell separation.

The method may further comprise stopping treatment of the reporter cells with the agent and repeating the separating, sequencing, calculating, and identifying steps one or more times. The steps may be repeated at least 48 hours after stopping treatment of the reported cells with the agent.

The method may further comprise measuring expression level of protein domains. The expression level of the protein domains can be determined using any methods known in the art, including immunoblotting and immunoassays for the protein itself or any tags or labels thereof. The expression level may be determined by measuring a relative presence or absence of the tag on the DNA binding domain.

The methods may identify a transcriptional repressor domain. The methods may comprise, a) preparing a domain library comprising a plurality of nucleic acid sequences each configured to express a fusion protein comprising a protein domain linked to an inducible DNA binding domain; b) transforming reporter cells with the domain library, wherein a reporter cell comprises a two-part reporter gene comprising a surface marker and a fluorescent protein under the control of a strong promoter, wherein the two-part reporter gene is capable of being silenced by a putative transcriptional repressor domain following treatment with an agent configured to induce the inducible DNA binding domain; c) treating the reporter cells with the agent for a length of time necessary for protein and mRNA degradation in the cell; d) separating reporter cells based on presence or absence of the surface marker, the fluorescent protein, or a combination thereof; e) sequencing the protein domains from the separated reporter cells; f) calculating for each protein domain sequence a ratio of sequencing counts from reporter cells not having the surface marker, the fluorescent protein, or a combination thereof to sequencing counts from reporter cells having the surface marker, the fluorescent protein, or a combination thereof; and g) identifying protein domains as transcriptional repressor.

The reporter cells may be treated with the agent for at least 3 days. For, example the reporter cells may be treated with the agent for at least 3 days, at least 4 days, at least 5 days, at least 6 days, at least 7 days, at least 8 days, at least 9 days, at least 10 days, at least 14 days, or more. The reporter cells may be treated with the agent for 3-12 days, 3-10 days, 3-7 days, or 3-5 days.

The protein domain may be identified as a transcriptional repressor when log2 of the ratio of sequencing counts from reporter cells not having the surface marker, the fluorescent protein, or a combination thereof to sequencing counts from reporter cells having the surface marker, the fluorescent protein, or a combination thereof is at least two standard deviations from (e.g., greater than) the mean of a negative control (See FIG. 1C, for example).

The methods may identify a transcriptional activator domain. The methods may comprise, a) preparing a domain library comprising a plurality of nucleic acid sequences each configured to express a fusion protein comprising a protein domain linked to an inducible DNA binding domain; b) transforming reporter cells with the domain library, wherein the reporter cells comprises a two-part reporter gene comprising a surface marker and a fluorescent protein under the control of a weak promoter, wherein the two-part reporter gene is capable of being activated by a putative transcriptional activator domain following treatment with an agent configured to induce the inducible DNA binding domain; c) treating the reporter cells with the agent for a length of time necessary for protein and mRNA production in the cell; d) separating reporter cells based on presence or absence of the surface marker, the fluorescent protein, or a combination thereof; e) sequencing the protein domains from the separated reporter cells; f) calculating for each protein domain sequence a ratio of sequencing counts from reporter cells not having the surface marker, the fluorescent protein, or a combination thereof to sequencing counts from reporter cells having the surface marker, the fluorescent protein, or a combination thereof; and g) identifying protein domains as transcriptional repressor.

The reporter cells may be treated with the agent for at least 24 hours. For, example the reporter cells may be treated with the agent for at least 24 hours (1 day), at least 36 hours, at least 48 hours (2 days), at least 60 hours, at least 72 hours (3 days), at least 94 hours, at least 106 hours (4 days) or more. The reporter cells may be treated for between 24 and 72 hours or between 36 and 60 hours.

The protein domain may be identified as a transcriptional activator when log2 of the ratio of sequencing counts from reporter cells not having the surface marker, the fluorescent protein, or a combination thereof to sequencing counts from reporter cells having the surface marker, the fluorescent protein, or a combination thereof is at least two standard deviations from (e.g., less than) the mean of a negative control. (See FIG. 5B, for example).

### 3. Transcription Factors

The present disclosure also provides synthetic transcription factors comprising one or more transcriptional effector domains fused to a heterologous DNA binding domain. As used herein, the term "transcription factor" refers to a protein or polypeptide that interacts with, directly or indirectly, specific DNA sequences associated with a genomic locus or gene of interest to block or recruit RNA polymerase activity to the promoter site for a gene or set of genes.

The synthetic transcription factor may comprise one or more transcriptional activator domains, one or more transcriptional repressor domains, or a combination thereof fused to a heterologous DNA binding domain. The at least one of the one or more transcriptional activator domains or at least one of the one or more transcriptional repressor domains may comprise an amino acid sequence having at least 70% (e.g., at least 70%, at least 75%, at least 80%, at least 85%, at least 90%, at least 95%, at least 98%, 99%) identity to any of SEQ ID NOs: 1-896. The one or more transcriptional activator domain, the one or more transcriptional repressor domain, or combination thereof may be identified by the methods disclosed herein.

The synthetic transcription factor may comprise two or more transcription effector domains (e.g., transcriptional activator domains, transcriptional repressor domains, or a combination thereof) fused to a heterologous DNA binding domain. The synthetic transcription factor may comprise two or more transcriptional activator domains or two or more transcriptional repressors domains fused to a heterologous DNA binding domain. The two or more effector domains can be fused to the DNA binding domain in any orientation, and may be separated from each other with an amino acid linker.

When the synthetic transcription factor comprises more than one transcription effector domains, the synthetic transcription factor may comprise at least one transcriptional activator domain or at least one transcriptional repressor domain as disclosed herein with at least one additional effector domain known in the art. See for example, Tycko J. et al., Cell. 2020 Dec 23;183(7):2020-2035. In some embodiments, the one or more transcriptional activator domain, the one or more transcriptional repressor domain is identified by the methods described herein.

When the synthetic transcription factor comprises more than one transcription effector domains, at least one of the one or more transcriptional activator domains may comprise an amino acid sequence having at least 70% identity to any of SEQ ID NOs: 563-664. At least one of the one or more transcriptional activator domains may comprise an amino acid sequence having at least 70% identity to any of SEQ ID NOs: 563-596. At least one of the one or more transcriptional activator domain may be selected from those found in Table 2.

When the synthetic transcription factor comprises more than one transcription effector domains, at least one of the one or more transcriptional repressor domains may comprise an amino acid sequence having at least 70% identity to any of SEQ ID NOs: 1-562 and 665-896. At least one of the one or more transcriptional repressor domains may comprise an amino acid sequence having at least 70% identity to any of SEQ ID NO: 666. At least one of the one or more transcriptional repressor domains may be selected from those found in any of Tables 1, 3, or 4.

The DNA binding domain is any polypeptide which is capable of binding double- or single-stranded DNA, generally or with sequence specificity. DNA binding domains include those polypeptides having helix-turn-helix motifs, zinc fingers, leucine zippers, HMG-box (high mobility group box) domains, winged helix region, winged helix-turn-helix region, helix-loop-helix region, immunoglobulin fold, B3 domain, Wor3 domain, TAL effector DNA-binding domain and the like. The heterologous DNA binding domains may be a natural binding domain. The heterologous DNA binding domain may comprise a programmable DNA binding domain, e.g., a DNA binding domain engineered, for example by altering one or more amino acid of a natural DNA binding domain to bind to a predetermined nucleotide sequence.

The DNA binding domain may be capable of binding directly to the target DNA sequences.

The DNA-binding domain may be derived from domains found in naturally occurring Transcription activator-like effectors (TALEs), such as AvrBs3, Hax2, Hax3 or Hax4 (Bonas et al. 1989. Mol Gen Genet 218(1): 127-36; Kay et al. 2005 Mol Plant Microbe Interact 18(8): 838-48). TALEs have a modular DNA-binding domain consisting of repetitive sequences of residues; each repeat region consists of 34 amino acids. A pair of residues at the 12th and 13th position of each repeat region determines the nucleotide specificity and combining of the regions allows synthesis of sequence-specific TALE DNA-binding domains. The TALE DNA binding domains may be engineered using known methods to provide a DNA binding domain with chosen specificity for any target sequence. The DNA binding domain may comprise multiple (e.g., 2, 3, 4, 5, 6, 10, 20, or more) Tal effector DNA-binding motifs. In particular, any number of nucleotide-specific Tal effector motifs can be combined to form a sequence-specific DNA-binding domain to be employed in the present transcription factor.

The DNA binding domain may associate with the target DNA in concert with an exogenous factor.

The DNA binding domain may be derived from a Clustered Regularly Interspaced Short Palindromic Repeats associated (Cas) protein (e.g., catalytically dead Cas9) and associates with the target DNA through a guide RNA. The gRNA itself comprises a sequence complementary to one strand of the DNA target sequence and a scaffold sequence which binds and recruits Cas9 to the target DNA sequence. The transcription factors described herein may be useful for CRISPR interference (CRISPRi) or CRISPR activation (CRISPRa).

The guide RNA (gRNA) may be a crRNA, crRNA/tracrRNA (or single guide RNA, sgRNA). The gRNA may be a non-naturally occurring gRNA. The terms "gRNA," "guide RNA" and "guide sequence" may be used interchangeably throughout and refer to a nucleic acid comprising a sequence that determines the binding specificity of the Cas protein. A gRNA hybridizes to (complementary to, partially or completely) the DNA target sequence.

The gRNA or portion thereof that hybridizes to the target nucleic acid (a target site) may be any length necessary for selective hybridization. gRNAs or sgRNA(s) can be between about 5 and about 100 nucleotides long, or longer (e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 60, 61, 62, 63, 63, 64, 65, 66, 67, 68, 69, 70, 71, 72, 73, 74, 75, 76, 77, 78, 79, 80, 81, 82, 83, 84, 85, 86, 87, 88, 89, 90, 91 92, 93, 94, 95, 96, 97, 98, 99, or 100 nucleotides in length, or longer).

To facilitate gRNA design, many computational tools have been developed (See Prykhozhij et al. (PLoS ONE, 10(3): (2015)); Zhu et al. (PLoS ONE, 9(9) (2014)); Xiao et al. (Bioinformatics. Jan 21 (2014)); Heigwer et al. (Nat Methods, 11(2): 122-123 (2014)). Methods and tools for guide RNA design are discussed by Zhu (Frontiers in Biology, 10 (4) pp 289-296 (2015)). Additionally, there are many publicly available software tools that can be used to facilitate the design of sgRNA(s); including but not limited to, Genscript Interactive CRISPR gRNA Design Tool, WU-CRISPR, and Broad Institute GPP sgRNA Designer. There are also publicly available pre-designed gRNA sequences to target many genes and locations within the genomes of many species (human, mouse, rat, zebrafish, C. elegans), including but not limited to, IDT DNA Predesigned Alt-R CRISPR-Cas9 guide RNAs, Addgene Validated gRNA Target Sequences, and GenScript Genome-wide gRNA databases.

The present disclosure also provides nucleic acids encoding a synthetic transcription factor or a transcriptional effector (e.g., activator or repressor) domain, as disclosed herein. For example, the effector domains may be encoded by nucleic acids disclosed in Tables 1-3. The effector domains may be encoded by nucleic acids having at least 70% identity to any of SEQ ID NOs: 897-1329. The nucleic acid may encode one or more synthetic transcription factor or one or more effector domain.

Nucleic acids of the present disclosure can comprise any of a number of promoters known to the art, wherein the promoter is constitutive, regulatable or inducible, cell type specific, tissue-specific, or species specific. In addition to the sequence sufficient to direct transcription, a promoter sequence of the invention can also include sequences of other regulatory elements that are involved in modulating transcription (e.g., enhancers, Kozak sequences and introns). Many promoter/regulatory sequences useful for driving constitutive expression of a gene are available in the art and include, but are not limited to, for example, CMV (cytomegalovirus promoter), EF1a (human elongation factor 1 alpha promoter), SV40 (simian vacuolating virus 40 promoter), PGK (mammalian phosphoglycerate kinase promoter), Ubc (human ubiquitin C promoter), human beta-actin promoter, rodent beta-actin promoter, CBh (chicken beta-actin promoter), CAG (hybrid promoter contains CMV enhancer, chicken beta actin promoter, and rabbit beta-globin splice acceptor), TRE (Tetracycline response element promoter), H1 (human polymerase III RNA promoter), U6 (human U6 small nuclear promoter), and the like. Additional promoters that can be used for expression of the components of the present system, include, without limitation, cytomegalovirus (CMV) intermediate early promoter, a viral LTR such as the Rous sarcoma virus LTR, HIV-LTR, HTLV-1 LTR, Maloney murine leukemia virus (MMLV) LTR, myeoloproliferative sarcoma virus (MPSV) LTR, spleen focus-forming virus (SFFV) LTR, the simian virus 40 (SV40) early promoter, herpes simplex tk virus promoter, elongation factor 1-alpha (EF1-α) promoter with or without the EF1-α intron. Additional promoters include any constitutively active promoter. Alternatively, any regulatable promoter may be used, such that its expression can be modulated within a cell.

Moreover, inducible expression can be accomplished by placing the nucleic acid encoding such a molecule under the control of an inducible promoter/regulatory sequence. Promoters that are well known in the art can be induced in response to inducing agents such as metals, glucocorticoids, tetracycline, hormones, and the like, are also contemplated for use with the invention. Thus, it will be appreciated that the present disclosure includes the use of any promoter/regulatory sequence known in the art that is capable of driving expression of the desired protein operably linked thereto.

The present disclosure also provides for vectors containing the nucleic acids and cells containing the nucleic acids or vectors, thereof. The vectors may be used to propagate the nucleic acid in an appropriate cell and/or to allow expression from the nucleic acid (e.g., an expression vector). The person of ordinary skill in the art would be aware of the various vectors available for propagation and expression of a nucleic acid sequence.

To construct cells that express the present transcription factors, expression vectors for stable or transient expression of the present system may be constructed via conventional methods and introduced into cells. For example, nucleic acids encoding the components the disclose transcription factors, or other nucleic acids or proteins, may be cloned into a suitable expression vector, such as a plasmid or a viral vector in operable linkage to a suitable promoter. The selection of expression vectors/plasmids/viral vectors should be suitable for integration and replication in eukaryotic cells.

Vectors of the present disclosure can drive the expression of one or more sequences in mammalian cells using a mammalian expression vector. Examples of mammalian expression vectors include pCDM8 (Seed, Nature (1987) 329:840) and pMT2PC (Kaufman, et al., EMBO J. (1987) 6:187). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, et al., MOLECULAR CLONING: A LABORATORY MANUAL. 2nd eds., Cold Spring Harbor Laboratory, Cold Spring Harbor Laboratory Press, Cold Spring Harbor, N.Y., 1989.

The vectors of the present disclosure may direct the expression of the nucleic acid in a particular cell type (e.g., tissue-specific regulatory elements are used to express the nucleic acid). Such regulatory elements include promoters that may be tissue specific or cell specific. The term "tissue specific" as it applies to a promoter refers to a promoter that is capable of directing selective expression of a nucleotide sequence of interest to a specific type of tissue (e.g., seeds) in the relative absence of expression of the same nucleotide sequence of interest in a different type of tissue. The term "cell type specific" as applied to a promoter refers to a promoter that is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, e.g., immunohistochemical staining.

Additionally, the vector may contain, for example, some or all of the following: a selectable marker gene for selection of stable or transient transfectants in host cells; transcription termination and RNA processing signals; 5'-and 3'-untranslated regions; internal ribosome binding sites (IRESes), versatile multiple cloning sites; and reporter gene for assessing expression of the chimeric receptor. Suitable vectors and methods for producing vectors containing transgenes are well known and available in the art. Selectable markers include chloramphenicol resistance, tetracycline resistance, spectinomycin resistance, neomycin, streptomycin resistance, erythromycin resistance, rifampicin resistance, bleomycin resistance, thermally adapted kanamycin resistance, gentamycin resistance, hygromycin resistance, trimethoprim resistance, dihydrofolate reductase (DHFR), GPT; the URA3, HIS4, LEU2, and TRP1 genes of S. cerevisiae.

When introduced into a cell, the vectors may be maintained as an autonomously replicating sequence or extrachromosomal element or may be integrated into host DNA.

Thus, the disclosure further provides for cells comprising a synthetic transcription factor, a nucleic acid, or a vector, as disclosed herein.

Conventional viral and non-viral based gene transfer methods can be used to introduce the nucleic acids into cells, tissues, or a subject. Such methods can be used to administer the nucleic acids to cells in culture, or in a host organism. Non-viral vector delivery systems include DNA plasmids, cosmids, RNA (e.g., a transcript of a vector described herein), a nucleic acid, and a nucleic acid complexed with a delivery vehicle.

Viral vector delivery systems include DNA and RNA viruses, which have either episomal or integrated genomes after delivery to the cell. A variety of viral constructs may be used to deliver the present nucleic acids to the cells, tissues and/or a subject. Viral vectors include, for example, retroviral, lentiviral, adenoviral, adeno-associated and herpes simplex viral vectors. Nonlimiting examples of such recombinant viruses include recombinant adeno-associated virus (AAV), recombinant adenoviruses, recombinant lentiviruses, recombinant retroviruses, recombinant herpes simplex viruses, recombinant poxviruses, phages, etc. The present disclosure provides vectors capable of integration in the host genome, such as retrovirus or lentivirus. See, e.g., Ausubel et al., Current Protocols in Molecular Biology, John Wiley & Sons, New York, 1989; Kay, M. A., et al., 2001 Nat. Medic. 7(1):33-40; and Walther W. and Stein U., 2000 Drugs, 60(2): 249-71.

The nucleic acids or transcription factors may be delivered by any suitable means. The nucleic acids or proteins thereof may be delivered in vivo. The nucleic acids or proteins thereof may be delivered to isolated/cultured cells in vitro or ex vivo to provide modified cells useful for in vivo delivery to patients afflicted with a disease or condition.

Vectors according to the present disclosure can be transformed, transfected, or otherwise introduced into a wide variety of host cells. Transfection refers to the taking up of a vector by a cell whether or not any coding sequences are in fact expressed. Numerous methods of transfection are known to the ordinarily skilled artisan, for example, lipofectamine, calcium phosphate co-precipitation, electroporation, DEAE-dextran treatment, microinjection, viral infection, and other methods known in the art. Transduction refers to entry of a virus into the cell and expression (e.g., transcription and/or translation) of sequences delivered by the viral vector genome. In the case of a recombinant vector, "transduction" generally refers to entry of the recombinant viral vector into the cell and expression of a nucleic acid of interest delivered by the vector genome.

Methods of delivering vectors to cells are well known in the art and may include DNA or RNA electroporation, transfection reagents such as liposomes or nanoparticles to delivery DNA or RNA; delivery of DNA, RNA, or protein by mechanical deformation (see, e.g., Sharei et al. Proc. Natl. Acad. Sci. USA (2013) 110(6): 2082-2087); or viral transduction. The vectors may be delivered to host cells by viral transduction. Nucleic acids can be delivered as part of a larger construct, such as a plasmid or viral vector, or directly, e.g., by electroporation, lipid vesicles, viral transporters, microinjection, and biolistics (high-speed particle bombardment). Similarly, the construct containing the one or more transgenes can be delivered by any method appropriate for introducing nucleic acids into a cell. The construct or the nucleic acid encoding the components of the present system may be a DNA molecule. The nucleic acid encoding the components of the present system may be a DNA vector and may be electroporated to cells. The nucleic acid encoding the components of the present system may be an RNA molecule, which may be electroporated to cells.

Additionally, delivery vehicles such as nanoparticle- and lipid-based delivery systems can be used. Further examples of delivery vehicles include lentiviral vectors, ribonucleoprotein (RNP) complexes, lipid-based delivery system, gene gun, hydrodynamic, electroporation or nucleofection microinjection, and biolistics. Various gene delivery methods are discussed in detail by Nayerossadat et al. (Adv Biomed Res. 2012; 1: 27) and Ibraheem et al. (Int J Pharm. 2014 Jan 1;459(1-2):70-83).

As such, the disclosure provides an isolated cell comprising the vector(s) or nucleic acid(s) disclosed herein. Preferred cells are those that can be easily and reliably grown, have reasonably fast growth rates, have well characterized expression systems, and can be transformed or transfected easily and efficiently. Examples of suitable prokaryotic cells include, but are not limited to, cells from the genera *Bacillus* (such as *Bacillus subtilis* and *Bacillus brevis), Escherichia* (such as *E. coli), Pseudomonas, Streptomyces, Salmonella,* and *Envinia.* Suitable eukaryotic cells are known in the art and include, for example, yeast cells, insect cells, and mammalian cells. Examples of suitable yeast cells include those from the genera *Kluyveromyces, Pichia, Rhino-sporidium, Saccharomyces,* and *Schizosaccharomyces.* Exemplary insect cells include Sf-9 and HIS (Invitrogen, Carlsbad, Calif.) and are described in, for example, Kitts et al., Biotechniques, 14: 810-817 (1993); Lucklow, Curr. Opin. Biotechnol., 4: 564-572 (1993); and Lucklow et al., J. Virol., 67: 4566-4579 (1993). Desirably, the cell is a mammalian cell. The cell may be a human cell. A number of suitable mammalian and human host cells are known in the art, and many are available from the American Type Culture Collection (ATCC, Manassas, Va.). Examples of suitable mammalian cells include, but are not limited to, Chinese hamster ovary cells (CHO) (ATCC No. CCL61), CHO DHFR-cells (Urlaub et al., Proc. Natl. Acad. Sci. USA, 97: 4216-4220 (1980)), human embryonic kidney (HEK) 293 or 293T cells (ATCC No. CRL1573), and 3T3 cells (ATCC No. CCL92). Other suitable mammalian cell lines are the monkey COS-1 (ATCC No. CRL1650) and COS-7 cell lines (ATCC No. CRL1651), as well as the CV-1 cell line (ATCC No. CCL70). Further exemplary mammalian host cells include primate, rodent, and human cell lines, including transformed cell lines. Normal diploid cells, cell strains derived from *in vitro* culture of primary tissue, as well as primary explants, are also suitable. Other suitable mammalian cell lines include, but are not limited to, mouse neuroblastoma N2A cells, HeLa, HEK, A549, HepG2, mouse L-929 cells, and BHK or HaK hamster cell lines.

Methods for selecting suitable mammalian cells and methods for transformation, culture, amplification, screening, and purification of cells are known in the art.

The present description is also directed to compositions or systems comprising a synthetic transcription factor, a nucleic acid, a vector, or a cell, as described herein. The compositions or system may comprise two or more synthetic transcription factors, nucleic acids, vectors, or cells.

The composition or system may further comprise a gRNA. The gRNA may be encoded on the same nucleic acid as a synthetic transcription factor or a different nucleic acid. The vector encoding a synthetic transcription factor may further encode a gRNA, under the same or different promoter. The gRNA may be encoded on its own vector, separated from that of the transcription factor.

### 4. Methods of Modulating Gene Expression

The present disclosure also provides methods of modulating the expression of at least one target gene in a cell, the method comprising introducing into the cell at least one synthetic transcription factor, nucleic acid, vector, or composition or system as described herein. The gene expression of at least two genes may be modulated.

Modulation of expression comprises increasing or decreasing gene expression compared to normal gene expression for the target gene. When the gene expression of at least two genes is modulation, both genes may have increased gene expression, both gene may have decreased gene expression, or one gene may have increased gene expression and the other may have decreased gene expression.

The cell may be a prokaryotic or eukaryotic cell. The cell may be a eukaryotic cell. The cell may be in vitro. The cell may be ex vivo.

The cell may be in an organism or host, such that introducing the disclosed systems, compositions, vectors into the cell comprises administration to a subject. The method may comprise providing or administering to the subject, in vivo, or by transplantation of ex vivo treated cells, at least one synthetic transcription factor, nucleic acid, vector, or composition or system as described herein.

A "subject" may be human or non-human and may include, for example, animal strains or species used as "model systems" for research purposes, such a mouse model as described herein. Likewise, subject may include either adults or juveniles (e.g., children). Moreover, subject may mean any living organism, preferably a mammal (e.g., human or non-human) that may benefit from the administration of compositions contemplated herein. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish, and the like. The mammal may be a human.

As used herein, the terms "providing", "administering," "introducing," are used interchangeably herein and refer to the placement of the systems of the disclosure into a subject by a method or route which results in at least partial localization of the system to a desired site. The systems can be administered by any appropriate route which results in delivery to a desired location in the subject.

### 5. Kits

Also disclosed herein are kits including at least one or all of at least one nucleic acid encoding an effector domain, or a DNA binding domain, or a combination thereof, at least one synthetic transcription factor, or nucleic acid encoding thereof, vectors encoding at least one effector domain or at least one synthetic transcription factor, a composition or system as described herein, a cell comprising an effector domain, a DNA binding domain, a synthetic transcription factor, or a nucleic acid encoding any of thereof, a reporter cell as described herein and a two-part reporter gene as described herein or a nucleic acid encoding thereof.

The kits can also comprise instructions for using the components of the kit. The instructions are relevant materials or methodologies pertaining to the kit. The materials may include any combination of the following: background information, list of components, brief or detailed protocols for using the compositions, trouble-shooting, references, technical support, and any other related documents. Instructions can be supplied with the kit or as a separate member component, either as a paper form or an electronic form which may be supplied on computer readable memory device or downloaded from an internet website, or as recorded presentation.

It is understood that the disclosed kits can be employed in connection with the disclosed methods. The kit may include instructions for use in any of the methods described herein. The instructions can comprise a description of use of the components for the methods of identifying repressor domains or methods of modulating gene expression.

The kits provided herein are in suitable packaging. Suitable packaging includes, but is not limited to, vials, bottles, jars, flexible packaging, and the like.

Kits optionally may provide additional components such as buffers and interpretive information. Normally, the kit comprises a container and a label or package insert(s) on or associated with the container. The disclosure may provide articles of manufacture comprising contents of the kits described above.

The kit may further comprise a device for holding or administering the present system or composition. The device may include an infusion device, an intravenous solution bag, a hypodermic needle, a vial, and/or a syringe.

The present disclosure also provides for kits for performing the methods or producing the components in vitro. The kit may include the components of the present system. Optional components of the kit include one or more of the following: (1) buffer constituents, (2) control plasmid, (3) sequencing primers.

### 6. Examples

Human gene expression is regulated by thousands of proteins that activate or repress transcription. We lack a complete and quantitative description of these proteins' effector domains, the domains sufficient to mediate changes in gene expression. To systematically measure transcriptional effector domains in human cells, provided herein is a high-throughput assay in which libraries of protein domains are fused to a DNA-binding domain and recruited to a reporter gene. The cells are then separated by reporter expression level and the library of protein domains is sequenced. The reporter is a synthetic surface marker that facilitates simple separation of tens of millions of cells into high- and low-expression populations, using magnetic beads.

Gene silencing and epigenetic memory was quantified after recruitment of all nuclear protein domains of ≤80 amino acids. Using the measurements for the complete families of >300 KRAB domains and >200 Homeodomains, relationships were discovered between transcription factor's repressor domain strength and their evolutionary history and developmental role. Further, a deep mutational scan of the ZNF10 KRAB effector function and identified substitutions with enhanced stability and repression compared to the KRAB domain used in CRISPRi. To search for effector domains beyond previously annotated regions, the sequence of 238 repressor complex proteins was tiled and novel repressor domains as short as 10 amino acids were discovered in unannotated regions of large chromatin regulators, including the non-canonical polycomb 1.6 recruitment protein MGA. Greater than 20 repressors were individually characterized and all of them were found to silence a reporter gene in an all-or-nothing fashion at the single-cell level, but with distinct dynamics of silencing and epigenetic memory.

In addition, new activator domains in nuclear proteins were discovered, including a highly divergent acidic KRAB domain variant.

Together, these results demonstrate a strategy for systematic measurement of transcriptional effector domain activity in human cells, and expand the number of compact transcriptional effector domains that can be applied in synthetic transcription and epigenetic perturbation technologies.

Problems addressed by the present technology:
i. Unknown which genes have effector function
ii. Within known TF/CR genes, often unknown which domains have this function
iii. Within domain families including known effector domains, unknown which family members have this function
iv. Within known effector domains, unknown which residues are necessary, and how mutations reduce or enhance function

The systems and methods provided herein can measure regulatory domains of activators and repressors capability to change the output from a reporter promoter. Historically, this requires low throughput work so relatively few effector domains have been measured. The systems and methods provided herein off an alternative high-throughput assay.

The systems and methods find use, for example, for: a. understanding gene regulation, predicting function of non-coding regulatory elements that these proteins bind to; and b. identifying effector domains for epigenome perturbation tools.

Previously, a limited number of transcriptional effector domains were available for the engineering of synthetic transcription factors. To address this limitation, provided herein is a high-throughput approach to screening and quantifying the function of transcriptional effectors domains. This approach enabled the discovery of hundreds of effector domains that can upregulate or downregulate transcription in a targeted manner when fused onto a DNA binding domain. This process also identifies mutants of effector domains with enhanced activity. These effector domains can be used to engineer synthetic transcription factors for applications in gene and cell therapy, synthetic biology, and functional genomics.

The new transcriptional effector domains provided herein have several advantages for applications that rely on synthetic transcription factors. We identify short domains (≤80 amino acids) and a high-throughput process for shortening them further to the minimally sufficient sequence, which is an advantage for delivery (e.g., packaging in viral vectors). **In** some cases, we identify potent effector domains that are as short as 10 amino acids. The domains are extracted from human proteins, which provides the advantage of reducing immunogenicity in comparison to viral effector domains. Most of these domains have not been reported as transcriptional effectors previously.

By performing high-throughput recruitment with the Pfam domain library against both a strong pEF promoter and a weak minCMV promoter, both repressor and activator domains were able to be measured. One possible reason that many more repressors were found is that they are more often autonomous stably-folding sequences which meet the Pfam definition of a domain while TADs are more often disordered or low-complexity regions that are not annotated as domains. Another possible reason could be that co-activators are more limiting in the nucleus than co-repressors (Gillespie Mol Cell 2020), which implies lower expression of activator domains could result in greater activation strength, but this effect would not be expected to completely mask signal in the screen. New library designs that tile transcription factors or focus on regions with TAD-like signatures (e.g., acidity) will uncover additional activator domains.

**In** addition, a high-throughput process for testing mutations in these domains in order to identify enhanced variants is disclosed herein. The high-throughput approach is more readily enabled by development of an artificial cell surface marker that provides more efficient, inexpensive, and rapid screening of these libraries using magnetic separation. This is an advantage over the more conventional approach of sorting libraries based on fluorescent reporter gene expression.

### Example 1

### HT-recruit identifies hundreds of repressor domains in human proteins

In order to turn the classical recruitment reporter assay into a high-throughput assay of transcriptional domains, two problems were solved: (1) modification of the reporter to make it compatible with rapid screening of libraries of tens of thousands of domains, and (2) development of a strategy to generate a library of candidate effector domains. To improve on the previously published fluorescent reporter (Bintu et al., 2016), a synthetic surface marker was engineered to enable facile magnetic separation of large numbers of cells and the reporter was integrated in a suspension cell line amenable to cell culture in large volume spinner flasks. Specifically, K562 reporter cells with 9xTetO binding sites upstream of a strong constitutive pEF1a promoter that drives expression of a two-part reporter consisting of a synthetic surface marker (the human IgG1 Fc region linked to an Igκ leader and PDGFRβ transmembrane domain) and a fluorescent citrine protein (FIG. 1) were generated. Flow cytometry confirmed that recruitment of a known repressor domain, the KRAB domain from the zinc finger transcription factor ZNF10, at the TetO sites silenced this reporter in a doxycycline-dependent manner within 5 days (FIGS. 7and 16A and 16B). Magnetic separation with ProG Dynabeads that bind to the synthetic surface marker separated cells with the reporter ON from OFF cells (FIGS. 7 and 16C).

Sequences were pulled from the UniProt database for Pfam-annotated domains in human proteins that can localize to the nucleus (including non-exclusively nuclear-localized proteins). In total, 14,657 domains were retrieved. Of these, 72% were less than or equal to 80 amino acids (AA) long (FIG. 1), which made them compatible with pooled synthesis as 300 base oligonucleotides. For domains shorter than 80 AA, the domain sequence was extended on both ends with the adjacent residues from the native protein sequence in order to reach a length of 80 AA and avoid PCR amplification biases. 861 negative controls that were either random 80 AA sequences or 80 AA sequences tiled along the DMD protein with a 10 AA tiling window were added. The DMD protein was not localized in the nucleus (Chevron et al., 1994), and thus unlikely to feature domains with transcriptional activity. The library was cloned for lentiviral expression as a fusion protein with either the rTetR doxycycline-inducible DNA-binding domain alone, or with a 3X-FLAG-tagged rTetR (FIGS. 17A and 8) and delivered to K562 reporter cells (FIG. 1).

Before assaying for transcriptional activity, it was determined which protein domains were well-expressed in K562 cells using a high-throughput approach (FIGS. 17A and 8). The library of cells was stained with an anti-FLAG fluorescent-labeled antibody, sorted the cells into two bins (FIGS. 17B and 8), genomic DNA was extracted, and the frequency of each domain by amplicon sequencing was counted. The sequencing counts were used to compute the enrichment ratio in the FLAGhigh versus FLAGlow population for each domain, as a measure of expression level. These measurements were reproducible between separately transduced biological replicates (r² = 0.82, FIGS. 17C and 8), and highly correlated with individual domain fusion expression levels measured by Western blot (r²=0.92, FIGS. 17D and 17E and 8). Native Pfam domains were significantly better-expressed than the random sequence controls (p<1e-5, Mann Whitney test), while the Pfam domains and the DMD tiling controls were similarly well-expressed (FIGS. 17F and 8). A threshold was set to identify well-expressed domains with a FLAGhigh:FLAGlow ratio one standard deviation above the median of the random controls. By this definition, 66% of the Pfam domains were well-expressed; these domains were the focus of further analysis.

The Pfam domain library was screened for transcriptional repressors. The pooled library of cells was treated with doxycycline for 5 days, which gave sufficient time after transcriptional silencing for the reporter mRNA and protein to degrade and dilute out due to cell division, resulting in a clear bimodal mixture of 'ON' and 'OFF' cells (FIGS. 18A and 9). Then, magnetic cell separation (FIGS. 18A and 9) and domain sequencing were performed, then the log2(OFF:ON) ratio was computed for each library member using the read counts in the unbound and bead-bound populations (FIG. 1). For clarity, the bead-bound population was referred to as 'ON' and the unbound population as 'OFF'. The measurements were highly reproducible between separately transduced biological replicates (r² = 0.96, FIG. 1). Domains were called as hits when they caused repression that was more than 2 standard deviations above the mean of the poorly expressed negative controls. This resulted in 446 repressor hits at day 5, with domains from 63 domain families (FIG. 12A). These repressor domains are found in 451 human proteins, because in some cases the exact same domain sequence occurs in multiple genes. Known repressor domains (e.g., KRAB from human ZNF10, Chromoshadow from CBX5) from 10 domain families described as repressors or co-repressor-binding domains by Pfam were among the hits. To measure epigenetic memory, additional time points were taken at days 9 and 13. The set of proteins containing hits was significantly enriched for transcription factors and chromatin regulators when compared to all nuclear proteins used in the library, but different categories of proteins were differentially enriched when classified by their memory levels (FIGS. 18B and 9). Specifically, the repressors with high memory (cells remaining OFF) at day 13 were most enriched for C2H2 zinc finger transcription factors which include KRAB ZNF proteins, and the repressors with low memory were most enriched for homeodomain transcription factors which include the Hox proteins. Overall, the very high reproducibility and identification of expected positive control repressor domains among the hits suggested the screening method, called HT-recruit, yielded reliable results. Amino acid and nucleic acid sequences for repressors identified in the nuclear Pfam domain library are shown in Table 1, with higher scores indicating increased repression.

One of the strongest hits was the YAF2_RYBP, a domain present in the RING1- and YY1-binding protein (RYBP) and its paralog YY1-associated Factor 2 (YAF2), which are both components of the polycomb repressive complex 1 (PRC1) (Chittock et al., 2017; Garcia et al., 1999). The domain from the RYBP protein as annotated by Pfam (which is just 32 amino acids, thus shorter than the version synthesized in the 80 AA domain library) was individually tested and rapid silencing of the reporter gene was confirmed (FIG. 12B). RYBP-mediated silencing was also demonstrated in a recent report of full-length RYBP protein recruitment in mouse embryonic stem cells (Moussa et al., 2019; Zhao et al., 2020). The result established that the 32 AA RYBP domain, which has been shown by surface plasmon resonance to be the minimal required domain to bind the polycomb histone modifier enzyme RING1B (Wang et al., 2010), was sufficient to mediate silencing in cells.

To quantify repression kinetics, the citrine level distributions were gated to calculate a percentage of silenced cells with normalization of the uniform low level of background silencing in the untreated cells, and then the data was fit to a model with an exponential silencing rate during doxycycline treatment and an exponential decay (or reactivation) after doxycycline removal that plateaus at a constant irreversibly silent percentage of cells (FIG. 12C). Using this approach, the repressor function of SUMO3, the Chromo domain from MPP8, the Chromoshadow domain from CBX1, and the SAM_1/SPM domain from SCMH1 (FIGS. 18C - 18F and 9), which all had previous support for repressor function from recruitment or co-repressor binding assays, were also validated (Chang et al., 2011; Chupreta et al., 2005; Frey et al., 2016; Lechner et al., 2000). Silencing rates from all individual measurements (for the repressor hits above and the other hits discussed below, FIGS. 18C - 18K and 9) correlated well with the high-throughput measurements of silencing at day 5 (R² = 0.86, FIG. 12D). These individual validations were performed using a new variant of the DNA binding domain rTetR (SE-G72P) that was engineered to mitigate leakiness in the absence of doxycycline in yeast (Roney et al., 2016), and which was found to not leak in human cells (FIGS. 19A and 19B), making it a useful tool for mammalian synthetic biology. This new rTetR variant has the same silencing strength at maximum doxycycline recruitment as the original rTetR (FIG. 19C), which was also evidenced by the high correlation between individual validations and screen scores (FIG. 12D). Together, these validation experiments demonstrated that HT-recruit both successfully identified bona fide repressors and quantified the repression strength for each domain with accuracy comparable to individual flow cytometry experiments.

### Example 2

### Identification of domains of unknown function that repress transcription

Over 22% of the Pfam domain families are labeled as Domains of Unknown Function (DUFs), while others are not named using this label but are nevertheless DUFs (El-Gebali et al., 2019). These domains have recognizable sequence conservation but lack experimental characterization. As such, the high-throughput domain screen described herein offered the opportunity to associate initial functions with DUFs. First, DUF3669 domains were identified as repressor hits and individually validated by flow cytometry (FIGS. 12A-12C). These DUFs are natively found in KRAB zinc finger proteins, which is a gene family containing many repressive transcription factors. Concordant results demonstrating transcriptional repression after recruitment of two DUF3669 family domains were recently published (Al Chiblak et al., 2019), and the high-throughput results expand this finding to include the four remaining untested DUF3669 sequences. The HNF3 C-terminal domain, HNF_C, is another DUF, although it has a more specific name because it is only found in Hepatocyte Nuclear Factors 3 alpha and beta (also known as FOXA1 and 2). The HNF_C domains from both FOXA1 and 2 were also found as repressor hits. They both include a EH1 (engrailed homology 1) motif, characterized by the FxIxxIL sequence, that has been nominated as a candidate repressor motif (Copley, 2005).

All three of the IRF-2BP1_2 N-terminal zinc finger domains (Childs and Goodbourn, 2003), an uncharacterized domain found in the interferon regulatory factor 2 (IRF2) co-repressors IRF2BP1, IRF2BP2, and IRF2BPL, were repressor hits. The Cyt-b5 domain in the DNA repair factor HERC2 E3 ligase (Mifsud and Bateman, 2002) was another functionally uncharacterized domain that was validated as a strong repressor hit (FIGS. 18G and 9). The SH3_9 domain in BIN1 is a largely uncharacterized variant of the SH3 protein-binding domain, which was also validated as a repressor (FIGS. 18H and 9). BIN1 is a Myc-interacting protein and tumor suppressor (Elliott et al., 1999) that is also associated with Alzheimer's disease risk (Nott et al., 2019). Concordant with the results, both full-length BIN1 and a Myc-binding domain deletion mutant were previously shown to repress transcription in a Gal4 recruitment assay in HeLa cells (Elliott et al., 1999), and the BIN1 yeast homolog hob1 has been linked to transcriptional repression and histone methylation (Ramalingam and Prendergast, 2007). In addition, the repressor activity of the HMG_box domain from the transcription factor TOX and of the zf-C3HC4_2 RING finger domain from the polycomb component PCGF2 were validated (FIGS. 18I and 18J). Lastly, DUF1087 was found in CHD chromatin remodelers and, although its high-throughput measurement was just below the screen significance threshold (FIG. 12A), the CHD3 DUF1087 was validated as a weak repressor by individual flow cytometry (FIGS. 12B and 12C). Together, these results demonstrated that high-throughput protein domain screens can assign initial functions to DUFs and expand understanding of the functions of incompletely characterized domains.

### Example 3

### A random sequence with strong repressor activity

Random sequences have not previously been tested for repressor activity. Surprisingly, one of the random 80 AA sequences, which were designed as negative controls, was a strong repressor hit with an average log2(OFF:ON) = 4.0, despite having a weak expression level below the threshold. Individual validation by flow cytometry confirmed that this sequence fully silenced the population of reporter cells after 5 days of recruitment with moderate epigenetic memory up to two weeks after doxycycline removal (FIGS. 18K and 9). One additional random sequence showed a repression score marginally above the hit threshold.

### Example 4

### Repressor KRAB domains are found in younger proteins

The data provided an opportunity to analyze the function of all effector domains in the largest family of transcription factors: the KRAB domains. The KRAB gene family includes some of the strongest known repressor domains (such as the KRAB in ZNF10). Previous studies of a subset of repressive KRAB domains revealed that they can repress transcription by interacting with the co-repressor KAP1, which in turn interacts with chromatin regulators such as SETDB1 and HP1 (Cheng et al., 2014). However, it remains unclear how many of the KRAB domains are repressors, and whether the recruitment of KAP1 is necessary or sufficient for repression across all KRABs.

The library included 335 human KRAB domains, and 92.1% were found as repressor hits after filtering for domains that were well-expressed. 9 repressor hit and 2 non-hit KRAB domains were individually validated by flow cytometry and these categorizations were confirmed in every case (FIG. 19D). Then, the domain recruitment results were compared with previously published immunoprecipitation mass spectrometry data generated from full-length KRAB protein pulldowns (Helleboid et al., 2019) and all but one of the non-repressive KRABs were in proteins that do not interact with KAP1 (the one exceptional KRAB was lowly expressed), and all of the repressor hit KRAB domains were KAP1 interactors (p<1e-9, Fisher's exact test, FIG. 2). Furthermore, available ChiP-seq and ChIP-exo datasets was analyzed (ENCODE Project Consortium et al., 2020; Imbeault et al., 2017; Najafabadi et al., 2015; Schmitges et al., 2016) and repressive KRAB domains were from KRAB Zinc Finger proteins that co-localize with KAP1, in contrast to non-repressive KRAB domains (FIG. 2).

Interestingly, repressive KRAB domains were mostly found in proteins with the simplest domain architecture consisting of just a KRAB domain and a zinc-finger array, while the non-repressive KRAB domains were mostly found in genes that also include a DUF3669 or SCAN domain (FIG. 2). In fact, only one KRAB in a DUF3669-containing gene, ZNF783, was a repressor. ZNF783 is an uncharacterized DUF3669-KRAB-containing gene that uniquely lacks a zinc finger array (despite its name), suggesting it is distinctive among this class of transcription factors in both its effector function and its mode of localizing to targets.

The compound domain architecture that included a SCAN or DUF3669 is more common in evolutionary old KRAB genes (Imbeault et al., 2017). Here, a clear relationship was observed between the evolutionary age of the KRAB genes and the KRAB repressor strength, with KRAB domains from genes pre-dating the marsupial-human common ancestor having no repressor activity, and KRAB domains from genes that evolved later consistently functioning as strong repressors (FIG. 2). Together, these results support a model of an ancient generation of non-repressor KRAB genes followed by a more recent massive expansion of repressor KRAB genes that recruit KAP1 to silence genomic targets.

### Example 5

### Deep mutational scan of the CRISPRi ZNF10 KRAB effector identifies mutations that modulate gene silencing

The KRAB domain from ZNF10 has been extensively used in synthetic biology applications for gene repression and is fused to dCas9 in the programmable epigenetic and transcriptional control tool known as CRISPR interference (Gilbert et al., 2014). To better understand its sequence-function relationships, a deep mutational scan (DMS) of this KRAB was performed domain using HT-recruit. A library with all possible single substitutions and all consecutive double and triple substitutions was designed (FIG. 3). To improve the ability to unambiguously align sequencing reads, variable codon usage was used to implement silent barcodes in the domain coding sequence such that the DNA sequences were more unique than the amino acid sequences (FIG. 3). HT-recruit was performed using the reporter and workflow in FIG. 1: 5 days of doxycycline induction and magnetic separation of ON and OFF cells at days 5, 9, and 13 (FIGS. 20A and 10). These measurements were highly reproducible and showed a general trend of increasing deleteriousness with increasing mutation length from singles to triples, as expected (FIGS. 20B and 10). Further, these results were compared with the KRAB amino acid conservation and a striking correlation was found between conservation and deleteriousness of mutations (FIG. 3). Amino acid and nucleic acid sequences for KRAB repressor mutants identified are shown in Table 3. Each repressor mutant score is shown relative to 0 for the wild-type sequence, with higher scores representing more enhanced KRAB transcriptional repression.

The ZNF10 KRAB effector has 3 components: the A-box which is necessary for binding KAP1 (Peng et al., 2009), the B-box which is thought to potentiate KAP1 binding (Peng et al., 2007), and an N-terminal extension that is natively found on a separate exon upstream of the KRAB domain (FIG. 3). Mutations at numerous positions in the A-box dramatically lowered repressor activity relative to the wildtype sequence (FIG. 3). Several of these mutations had previously been tested with a recruitment CAT assay in COS and 3T3 cells; those data correlated well with measurements from the deep mutational scan in K562 cells (FIG. 3). The complete lack of silencing function in an A-box KRAB mutant was also individually validated (FIG. 3). The mutational impacts across the Abox appeared to be periodic, suggesting the angle of these residues along an alpha helix could be functionally relevant (FIG. 3). These residues were designated as necessary for silencing (p<1e-5, Wilcoxon rank sum test comparing distribution of all substitutions against wild-type at day 5) and 12 necessary residues with strong mutational impacts in the A-box and one residue with significant but weak effects in the B-box were found (FIG. 3).

These substitutions were mapped onto an aligned mouse KRAB A-box structure (PDB: 1v65, 55% identity, 69% similarity in A-box [V13-Y54], FIGS. 20C and 10) and the necessary residues were found to be similarly oriented in 3D space, suggestive of a binding interface (FIGS. 3 and 20D, red, and 10). These residues may be important for KAP1 binding as 10 out of 12 of these A-box residues were in fact shown to facilitate KAP1 binding in a previous recombinant protein binding assay (Peng et al., 2009) using KRAB-O, which aligns to ZNF10 KRAB 12-71 (50% identity, 75% similarity) in a region containing all 12 of the necessary residues (red KRAB-O residues, FIGS. 20C and 10). The remaining 8 out of 8 residues previously found unnecessary for binding were also not necessary for repression in the DMS (p<1e-4, Fisher's exact test, grey KRAB-O residues, FIGS. 20C and 10). The DMS day 5 silencing scores were inspected for the individual single, double, and triple alanine substitutions used in the binding assay, and perfect agreement was found: mutations that ablated binding also abolished silencing (Z-score<-4 compared to wild-type distribution), and mutations that did not affect binding also did not affect silencing (|Z-score|<0.6) (p<0.01, n = 12 mutations, Fisher's exact test). This high validation rate, and their positioning in the 3D structure, suggests the remaining 2 out of 12 necessary A-box residues from the DMS (V41 and N45) may also be involved in KAP1 binding.

In contrast to the A-box, B-box mutations showed relatively little effect at the end of recruitment (day 5), with only one statistically significant position (P59) showing consistent but weak effects. Meanwhile P59 and 4 other positions (K58, I62, L65, E66) showed a significant effect on memory after doxycycline removal as measured at day 9 (FIG. 3). Individual validations were performed for 4 significant positions and, as in the high-throughput experiment, the B-box mutants were strong gene silencers after day 5 of recruitment but showed reduced memory after doxycycline release (FIGS. 3 and 20E and 10). To interpret this result, the previously proposed gene silencing model in which silenced cells pass through a 'reversibly silent' state before entering an 'irreversibly silent' state was considered (Bintu et al., 2016). The B-box mutant memory reduction may be the result of a moderate silencing speed reduction, resulting in fewer cells committing to the irreversibly silent state by day 5, and that the mutational impact on silencing speed was masked because reversibly silent and irreversibly silent cells are indistinguishable at day 5. To test this possibility, the silencing time course was repeated with a 100-fold lower dose of doxycycline in order to tune down the recruitment strength. In this regime, the B-box mutations reduced silencing speed before day 5 (FIGS. 20E and 10). This result shows the B-box has a partial contribution to KRAB silencing speed.

Lastly, the KRAB N-terminus contained residues where many substitutions consistently enhanced silencing relative to wild-type (FIGS. 3, blue, day 13 panels). In particular, nearly all substitutions for the tryptophan at position 8 led to higher numbers of cells silenced relative to wild-type at day 13 (which is the time point with the most dynamic range to detect silencing levels above wild-type). This was the only significant position for enhanced silencing (FIG. 3). The memory enhancement for two of the highest-ranked of these mutants (WSR8EEE and AW7EE) was individually validated with high-doxycycline recruitment (FIGS. 3 and 20E and 10).

This silencing enhancement may have been a result of enhanced KRAB protein expression level. To investigate the relationship between protein expression level and KRAB silencing strength, the high-throughput FLAG-tag expression level measurements for the set of KAP1-binding KRAB domains was inspected and a significant correlation was found between KRAB expression level and silencing at day 13 (r² = 0.49, FIGS. 20F and 10). Most relevant to the deep mutational scan results, ZNF10 KRAB had lower expression levels compared to other KRAB domains that showed higher day 13 silencing levels, implying that it could be improved via mutations. Notably, the N-terminus was very poorly conserved (FIG. 3) and was in fact uniquely found in the KRAB from ZNF10 by BLAST, suggesting that stability-improving mutations in the N-terminus would be unlikely to interfere with KRAB function. In addition, across the entire domain expression higher tryptophan (W) frequency was observed in a domain that was negatively correlated with expression level while higher glutamic acid (E) frequency was positively correlated with expression level (FIGS. 20G and 10). This amino acid composition trend further suggested that the N-terminal KRAB mutant enhancements could be due to improved expression level, as substituting out the tryptophan from KRAB position 8 enhanced its effector function and that this enhancement was most pronounced when substituting with glutamic acid. A Western blot for the ZNF10 KRAB variants confirmed that the N-terminal glutamic acid substitution mutants were more highly expressed than the wild-type (FIGS. 20H and 10). Together, these results demonstrated the use of a deep mutational scan both to map sequence-to-function for a human transcriptional repressor and to improve effectors by incorporating expression-enhancing substitutions into poorly conserved positions.

### Example 6

### Homeodomain repressor strength is colinear with Hox gene organization

The second largest domain family that included repressor hits in the screen was the homeodomain family. Homeodomains are composed of 3 helices and are sequence-specific DNA binding domains that make base contacts through Helix 3 (Lynch et al., 2006). In some cases, they are also known to act as repressors (Holland et al., 2007; Schnabel and Abate-Shen, 1996). The library included the homeodomains from 216 human genes, and 26% were repressor hits. The repressors were found in 4 out of the 11 subclasses of homeodomains: PRD, NKL, HOXL, and LIM (FIG. 13A). These recruitment assay results suggested that transcriptional repression could be a widespread, though not ubiquitous, function of homeodomain transcription factors.

Next, the HOXL subclass results were inspected more closely. This subclass contained the Hox genes, a subset of 39 homeodomain transcription factors that are master regulators of cell fate and specify regions of the body plan along the anterior-posterior axis during embryogenesis. These genes are found in four Hox paralog clusters (A to D) arranged co-linearly from 3' to 5' corresponding to the temporal order and spatial patterning of their expression along the anterior-posterior axis (Gilbert, 1971). Interestingly, the repressor strength of their homeodomains was also collinear with their arrangement in the Hox clusters, such that the more 5' gene homeodomains were stronger repressors (Spearman's ρ = 0. 82, FIG. 13B). This correlation suggested a possible link between homeodomain repressor function and Hox gene expression timing and anterior-posterior axis spatial patterning.

Multiple sequence alignment of the Hox homeodomains revealed an RKKR (SEQ ID NO: 1330) motif present in the N-terminal arm of the 11 strongest repressor domains (FIG. 13C). The motif resided in a basic context in the strongest repressors, while the lower ranked domains lack the motif but still contained some basic residues in the disordered N-terminal arm, resulting in a significant correlation between repression strength and the number of positively charged amino acids arginine and lysine (R² = 0.85, FIGS. 13C - 13E).

Outside the Hox homeodomains, 99.5% of the repressor hits in the Pfam nuclear protein domain library did not contain the RKKR (SEQ ID NO: 1330) motif, while many non-hits did. Also, there was no correlation between net domain charge and repression strength at day 5 when considering the full library of domains (R² = 0.04). Together, these results suggested the RKKR (SEQ ID NO: 1330) motif and charge contributed to Hox homeodomain repression in the recruitment assay, but they were not sufficient for repression when found in the context of other domains.

### Example 7

### Discovery of transcriptional activators by HT-recruit to a minimal promoter

It was established that a reporter K562 line with a weak minimal CMV (min CMV) promoter that could be activated upon recruitment of fusions between rTetR and activation domains (FIG. 14A). To perform the activator screen, lentivirus was used to deliver the nuclear Pfam domain library to these reporter cells, rTetR-mediated recruitment was induced with doxycycline for 48 hours, the cells (FIG. 21A) were magnetically separated and the domains in the two resulting cell populations were sequenced. An enrichment ratio from the sequencing counts in the bead-bound (ON) and unbound (OFF) populations for each domain as a measure of transcriptional activation strength was computed and hits were two standard deviations past the mean of the poorly expressed negative controls (FIG. 14B). The hits included three previously known transcriptional activation domain families that were present in the library: FOXO-TAD from FOXO1/3/6, LMSTEN from Myb/Myb-A, and TORC_C from CRTC1/2/3. Activation strength measurements for the hits were highly reproducible between separately transduced biological replicates (r² = 0.89, FIG. 14B). This second screen with the short nuclear domain library established that HT-recruit can be used to measure either activation or repression by changing the reporter's promoter. Amino acid and nucleic acid sequences for activators identified in the nuclear Pfam domain library are shown in Table 2, with lower scores indicated stronger activators.

In total, 48 hits from 26 domain families were found. Beyond the three known activator domain families above, the remaining families with an activator hit were not previously annotated on Pfam as activator domains (FIG. 14C). Overall, fewer activators were found than repressors, which may simply be because activators are often disordered or low-complexity regions (Liu et al., 2006) that are frequently not annotated as Pfam domains. However, the proteins containing activator domains were significantly enriched for gene ontology terms such as 'positive regulation of transcription' and the strongest enrichment was for 'signaling', which reflects that many of their source proteins are activating factors (FIG. 21B). Further, the hits were significantly more acidic than non-hits (p ≤ 1e-5, Mann Whitney test, FIG. 14D), a common property in activation domains (Mitchell and Tjian, 1989; Staller et al., 2018).

Several hits were not sourced from sequence-specific transcription factors where classical activator domains are expected but were instead nonclassical activators from co-activator and transcriptional machinery proteins including Med9, TFIIEβ, and NCOA3. In particular, the Med9 domain, whose ortholog directly binds other mediator complex components in yeast (Takahashi et al., 2009), was a strong activator with an average log2(OFF:ON) = -5.5, despite its weak expression level. Nonclassical activators have previously been reported to work individually in yeast (Gaudreau et al., 1999) but only weakly when individually recruited in mammalian cells (Nevado et al., 1999). One exception is TATA-binding protein (Dorris and Struhl, 2000). By screening more nonclassical sequences, more exceptions to this notion were found.

For all tested domains, doxycycline-dependent activation of the reporter gene was confirmed using both the extended 80 AA sequence from the library and the trimmed Pfam-annotated domain (FIG. 21C). The previously annotated FOXO-TAD and LMSTEN were strong activators, in both their extended and trimmed versions. The activator function of DUF3446 from the transcription factor EGR3 and the largely uncharacterized QLQ domain from the SWI/SNF family SMARCA2 protein was also confirmed. Further, it was confirmed that the Dpy-30 motif domain, a DUF found in the Dpy-30 protein, was a weak activator. Dpy-30 was a core subunit of histone methyltransferase complexes that write H3K4me3 (Hyun et al., 2017), a chromatin mark associated with transcriptionally active chromatin regions (Sims et al., 2003). In total, 11 hit domains (including nonclassical hits Med9 and Nuc_rec_co-act from NCOA3) were tested and all were found to significantly activate the reporter, when using the extended 80 AA sequence from the library. Together, the screen and validations demonstrated that the unbiased nuclear protein domain library can be productively re-screened to uncover domains with distinct functions, and that a diverse set of domains beyond classical activation domains (and including DUFs) can activate transcription upon recruitment.

### Example 8

### Discovery of KRAB activator domains

Surprisingly, the strongest activator in the library was the KRAB domain from ZNF473 (FIG. 5B). Three other KRAB domains (from ZFP28, ZNF496, and ZNF597) were also activator hits, all of which were stably expressed and not repressors. One of these domains, from ZNF496, had previously been reported as an activator when recruited individually in HT1080 cells (Losson and Nielsen, 2010). Interestingly, ZFP28 contains two KRAB domains; KRAB_1 was a repressor and KRAB_2 was an activator. Previous affinity-purification/mass-spectrometry performed on full-length ZFP28 identified significant interactions with both repressor and activator proteins (Schmitges et al., 2016). The activator KRAB domains were significantly more acidic than nonactivator KRABs (p = 0.01, Mann Whitney test, FIG. 14D). Sequence analysis showed they were divergent from the consensus KRAB sequence while sharing homology to each other and formed a variant KRAB subcluster (FIG. 14E). Previous phylogenetic analysis has linked the variant KRAB cluster to a lack of KAP1 binding and older evolutionary age (Helleboid et al., 2019). More specifically, two of the activator KRAB source proteins (ZNF496 and ZNF597) had previously been tested with co-immunoprecipitation mass-spectrometry and were not found to interact with KAP1 (Helleboid et al., 2019).

It was individually validated that the KRAB from ZNF473 as a strong activator and KRAB_2 from ZFP28 as a moderate strength activator (FIG. 14F), using the same 80 AA sequence centered on the KRAB domains that was used in the library. Further, the trimmed 41 AA KRAB from ZNF473 was sufficient for strong activation, while the trimmed 37 AA KRAB_2 from ZFP28 did not activate, implying some of the surrounding sequence was required for activation (FIG. 21C). Next, available ChiP-seq and ChIP-exo datasets were inspected (ENCODE Project Consortium et al., 2020; Imbeault et al., 2017; Najafabadi et al., 2015; Schmitges et al., 2016) and it was found that ZNF473 co-localized with the active chromatin mark H3K27ac, in contrast to the repressive ZNF10 (FIG. 14G). Upon manual inspection, the most significant ZNF473 peaks were found near the transcription start site of genes (CASC3, STAT6, WASF2, ZKSCAN2) and a lncRNA (LINC00431). Meanwhile, ZFP28 did not co-localize with H3K27ac, perhaps indicating its KAP1-binding repressor KRAB_1 domain was generally the dominant effector over its moderate strength activator KRAB_2 domain. Looking beyond these individual KRAB proteins, the zinc finger proteins that contain a repressor KRAB did not co-localize with H3K27ac while the non-repressive KRAB proteins as a group did include colocalized peaks (FIG. 14G). Together, the results support that variant KRAB proteins are functionally diverse, sometimes functioning as transcriptional activators.

### Example 9

### Tiling library uncovers effector domains in unannotated regions of nuclear proteins

Pfam annotations provided one useful means of filtering the nuclear proteome to generate a relatively compact library, but Pfam is likely currently missing many of the human effector domains. In order to discover effector domains in unannotated regions of proteins, a tiling library was designed by curating a list of 238 proteins from silencer complexes and tiling their sequences with 80 amino acids separated by a 10 amino acid tiling window (FIG. 15A).High-throughput recruitment to the strong pEF reporter was performed and time points were taken after 5 days of doxycycline to measure silencing, and again at day 13 (8 days after doxycycline release) to measure epigenetic memory (FIG. 22A). 4.3% of the tiles scored as hits at day 5 (FIG. 15B) and their repressor strength measurements were reproducible (r² = 0.72 , FIG. 22B). Altogether, the tiling screen found short repressor domains in 141/238 proteins. Some of these hits include positive controls overlapping annotated domains: for example, by tiling ZNF57 and ZNF461, the KRAB domains of these transcription factors were identified as repressive effectors, and not the rest of the sequence (FIG. 22C). Similarly, the tiling strategy identified the RYBP repressive domain annotated by Pfam, and both the 80 AA tile and the 32 AA Pfam domain silenced with similar strength and epigenetic memory in individual validations (FIG. 22D). Repressors in REST (overlapping the CoREST binding domain (Ballas et al., 2001)), DNMT3b (overlapping the DNMT1 and DNMT3a binding domain (Kim et al., 2002)), and CBX7 (overlapping the PcBox that recruits PRC1 (Li et al., 2010)) were also identified and validated (FIGS. 22E - 22G). Another category of tiling hits was not annotated as domains in Pfam, but previous reports were found of their repressor function in the literature. For example, amino acids 121-220 of CTCF had a strong repressive function in the screen and when validated individually (FIGS. 15C and 15E), consistent with previous recruitment studies in HeLa, HEK293, and COS-7 cells (Drueppel et al., 2004). Together, these results established that high-throughput recruitment of protein tiles was an effective strategy to identify bonafide repressor domains. Amino acid sequences for repressors identified in the tiling library are shown in Table 4, with higher scores indicating increased repression.

Novel unannotated repressor domains were also discovered. For example, BAZ2A (also known as TIP5) is a nuclear remodeling complex (NoRC) component that mediates transcriptional silencing of some rDNA (Guetg et al., 2010), but does not have any annotated effector domains. The BAZ2A tiling data showed a peak of repressor function in a glutamine-rich region and it was individually validated as a moderate strength repressor (FIGS. 15D and 15E). Repressor tiles were found in unannotated regions of three TET DNA demethylases (TET1/2/3). Unexpectedly, repressor tiles were also identified in the control protein DMD, which was validated by flow cytometry (FIG. 22H).

A MGA, which is thought to repress transcription by binding the genome at E-box motifs and recruiting the non-canonical polycomb 1.6 complex (Blackledge et al., 2014; Jolma et al., 2013; Stielow et al., 2018), tiling experiment revealed two domains with repressor function, located adjacent to the two known DNA binding domains, called here Repressor 1 and Repressor 2 (FIG. 15F). These repressor domains were individually validated and distinct dynamics of silencing and degrees of memory were observed; the first domain (amino acids 341-420) featured slow silencing but strong memory, while the second domain (amino acids 2381-2460), featured rapid silencing but weak memory with fast reactivation (FIG. 15G). These appear to be the first effector domains isolated from a protein in the ncPRC1.6 silencing complex.

Next, it was attempted to identify the minimal necessary sequence for repressor function in each independent domain by examining the overlap in all tiles covering a protein region that shows repressor function and determining which contiguous sequence of amino acids is present in all the repressive tiles (FIG. 15H). Using this approach, two candidate minimized effector domains for MGA were generated: the 10 amino acid sequence MGA[381-390] and the 30 amino acid sequence MGA[2431-2460], which both overlapped conserved regions with ConSurf-predicted functional exposed residues. Individual validation experiments demonstrated that both minimized candidates can efficiently silence the reporter (FIG. 15I).

### Materials and Methods

### Cell lines and cell culture

All experiments were carried out in K562 cells (ATCC CCL-243). Cells were cultured in a controlled humidified incubator at 37°C and 5% CO2, in RPMI 1640 (Gibco) media supplemented with 10% FBS (Hyclone), penicillin (10,000 I.U./mL), streptomycin (10,000 ug/mL), and L-glutamine (2 mM). HEK293FT and HEK293T-LentiX cells were grown in DMEM (Gibco) media supplemented with 10% FBS (Hyclone), penicillin (10,000 I.U./mL), and streptomycin (10,000 ug/mL) and used to produce lentivirus. Reporter cell lines were generated by TALEN-mediated homology-directed repair to integrate a donor construct into the AAVS1 locus as follows: 1.2×10⁶ K562 cells were electroporated in Amaxa solution (Lonza Nucleofector 2b, setting T0-16) with 1000 ng of reporter donor plasmid , and 500 ng of each TALEN-L (Addgene #35431) and TALEN-R (Addgene #35432) plasmid (targeting upstream and downstream the intended DNA cleavage site, respectively). After 7 days, the cells were treated with 1000 ng/mL puromycin antibiotic for 5 days to select for a population where the donor was stably integrated in the intended locus, which provides a promoter to express the PuroR resistance gene. Fluorescent reporter expression was measured by microscopy and by flow cytometry (BD Accuri).

### Nuclear protein Pfam domain library design

The UniProt database (UniProt Consortium, 2015) was queried for human genes that can localize to the nucleus. Subcellular location information on UniProt was determined from publications or 'by similarity' in cases where there was only a publication on a similar gene (e.g., ortholog) and was manually reviewed. Pfam-annotated domains were then retrieved using the ProDy searchPfam function (Bakan et al., 2011). domains that were 80 amino acids or shorter were filtered for and the C2H2 Zinc finger DNA-binding domains, which are highly abundant, repetitive, were excluded and not expected to function as transcriptional effectors. The sequence of the annotated domain was retrieved and it was extended equally on either side to reach 80 amino acids total. Duplicate sequences were removed, then codon optimization was performed for human codon usage, removing BsmBI sites and constraining GC content to between 20% and 75% in every 50 nucleotide window (performed with DNA chisel (Zulkower and Rosser, 2020)). 499 random controls of 80 amino acids lacking stop codons were computationally generated as controls. 362 elements tiling the DMD protein in 80 amino acid tiles with a 10 amino acid sliding window were also included as controls because DMD was not thought to be a transcriptional regulator. In total, the library consists of 5,955 elements.

### Silencer tiling library design

216 proteins involved in transcriptional silencing were curated from a database of transcriptional regulators (Lambert et al., 2018). 32 proteins likely to be involved in transcriptional silencing were manually added and then an unbiased protein tiling library was generated. To do this, the canonical transcript for each gene was retrieved from the Ensembl BioMart (Kinsella et al., 2011) using the Python API. If no canonical transcript was found, the longest transcript with a CDS was retrieved. The coding sequences were divided into 80 amino acid tiles with a 10 amino acid sliding window between tiles. For each gene, a final tile was included, spanning from 80 amino acids upstream of the last residue to that last residue, such that the C-terminal region would be included in the library. Duplicate protein sequences were removed, and codon optimization was performed for human codon usage, removing BsmBI sites and constraining GC content to between 20% and 75% in every 50 nucleotide window (performed with DNA chisel (Zulkower and Rosser, 2020)). 361 DMD tiling negative controls were included, as in the previous library design, resulting in 15,737 library elements in total.

### KRAB deep mutational scan library design

A deep mutational scan of ZNF10 KRAB domain sequence, as used in CRISPRi (Gilbert et al., 2014), was designed with all possible single substitutions and all consecutive double and triple substitutions of the same amino acid (e.g., substitution with AAA). These amino acid sequences were reverse translated into DNA sequences using a probabilistic codon optimization algorithm, such that each DNA sequence contains some variation beyond the substituted residues, which improves the ability to unambiguously align sequencing reads to unique library members. In addition, all Pfam-annotated KRAB domains from human KRAB genes found on InterPro were included, similarly as in the previous nuclear Pfam domain library. Tiling sequences, as designed in the previous tiling library, were also included for five KRAB Zinc Finger genes. 300 random control sequences and 200 tiles from the DMD gene were included as negative controls. During codon optimization, BsmBI sites were removed and GC content was constrained to be between 30% and 70% in every 80 nucleotide window (performed with DNA chisel (Zulkower and Rosser, 2020)). The total library size was 5,731 elements.

### Domain library cloning

Oligonucleotides with lengths up to 300 nucleotides were synthesized as pooled libraries (Twist Biosciences) and then PCR amplified. 6x 50 ul reactions were set up in a clean PCR hood to avoid amplifying contaminating DNA. For each reaction, 5 ng of template, 0.1 µl of each 100 µM primer, 1 µl of Herculase II polymerase (Agilent), 1 µl of DMSO, 1 µl of 10 nM dNTPs, and 10 µl of 5x Herculase buffer was used. The thermocycling protocol was 3 minutes at 98°C, then cycles of 98°C for 20 seconds, 61°C for 20 seconds, 72°C for 30 seconds, and then a final step of 72°C for 3 minutes. The default cycle number was 29x, and this was optimized for each library to find the lowest cycle that resulted in a clean visible product for gel extraction (in practice, 25 cycles was the minimum). After PCR, the resulting dsDNA libraries were gel extracted by loading ≥4 lanes of a 2% TBE gel, excising the band at the expected length (around 300 bp), and using a QIAgen gel extraction kit. The libraries were cloned into a lentiviral recruitment vector pJT050 with 4x10 µl GoldenGate reactions (75 ng of pre-digested and gel-extracted backbone plasmid, 5 ng of library, 0.13 µl of T4 DNA ligase (NEB, 20000 U/µl), 0.75 µl of Esp3I-HF (NEB), and 1 µl of 10x T4 DNA ligase buffer) with 30 cycles of digestion at 37°C and ligation at 16°C for 5 minutes each, followed by a final 5 minute digestion at 37°C and then 20 minutes of heat inactivation at 70°C. The reactions were then pooled and purified with MinElute columns (QIAgen), eluting in 6 ul of ddH2O. 2 µl per tube was transformed into two tubes of 50 µl of electrocompetent cells (Lucigen DUO) following the manufacturer's instructions. After recovery, the cells were plated on 3 - 7 large 10" x 10" LB plates with carbenicillin. After overnight growth at 37°C, the bacterial colonies were scraped into a collection bottle and plasmid pools were extracted with a HiSpeed Plasmid Maxiprep kit (QIAgen). 2 - 3 small plates were prepared in parallel with diluted transformed cells in order to count colonies and confirm the transformation efficiency was sufficient to maintain at least 30x library coverage. To determine the quality of the libraries, the domains were amplified from the plasmid pool and from the original oligo pool by PCR with primers with extensions that include Illumina adapters and sequenced. The PCR and sequencing protocol were the same as described below for sequencing from genomic DNA, except these PCRs use 10 ng of input DNA and 17 cycles. These sequencing datasets were analyzed as described below to determine the uniformity of coverage and synthesis quality of the libraries. In addition, 20 - 30 colonies from the transformations were Sanger sequenced (Quintara) to estimate the cloning efficiency and the proportion of empty backbone plasmids in the pools.

### High-throughput recruitment to measure repressor activity

Large scale lentivirus production and spinfection of K562 cells were performed. To generate sufficient lentivirus to infect the libraries into K562 cells, HEK293T cells were plated on four 15-cm tissue culture plates. On each plate, 9×105 HEK293T cells were plated in 30 mL of DMEM, grown overnight, and then transfected with 8 µg of an equimolar mixture of the three third-generation packaging plasmids and 8 µg of rTetR-domain library vectors using 50 µl of polyethylenimine (PEI, Polysciences #23966). After 48 hours and 72 hours of incubation, lentivirus was harvested. The pooled lentivirus was filtered through a 0.45-µm PVDF filter (Millipore) to remove any cellular debris. For the nuclear Pfam domain repressor screen, 4.5×10⁷ K562 reporter cells were infected with the lentiviral library by spinfection for 2 hours, with two separate biological replicates of the infection. Infected cells grew for 3 days and then the cells were selected with blasticidin (10 µg/mL, Sigma). Infection and selection efficiency were monitored each day using flow cytometry to measure mCherry (BD Accuri C6). Cells were maintained in spinner flasks in log growth conditions each day by diluting cell concentrations back to a 5×10⁵ cells/mL, with at least 1.5×10⁸ cells total remaining per replicate such that the lowest maintenance coverage was >25,000× cells per library element (a very high coverage level that compensates for losses from incomplete blasticidin selection, library preparation, and library synthesis errors). On day 6 post-infection, recruitment was induced by treating the cells with 1000 ng/ml doxycycline (Fisher Scientific) for 5 days, then cells were spun down out of doxycycline and blasticidin and maintained in untreated RPMI media for 8 more days, up to Day 13 counting from the addition of doxycycline. 2.5×10⁸ cells were taken for measurements at each timepoint (days 5, 9, and 13). The protocol was similar for the KRAB DMS, but doxycycline was added on day 8 post-infection, >12,500× coverage, and 2×10⁸ - 2.2×10⁸ cells were taken for each timepoint. The protocol was similar for the tiling screen, but 9.6×10⁷ cells were infected, doxycycline was added on day 8 post-infection, at least 2×10⁸ cells were maintained at each passage for >12,500× coverage, and 2×10⁸ - 2.7×10⁸ cells were taken for each timepoint.

### High-throughput recruitment to measure transcriptional activation activity

For the nuclear Pfam domain activator screen, lentivirus for the nuclear Pfam library in the rTetR(SE-G72P)-3XFLAG vector was generated as for the repressor screen, and 3.8×10⁷ K562-pDY32 minCMV reporter cells were infected with the lentiviral library by spinfection for 2 hours, with two separate biological replicates of the infection. Infected cells grew for 2 days and then the cells were selected with blasticidin (10 µg/mL, Sigma). Infection and selection efficiency were monitored each day using flow cytometry to measure mCherry (BD Accuri C6). Cells were maintained in spinner flasks in log growth conditions each day by diluting cell concentrations back to a 5×10⁵ cells/mL, with at least 1×10⁸ total cells remaining per replicate such that the lowest maintenance coverage was >18,000× cells per library element. On day 7 post-infection, recruitment was induced by treating the cells with 1000 ng/ml doxycycline (Fisher Scientific) for 2 days, then cells were spun down out of doxycycline and blasticidin and maintained in untreated RPMI media for 4 more days. 2×10⁸ cells were taken for measurements at the day 2 time point. There was no evidence of activation memory at day 4 post-doxycycline removal, as determined by the absence of citrine positive cells by flow cytometry, so no additional time points were collected.

### Magnetic separation of reporter cells

At each timepoint, cells were spun down at 300 × g for 5 minutes and media was aspirated. Cells were then resuspended in the same volume of PBS (Gibco) and the spin down and aspiration was repeated, to wash the cells and remove any IgG from serum. Dynabeads^{™} M-280 Protein G (ThermoFisher 10003D) were resuspended by vortexing for 30 seconds. 50 mL of blocking buffer was prepared per 2×10⁸ cells by adding 1 gram of biotin-free BSA (Sigma Aldrich) and 200 µl of 0.5 M pH 8.0 EDTA (ThemoFisher 15575020) into DPBS (Gibco), vacuum filtering with a 0.22-um filter (Millipore), and then kept on ice. 60 µl of beads was prepared for every 1×10⁷ cells, by adding 1 mL of buffer per 200 µl of beads, vortexing for 5 seconds, placing on a magnetic tube rack (Eppendorf), waiting one minute, removing supernatant, and finally removing the beads from the magnet and resuspending in 100 - 600 µl of blocking buffer per initial 60 µl of beads. For the KRAB DMS only, 30 µl of beads was prepared for every 1×10⁷ cells, in the same way. Beads were added to cells at no more than 1×10⁷ cells per 100 µl of resuspended beads, and then incubated at room temperature while rocking for 30 minutes. For a sample with 2×10⁸ cells, 1.2 mL of beads were used, resuspended in 12 mL of blocking buffer, in a 15 mL Falcon tube and a large magnetic rack. For a sample with <5×10⁷ cells, non-stick Ambion 1.5 mL tubes and a small magnetic rack were used. After incubation, the bead and cell mixture were placed on the magnetic rack for >2 minutes. The unbound supernatant was transferred to a new tube, placed on the magnet again for >2 minutes to remove any remaining beads, and then the supernatant was transferred and saved as the unbound fraction. Then, the beads were resuspended in the same volume of blocking buffer, magnetically separated again, the supernatant was discarded, and the tube with the beads was kept as the bound fraction. The bound fraction was resuspended in blocking buffer or PBS to dilute the cells (the unbound fraction is already dilute). Flow cytometry (BD Accuri) was performed using a small portion of each fraction to estimate the number of cells in each fraction (to ensure library coverage was maintained) and to confirm separation based on citrine reporter levels (the bound fraction should be >90% citrine positive, while the unbound fraction is more variable depending on the initial distribution of reporter levels). Finally, the samples were spun down and the pellets were frozen at -20°C until genomic DNA extraction.

### High-throughput measurement of domain fusion protein expression level

The expression level measurements were made in K562-pDY32 cells (with citrine OFF) infected with the 3XFLAG-tagged nuclear Pfam domain library. 1×10⁸ cells per biological replicate were used after 5 days of blasticidin selection (10 µg/mL, Sigma), which was 7 days post-infection. 1×10⁶ control K562-JT039 cells (citrine ON, no lentiviral infection) were spiked into each replicate. Fix Buffer I (BD Biosciences, BDB557870) was preheated to 37°C for 15 minutes and Permeabilization Buffer III (BD Biosciences, BDB558050) and PBS (Gibco) with 10% FBS (Hyclone) were chilled on ice. The library of cells expressing domains was collected and cell density was counted by flow cytometry (BD Accuri). To fix, cells were resuspended in a volume of Fix Buffer I (BD Biosciences, BDB557870) corresponding to pellet volume, with 20 µl per 1 million cells, at 37°C for 10 - 15 minutes. Cells were washed with 1 mL of cold PBS containing 10% FBS, spun down at 500 × g for 5 minutes and then supernatant was aspirated. Cells were permeabilized for 30 minutes on ice using cold BD Permeabilization Buffer III (BD Biosciences, BDB558050), with 20 µl per 1 million cells, which was added slowly and mixed by vortexing. Cells were then washed twice in 1 ml PBS+10% FBS, as before, and then supernatant was aspirated. Antibody staining was performed for 1 hour at room temperature, protected from light, using 5 µl/ 1×10⁶ cells of α-FLAG-Alexa647 (RNDsystems, IC8529R). The cells were washed and resuspended at a concentration of 3×10⁷ cells / ml in PBS+10%FBS. Cells were sorted into two bins based on the level of APC-A fluorescence (Sony SH800S) after gating for mCherry positive viable cells. A small number of unstained control cells was also analyzed on the sorter to confirm staining was above background. The spike-in citrine positive cells were used to assess the background level of staining in cells known to lack the 3XFLAG tag, and the gate for sorting was drawn above that level. After sorting, the cellular coverage ranged from 336 - 1,295 cells per library element across samples. The sorted cells were spun down at 500 × g for 5 minutes and then resuspended in PBS. Genomic DNA extraction was performed following the manufacturer's instructions (QIAgen Blood Maxi kit was used for samples with >1×107 cells, and QIAamp DNA Mini kit with one column per up to 5×10⁶ cells was used for samples with ≤1×10⁷ cells) with one modification: the Proteinase K + AL buffer incubation was performed overnight at 56 °C.

### Library preparation and sequencing

Genomic DNA was extracted using a Blood & Tissue kit (QIAgen) following the manufacturer's instructions with up to 1.25×10⁸ cells per column. DNA was eluted in EB and not AE to avoid subsequence PCR inhibition. The domain sequences were amplified by PCR with primers containing Illumina adapters as extensions. A test PCR was performed using 5 µg of genomic DNA in a 50 µl (half-size) reaction to verify if the PCR conditions would result in a visible band at the expected size for each sample. Then, 12 - 24x 100 µl reactions were set up on ice (in a clean PCR hood to avoid amplifying contaminating DNA), with the number of reactions depending on the amount of genomic DNA available in each experiment. 10 µg of genomic DNA, 0.5 µl of each 100 µM primer, and 50 µl of NEBnext 2x Master Mix (NEB) was used in each reaction. The thermocycling protocol was to preheat the thermocycler to 98°C, then add samples for 3 minutes at 98°C, then 32x cycles of 98°C for 10 seconds, 63°C for 30 seconds, 72°C for 30 seconds, and then a final step of 72°C for 2 minutes. All subsequent steps were performed outside the PCR hood. The PCR reactions were pooled and ≥140 µl were run on at least three lanes of a 2% TBE gel alongside a 100-bp ladder for at least one hour, the library band around 395 bp was cut out, and DNA was purified using the QIAquick Gel Extraction kit (QIAgen) with a 30 ul elution into non-stick tubes (Ambion). A confirmatory gel was run to verify that small products were removed. These libraries were then quantified with a Qubit HS kit (Thermo Fisher), pooled with 15% PhiX control (Illumina), and sequenced on an Illumina NextSeq with a High output kit using a single end forward read (266 or 300 cycles) and 8 cycle index reads.

### Domain sequencing analysis

Sequencing reads were demultiplexed using bcl2fastq (Illumina). A Bowtie reference was generated using the designed library sequences with the script 'makeIndices.py' and reads were aligned with 0 mismatch allowance using the script 'makeCounts.py'. The enrichments for each domain between OFF and ON (or FLAGhigh and FLAGlow) samples were computed using the script 'makeRhos.py'. Domains with <5 reads in both samples for a given replicate were dropped from that replicate (assigned 0 counts), whereas domains with <5 reads in one sample would have those reads adjusted to 5 in order to avoid the inflation of enrichment values from low depth. For all of the nuclear domain screens, domains with ≤ 5 counts in both replicates of a given condition were filtered out of downstream analysis. For the nuclear domain expression screen, well-expressed domains were those with a log2(FLAGhigh:FLAGlow) ≥ 1 standard deviation above the median of the random controls. For the nuclear Pfam domain repressor screen, hits were domains with log2(OFF:ON) ≥ 2 standard deviations above the mean of the poorly expressed domains. For the nuclear domain activator screen, hits were domains with log2(OFF:ON) ≤ 2 standard deviations below the mean of the poorly expressed domains. For the silencer tiling screen, tiles with ≤20 counts in both replicates of a given condition were filtered out and hits were tiles with log2(OFF:ON) ≥ 2 standard deviations above the mean of the random and DMD tiling controls. Gene ontology analysis enrichments were computed using the PantherDB web tool (www.pantherdb.org). The background sets were all proteins containing domains that were well-expressed and measured in the experiment after count filters were applied. P-values for statistical significance were calculated using Fisher's exact test, the False Discovery Rate (FDR) was computed, and only the most significant results, all with FDR<10%, were shown.

### Western blot and co-immunoprecipitation

Cells transduced with a lentiviral vector containing an rTetR-fusion-T2A-mCherry-BSD were selected with blasticidin (10 µg/mL) were selected until mCherry was > 80%. Cells were lysed in lysis buffer (1% Triton X-100, 150mM NaCl, 50mM Tris pH 7.5, 1mM EDTA, Protease inhibitor cocktail). Protein amounts were quantified using the DC Protein Assay kit (Bio-Rad). Equal amounts were loaded onto a gel and transferred to a nitrocellulose or PVDF membrane. Membrane was probed using GATA1 antibody (1: 1000, rabbit, Cell Signaling Technologies cat no. 3535S) and GAPDH antibody (1:2000, mouse, ThermoFisher cat no. AM4300) or FLAG M2 monoclonal antibody (1:1000, mouse, Sigma-Aldrich, catalog number F1804) and Histone 3 antibody (1:1000, mouse, Abcam cat no. AB1791) as primary antibodies. Donkey anti-rabbit IRDye 680 LT and goat anti-mouse IRDye 800CW (1:20,000 dilution, LI-COR Biosciences, cat nos. 926-68023 and 926-32210, respectively) or Goat anti-mouse IRDye 680 RD and goat anti-rabbit IRDye 800CW (1:20,000 dilution, LI-COR Biosciences, cat nos. 926-68070 and 926-32211, respectively) were used as secondary antibodies, respectively

Blots were imaged on a LiCor Odyssey CLx. Band intensities were quantified using ImageJ.

### Individual repressor recruitment assays

Individual effector domains were cloned as fusions with rTetR or rTetR(SE-G72P) with or without a 3XFLAG tag (see figure legends), upstream of a T2A-mCherry-BSD marker using GoldenGate cloning into backbones pJT050 or pJT126. K562-pJT039-pEF-citrine reporter cells were then transduced with this lentiviral vector and, 3 days later, selected with blasticidin (10 µg/mL) until >80% of the cells were mCherry positive (6-7 days). Cells were split into separate wells of a 24-well plate and either treated with doxycycline (Fisher Scientific) or left untreated. After 5 days of treatment, doxycycline was removed by spinning down the cells, replacing media with DPBS (Gibco) to dilute any remaining doxycycline, and then spinning down the cells again and transferring them to fresh media. Timepoints were measured every 2-3 days by flow cytometry analysis of >7,000 cells (either a BD Accuri C6 or Beckman Coulter CytoFLEX). Data was analyzed using Cytoflow and custom Python scripts. Events were gated for viability and for mCherry as a delivery marker. To compute a fraction of OFF cells during doxycycline treatment, a 2 component Gaussian mixture model was fitted to the untreated rTetR-only negative control cells which fits both the ON peak and the subpopulation of background-silenced OFF cells, and then set a threshold that was 2 standard deviations below the mean of the ON peak in order to label cells that have silenced as OFF. Using the time-matched untreated control, the background normalized percentage of cells was calculated *Cells_{OFF,normalized} = Cells*_{*OFF*,+*dox*}|(1 *- Cells_{OFF,untreated}*). Two independently transduced biological replicates were used. A gene silencing model, consisting of the increasing form of the exponential decay (e.g., exponential decay subtracted from 1) during the doxycycline treatment phase and an exponential decay during the doxycycline removal phase with additional parameters for lag times before silencing and reactivation initiate, was fit to the normalized data using SciPy.

### Individual activator recruitment assays

Domains were cloned as a fusion with rTetR(SE-G72P) upstream of a T2A-mCherry-BSD marker, using GoldenGate cloning in the backbone pJT126. K562 pDY32 minCMV citrine reporter cells were then transduced with each lentiviral vector and, 3 days later, selected with blasticidin (10 µg/mL) until >80% of the cells were mCherry positive (6-7 days). Cells were split into separate wells of a 24-well plate and either treated with doxycycline or left untreated. Timepoints were measured by flow cytometry analysis of >15,000 cells (Biorad ZE5). To compute a fraction of ON cells during doxycycline treatment, a Gaussian model was fitted to the untreated rTetR-only negative control cells which fits the OFF peak, and then set a threshold that was 2 standard deviations above the mean of the OFF peak in order to label cells that have activated as ON. Two independently transduced biological replicates were used.

### Flow cytometry for FLAG-tagged protein levels

Staining of FLAG-tagged fusion protein levels was performed. Specifically, K562 cells were transduced with lentivirus to express the fusion proteins, selected with blasticidin, and then were fixed with Fix Buffer I (BD Biosciences) for 15 minutes at 37°C. Cells were washed with cold PBS with 10% FBS once and then permeabilized on ice for 30 min using Perm Buffer III (BD Biosciences). Cells were washed twice and then stained with anti-FLAG (XX) for 1 hour at 4°C. After a final round of washing, flow cytometry was performed using a CytoFLEX (Beckman Coulter) flow cytometer. The data was analyzed with CytoFlow by gating the cells on mCherry expression and then plot the FLAG-tagged protein level in mCherry+ and non-transduced cells. This approach controls for variability in staining efficiency as the two cell groups are mixed within the same sample.

### Phylogenetic and alignment analyses

KRAB and homeodomain sequences were retrieved from Pfam and extended, using surrounding native sequence, to reach 80 AA. Well-expressed domains were selected for alignment. Phylogenetic trees and sequence alignments were obtained using the alignment website Clustal Omega using default parameters (McWilliam et al., 2013; Sievers et al., 2011), and the 52 phylogenetic neighbor-joining tree without distance corrections was built with default parameters in Jalview (Waterhouse et al., 2009). Alignment visualization was performed in Jalview.

### Analysis of amino acid residue conservation

Protein sequences were submitted to the ConSurf webserver and analyzed using the ConSeq method. Briefly, ConSeq selects up to 150 homologs for a multiple string alignment, by sampling from the list of homologs with 35-95% sequence identity. Then, a phylogenetic tree is re-constructed and conservation is scored using Rate4Site. ConSurf provides normalized scores, so that the average score for all residues is zero, and the standard deviation is one. The conservation scores calculated by ConSurf are a relative measure of evolutionary conservation at each residue in the protein and the lowest score represents the most conserved position in the protein. The uniqueness of the ZNF10 KRAB N-terminal extension was determined by protein BLAST to all human proteins and searching for other zinc finger protein among the BLAST matches (Johnson et al., 2008).

### ChIP-seq and ChIP-exo analysis

External ChIP datasets were retrieved from multiple sources. ENCODE ChIP-seq data was processed with the uniform processing pipeline of ENCODE (ENCODE Project Consortium et al., 2020), and narrow peaks below IDR threshold 0.05 were retrieved. KRAB ZNF ChIP-exo data from tagged KRAB ZNF overexpression in HEK293 cells and KAP1 ChIP-exo data from H1 hESCs was obtained from GEO accession GSE78099 (Imbeault et al., 2017). Reads were trimmed to a uniform length of 36 basepairs and mapped to the hg38 version of the human genome using Bowtie (version 1.0.1; (Langmead et al., 2009)), allowing for up to 2 mismatches and only retaining unique alignments. Peak were called using MACS2 (version 2.1.0) (Feng et al., 2012) with the following settings: "-g hs -f BAM --keep-dup all --shift -75 --extsize 150 -- nomodel". Browser tracks were generated using Python scripts. For some KRAB ZNFs where ChIP-exo data was not available, ChIP-seq data from tagged KRAB ZNF overexpression in HEK293 cells was obtained from GEO accessions GSE76496 (Schmitges et al., 2016) and GSE52523 (Najafabadi et al., 2015). KRAB ZNF peaks were defined as solo binding sites if no other KRAB ZNF in the dataset had a peak less than 250 basepairs away. ENCODE H3K27ac ChIP-seq datasets for H1 cells were processed with the ENCODE pipeline (ENCODE Project Consortium et al., 2020), narrow peaks were called with MACS2, and peaks below IDR threshold 0.05 were retrieved.

### External datasets

ChIP-seq and ChIP-exo data for KRAB ZNF, KAP1, and H3K27ac (ENCODE Project Consortium et al., 2020; Imbeault et al., 2017; Najafabadi et al., 2015; Schmitges et al., 2016), KRAB ZNF gene evolutionary age (Imbeault et al., 2017), KRAB ZNF protein co-immunoprecipitation/mass spectrometry data (Helleboid et al., 2019), and CAT assays for KRAB repressor activity (Margolin et al., 1994; Witzgall et al., 1994) were retrieved from previously published studies.

**Table 1: Pfam Repressors**

| **Gene** | **Pfam Domain sequence** | **Extended Domain sequence** | **Extended Domain DNA sequence** | **Avg Repr D5** |
|---|---|---|---|---|
| ZFP28 | LVTFGDVAVDFS QEEWEWLNPIQ RNLYRKVMLENY RNLASL (SEQ ID NO: 1) | NKKLEAVGTGIEPKAMSQGLVT FGDVAVDFSQEEWEWLNPIQR NLYRKVMLENYRNLASLGLCVS KPDVISSLEQGKEPW (SEQ ID NO: 2) | AACAAGAAGCTGGAGGCCGTGGGCACCGGCATCGAGCCCAAGGCCATGAGCCA GGGCCTGGTGACCTTCGGCGACGTGGCCGTGGACTTCAGCCAGGAGGAGTGGG AGTGGCTGAACCCCATCCAGAGAAACCTGTACAGAAAGGTGATGCTGGAGAAC TACAGAAACCTGGCCAGCCTGGGCCTGTGCGTGAGCAAGCCCGACGTGATCAG CAGCCTGGAGCAGGGCAAGGAGCCCTGG (SEQ ID NO: 1177) | 7.89762464 |
| ZN334 | PVSFQDLTVNFT QEEWQQLDPAQ RLLYRDVMLENY SNLVSV (SEQ ID NO: 3) | KMKKFQIPVSFQDLTVNFTQEE WQQLDPAQRLLYRDVMLENYS NLVSVGYHVSKPDVIFKLEQGEE PWIVEEFSNQNYPD (SEQ ID NO: 4) | AAGATGAAGAAGTTCCAGATCCCCGTGAGCTTCCAGGACCTGACCGTGAACTTC ACCCAGGAGGAGTGGCAGCAGCTGGACCCCGCCCAGAGACTGCTGTACAGAGA TGTGATGCTGGAGAACTACAGCAACCTGGTGAGCGTGGGCTACCACGTGAGCA AGCCCGACGTGATCTTCAAGCTGGAGCAGGGCGAGGAGCCCTGGATCGTGGAG GAGTTCAGCAACCAGAACTACCCCGAC (SEQ ID NO: 897) | 7.84632686 |
| ZN568 | TVTFKDVAVDLT QEEWEQMKPAQ RNLYRDVMLENY SNLVTV (SEQ ID NO: 5) | CSQESALSEEEEDTTRPLETVTF KDVAVDLTQEEWEQMKPAQR NLYRDVMLENYSNLVTVGCQV TKPDVIFKLEQEEEPW (SEQ ID NO: 6) | TGCAGCCAGGAGAGCGCCCTGAGCGAGGAGGAGGACACCACCAGACCCC TGGAGACCGTGACCTTCAAGGACGTGGCCGTGGACCTGACCCAGGAGGAGTGG GAGCAGATGAAGCCCGCCCAGAGAAACCTGTACAGAGATGTGATGCTGGAGAA CTACAGCAACCTGGTGACCGTGGGCTGCCAGGTGACCAAGCCCGACGTGATCTT CAAGCTGGAGCAGGAGGAGGAGCCCTGG (SEQ ID NO: 898) | 7.83993427 |
| ZN37A | SVSFRDVTVGFT QEEWQHLDPAQ RTLYRDVMLENY SHLVSV (SEQ ID NO: 7) | ITSQGSVSFRDVTVGFTQEEWQ HLDPAQRTLYRDVMLENYSHLV SVGYCIPKPEVILKLEKGEEPWIL EEKFPSQSHLEL (SEQ ID NO: 8) | ATCACCAGCCAGGGCAGCGTGAGCTTCAGATGTGACCGTGGGCTTCACCCA GGAGGAGTGGCAGCACCTGGACCCCGCCCAGAGAACCCTGTACAGAGATGTGA TGCTGGAGAACTACAGCCACCTGGTGAGCGTGGGCTACTGCATCCCCAAGCCCG AGGTGATCCTGAAGCTGGAGAAGGGCGAGGAGCCCTGGATCCTGGAGGAGAA GTTCCCCAGCCAGAGCCACCTGGAGCTG (SEQ ID NO: 899) | 7.81563526 |
| ZN181 | QVTFNDVAIDFT HEEWGWLSSAQ RDLYKDVMVQN YENLVS (SEQ ID NO: 9) | PQVTFNDVAIDFTHEEWGWLS SAQRDLYKDVMVQNYENLVSV AGLSVTKPYVITLLEDGKEPWM MEKKLSKGMIPDWESR (SEQ ID NO: 10) | CCCCAGGTGACCTTCAACGACGTGGCCATCGACTTCACCCACGAGGAGTGGGGC TGGCTGAGCAGCGCCCAGAGAGACCTGTACAAGGACGTGATGGTGCAGAACTA CGAGAACCTGGTGAGCGTGGCCGGCCTGAGCGTGACCAAGCCCTACGTGATCA CCCTGCTGGAGGACGGCAAGGAGCCCTGGATGATGGAGAAGAAGCTGAGCAA GGGCATGATCCCCGACTGGGAGAGCAGA (SEQ ID NO: 900) | 7.80113461 |
| ZN510 | SVSFKDVTIEFTQ EEWQQMAPVQ KNLYRDVMLENY SNLVSV (SEQ ID NO: 11) | PLRFSTLFQEQQKMNISQASVS FKDVTIEFTQEEWQQMAPVQK NLYRDVMLENYSNLVSVGYCCF KPEVIFKLEQGEEPW (SEQ ID NO: 12) | CCCCTGAGATTCAGCACCCTGTTCCAGGAGCAGCAGAAGATGAACATCAGCCAG GCCAGCGTGAGCTTCAAGGACGTGACCATCGAGTTCACCCAGGAGGAGTGGCA GCAGATGGCCCCCGTGCAGAAGAACCTGTACAGAGATGTGATGCTGGAGAACT ACAGCAACCTGGTGAGCGTGGGCTACTGCTGCTTCAAGCCCGAGGTGATCTTCA AGCTGGAGCAGGGCGAGGAGCCCTGG (SEQ ID NO: 901) | 7.65619264 |
| ZN862 | PVVFEDVAVYFT REEWGMLDKRQ KELYRDVMRMN YELLASL (SEQ ID NO: 13) | QDPSAEGLSEEVPVVFEELPVVF EDVAVYFTREEWGMLDKRQKE LYRDVMRMNYELLASLGPAAA KPDLISKLERRAAPW (SEQ ID NO: 14) | CAGGACCCCAGCGCCGAGGGCCTGAGCGAGGAGGTGCCCGTGGTGTTCGAGG AGCTGCCCGTGGTGTTCGAGGACGTGGCCGTGTACTTCACCAGAGAGGAGTGG GGCATGCTGGACAAGAGACAGAAGGAGCTGTACAGAGATGTGATGAGAATGA ACTACGAGCTGCTGGCCAGCCTGGGCCCCGCCGCCGCCAAGCCTGACCTGATCA GCAAGCTGGAGAGAAGAGCCGCCCCCTGG (SEQ ID NO: 902) | 7.64282609 |
| ZN140 | SVTFRDVAIDFSQ EEWKWLQPAQR DLYRCVMLENYG HLVSL (SEQ ID NO: 15) | SQGSVTFRDVAIDFSQEEWKW LQPAQRDLYRCVMLENYGHLV SLGLSISKPDVVSLLEQGKEPWL GKREVKRDLFSVSES (SEQ ID NO: 16) | AGCCAGGGCAGCGTGACCTTCAGAGATGTGGCCATCGACTTCAGCCAGGAGGA GTGGAAGTGGCTGCAGCCGCCCAGAGAGACCTGTACAGATGCGTGATGCTGG AGAACTACGGCCACCTGGTGAGCCTGGGCCTGAGCATCAGCAAGCCCGACGTG GTGAGCCTGCTGGAGCAGGGCAAGGAGCCCTGGCTGGGCAAGAGAGAGGTGA AGAGAGACCTGTTCAGCGTGAGCGAGAGC (SEQ ID NO: 903) | 7.59939471 |
| ZN208 | SLTFRDVAIEFSLE EWQCLDTAQQN LYRNVMLENYRN LVFL (SEQ ID NO: 17) | GSLTFRDVAIEFSLEEWQCLDTA QQNLYRNVMLENYRNLVFLGIA AFKPDLIIFLEEGKESWNMKRH EMVEESPVICSHF (SEQ ID NO: 18) | GGCAGCCTGACCTTCAGAGATGTGGCCATCGAGTTCAGCCTGGAGGAGTGGCA GTGCCTGGACACCGCCCAGCAGAACCTGTACAGAAACGTGATGCTGGAGAACT ACAGAAACCTGGTGTTCCTGGGCATCGCCGCCTTCAAGCCCGACCTGATCATCTT CCTGGAGGAGGGCAAGGAGAGCTGGAACATGAAGAGACACGAGATGGTGGAG GAGAGCCCCGTGATCTGCAGCCACTTC (SEQ ID NO: 904) | 7.57602814 |
| ZN248 | QVSFKDVCVDFT QEEWYLLDPAQK ILYRDVILENYSNL VSV (SEQ ID NO: 19) | NKSQEQVSFKDVCVDFTQEEW YLLDPAQKILYRDVILENYSNLVS VGYCITKPEVIFKIEQGEEPWILE KGFPSQCHPER (SEQ ID NO: 20) | AACAAGAGCCAGGAGCAGGTGAGCTTCAAGGACGTGTGCGTGGACTTCACCCA GGAGGAGTGGTACCTGCTGGACCCCGCCCAGAAGATCCTGTACAGAGATGTGA TCCTGGAGAACTACAGCAACCTGGTGAGCGTGGGCTACTGCATCACCAAGCCCG AGGTGATCTTCAAGATCGAGCAGGGCGAGGAGCCCTGGATCCTGGAGAAGGGC TTCCCCAGCCAGTGCCACCCCGAGAGA (SEQ ID NO: 905) | 7.53353306 |
| ZN571 | LVTFRDVAIDFSQ EEWECLDPAQRD LYRDVMLENYSN LISL (SEQ ID NO: 21) | PHLLVTFRDVAIDFSQEEWECL DPAQRDLYRDVMLENYSNLISL DLESSCVTKKLSPEKEIYEMESL QWENMGKRINHHL (SEQ ID NO: 22) | CCCCACCTGCTGGTGACCTTCAGAGATGTGGCCATCGACTTCAGCCAGGAGGAG TGGGAGTGCCTGGACCCCGCCCAGAGAGACCTGTACAGAGATGTGATGCTGGA GAACTACAGCAACCTGATCAGCCTGGACCTGGAGAGCAGCTGCGTGACCAAGA AGCTGAGCCCCGAGAAGGAGATCTACGAGATGGAGAGCCTGCAGTGGGAGAA CATGGGCAAGAGAATCAACCACCACCTG (SEQ ID NO: 906) | 7.45303805 |
| ZN699 | SVVFEDVAVDFT QEEWALLDLAQR NLYRDVMLENFQ NLASL (SEQ ID NO: 23) | EEERKTAELQKNRIQDSVVFED VAVDFTQEEWALLDLAQRNLY RDVMLENFQNLASLGYPLHTP HLISQWEQEEDLQTVK (SEQ ID NO: 24) | GAGGAGGAGAGAAAGACCGCCGAGCTGCAGAAGAACAGAATCCAGGACAGCG TGGTGTTCGAGGACGTGGCCGTGGACTTCACCCAGGAGGAGTGGGCCCTGCTG GACCTGGCCCAGAGAAACCTGTACAGAGATGTGATGCTGGAGAACTTCCAGAA CCTGGCCAGCCTGGGCTACCCCCTGCACACCCCCCACCTGATCAGCCAGTGGGA GCAGGAGGAGGACCTGCAGACCGTGAAG (SEQ ID NO: 907) | 7.44633076 |
| ZN726 | LLTFRDVAIEFSLE EWQCLDTAQKN LYRNVMLENYRN LAFL (SEQ ID NO: 25) | GLLTFRDVAIEFSLEEWQCLDTA QKNLYRNVMLENYRNLAFLGIA VSKPDLIICLEKEKEPWNMKRD EMVDEPPGICPHF (SEQ ID NO: 26) | GGCCTGCTGACCTTCAGAGATGTGGCCATCGAGTTCAGCCTGGAGGAGTGGCA GTGCCTGGACACCGCCCAGAAGAACCTGTACAGAAACGTGATGCTGGAGAACT ACAGAAACCTGGCCTTCCTGGGCATCGCCGTGAGCAAGCCCGACCTGATCATCT GCCTGGAGAAGGAGAAGGAGCCCTGGAACATGAAGAGAGATGAGATGGTGGA CGAGCCCCCCGGCATCTGCCCCCACTTC (SEQ ID NO: 908) | 7.44588981 |
| ZIK1 | CVTFEDIAIYFSQ DEWGLLDEAQRL LYLEVMLENFALV ASL (SEQ ID NO: 27) | RAPTQVTVSPETHMDLTKGCV TFEDIAIYFSQDEWGLLDEAQRL LYLEVMLENFALVASLGCGHGT EDEETPSDQNVSVG (SEQ ID NO: 28) | AGAGCCCCCACCCAGGTGACCGTGAGCCCCGAGACCCACATGGACCTGACCAA GGGCTGCGTGACCTTCGAGGACATCGCCATCTACTTCAGCCAGGACGAGTGGG GCCTGCTGGACGAGGCCCAGAGACTGCTGTACCTGGAGGTGATGCTGGAGAAC TTCGCCCTGGTGGCCAGCCTGGGCTGCGGCCACGGCACCGAGGACGAAGAGAC CCCTAGCGACCAGAACGTGAGCGTGGGC (SEQ ID NO: 909) | 7.43302782 |
| ZNF2 | SVTFEDVAVVFT DEEWSRLVPIQR DLYKEVMLENYN SIVSL (SEQ ID NO: 29) | AAVSPTTRCQESVTFEDVAVVF TDEEWSRLVPIQRDLYKEVMLE NYNSIVSLGLPVPQPDVIFQLKR GDKPWMVDLHGSE (SEQ ID NO: 30) | GCCGCCGTGAGCCCCACCACCAGATGCCAGGAGAGCGTGACCTTCGAGGACGT GGCCGTGGTGTTCACCGACGAGGAGTGGAGCAGACTGGTGCCCATCCAGAGAG ACCTGTACAAGGAGGTGATGCTGGAGAACTACAACAGCATCGTGAGCCTGGGC CTGCCCGTGCCCCAGCCCGACGTGATCTTCCAGCTGAAGAGAGGCGACAAGCCC TGGATGGTGGACCTGCACGGCAGCGAG (SEQ ID NO: 910) | 7.40745859 |
| Z705F | KVTFEDVAIDFTQ EEWDMMDTSKR KLYRDVMLENIS HLVSL (SEQ ID NO: 31) | HSLEKVTFEDVAIDFTQEEWD MMDTSKRKLYRDVMLENISHL VSLGYQISKSYIILQLEQGKELW REGRVFLQDQNPDRE (SEQ ID NO: 32) | CACAGCCTGGAGAAGGTGACCTTCGAGGACGTGGCCATCGACTTCACCCAGGA GGAGTGGGACATGATGGACACCAGCAAGAGAAAGCTGTACAGAGATGTGATG CTGGAGAACATCAGCCACCTGGTGAGCCTGGGCTACCAGATCAGCAAGAGCTA CATCATCCTGCAGCTGGAGCAGGGCAAGGAGCTGTGGAGAGAGGGCAGAGTG TTCCTGCAGGACCAGAACCCCGACAGAGAG (SEQ ID NO: 911) | 7.40598629 |
| ZNF14 | SVSFEDVAVNFTL EEWALLDSSQKK LYEDVMQETFKN LVCL (SEQ ID NO: 33) | DSVSFEDVAVNFTLEEWALLDS SQKKLYEDVMQETFKNLVCLGK KWEDQDIEDDHRNQGKNRRC HMVERLCESRRGSKCG (SEQ ID NO: 34) | GACAGCGTGAGCTTCGAGGACGTGGCCGTGAACTTCACCCTGGAGGAGTGGGC CCTGCTGGACAGCAGCCAGAAGAAGCTGTACGAGGACGTGATGCAGGAGACCT TCAAGAACCTGGTGTGCCTGGGCAAGAAGTGGGAGGACCAGGACATCGAGGA CGACCACAGAAACCAGGGCAAGAACAGAAGATGCCACATGGTGGAGAGACTGT GCGAGAGCAGAAGAGGCAGCAAGTGCGGC (SEQ ID NO: 912) | 7.3912024 |
| ZN471 | LVTFKDVAIDFSQ EEWQWMNPAQ KRLYRSMMLENY QSLVSL (SEQ ID NO: 35) | NVEVVKVMPQDLVTFKDVAID FSQEEWQWMNPAQKRLYRS MMLENYQSLVSLGLCISKPYVIS LLEQGREPWEMTSEMTR (SEQ ID NO: 36) | AACGTGGAGGTGGTGAAGGTGATGCCCCAGGACCTGGTGACCTTCAAGGACGT GGCCATCGACTTCAGCCAGGAGGAGTGGCAGTGGATGAACCCCGCCCAGAAGA GACTGTACAGAAGCATGATGCTGGAGAACTACCAGAGCCTGGTGAGCCTGGGC CTGTGCATCAGCAAGCCCTACGTGATCAGCCTGCTGGAGCAGGGCAGAGAGCC CTGGGAGATGACCAGCGAGATGACCAGA (SEQ ID NO: 913) | 7.38691832 |
| ZN624 | SVTFKDVAIDFTL EEWRLMDPTQR NLHKDVMLENYR NLVSL (SEQ ID NO: 37) | TQPDEDLHLQAEETQLVKESVT FKDVAIDFTLEEWRLMDPTQR NLHKDVMLENYRNLVSLGLAVS KPDMISHLENGKGPW (SEQ ID NO: 38) | ACCCAGCCCGACGAGGACCTGCACCTGCAGGCCGAGGAGACCCAGCTGGTGAA GGAGAGCGTGACCTTCAAGGACGTGGCCATCGACTTCACCCTGGAGGAGTGGA GACTGATGGACCCCACCCAGAGAAACCTGCACAAGGACGTGATGCTGGAGAAC TACAGAAACCTGGTGAGCCTGGGCCTGGCCGTGAGCAAGCCCGACATGATCAG CCACCTGGAGAACGGCAAGGGCCCCTGG (SEQ ID NO: 914) | 7.37615807 |
| ZNF84 | SFSFDDLSVDFTQ KEWQLLDPSQKN LYKDVMLENYSSL VSL (SEQ ID NO: 39) | TMLQESFSFDDLSVDFTQKEW QLLDPSQKNLYKDVMLENYSSL VSLGYEVMKPDVIFKLEQGEEP WVGDGEIPSSDSPEV (SEQ ID NO: 40) | ACCATGCTGCAGGAGAGCTTCAGCTTCGACGACCTGAGCGTGGACTTCACCCAG AAGGAGTGGCAGCTGCTGGACCCCAGCCAGAAGAACCTGTACAAGGACGTGAT GCTGGAGAACTACAGCAGCCTGGTGAGCCTGGGCTACGAGGTGATGAAGCCCG ACGTGATCTTCAAGCTGGAGCAGGGCGAGGAGCCCTGGGTGGGCGACGGCGA GATCCCTAGCAGCGACAGCCCCGAGGTG (SEQ ID NO: 915) | 7.37354184 |
| ZNF7 | VVTFGDVAVHFS REEWQCLDPGQ RALYREVMLENH SSVAG (SEQ ID NO: 41) | EVVTFGDVAVHFSREEWQCLD PGQRALYREVMLENHSSVAGL AGFLVFKPELISRLEQGEEPWVL DLQGAEGTEAPRTSK (SEQ ID NO: 42) | GAGGTGGTGACCTTCGGCGACGTGGCCGTGCACTTCAGCAGAGAGGAGTGGCA GTGCCTGGACCCCGGCCAGAGAGCCCTGTACAGAGAGGTGATGCTGGAGAACC ACAGCAGCGTGGCCGGCCTGGCCGGCTTCCTGGTGTTCAAGCCCGAGCTGATCA GCAGACTGGAGCAGGGCGAGGAGCCCTGGGTGCTGGACCTGCAGGGCGCCGA AGGCACAGAGGCCCCCAGAACCAGCAAG (SEQ ID NO: 916) | 7.35816861 |
| ZN891 | PMTFKDVAVEFT QEEWMMLDSA QRSLYRDVMLEN YRNLTSV (SEQ ID NO: 43) | RNAEEERMIAVFLTTWLQEPM TFKDVAVEFTQEEWMMLDSA QRSLYRDVMLENYRNLTSVEYQ LYRLTVISPLDQEEIRN (SEQ ID NO: 44) | AGAAACGCCGAGGAGGAGAGAATGATCGCCGTGTTCCTGACCACCTGGCTGCA GGAGCCCATGACCTTCAAGGACGTGGCCGTGGAGTTCACCCAGGAGGAGTGGA TGATGCTGGACAGCGCCCAGAGAAGCCTGTACAGAGATGTGATGCTGGAGAAC TACAGAAACCTGACCAGCGTGGAGTACCAGCTGTACAGACTGACCGTGATCAGC CCCCTGGACCAGGAGGAGATCAGAAAC (SEQ ID NO: 917) | 7.35404032 |
| ZN337 | FLAFGDVTVDFT QKEWRLLSPAQR ALYREVTLENYSH LVSL (SEQ ID NO: 45) | GPQGARRQAFLAFGDVTVDFT QKEWRLLSPAQRALYREVTLEN YSHLVSLGILHSKPELIRRLEQGE VPWGEERRRRPGP (SEQ ID NO: 46) | GGCCCCCAGGGCGCCAGAAGACAGGCCTTCCTGGCCTTCGGCGACGTGACCGT GGACTTCACCCAGAAGGAGTGGAGACTGCTGAGCCCCGCCCAGAGAGCCCTGT ACAGAGAGGTGACCCTGGAGAACTACAGCCACCTGGTGAGCCTGGGCATCCTG CACAGCAAGCCCGAGCTGATCAGAAGACTGGAGCAGGGCGAGGTGCCCTGGG GCGAGGAGAGAAGAAGAAGACCCGGCCCC (SEQ ID NO: 918) | 7.3403856 |
| Z705G | KLTFEDVAIDFTQ EEWAMMDTSKR KLYRDVMLENIS HLVSL (SEQ ID NO: 47) | HSLKKLTFEDVAIDFTQEEWAM MDTSKRKLYRDVMLENISHLVS LGYQISKSYIILQLEQGKELWRE GRVFLQDQNPNRE (SEQ ID NO: 48) | CACAGCCTGAAGAAGCTGACCTTCGAGGACGTGGCCATCGACTTCACCCAGGAG GAGTGGGCCATGATGGACACCAGCAAGAGAAAGCTGTACAGAGATGTGATGCT GGAGAACATCAGCCACCTGGTGAGCCTGGGCTACCAGATCAGCAAGAGCTACA TCATCCTGCAGCTGGAGCAGGGCAAGGAGCTGTGGAGAGAGGGCAGAGTGTTC CTGCAGGACCAGAACCCCAACAGAGAG (SEQ ID NO: 919) | 7.33888308 |
| ZN529 | LVTLRDVVINFSQ EEWEYLDSAQRN LYWDVMMENYS NLLSL (SEQ ID NO: 49) | MPEVEFPDQFFTVLTMDHELV TLRDVVINFSQEEWEYLDSAQR NLYWDVMMENYSNLLSLDLES RNETKHLSVGKDIIQN (SEQ ID NO: 50) | ATGCCCGAGGTGGAGTTCCCCGACCAGTTCTTCACCGTGCTGACCATGGACCAC GAGCTGGTGACCCTGAGAGATGTGGTGATCAACTTCAGCCAGGAGGAGTGGGA GTACCTGGACAGCGCCCAGAGAAACCTGTACTGGGACGTGATGATGGAGAACT ACAGCAACCTGCTGAGCCTGGACCTGGAGAGCAGAAACGAGACCAAGCACCTG AGCGTGGGCAAGGACATCATCCAGAAC (SEQ ID NO: 920) | 7.33722191 |
| ZN729 | PLTFRDVTIEFSLE EWQCLDTVQQN LYRDVMLENYRN LVFL (SEQ ID NO: 51) | PGAPGSLEMGPLTFRDVTIEFSL EEWQCLDTVQQNLYRDVMLE NYRNLVFLGMAVFKPDLITCLK QGKEPWNMKRHEMVT (SEQ ID NO: 52) | CCCGGCGCCCCCGGCAGCCTGGAGATGGGCCCCCTGACCTTCAGAGATGTGACC ATCGAGTTCAGCCTGGAGGAGTGGCAGTGCCTGGACACCGTGCAGCAGAACCT GTACAGAGATGTGATGCTGGAGAACTACAGAAACCTGGTGTTCCTGGGCATGG CCGTGTTCAAGCCCGACCTGATCACCTGCCTGAAGCAGGGCAAGGAGCCCTGGA ACATGAAGAGACACGAGATGGTGACC (SEQ ID NO: 921) | 7.33489189 |
| ZN419 | YVTFEDVAVYFS QEEWRLLDDAQ RLLYRNVMLENF TLLASL (SEQ ID NO: 53) | RDPAQVPVAADLLTDHEEGYVT FEDVAVYFSQEEWRLLDDAQRL LYRNVMLENFTLLASLGLASSKT HEITQLESWEEPF (SEQ ID NO: 54) | AGAGACCCCGCCCAGGTGCCCGTGGCCGCCGACCTGCTGACCGACCACGAAGA AGGCTACGTGACCTTCGAGGACGTGGCCGTGTACTTCAGCCAGGAGGAGTGGA GACTGCTGGACGACGCCCAGAGACTGCTGTACAGAAACGTGATGCTGGAGAAC TTCACCCTGCTGGCCAGCCTGGGCCTGGCCAGCAGCAAGACCCACGAGATCACC CAGCTGGAGAGCTGGGAGGAGCCCTTC (SEQ ID NO: 922) | 7.33241867 |
| Z705A | KVTFEDVAIDFTQ EEWAMMDTSKR KLYRDVMLENIS HLVSL (SEQ ID NO: 55) | HSLKKVTFEDVAIDFTQEEWA MMDTSKRKLYRDVMLENISHL VSLGYQISKSYIILQLEQGKELW REGREFLQDQNPDRE (SEQ ID NO: 56) | CACAGCCTGAAGAAGGTGACCTTCGAGGACGTGGCCATCGACTTCACCCAGGA GGAGTGGGCCATGATGGACACCAGCAAGAGAAAGCTGTACAGAGATGTGATGC TGGAGAACATCAGCCACCTGGTGAGCCTGGGCTACCAGATCAGCAAGAGCTAC ATCATCCTGCAGCTGGAGCAGGGCAAGGAGCTGTGGAGAGAGGGCAGAGAGT TCCTGCAGGACCAGAACCCCGACAGAGAG (SEQ ID NO: 923) | 7.32024193 |
| ZNF45 | AVTFKDVAVVFS EEELQLLDLAQRK LYRDVMLENFRN VVSV (SEQ ID NO: 57) | TKSKEAVTFKDVAVVFSEEELQL LDLAQRKLYRDVMLENFRNVVS VGHQSTPDGLPQLEREEKLWM MKMATQRDNSSGAK (SEQ ID NO: 58) | ACCAAGAGCAAGGAGGCCGTGACCTTCAAGGACGTGGCCGTGGTGTTCAGCGA GGAGGAGCTGCAGCTGCTGGACCTGGCCCAGAGAAAGCTGTACAGAGATGTGA TGCTGGAGAACTTCAGAAACGTGGTGAGCGTGGGCCACCAGAGCACCCCCGAC GGCCTGCCCCAGCTTGAGAGAGAGGAGAAGCTGTGGATGATGAAGATGGCCAC CCAGAGAGACAACAGCAGCGGCGCCAAG (SEQ ID NO: 924) | 7.31275735 |
| ZN302 | QVTFSDVAIDFSH EEWACLDSAQR DLYKDVMVQNY ENLVSV (SEQ ID NO: 59) | SQVTFSDVAIDFSHEEWACLDS AQRDLYKDVMVQNYENLVSVG LSVTKPYVIMLLEDGKEPWMM EKKLSKAYPFPLSHSV (SEQ ID NO: 60) | AGCCAGGTGACCTTCAGCGACGTGGCCATCGACTTCAGCCACGAGGAGTGGGC CTGCCTGGACAGCGCCCAGAGAGACCTGTACAAGGACGTGATGGTGCAGAACT ACGAGAACCTGGTGAGCGTGGGCCTGAGCGTGACCAAGCCCTACGTGATCATG CTGCTGGAGGACGGCAAGGAGCCCTGGATGATGGAGAAGAAGCTGAGCAAGG CCTACCCCTTCCCCCTGAGCCACAGCGTG (SEQ ID NO: 925) | 7.27433142 |
| ZN486 | SLQFRDVAVEFSL EEWHCLDTAQQ NLYRDVMLENYR HLVFL (SEQ ID NO: 61) | PGPLRSLEMESLQFRDVAVEFSL EEWHCLDTAQQNLYRDVMLE NYRHLVFLGIIVSKPDLITCLEQG IKPLTMKRHEMIA (SEQ ID NO: 62) | CCCGGCCCCCTGAGAAGCCTGGAGATGGAGAGCCTGCAGTTCAGAGATGTGGC CGTGGAGTTCAGCCTGGAGGAGTGGCACTGCCTGGACACCGCCCAGCAGAACC TGTACAGAGATGTGATGCTGGAGAACTACAGACACCTGGTGTTCCTGGGCATCA TCGTGAGCAAGCCCGACCTGATCACCTGCCTGGAGCAGGGCATCAAGCCCCTGA CCATGAAGAGACACGAGATGATCGCC (SEQ ID NO: 926) | 7.27242434 |
| ZN621 | SVTFEDVAVYFT QNQWASLDPAQ RALYGEVMLENY ANVAS (SEQ ID NO: 63) | LQTTWPQESVTFEDVAVYFTQ NQWASLDPAQRALYGEVMLE NYANVASLVAFPFPKPALISHLE RGEAPWGPDPWDTEIL (SEQ ID NO: 64) | CTGCAGACCACCTGGCCCCAGGAGAGCGTGACCTTCGAGGACGTGGCCGTGTA CTTCACCCAGAACCAGTGGGCCAGCCTGGACCCCGCCCAGAGAGCCCTGTACGG CGAGGTGATGCTGGAGAACTACGCCAACGTGGCCAGCCTGGTGGCCTTCCCCTT CCCCAAGCCCGCCCTGATCAGCCACCTGGAGAGAGGCGAGGCCCCCTGGGGAC CTGACCCCTGGGACACCGAGATCCTG (SEQ ID NO: 927) | 7.25940008 |
| ZN688 | TVSFADVAVYFSP EEWGCLRPAQR ALYRDVMQETYG HLGAL (SEQ ID NO: 65) | APLLAPRPGETRPGCRKPGTVS FADVAVYFSPEEWGCLRPAQR ALYRDVMQETYGHLGALGFPG PKPALISWMEQESEAW (SEQ ID NO: 66) | GCCCCCCTGCTGGCCCCCAGACCCGGCGAGACCAGACCCGGCTGCAGAAAGCC CGGCACAGTGAGCTTCGCCGACGTGGCCGTGTACTTCAGCCCCGAGGAGTGGG GCTGCCTGAGACCCGCCCAGAGAGCCCTGTACAGAGATGTGATGCAGGAGACC TACGGCCACCTGGGCGCCCTGGGCTTCCCCGGCCCCAAACCTGCTCTGATCAGCT GGATGGAGCAGGAGAGCGAGGCCTGG (SEQ ID NO: 928) | 7.2566174 |
| ZN33A | SVSFKDVTVGFT QEEWQHLDPSQ RALYRDVMLENY SNLVSV (SEQ ID NO: 67) | NKVEQKSQESVSFKDVTVGFTQ EEWQHLDPSQRALYRDVMLEN YSNLVSVGYCVHKPEVIFRLQQ GEEPWKQEEEFPSQS (SEQ ID NO: 68) | AACAAGGTGGAGCAGAAGAGCCAGGAGAGCGTGAGCTTCAAGGACGTGACCG TGGGCTTCACCCAGGAGGAGTGGCAGCACCTGGACCCCAGCCAGAGAGCCCTG TACAGAGATGTGATGCTGGAGAACTACAGCAACCTGGTGAGCGTGGGCTACTG CGTGCACAAGCCCGAGGTGATCTTCAGACTGCAGCAGGGCGAGGAGCCCTGGA AGCAGGAGGAGGAGTTCCCCAGCCAGAGC (SEQ ID NO: 929) | 7.23239827 |
| ZN554 | LVTFEDVSMDFS QEEWELLEPAQK NLYREVMLENYR NVVSL (SEQ ID NO: 69) | CFSQEERMAAGYLPRWSQELV TFEDVSMDFSQEEWELLEPAQ KNLYREVMLENYRNVVSLEALK NQCTDVGIKEGPLSPA (SEQ ID NO: 70) | TGCTTCAGCCAGGAGGAGAGAATGGCCGCCGGCTACCTGCCCAGATGGAGCCA GGAGCTGGTGACCTTCGAGGACGTGAGCATGGACTTCAGCCAGGAGGAGTGG GAGCTGCTGGAGCCCGCCCAGAAGAACCTGTACAGAGAGGTGATGCTGGAGAA CTACAGAAACGTGGTGAGCCTGGAGGCCCTGAAGAACCAGTGCACCGACGTGG GCATCAAGGAGGGCCCCCTGAGCCCCGCC (SEQ ID NO: 930) | 7.22964061 |
| ZN878 | SVAFEDVAVNFT QEEWALLDPSQK NLYREVMQETLR NLTSI (SEQ ID NO: 71) | DSVAFEDVAVNFTQEEWALLD PSQKNLYREVMQETLRNLTSIG KKWNNQYIEDEHQNPRRNLRR LIGERLSESKESHQHG (SEQ ID NO: 72) | GACAGCGTGGCCTTCGAGGACGTGGCCGTGAACTTCACCCAGGAGGAGTGGGC CCTGCTGGACCCCAGCCAGAAGAACCTGTACAGAGAGGTGATGCAGGAGACCC TGAGAAACCTGACCAGCATCGGCAAGAAGTGGAACAACCAGTACATCGAGGAC GAGCACCAGAACCCCAGAAGAAACCTGAGAAGACTGATCGGCGAGAGACTGAG CGAGAGCAAGGAGAGCCACCAGCACGGC (SEQ ID NO: 931) | 7.21922256 |
| ZN772 | QVNFEDVFVYFS QEEWVLLDEAQR LLYRDVMLENFA LMASL (SEQ ID NO: 73) | MGPAQVPMNSEVIVDPIQGQ VNFEDVFVYFSQEEWVLLDEAQ RLLYRDVMLENFALMASLGHTS FMSHIVASLVMGSEPW (SEQ ID NO: 74) | ATGGGCCCCGCCCAGGTGCCCATGAACAGCGAGGTGATCGTGGACCCCATCCA GGGCCAGGTGAACTTCGAGGACGTGTTCGTGTACTTCAGCCAGGAGGAGTGGG TGCTGCTGGACGAGGCCCAGAGACTGCTGTACAGAGATGTGATGCTGGAGAAC TTCGCCCTGATGGCCAGCCTGGGCCACACCAGCTTCATGAGCCACATCGTGGCC AGCCTGGTGATGGGCAGCGAGCCCTGG (SEQ ID NO: 932) | 7.1961596 |
| ZN224 | AMTFKDVAVVFT EEELGLLDLAQRK LYRDVMLENFRN LLSV (SEQ ID NO: 75) | TTFKEAMTFKDVAVVFTEEELG LLDLAQRKLYRDVMLENFRNLL SVGHQAFHRDTFHFLREEKIW MMKTAIQREGNSGDK(SEQ ID NO: 76) | ACCACCTTCAAGGAGGCCATGACCTTCAAGGACGTGGCCGTGGTGTTCACCGAG GAGGAGCTGGGCCTGCTGGACCTGGCCCAGAGAAAGCTGTACAGAGATGTGAT GCTGGAGAACTTCAGAAACCTGCTGAGCGTGGGCCACCAGGCCTTCCACAGAG ACACCTTCCACTTCCTGAGAGAGGAGAAGATCTGGATGATGAAGACCGCCATCC AGAGAGAGGGCAACAGCGGCGACAAG (SEQ ID NO: 933) | 7.18876477 |
| ZN184 | AVTFKDVIVDFTQ EEWKQLDPGQR DLFRDVTLENYTH LVSI (SEQ ID NO: 77) | DSTLLQGGHNLLSSASFQEAVT FKDVIVDFTQEEWKQLDPGQR DLFRDVTLENYTHLVSIGLQVSK PDVISQLEQGTEPW (SEQ ID NO: 78) | GACAGCACCCTGCTGCAGGGCGGCCACAACCTGCTGAGCAGCGCCAGCTTCCA GGAGGCCGTGACCTTCAAGGACGTGATCGTGGACTTCACCCAGGAGGAGTGGA AGCAGCTGGACCCCGGCCAGAGAGACCTGTTCAGAGATGTGACCCTGGAGAAC TACACCCACCTGGTGAGCATCGGCCTGCAGGTGAGCAAGCCCGACGTGATCAGC CAGCTGGAGCAGGGCACCGAGCCCTGG (SEQ ID NO: 934) | 7.18783852 |
| ZN544 | SVCFEDVAMAFT QEEWEQLDLAQ RTLYREVTLETWE HIVSL (SEQ ID NO: 79) | EARSMLVPPQASVCFEDVAMA FTQEEWEQLDLAQRTLYREVTL ETWEHIVSLGLFLSKSDVISQLE QEEDLCRAEQEAPR (SEQ ID NO: 80) | GAGGCCAGAAGCATGCTGGTGCCCCCCAGGCCAGCGTGTGCTTCGAGGACGT GGCCATGGCCTTCACCCAGGAGGAGTGGGAGCAGCTGGACCTGGCCCAGAGAA CCCTGTACAGAGAGGTGACCCTGGAGACCTGGGAGCACATCGTGAGCCTGGGC CTGTTCCTGAGCAAGAGCGACGTGATCAGCCAGCTGGAGCAGGAGGAGGACCT GTGCAGAGCCGAGCAGGAGGCCCCCAGA (SEQ ID NO: 935) | 7.18695522 |
| ZNF57 | SVVFEDVAVDFTL EEWALLDSAQRD LYRDVMLETFRN LASV (SEQ ID NO: 81) | DSVVFEDVAVDFTLEEWALLDS AQRDLYRDVMLETFRNLASVD DGTQFKANGSVSLQDMYGQE KSKEQTIPNFTGNNSCA (SEQ ID NO: 82) | GACAGCGTGGTGTTCGAGGACGTGGCCGTGGACTTCACCCTGGAGGAGTGGGC CCTGCTGGACAGCGCCCAGAGAGACCTGTACAGAGATGTGATGCTGGAGACCT TCAGAAACCTGGCCAGCGTGGACGACGGCACCCAGTTCAAGGCCAACGGCAGC GTGAGCCTGCAGGACATGTACGGCCAGGAGAAGAGCAAGGAGCAGACCATCCC CAACTTCACCGGCAACAACAGCTGCGCC (SEQ ID NO: 936) | 7.1854619 |
| ZN283 | LVTFRDVAIDFSQ EEWECLDPAQRD LYVDVMLENYSN LVSL (SEQ ID NO: 83) | EESHGALISSCNSRTMTDGLVTF RDVAIDFSQEEWECLDPAQRDL YVDVMLENYSNLVSLDLESKTYE TKKIFSENDIFE (SEQ ID NO: 84) | GAGGAGAGCCACGGCGCCCTGATCAGCAGCTGCAACAGCAGAACCATGACCGA CGGCCTGGTGACCTTCAGAGATGTGGCCATCGACTTCAGCCAGGAGGAGTGGG AGTGCCTGGACCCCGCCCAGAGAGACCTGTACGTGGACGTGATGCTGGAGAAC TACAGCAACCTGGTGAGCCTGGACCTGGAGAGCAAGACCTACGAGACCAAGAA GATCTTCAGCGAGAACGACATCTTCGAG (SEQ ID NO: 937) | 7.16688066 |
| ZN549 | HVTFEDIAVYFSQ EEWGLLDEAQRC LYHDVMLENFSL MASV (SEQ ID NO: 85) | VITPQIPMVTEEFVKPSQGHVT FEDIAVYFSQEEWGLLDEAQRC LYHDVMLENFSLMASVGCLHGI EAEEAPSEQTLSAQ (SEQ ID NO: 86) | GTGATCACCCCCCAGATCCCCATGGTGACCGAGGAGTTCGTGAAGCCCAGCCAG GGCCACGTGACCTTCGAGGACATCGCCGTGTACTTCAGCCAGGAGGAGTGGGG CCTGCTGGACGAGGCCCAGAGATGCCTGTACCACGACGTGATGCTGGAGAACTT CAGCCTGATGGCCAGCGTGGGCTGCCTGCACGGCATCGAGGCCGAGGAGGCTC CTAGCGAGCAGACCCTGAGCGCCCAG (SEQ ID NO: 938) | 7.14938492 |
| ZN211 | SVTFEDVAVYFS WEEWDLLDEAQ KHLYFDVMLENF ALTSSL (SEQ ID NO: 87) | VQLRPQTRMATALRDPASGSV TFEDVAVYFSWEEWDLLDEAQ KHLYFDVMLENFALTSSLGCWC GVEHEETPSEQRISGE (SEQ ID NO: 88) | GTGCAGCTGAGACCCCAGACCAGAATGGCCACCGCCCTGAGAGACCCCGCCAG CGGCAGCGTGACCTTCGAGGACGTGGCCGTGTACTTCAGCTGGAGGAGTGGG ACCTGCTGGACGAGGCCCAGAAGCACCTGTACTTCGACGTGATGCTGGAGAACT TCGCCCTGACCAGCAGCCTGGGCTGCTGGTGCGGCGTGGAGCACGAGGAGACC CCCAGCGAGCAGAGAATCAGCGGCGAG (SEQ ID NO: 939) | 7.14721188 |
| ZN506 | PLQFRDVAIEFSL EEWHCLDAAQR NLYRDVMLENYR NLIFL (SEQ ID NO: 89) | GPLQFRDVAIEFSLEEWHCLDA AQRNLYRDVMLENYRNLIFLGI VVSKPNLITCLEQGKKPLTMKR HEMIAKPPVMYSHF (SEQ ID NO: 90) | GGCCCCCTGCAGTTCAGAGATGTGGCCATCGAGTTCAGCCTGGAGGAGTGGCA CTGCCTGGACGCCGCCCAGAGAAACCTGTACAGAGATGTGATGCTGGAGAACT ACAGAAACCTGATCTTCCTGGGCATCGTGGTGAGCAAGCCCAACCTGATCACCT GCCTGGAGCAGGGCAAGAAGCCCCTGACCATGAAGAGACACGAGATGATCGCC AAGCCCCCCGTGATGTACAGCCACTTC (SEQ ID NO: 940) | 7.1466168 |
| ZN615 | SLTLEDVAVDFT WEEWQFLSPAQ KDLYRDVMLENY SNLVAV (SEQ ID NO: 91) | MQAQESLTLEDVAVDFTWEE WQFLSPAQKDLYRDVMLENYS NLVAVGYQASKPDALSKLERGE ETCTTEDEIYSRICSEI (SEQ ID NO: 92) | ATGCAGGCCCAGGAGAGCCTGACCCTGGAGGACGTGGCCGTGGACTTCACCTG GGAGGAGTGGCAGTTCCTGAGCCCCGCCCAGAAGGACCTGTACAGAGATGTGA TGCTGGAGAACTACAGCAACCTGGTGGCCGTGGGCTACCAGGCCAGCAAGCCC GACGCCCTGAGCAAGCTGGAGAGAGGCGAGGAGACCTGCACCACCGAGGACG AGATCTACAGCAGAATCTGCAGCGAGATC (SEQ ID NO: 941) | 7.13864847 |
| ZN253 | PLQFRDVAIEFSL EEWHCLDTAQR NLYRDVMLENYR NLVFL (SEQ ID NO: 93) | GPLQFRDVAIEFSLEEWHCLDT AQRNLYRDVMLENYRNLVFLGI VVSKPDLVTCLEQGKKPLTMER HEMIAKPPVMSSHF (SEQ ID NO: 94) | GGCCCCCTGCAGTTCAGAGATGTGGCCATCGAGTTCAGCCTGGAGGAGTGGCA CTGCCTGGACACCGCCCAGAGAAACCTGTACAGAGATGTGATGCTGGAGAACT ACAGAAACCTGGTGTTCCTGGGCATCGTGGTGAGCAAGCCCGACCTGGTGACCT GCCTGGAGCAGGGCAAGAAGCCCCTGACCATGGAGAGACACGAGATGATCGCC AAGCCCCCCGTGATGAGCAGCCACTTC (SEQ ID NO: 942) | 7.12597439 |
| ZN226 | AVTFKDVAVAFT EEELGLLGPAQRK LYRDVMVENFRN LLSV (SEQ ID NO: 95) | NMFKEAVTFKDVAVAFTEEELG LLGPAQRKLYRDVMVENFRNLL SVGHPPFKQDVSPIERNEQLWI MTTATRRQGNLGEK (SEQ ID NO: 96) | AACATGTTCAAGGAGGCCGTGACCTTCAAGGACGTGGCCGTGGCCTTCACCGAG GAGGAGCTGGGCCTGCTGGGCCCTGCTCAGAGAAAGCTGTACAGAGATGTGAT GGTGGAGAACTTCAGAAACCTGCTGAGCGTGGGCCACCCCCCCTTCAAGCAGG ACGTGAGCCCCATCGAGAGAAACGAGCAGCTGTGGATCATGACCACCGCCACC AGAAGACAGGGCAACCTGGGCGAGAAG (SEQ ID NO: 943) | 7.12032078 |
| ZN730 | ALTFRDVAIEFSLE EWQCLDTEQQN LYRNVMLDNYR NLVFL (SEQ ID NO: 97) | GALTFRDVAIEFSLEEWQCLDTE QQNLYRNVMLDNYRNLVFLGI AVSKPDLITCLEQEKEPWNLKT HDMVAKPPVICSHI (SEQ ID NO: 98) | GGCGCCCTGACCTTCAGAGATGTGGCCATCGAGTTCAGCCTGGAGGAGTGGCA GTGCCTGGACACCGAGCAGCAGAACCTGTACAGAAACGTGATGCTGGACAACT ACAGAAACCTGGTGTTCCTGGGCATCGCCGTGAGCAAGCCCGACCTGATCACCT GCCTGGAGCAGGAGAAGGAGCCCTGGAACCTGAAGACCCACGACATGGTGGCC AAGCCCCCCGTGATCTGCAGCCACATC (SEQ ID NO: 944) | 7.1167303 |
| Z585A | SVSFRDVAIDFSR EEWRHLDPSQR NLYRDVMLETYS HLLSV (SEQ ID NO: 99) | SPQKSSALAPEDHGSSYEGSVSF RDVAIDFSREEWRHLDPSQRNL YRDVMLETYSHLLSVGYQVPEA EVVMLEQGKEPWA (SEQ ID NO: 100) | AGCCCCCAGAAGAGCAGCGCCCTGGCCCCCGAGGACCACGGCAGCAGCTACGA GGGAAGCGTGAGCTTCAGAGATGTGGCCATCGACTTCAGCAGAGAGGAGTGGA GACACCTGGACCCCAGCCAGAGAAACCTGTACAGAGATGTGATGCTGGAGACC TACAGCCACCTGCTGAGCGTGGGCTACCAGGTGCCCGAGGCCGAGGTGGTGAT GCTGGAGCAGGGCAAGGAGCCCTGGGCC (SEQ ID NO: 945) | 7.11150182 |
| ZN732 | LLTFRDVAIEFSPE EWKCLDPAQQN LYRDVMLENYRN LISL (SEQ ID NO: 101) | ELLTFRDVAIEFSPEEWKCLDPA QQNLYRDVMLENYRNLISLGVA ISNPDLVIYLEQRKEPYKVKIHET VAKHPAVCSHF (SEQ ID NO: 102) | GAGCTGCTGACCTTCAGAGATGTGGCCATCGAGTTCAGCCCCGAGGAGTGGAA GTGCCTGGACCCCGCCCAGCAGAACCTGTACAGAGATGTGATGCTGGAGAACT ACAGAAACCTGATCAGCCTGGGCGTGGCCATCAGCAACCCCGACCTGGTGATCT ACCTGGAGCAGAGAAAGGAGCCCTACAAGGTGAAGATCCACGAGACCGTGGCC AAGCACCCCGCCGTGTGCAGCCACTTC (SEQ ID NO: 946) | 7.10058289 |
| ZN681 | PLKFRDVAIEFSLE EWQCLDTIQQNL YRNVMLENYRNL VFL (SEQ ID NO: 103) | EPLKFRDVAIEFSLEEWQCLDTI QQNLYRNVMLENYRNLVFLGIV VSKPDLITCLEQEKEPWTRKRH RMVAEPPVICSHF (SEQ ID NO: 104) | GAGCCCCTGAAGTTCAGAGATGTGGCCATCGAGTTCAGCCTGGAGGAGTGGCA GTGCCTGGACACCATCCAGCAGAACCTGTACAGAAACGTGATGCTGGAGAACTA CAGAAACCTGGTGTTCCTGGGCATCGTGGTGAGCAAGCCCGACCTGATCACCTG CCTGGAGCAGGAGAAGGAGCCCTGGACCAGAAAGAGACACAGAATGGTGGCC GAGCCCCCCGTGATCTGCAGCCACTTC (SEQ ID NO: 947) | 7.09555392 |
| ZN667 | PITFGDLAIYFSQE EWEWLSPIQKDL YEDVMLENYRNL VSL (SEQ ID NO: 105) | PSARGKSKSKAPITFGDLAIYFSQ EEWEWLSPIQKDLYEDVMLEN YRNLVSLGLSFRRPNVITLLEKGK APWMVEPVRRR (SEQ ID NO: 106) | CCCAGCGCCAGAGGCAAGAGCAAGAGCAAGGCCCCCATCACCTTCGGCGACCT GGCCATCTACTTCAGCCAGGAGGAGTGGGAGTGGCTGAGCCCCATCCAGAAGG ACCTGTACGAGGACGTGATGCTGGAGAACTACAGAAACCTGGTGAGCCTGGGC CTGAGCTTCAGAAGACCCAACGTGATCACCCTGCTGGAGAAGGGCAAGGCCCC CTGGATGGTGGAGCCCGTGAGAAGAAGA (SEQ ID NO: 948) | 7.08035538 |
| ZN649 | SLTLEDVAVDFT WEEWQFLSPAQ KDLYRDVMLENY SNLVSV (SEQ ID NO: 107) | TKAQESLTLEDVAVDFTWEEW QFLSPAQKDLYRDVMLENYSNL VSVGYQAGKPDALTKLEQGEPL WTLEDEIHSPAHPEI (SEQ ID NO: 108) | ACCAAGGCCCAGGAGAGCCTGACCCTGGAGGACGTGGCCGTGGACTTCACCTG GGAGGAGTGGCAGTTCCTGAGCCCCGCCCAGAAGGACCTGTACAGAGATGTGA TGCTGGAGAACTACAGCAACCTGGTGAGCGTGGGCTACCAGGCCGGCAAGCCC GACGCCCTGACCAAGCTGGAGCAGGGCGAGCCTCTGTGGACCCTGGAGGACGA GATCCACAGCCCCGCCCACCCCGAGATC (SEQ ID NO: 949) | 7.07364506 |
| ZN470 | SVTFTDVAIDFSQ DEWEWLNLAQR SLYKKVMLENYR NLVSV(SEQ ID NO: 109) | SQEEVEVAGIKLCKAMSLGSVT FTDVAIDFSQDEWEWLNLAQR SLYKKVMLENYRNLVSVGLCISK PDVISLLEQEKDPW (SEQ ID NO: 110) | AGCCAGGAGGAGGTGGAGGTGGCCGGCATCAAGCTGTGCAAGGCCATGAGCC TGGGCAGCGTGACCTTCACCGACGTGGCCATCGACTTCAGCCAGGACGAGTGG GAGTGGCTGAACCTGGCCCAGAGAAGCCTGTACAAGAAGGTGATGCTGGAGAA CTACAGAAACCTGGTGAGCGTGGGCCTGTGCATCAGCAAGCCCGACGTGATCA GCCTGCTGGAGCAGGAGAAGGACCCCTGG (SEQ ID NO: 950) | 7.07241961 |
| ZN484 | SVSFKDVTVDFSR DEWQQLDLAQK SLYREVMLENYF NLISV (SEQ ID NO: 111) | TKSLESVSFKDVTVDFSRDEWQ QLDLAQKSLYREVMLENYFNLIS VGCQVPKPEVIFSLEQEEPCML DGEIPSQSRPDGD (SEQ ID NO: 112) | ACCAAGAGCCTGGAGAGCGTGAGCTTCAAGGACGTGACCGTGGACTTCAGCAG AGATGAGTGGCAGCAGCTGGACCTGGCCCAGAAGAGCCTGTACAGAGAGGTG ATGCTGGAGAACTACTTCAACCTGATCAGCGTGGGCTGCCAGGTGCCCAAGCCC GAGGTGATCTTCAGCCTGGAGCAGGAGGAGCCCTGCATGCTGGACGGCGAGAT CCCCAGCCAGAGCAGACCCGACGGCGAC (SEQ ID NO: 951) | 7.07124789 |
| ZN431 | TLTFRDVAIEFSLE EWECLNPAQQN LYMNVMLENYK NLVFL (SEQ ID NO: 113) | SGCPGAERNLLVYSYFEKETLTF RDVAIEFSLEEWECLNPAQQNL YMNVMLENYKNLVFLGVAVSK QDPVTCLEQEKEPW (SEQ ID NO: 114) | AGCGGCTGCCCCGGCGCCGAGAGAAACCTGCTGGTGTACAGCTACTTCGAGAA GGAGACCCTGACCTTCAGAGATGTGGCCATCGAGTTCAGCCTGGAGGAGTGGG AGTGCCTGAACCCCGCCCAGCAGAACCTGTACATGAACGTGATGCTGGAGAACT ACAAGAACCTGGTGTTCCTGGGCGTGGCCGTGAGCAAGCAGGACCCCGTGACC TGCCTGGAGCAGGAGAAGGAGCCCTGG (SEQ ID NO: 952) | 7.06946125 |
| ZN382 | SVSFKDVTVDFT QEEWQQLDPAQ KALYRDVMLENY CHFVSV (SEQ ID NO: 115) | PLQGSVSFKDVTVDFTQEEWQ QLDPAQKALYRDVMLENYCHF VSVGFHMAKPDMIRKLEQGEE LWTQRIFPSYSYLEEDG (SEQ ID NO: 116) | CCCCTGCAGGGCAGCGTGAGCTTCAAGGACGTGACCGTGGACTTCACCCAGGA GGAGTGGCAGCAGCTGGACCCCGCCCAGAAGGCCCTGTACAGAGATGTGATGC TGGAGAACTACTGCCACTTCGTGAGCGTGGGCTTCCACATGGCCAAGCCCGACA TGATCAGAAAGCTGGAGCAGGGCGAGGAGCTGTGGACCCAGAGAATCTTCCCC AGCTACAGCTACCTGGAGGAGGACGGC (SEQ ID NO: 953) | 7.06892645 |
| ZN254 | LLTFRDVAIEFSLE EWQHLDIAQQN LYRNVMLENYRN LAFL (SEQ ID NO: 117) | PGPPRSLEMGLLTFRDVAIEFSL EEWQHLDIAQQNLYRNVMLE NYRNLAFLGIAVSKPDLITCLEQ GKEPWNMKRHEMVD (SEQ ID NO: 118) | CCCGGCCCCCCAGAAGCCTGGAGATGGGCCTGCTGACCTTCAGAGATGTGGCC ATCGAGTTCAGCCTGGAGGAGTGGCAGCACCTGGACATCGCCCAGCAGAACCT GTACAGAAACGTGATGCTGGAGAACTACAGAAACCTGGCCTTCCTGGGCATCGC CGTGAGCAAGCCCGACCTGATCACCTGCCTGGAGCAGGGCAAGGAGCCCTGGA ACATGAAGAGACACGAGATGGTGGAC (SEQ ID NO: 954) | 7.06718937 |
| ZN124 | SVAFEDVAVNFT QEEWALLDPSQK NLYRDVMQETFR NLASI(SEQ ID NO: 119) | SGHPGSWEMNSVAFEDVAVN FTQEEWALLDPSQKNLYRDVM QETFRNLASIGNKGEDQSIEDQ YKNSSRNLRHIISHSGN (SEQ ID NO: 120) | AGCGGCCACCCCGGCAGCTGGGAGATGAACAGCGTGGCCTTCGAGGACGTGGC CGTGAACTTCACCCAGGAGGAGTGGGCCCTGCTGGACCCCAGCCAGAAGAACC TGTACAGAGATGTGATGCAGGAGACCTTCAGAAACCTGGCCAGCATCGGCAAC AAGGGCGAGGACCAGAGCATCGAGGACCAGTACAAGAACAGCAGCAGAAACC TGAGACACATCATCAGCCACAGCGGCAAC (SEQ ID NO: 955) | 7.0598763 |
| ZN607 | SITFGDVAIDFSH QEWEYLSLVQKT LYQEVMMENYD NLVS (SEQ ID NO: 121) | SYGSITFGDVAIDFSHQEWEYLS LVQKTLYQEVMMENYDNLVSL AGHSVSKPDLITLLEQGKEPWM IVREETRGECTDLD (SEQ ID NO: 122) | AGCTACGGCAGCATCACCTTCGGCGACGTGGCCATCGACTTCAGCCACCAGGAG TGGGAGTACCTGAGCCTGGTGCAGAAGACCCTGTACCAGGAGGTGATGATGGA GAACTACGACAACCTGGTGAGCCTGGCCGGCCACAGCGTGAGCAAGCCCGACC TGATCACCCTGCTGGAGCAGGGCAAGGAGCCCTGGATGATCGTGAGAGAGGAG ACCAGAGGCGAGTGCACCGACCTGGAC (SEQ ID NO: 956) | 7.05852729 |
| ZN317 | SVTFQDVAVDFT EKEWPLLDSSQR KLYKDVMLENYS NLTSL (SEQ ID NO: 123) | DLFVCSGLEPHTPSVGSQESVTF QDVAVDFTEKEWPLLDSSQRKL YKDVMLENYSNLTSLGYQVGKP SLISHLEQEEEPR (SEQ ID NO: 124) | GACCTGTTCGTGTGCAGCGGCCTGGAGCCCCACACCCCCAGCGTGGGCAGCCA GGAAAGCGTGACCTTCCAGGACGTGGCCGTGGACTTCACCGAGAAGGAGTGGC CCCTGCTGGACAGCAGCCAGAGAAAGCTGTACAAGGACGTGATGCTGGAGAAC TACAGCAACCTGACCAGCCTGGGCTACCAGGTGGGCAAGCCCAGCCTGATCAGC CACCTGGAGCAGGAGGAGGAGCCCAGA (SEQ ID NO: 957) | 7.05281313 |
| ZN620 | PVTFEDVAVYFT QNEWASLDSVQ RALYREVMLENY ANVAS (SEQ ID NO: 125) | FQTAWRQEPVTFEDVAVYFTQ NEWASLDSVQRALYREVMLEN YANVASLAFPFTTPVLVSQLEQ GELPWGLDPWEPMGRE (SEQ ID NO: 126) | TTCCAGACCGCCTGGAGACAGGAGCCCGTGACCTTCGAGGACGTGGCCGTGTA CTTCACCCAGAACGAGTGGGCCAGCCTGGACAGCGTGCAGAGAGCCCTGTACA GAGAGGTGATGCTGGAGAACTACGCCAACGTGGCCAGCCTGGCCTTCCCCTTCA CCACCCCCGTGCTGGTGAGCCAGCTGGAGCAGGGCGAGCTGCCCTGGGGACTG GACCCCTGGGAGCCTATGGGCAGAGAG (SEQ ID NO: 958) | 7.04082891 |
| ZN141 | LLTFRDVAIEFSPE EWKCLDPDQQN LYRDVMLENYRN LVSL (SEQ ID NO: 127) | ELLTFRDVAIEFSPEEWKCLDPD QQNLYRDVMLENYRNLVSLGV AISNPDLVTCLEQRKEPYNVKIH KIVARPPAMCSHF (SEQ ID NO: 128) | GAGCTGCTGACCTTCAGAGATGTGGCCATCGAGTTCAGCCCCGAGGAGTGGAA GTGCCTGGACCCCGACCAGCAGAACCTGTACAGAGATGTGATGCTGGAGAACT ACAGAAACCTGGTGAGCCTGGGCGTGGCCATCAGCAACCCCGACCTGGTGACCT GCCTGGAGCAGAGAAAGGAGCCCTACAACGTGAAGATCCACAAGATCGTGGCC AGACCCCCCGCCATGTGCAGCCACTTC (SEQ ID NO: 959) | 7.03997569 |
| ZN584 | LVMFEDVTVYFS REEWGLLNVTQK GLYRDVMLENFA LVSSL (SEQ ID NO: 129) | AGEAEAQLDPSLQGLVMFEDV TVYFSREEWGLLNVTQKGLYRD VMLENFALVSSLGLAPSRSPVFT QLEDDEQSWVPSWV (SEQ ID NO: 130) | GCCGGCGAGGCCGAGGCCCAGCTGGACCCAGCCTGCAGGGCCTGGTTATGTT CGAGGACGTGACCGTGTACTTCAGCAGAGAGGAGTGGGGCCTGCTGAACGTGA CCCAGAAGGGCCTGTACAGAGATGTGATGCTGGAGAACTTCGCCCTGGTGAGC AGCCTGGGCCTGGCCCCCAGCAGAAGCCCCGTGTTCACCCAGCTGGAGGACGA CGAGCAGAGCTGGGTGCCCAGCTGGGTG (SEQ ID NO: 960) | 7.03820051 |
| ZN540 | LVTFRDVAIDFSQ KEWECLDTTQRK LYRDVMLENYNN LVSL (SEQ ID NO: 131) | AHALVTFRDVAIDFSQKEWECL DTTQRKLYRDVMLENYNNLVSL GYSGSKPDVITLLEQGKEPCVVA RDVTGRQCPGLLS (SEQ ID NO: 132) | GCCCACGCCCTGGTGACCTTCAGAGATGTGGCCATCGACTTCAGCCAGAAGGAG TGGGAGTGCCTGGACACCACCCAGAGAAAGCTGTACAGAGATGTGATGCTGGA GAACTACAACAACCTGGTGAGCCTGGGCTACAGCGGCAGCAAGCCCGACGTGA TCACCCTGCTGGAGCAGGGCAAGGAGCCCTGCGTGGTGGCCAGAGATGTGACC GGCAGACAGTGCCCCGGCCTGCTGAGC (SEQ ID NO: 961) | 7.03581318 |
| ZN75D | LLTFEDVAVYFSE EEWQLLNPLEKT LYNDVMQDIYET VISL (SEQ ID NO: 133) | KRIKHWKMASKLILPESLSLLTFE DVAVYFSEEEWQLLNPLEKTLY NDVMQDIYETVISLGLKLKNDT GNDHPISVSTSE (SEQ ID NO: 134) | AAGAGAATCAAGCACTGGAAGATGGCCAGCAAGCTGATCCTGCCCGAGAGCCT GAGCCTGCTGACCTTCGAGGACGTGGCCGTGTACTTCAGCGAGGAGGAGTGGC AGCTGCTGAACCCCCTGGAGAAGACCCTGTACAACGACGTGATGCAGGACATCT ACGAGACCGTGATCAGCCTGGGCCTGAAGCTGAAGAACGACACCGGCAACGAC CACCCCATCAGCGTGAGCACCAGCGAG (SEQ ID NO: 962) | 7.02809755 |
| ZN555 | SVVFEDVAVDFTL EEWALLDSAQRD LYRDVMLETFQN LASV(SEQ ID NO: 135) | DSVVFEDVAVDFTLEEWALLDS AQRDLYRDVMLETFQNLASVD DETQFKASGSVSQQDIYGEKIPK ESKIATFTRNVSWA (SEQ ID NO: 136) | GACAGCGTGGTGTTCGAGGACGTGGCCGTGGACTTCACCCTGGAGGAGTGGGC CCTGCTGGACAGCGCCCAGAGAGACCTGTACAGAGATGTGATGCTGGAGACCT TCCAGAACCTGGCCAGCGTGGACGACGAGACCCAGTTCAAGGCCAGCGGCAGC GTGAGCCAGCAGGACATCTACGGCGAGAAGATCCCCAAGGAGAGCAAGATCGC CACCTTCACCAGAAACGTGAGCTGGGCC (SEQ ID NO: 963) | 7.02680391 |
| ZN658 | SVSFQDVTVEFTR EEWQHLGPVERT LYRDVMLENYSH LISV (SEQ ID NO: 137) | NMSQASVSFQDVTVEFTREEW QHLGPVERTLYRDVMLENYSHL ISVGYCITKPKVISKLEKGEEPWS LEDEFLNQRYPGY (SEQ ID NO: 138) | AACATGAGCCAGGCCAGCGTGAGCTTCCAGGACGTGACCGTGGAGTTCACCAG AGAGGAGTGGCAGCACCTGGGCCCCGTGGAGAGAACCCTGTACAGAGATGTGA TGCTGGAGAACTACAGCCACCTGATCAGCGTGGGCTACTGCATCACCAAGCCCA AGGTGATCAGCAAGCTGGAGAAGGGCGAGGAGCCCTGGAGCCTGGAGGACGA GTTCCTGAACCAGAGATACCCCGGCTAC (SEQ ID NO: 964) | 7.01857786 |
| ZN684 | SVTFQDVAVDFT AEEWQLLDCAER TLYWDVMLENY RNLISV (SEQ ID NO: 139) | ISFQESVTFQDVAVDFTAEEWQ LLDCAERTLYWDVMLENYRNLI SVGCPITKTKVILKVEQGQEPW MVEGANPHESSPES (SEQ ID NO: 140) | ATCAGCTTCCAGGAGAGCGTGACCTTCCAGGACGTGGCCGTGGACTTCACCGCC GAGGAGTGGCAGCTGCTGGACTGCGCCGAGAGAACCCTGTACTGGGACGTGAT GCTGGAGAACTACAGAAACCTGATCAGCGTGGGCTGCCCCATCACCAAGACCAA GGTGATCCTGAAGGTGGAGCAGGGCCAGGAGCCCTGGATGGTGGAGGGCGCC AACCCCCACGAGAGCAGCCCCGAGAGC (SEQ ID NO: 965) | 7.01522838 |
| RBAK | PVSFKDVAVDFT QEEWQQLDPDE KITYRDVMLENYS HLVSV (SEQ ID NO: 141) | NTLQGPVSFKDVAVDFTQEEW QQLDPDEKITYRDVMLENYSHL VSVGYDTTKPNVIIKLEQGEEP WIMGGEFPCQHSPEA (SEQ ID NO: 142) | AACACCCTGCAGGGCCCCGTGAGCTTCAAGGACGTGGCCGTGGACTTCACCCAG GAGGAGTGGCAGCAGCTGGACCCCGACGAGAAGATCACCTACAGAGATGTGAT GCTGGAGAACTACAGCCACCTGGTGAGCGTGGGCTACGACACCACCAAGCCCA ACGTGATCATCAAGCTGGAGCAGGGCGAGGAGCCCTGGATCATGGGCGGCGA GTTCCCCTGCCAGCACAGCCCCGAGGCC (SEQ ID NO: 966) | 7.01040328 |
| ZN829 | PVMFRDVSIDFS QEEWECLDADQ MNLYKEVMLENF SNLVSV (SEQ ID NO: 143) | HPEEEERMHDELLQAVSKGPV MFRDVSIDFSQEEWECLDADQ MNLYKEVMLENFSNLVSVGLS NSKPAVISLLEQGKEPW (SEQ ID NO: 144) | CACCCCGAGGAGGAGGAGAGAATGCACGACGAGCTGCTGCAGGCCGTGAGCA AGGGCCCCGTGATGTTCAGAGATGTGAGCATCGACTTCAGCCAGGAGGAGTGG GAGTGCCTGGACGCCGACCAGATGAACCTGTACAAGGAGGTGATGCTGGAGAA CTTCAGCAACCTGGTGAGCGTGGGCCTGAGCAACAGCAAGCCCGCCGTGATCA GCCTGCTGGAGCAGGGCAAGGAGCCCTGG (SEQ ID NO: 967) | 7.0012394 |
| ZN582 | LFRDVAIVFSQEE WQWLAPAQRDL YRDVMLETYSNL VSL (SEQ ID NO: 145) | SLGSELFRDVAIVFSQEEWQWL APAQRDLYRDVMLETYSNLVSL GLAVSKPDVISFLEQGKEPWM VERVVSGGLCPVLES (SEQ ID NO: 146) | AGCCTGGGCAGCGAGCTGTTCAGAGATGTGGCCATCGTGTTCAGCCAGGAGGA GTGGCAGTGGCTGGCCCCCGCCCAGAGAGACCTGTACAGAGATGTGATGCTGG AGACCTACAGCAACCTGGTGAGCCTGGGCCTGGCCGTGAGCAAGCCCGACGTG ATCAGCTTCCTGGAGCAGGGCAAGGAGCCCTGGATGGTGGAGAGAGTGGTGA GCGGCGGCCTGTGCCCCGTGCTGGAGAGC (SEQ ID NO: 968) | 6.98988925 |
| ZN112 | MVTFKDVAVVFT EEELGLLDSVQRK LYRDVMLENFRN LLL (SEQ ID NO: 147) | TKFQEMVTFKDVAVVFTEEELG LLDSVQRKLYRDVMLENFRNLL LVAHQPFKPDLISQLEREEKLLM VETETPRDGCSGR (SEQ ID NO: 148) | ACCAAGTTCCAGGAGATGGTGACCTTCAAGGACGTGGCCGTGGTGTTCACCGA GGAGGAGCTGGGCCTGCTGGACAGCGTGCAGAGAAAGCTGTACAGAGATGTG ATGCTGGAGAACTTCAGAAACCTGCTGCTGGTGGCCCACCAGCCCTTCAAGCCC GACCTGATCAGCCAGCTGGAGAGAGAGGAGAAGCTGCTGATGGTGGAGACCG AGACCCCCAGAGATGGCTGCAGCGGCAGA (SEQ ID NO: 969) | 6.98982538 |
| ZN716 | LLTFRDIAIEFSLA EWQCLDHAQQN LYRDVMLENYRN LVSL (SEQ ID NO: 149) | AKRPGPPGSREMGLLTFRDIAIE FSLAEWQCLDHAQQNLYRDV MLENYRNLVSLGIAVSKPDLITC LEQNKEPQNIKRNE (SEQ ID NO: 150) | GCCAAGAGACCCGGCCCCCCGGCAGCAGAGAGATGGGCCTGCTGACCTTCAG AGACATCGCCATCGAGTTCAGCCTGGCCGAGTGGCAGTGCCTGGACCACGCCCA GCAGAACCTGTACAGAGATGTGATGCTGGAGAACTACAGAAACCTGGTGAGCC TGGGCATCGCCGTGAGCAAGCCCGACCTGATCACCTGCCTGGAGCAGAACAAG GAGCCCCAGAACATCAAGAGAAACGAG (SEQ ID NO: 970) | 6.98744382 |
| HKR1 | FVAFRDVAVYFT QEEWRLLSPAQR TLHREVMLETYN HLVSL (SEQ ID NO: 151) | TCMVHRQTMSCSGAGGITAFV AFRDVAVYFTQEEWRLLSPAQR TLHREVMLETYNHLVSLEIPSSK PKLIAQLERGEAPW (SEQ ID NO: 152) | ACCTGCATGGTGCACAGACAGACCATGAGCTGCAGCGGCGCCGGCGGCATCAC CGCCTTCGTGGCCTTCAGAGATGTGGCCGTGTACTTCACCCAGGAGGAGTGGAG ACTGCTGAGCCCCGCCCAGAGAACCCTGCACAGAGAGGTGATGCTGGAGACCT ACAACCACCTGGTGAGCCTGGAGATCCCCAGCAGCAAGCCCAAGCTGATCGCCC AGCTGGAGAGAGGCGAGGCCCCCTGG (SEQ ID NO: 971) | 6.98664414 |
| ZN350 | SITLEDVAVDFT WEEWQLLGAAQ KDLYRDVMLENY SNLVAV (SEQ ID NO: 153) | IQAQESITLEDVAVDFTWEEW QLLGAAQKDLYRDVMLENYSN LVAVGYQASKPDALFKLEQGEQ LWTIEDGIHSGACSDI (SEQ ID NO: 154) | ATCCAGGCCCAGGAGAGCATCACCCTGGAGGACGTGGCCGTGGACTTCACCTG GGAGGAGTGGCAGCTGCTGGGCGCCGCCCAGAAGGACCTGTACAGAGATGTG ATGCTGGAGAACTACAGCAACCTGGTGGCCGTGGGCTACCAGGCCAGCAAGCC CGACGCCCTGTTCAAGCTGGAGCAGGGCGAGCAGCTGTGGACCATCGAGGACG GCATCCACAGCGGCGCCTGCAGCGACATC (SEQ ID NO: 972) | 6.98636848 |
| ZN480 | HLTFRDVAIEFSQ AEWKCLDPAQR ALYKDVMLENYR NLVSL (SEQ ID NO: 155) | AQKRRKRKAKESGMALPQGHL TFRDVAIEFSQAEWKCLDPAQR ALYKDVMLENYRNLVSLGISLPD LNINSMLEQRREPW (SEQ ID NO: 156) | GCCCAGAAGAGAAGAAAGAGAAAGGCCAAGGAGAGCGGCATGGCCCTGCCCC AGGGCCACCTGACCTTCAGAGATGTGGCCATCGAGTTCAGCCAGGCCGAGTGG AAGTGCCTGGACCCCGCCCAGAGAGCCCTGTACAAGGACGTGATGCTGGAGAA CTACAGAAACCTGGTGAGCCTGGGCATCAGCCTGCCCGACCTGAACATCAACAG CATGCTGGAGCAGAGAAGAGAGCCCTGG (SEQ ID NO: 973) | 6.98462693 |
| ZN416 | CVTFEDVAIYFSQ EEWGLLDEAQRL LYRDVMLENFALI TA (SEQ ID NO: 157) | DSTSVPVTAEAKLMGFTQGCVT FEDVAIYFSQEEWGLLDEAQRL LYRDVMLENFALITALVCWHG MEDEETPEQSVSVEG (SEQ ID NO: 158) | GACAGCACCAGCGTGCCCGTGACCGCCGAGGCCAAGCTGATGGGCTTCACCCA GGGCTGCGTGACCTTCGAGGACGTGGCCATCTACTTCAGCCAGGAGGAGTGGG GCCTGCTGGACGAGGCCCAGAGACTGCTGTACAGAGATGTGATGCTGGAGAAC TTCGCCCTGATCACCGCCCTGGTGTGCTGGCACGGCATGGAGGACGAGGAGAC CCCCGAGCAGAGCGTGAGCGTGGAGGGC (SEQ ID NO: 974) | 6.97472813 |
| ZNF92 | PLTFRDVKIEFSLE EWQCLDTAQRN LYRDVMLENYRN LVFL (SEQ ID NO: 159) | GPLTFRDVKIEFSLEEWQCLDTA QRNLYRDVMLENYRNLVFLGIA VSKPDLITWLEQGKEPWNLKR HEMVDKTPVMCSHF (SEQ ID NO: 160) | GGCCCCCTGACCTTCAGAGATGTGAAGATCGAGTTCAGCCTGGAGGAGTGGCA GTGCCTGGACACCGCCCAGAGAAACCTGTACAGAGATGTGATGCTGGAGAACT ACAGAAACCTGGTGTTCCTGGGCATCGCCGTGAGCAAGCCCGACCTGATCACCT GGCTGGAGCAGGGCAAGGAGCCCTGGAACCTGAAGAGACACGAGATGGTGGA CAAGACCCCCGTGATGTGCAGCCACTTC (SEQ ID NO: 975) | 6.97138149 |
| ZN100 | PLTFRDVAIEFSLE EWQCLDSAQQG LYRKVMLENYRN LVF (SEQ ID NO: 161) | SGCPGAERSLLVQSYFEKGPLTF RDVAIEFSLEEWQCLDSAQQGL YRKVMLENYRNLVFLAGIALTKP DLITCLEQGKEP (SEQ ID NO: 162) | AGCGGCTGCCCCGGCGCCGAGAGAAGCCTGCTGGTGCAGAGCTACTTCGAGAA GGGCCCCCTGACCTTCAGAGATGTGGCCATCGAGTTCAGCCTGGAGGAGTGGC AGTGCCTGGACAGCGCCCAGCAGGGCCTGTACAGAAAGGTGATGCTGGAGAAC TACAGAAACCTGGTGTTCCTGGCCGGCATCGCCCTGACCAAGCCCGACCTGATC ACCTGCCTGGAGCAGGGCAAGGAGCCC (SEQ ID NO: 976) | 6.9692141 |
| ZN736 | VLTFRDVAVEFSP EEWECLDSAQQR LYRDVMLENYGN LVSL (SEQ ID NO: 163) | GVLTFRDVAVEFSPEEWECLDS AQQRLYRDVMLENYGNLVSLG LAIFKPDLMTCLEQRKEPWKVK RQEAVAKHPAGSFHF (SEQ ID NO: 164) | GGCGTGCTGACCTTCAGAGATGTGGCCGTGGAGTTCAGCCCCGAGGAGTGGGA GTGCCTGGACAGCGCCCAGCAGAGACTGTACAGAGATGTGATGCTGGAGAACT ACGGCAACCTGGTGAGCCTGGGCCTGGCCATCTTCAAGCCCGACCTGATGACCT GCCTGGAGCAGAGAAAGGAGCCCTGGAAGGTGAAGAGACAGGAGGCCGTGGC CAAGCACCCCGCCGGCAGCTTCCACTTC (SEQ ID NO: 977) | 6.95843452 |
| ZNF74 | SVSFKDVAVDFT QEEWGQLDSPQ RALYRDVMLENY QNLLAL (SEQ ID NO: 165) | KENLEDISGWGLPEARSKESVSF KDVAVDFTQEEWGQLDSPQRA LYRDVMLENYQNLLALGPPLHK PDVISHLERGEEPW (SEQ ID NO: 166) | AAGGAGAACCTGGAGGACATCAGCGGCTGGGGCCTGCCCGAGGCCAGAAGCA AGGAGAGCGTGAGCTTCAAGGACGTGGCCGTGGACTTCACCCAGGAGGAGTG GGGCCAGCTGGACAGCCCCCAGAGAGCCCTGTACAGAGATGTGATGCTGGAGA ACTACCAGAACCTGCTGGCCCTGGGCCCCCCCCTGCACAAGCCCGACGTGATCA GCCACCTGGAGAGAGGCGAGGAGCCCTGG (SEQ ID NO: 978) | 6.95809395 |
| CBX1 | EPERIIGATDSSG ELMFLMKWKNS DEADLVPAKEAN VKCPQVVISFYEE RLT (SEQ ID NO: 167) | EESEKPRGFARGLEPERIIGATDS SGELMFLMKWKNSDEADLVPA KEANVKCPQVVISFYEERLTWH SYPSEDDDKKDDK (SEQ ID NO: 168) | GAGGAGAGCGAGAAGCCCAGAGGCTTCGCCAGAGGCCTGGAGCCCGAGAGAA TCATCGGCGCCACCGACAGCAGCGGCGAGCTGATGTTCCTGATGAAGTGGAAG AACAGCGACGAGGCCGACCTGGTGCCCGCCAAGGAGGCCAACGTGAAGTGCCC CCAGGTGGTGATCAGCTTCTACGAGGAGAGACTGACCTGGCACAGCTACCCCAG CGAGGACGACGACAAGAAGGACGACAAG (SEQ ID NO: 979) | 6.95269512 |
| ZN443 | SVALEDVAVNFT REEWALLGPCQK NLYKDVMQETIR NLDC (SEQ ID NO: 169) | ASVALEDVAVNFTREEWALLGP CQKNLYKDVMQETIRNLDCVV MKWKDQNIEDQYRYPRKNLRC RMLERFVESKDGTQCG (SEQ ID NO: 170) | GCCAGCGTGGCCCTGGAGGACGTGGCCGTGAACTTCACCAGAGAGGAGTGGGC CCTGCTGGGCCCCTGCCAGAAGAACCTGTACAAGGACGTGATGCAGGAGACCA TCAGAAACCTGGACTGCGTGGTGATGAAGTGGAAGGACCAGAACATCGAGGAC CAGTACAGATACCCCAGAAAGAACCTGAGATGCAGAATGCTGGAGAGATTCGT GGAGAGCAAGGACGGCACCCAGTGCGGC (SEQ ID NO: 980) | 6.94561303 |
| ZN195 | LLTFRDVAIEFSLE EWKCLDLAQQN LYRDVMLENYRN LFSV (SEQ ID NO: 171) | TLLTFRDVAIEFSLEEWKCLDLA QQNLYRDVMLENYRNLFSVGL TVCKPGLITCLEQRKEPWNVKR QEAADGHPEMGFHH (SEQ ID NO: 172) | ACCCTGCTGACCTTCAGAGATGTGGCCATCGAGTTCAGCCTGGAGGAGTGGAA GTGCCTGGACCTGGCCCAGCAGAACCTGTACAGAGATGTGATGCTGGAGAACT ACAGAAACCTGTTCAGCGTGGGCCTGACCGTGTGCAAGCCCGGCCTGATCACCT GCCTGGAGCAGAGAAAGGAGCCCTGGAACGTGAAGAGACAGGAGGCCGCCGA CGGCCACCCCGAGATGGGCTTCCACCAC (SEQ ID NO: 981) | 6.9432522 |
| ZN530 | FVAFEDVAIYFSQ EEWELLDEMQRL LYRDVMLENFAV MASL (SEQ ID NO: 173) | AAALRAPTQQVFVAFEDVAIYF SQEEWELLDEMQRLLYRDVML ENFAVMASLGCWCGAVDEGT PSAESVSVEELSQGRTP (SEQ ID NO: 174) | GCCGCCGCCCTGAGAGCCCCCACCCAGCAGGTGTTCGTGGCCTTCGAGGACGTG GCCATCTACTTCAGCCAGGAGGAGTGGGAGCTGCTGGACGAGATGCAGAGACT GCTGTACAGAGATGTGATGCTGGAGAACTTCGCCGTGATGGCCAGCCTGGGCT GCTGGTGCGGCGCCGTGGACGAGGGAACACCTAGCGCCGAGAGCGTGAGCGT GGAGGAGCTGAGCCAGGGCAGAACCCCC (SEQ ID NO: 982) | 6.94292737 |
| ZN782 | SVSFQDVTVEFS QEEWQHMGPV ERTLYRDVMLEN YSHLVSV (SEQ ID NO: 175) | NTFQASVSFQDVTVEFSQEEW QHMGPVERTLYRDVMLENYSH LVSVGYCFTKPELIFTLEQGEDP WLLEKEKGFLSRNSP (SEQ ID NO: 176) | AACACCTTCCAGGCCAGCGTGAGCTTCCAGGACGTGACCGTGGAGTTCAGCCAG GAGGAGTGGCAGCACATGGGCCCGTGGAGAGAACCCTGTACAGAGATGTGAT GCTGGAGAACTACAGCCACCTGGTGAGCGTGGGCTACTGCTTCACCAAGCCCGA GCTGATCTTCACCCTGGAGCAGGGCGAGGACCCCTGGCTGCTGGAGAAGGAGA AGGGCTTCCTGAGCAGAAACAGCCCC (SEQ ID NO: 983) | 6.94217051 |
| ZN791 | SVAFEDVSVSFSQ EEWALLAPSQKK LYRDVMQETFKN LASI (SEQ ID NO: 177) | DSVAFEDVSVSFSQEEWALLAP SQKKLYRDVMQETFKNLASIGE KWEDPNVEDQHKNQGRNLRS HTGERLCEGKEGSQCA (SEQ ID NO: 178) | GACAGCGTGGCCTTCGAGGACGTGAGCGTGAGCTTCAGCCAGGAGGAGTGGG CCCTGCTGGCCCCCAGCCAGAAGAAGCTGTACAGAGATGTGATGCAGGAGACC TTCAAGAACCTGGCCAGCATCGGCGAGAAGTGGGAGGACCCCAACGTGGAGGA CCAGCACAAGAACCAGGGCAGAAACCTGAGAAGCCACACCGGCGAGAGACTGT GCGAGGGCAAGGAGGGCAGCCAGTGCGCC (SEQ ID NO: 984) | 6.93320479 |
| ZN331 | LVTFADVAIDFSQ EEWACLNSAQR DLYWDVMLENY SNLVSL (SEQ ID NO: 179) | AQGLVTFADVAIDFSQEEWACL NSAQRDLYWDVMLENYSNLVS LDLESAYENKSLPTEKNIHEIRAS KRNSDRRSKSLGR (SEQ ID NO: 180) | GCCCAGGGCCTGGTGACCTTCGCCGACGTGGCCATCGACTTCAGCCAGGAGGA GTGGGCCTGCCTGAACAGCGCCCAGAGAGACCTGTACTGGGACGTGATGCTGG AGAACTACAGCAACCTGGTGAGCCTGGACCTGGAGAGCGCCTACGAGAACAAG AGCCTGCCCACCGAGAAGAACATCCACGAGATCAGAGCCAGCAAGAGAAACAG CGACAGAAGAAGCAAGAGCCTGGGCAGA (SEQ ID NO: 985) | 6.92979428 |
| Z354C | PVTFRDVAVFFS QDEWLHLDSAQ RALYREVMLENY SSLVSL (SEQ ID NO: 181) | AVDLLSAQEPVTFRDVAVFFSQ DEWLHLDSAQRALYREVMLEN YSSLVSLGIPFSMPKLIHQLQQG EDPCMVEREVPSDT (SEQ ID NO: 182) | GCCGTGGACCTGCTGAGCGCCCAGGAGCCCGTGACCTTCAGAGATGTGGCCGT GTTCTTCAGCCAGGACGAGTGGCTGCACCTGGACAGCGCCCAGAGAGCCCTGTA CAGAGAGGTGATGCTGGAGAACTACAGCAGCCTGGTGAGCCTGGGCATCCCCT TCAGCATGCCCAAGCTGATCCACCAGCTGCAGCAGGGCGAGGACCCCTGCATG GTGGAGAGAGAGGTGCCCAGCGACACC (SEQ ID NO: 986) | 6.92855271 |
| ZN157 | SVSFEDVAVDFTR QEWHRLDPAQR TMHKDVMLETYS NLASV (SEQ ID NO: 183) | SPQRFPALIPGEPGRSFEGSVSF EDVAVDFTRQEWHRLDPAQRT MHKDVMLETYSNLASVGLCVA KPEMIFKLERGEELW (SEQ ID NO: 184) | AGCCCCCAGAGATTCCCCGCCCTGATCCCCGGCGAGCCCGGCAGAAGCTTCGAG GGCAGCGTGAGCTTCGAGGACGTGGCCGTGGACTTCACCAGACAGGAGTGGCA CAGACTGGACCCCGCCCAGAGAACCATGCACAAGGACGTGATGCTGGAGACCT ACAGCAACCTGGCCAGCGTGGGCCTGTGCGTGGCCAAGCCCGAGATGATCTTCA AGCTGGAGAGAGGCGAGGAGCTGTGG (SEQ ID NO: 987) | 6.92764017 |
| ZN727 | VLTFRDVAVEFSP EEWECLDSAQQR LYRDVMLENYGN LFSL (SEQ ID NO: 185) | RVLTFRDVAVEFSPEEWECLDS AQQRLYRDVMLENYGNLFSLG LAIFKPDLITYLEQRKEPWNARR QKTVAKHPAGSLHF (SEQ ID NO: 186) | AGAGTGCTGACCTTCAGAGATGTGGCCGTGGAGTTCAGCCCCGAGGAGTGGGA GTGCCTGGACAGCGCCCAGCAGAGACTGTACAGAGATGTGATGCTGGAGAACT ACGGCAACCTGTTCAGCCTGGGCCTGGCCATCTTCAAGCCCGACCTGATCACCTA CCTGGAGCAGAGAAAGGAGCCCTGGAACGCCAGAAGACAGAAGACCGTGGCC AAGCACCCCGCCGGCAGCCTGCACTTC (SEQ ID NO: 988) | 6.9257026 |
| ZN550 | LVTFKDVAVTFTR EEWRQLDLAQRT LYREVMLETCGLL VSL (SEQ ID NO: 187) | AETKDAAQMLVTFKDVAVTFT REEWRQLDLAQRTLYREVMLE TCGLLVSLGHRVPKPELVHLLEH GQELWIVKRGLSHAT (SEQ ID NO: 188) | GCCGAGACCAAGGACGCCGCCCAGATGCTGGTGACCTTCAAGGACGTGGCCGT GACCTTCACCAGAGAGGAGTGGAGACAGCTGGACCTGGCCCAGAGAACCCTGT ACAGAGAGGTGATGCTGGAGACCTGCGGCCTGCTGGTGAGCCTGGGCCACAGA GTGCCCAAGCCCGAGCTGGTGCACCTGCTGGAGCACGGCCAGGAGCTGTGGAT CGTGAAGAGAGGCCTGAGCCACGCCACC (SEQ ID NO: 989) | 6.92403295 |
| ZN793 | PVSFKDVVVGFT QEEWHRLSPAQ RALYRDVMLETY SNLVSV (SEQ ID NO: 189) | IEYQIPVSFKDVVVGFTQEEWH RLSPAQRALYRDVMLETYSNLV SVGYEGTKPDVILRLEQEEAPWI GEAACPGCHCWED (SEQ ID NO: 190) | ATCGAGTACCAGATCCCCGTGAGCTTCAAGGACGTGGTGGTGGGCTTCACCCAG GAGGAGTGGCACAGACTGAGCCCCGCCCAGAGAGCCCTGTACAGAGATGTGAT GCTGGAGACCTACAGCAACCTGGTGAGCGTGGGCTACGAGGGCACCAAGCCCG ACGTGATCCTGAGACTGGAGCAGGAGGAGGCCCCCTGGATCGGCGAGGCCGCT TGCCCTGGATGCCACTGCTGGGAGGAC (SEQ ID NO: 990) | 6.92326085 |
| ZN235 | AVTFKDVAVAFT EEELGLLDSAQRK LYRDVMLENFRN LVSV (SEQ ID NO: 191) | TKFQEAVTFKDVAVAFTEEELGL LDSAQRKLYRDVMLENFRNLVS VGHQSFKPDMISQLEREEKLW MKELQTQRGKHSGD (SEQ ID NO: 192) | ACCAAGTTCCAGGAGGCCGTGACCTTCAAGGACGTGGCCGTGGCCTTCACCGAG GAGGAGCTGGGCCTGCTGGACAGCGCCCAGAGAAAGCTGTACAGAGATGTGAT GCTGGAGAACTTCAGAAACCTGGTGAGCGTGGGCCACCAGAGCTTCAAGCCCG ACATGATCAGCCAGCTGGAGAGAGAGGAGAAGCTGTGGATGAAGGAGCTGCA GACCCAGAGAGGCAAGCACAGCGGCGAC (SEQ ID NO: 991) | 6.91826902 |
| ZNF8 | PVTFRDVAVDFT QEEWGQLDPTQ RILYRDVMLETFG HLLSI (SEQ ID NO: 193) | DEGVAGVMSVGPPAARLQEPV TFRDVAVDFTQEEWGQLDPTQ RILYRDVMLETFGHLLSIGPELPK PEVISQLEQGTELW (SEQ ID NO: 194) | GACGAGGGCGTGGCCGGCGTGATGAGCGTGGGCCCCCCGCTGCTAGACTTCA GGAGCCCGTGACCTTCAGAGATGTGGCCGTGGACTTCACCCAGGAGGAGTGGG GCCAGCTGGACCCCACCCAGAGAATCCTGTACAGAGATGTGATGCTGGAGACCT TCGGCCACCTGCTGAGCATCGGCCCCGAGCTGCCCAAGCCCGAGGTGATCAGCC AGCTGGAGCAGGGCACCGAGCTGTGG(SEQ ID NO: 992) | 6.91722882 |
| ZN724 | PLTFMDVAIEFSV EEWQCLDTAQQ NLYRNVMLENYR NLVFL (SEQ ID NO: 195) | GPLTFMDVAIEFSVEEWQCLDT AQQNLYRNVMLENYRNLVFLGI AVSKPDLITCLEQGKEPWNME RHEMVAKPPGMCCYF (SEQ ID NO: 196) | GGCCCCCTGACCTTCATGGACGTGGCCATCGAGTTCAGCGTGGAGGAGTGGCA GTGCCTGGACACCGCCCAGCAGAACCTGTACAGAAACGTGATGCTGGAGAACT ACAGAAACCTGGTGTTCCTGGGCATCGCCGTGAGCAAGCCCGACCTGATCACCT GCCTGGAGCAGGGCAAGGAGCCCTGGAACATGGAGAGACACGAGATGGTGGC CAAGCCCCCCGGCATGTGCTGCTACTTC (SEQ ID NO: 993) | 6.89904065 |
| ZN573 | LVTFRDVAIDFSR QEWEYLDPNQR DLYRDVMLENYR NLVSL (SEQ ID NO: 197) | HQVGLIRSYNSKTMTCFQELVT FRDVAIDFSRQEWEYLDPNQR DLYRDVMLENYRNLVSLGGHSI SKPVVVDLLERGKEP (SEQ ID NO: 198) | CACCAGGTGGGCCTGATCAGAAGCTACAACAGCAAGACCATGACCTGCTTCCAG GAGCTGGTGACCTTCAGAGATGTGGCCATCGACTTCAGCAGACAGGAGTGGGA GTACCTGGACCCCAACCAGAGAGACCTGTACAGAGATGTGATGCTGGAGAACT ACAGAAACCTGGTGAGCCTGGGCGGCCACAGCATCAGCAAGCCCGTGGTGGTG GACCTGCTGGAGAGAGGCAAGGAGCCC (SEQ ID NO: 994) | 6.89366942 |
| ZN577 | SLSFEDVAVGFTR EEWQFLDQSQK VLYKEVMLENYIN LVSI (SEQ ID NO: 199) | NATIVMSVRREQGSSSGEGSLS FEDVAVGFTREEWQFLDQSQK VLYKEVMLENYINLVSIGYRGTK PDSLFKLEQGEPPG (SEQ ID NO: 200) | AACGCCACCATCGTGATGAGCGTGAGAAGAGAGCAGGGCAGCAGCAGCGGCG AGGGCAGCCTGAGCTTCGAGGACGTGGCCGTGGGCTTCACCAGAGAGGAGTG GCAGTTCCTGGACCAGAGCCAGAAGGTGCTGTACAAGGAGGTGATGCTGGAGA ACTACATCAACCTGGTGAGCATCGGCTACAGAGGCACCAAGCCCGACAGCCTGT TCAAGCTGGAGCAGGGCGAGCCCCCGGC (SEQ ID NO: 995) | 6.89093009 |
| ZN789 | LLSFEDVAMYFTR EEWGHLNWGQ KDLYRDVMLENY RNMVLL (SEQ ID NO: 201) | FPPARGKELLSFEDVAMYFTREE WGHLNWGQKDLYRDVMLENY RNMVLLGFQFPKPEMICQLEN WDEQWILDLPRTGNRK (SEQ ID NO: 202) | TTCCCCCCGCCAGAGGCAAGGAGCTGCTGAGCTTCGAGGACGTGGCCATGTAC TTCACCAGAGAGGAGTGGGGCCACCTGAACTGGGGCCAGAAGGACCTGTACAG AGATGTGATGCTGGAGAACTACAGAAACATGGTGCTGCTGGGCTTCCAGTTCCC CAAGCCCGAGATGATCTGCCAGCTGGAGAACTGGGACGAGCAGTGGATCCTGG ACCTGCCCAGAACCGGCAACAGAAAG (SEQ ID NO: 996) | 6.88877268 |
| ZN718 | LLTFKDVAIEFSPE EWKCLDTSQQNL YRDVMLENYRNL VSL (SEQ ID NO: 203) | ELLTFKDVAIEFSPEEWKCLDTS QQNLYRDVMLENYRNLVSLGV SISNPDLVTSLEQRKEPYNLKIHE TAARPPAVCSHF (SEQ ID NO: 204) | GAGCTGCTGACCTTCAAGGACGTGGCCATCGAGTTCAGCCCCGAGGAGTGGAA GTGCCTGGACACCAGCCAGCAGAACCTGTACAGAGATGTGATGCTGGAGAACT ACAGAAACCTGGTGAGCCTGGGCGTGAGCATCAGCAACCCCGACCTGGTGACC AGCCTGGAGCAGAGAAAGGAGCCCTACAACCTGAAGATCCACGAGACCGCCGC CAGACCCCCCGCCGTGTGCAGCCACTTC (SEQ ID NO: 997) | 6.87598723 |
| ZN300 | LVSFKDVAVDFT QEEWQQLDPSQ RTLYRDVMLENY SHLVSM (SEQ ID NO: 205) | MKSQGLVSFKDVAVDFTQEEW QQLDPSQRTLYRDVMLENYSH LVSMGYPVSKPDVISKLEQGEE PWIIKGDISNWIYPDE (SEQ ID NO: 206) | ATGAAGAGCCAGGGCCTGGTGAGCTTCAAGGACGTGGCCGTGGACTTCACCCA GGAGGAGTGGCAGCAGCTGGACCCCAGCCAGAGAACCCTGTACAGAGATGTGA TGCTGGAGAACTACAGCCACCTGGTGAGCATGGGCTACCCCGTGAGCAAGCCC GACGTGATCAGCAAGCTGGAGCAGGGCGAGGAGCCCTGGATCATCAAGGGCG ACATCAGCAACTGGATCTACCCCGACGAG (SEQ ID NO: 998) | 6.87019452 |
| ZN383 | SVMFSDVSIDFS QEEWDCLDPVQ RDLYRDVMLENY GNLVSM (SEQ ID NO: 207) | AEGSVMFSDVSIDFSQEEWDCL DPVQRDLYRDVMLENYGNLVS MGLYTPKPQVISLLEQGKEPW MVGRELTRGLCSDLES (SEQ ID NO: 208) | GCCGAGGGCAGCGTGATGTTCAGCGACGTGAGCATCGACTTCAGCCAGGAGGA GTGGGACTGCCTGGACCCCGTGCAGAGAGACCTGTACAGAGATGTGATGCTGG AGAACTACGGCAACCTGGTGAGCATGGGCCTGTACACCCCCAAGCCCCAGGTG ATCAGCCTGCTGGAGCAGGGCAAGGAGCCCTGGATGGTGGGCAGAGAGCTGA CCAGAGGCCTGTGCAGCGACCTGGAGAGC (SEQ ID NO: 999) | 6.86203801 |
| ZN429 | PLTFTDVAIEFSLE EWQCLDTAQQN LYRNVMLENYRN LVFL (SEQ ID NO: 209) | GPLTFTDVAIEFSLEEWQCLDTA QQNLYRNVMLENYRNLVFLGIA VSKPDLITCLEKEKEPCKMKRHE MVDEPPVVCSHF (SEQ ID NO: 210) | GGCCCCCTGACCTTCACCGACGTGGCCATCGAGTTCAGCCTGGAGGAGTGGCAG TGCCTGGACACCGCCCAGCAGAACCTGTACAGAAACGTGATGCTGGAGAACTAC AGAAACCTGGTGTTCCTGGGCATCGCCGTGAGCAAGCCCGACCTGATCACCTGC CTGGAGAAGGAGAAGGAGCCCTGCAAGATGAAGAGACACGAGATGGTGGACG AGCCCCCCGTGGTGTGCAGCCACTTC (SEQ ID NO: 1000) | 6.85768103 |
| ZN677 | LFTFKDVAIEFSQ EEWECLDPAQRA LYRDVMLENYRN LLSL (SEQ ID NO: 211) | ALSQGLFTFKDVAIEFSQEEWE CLDPAQRALYRDVMLENYRNLL SLDEDNIPPEDDISVGFTSKGLS PKENNKEELYHLV (SEQ ID NO: 212) | GCCCTGAGCCAGGGCCTGTTCACCTTCAAGGACGTGGCCATCGAGTTCAGCCAG GAGGAGTGGGAGTGCCTGGACCCCGCCCAGAGAGCCCTGTACAGAGATGTGAT GCTGGAGAACTACAGAAACCTGCTGAGCCTGGACGAGGACAACATCCCCCCCG AGGACGACATCAGCGTGGGCTTCACCAGCAAGGGCCTGAGCCCCAAGGAGAAC AACAAGGAGGAGCTGTACCACCTGGTG (SEQ ID NO: 1001) | 6.85440091 |
| ZN850 | LVMFQDLSIDFS QEEWECLDAAQ KDLYRDVMMEN YSSLVSL (SEQ ID NO: 213) | NMEGLVMFQDLSIDFSQEEWE CLDAAQKDLYRDVMMENYSSL VSLGLSIPKPDVISLLEQGKEPW MVSRDVLGGWCRDSE (SEQ ID NO: 214) | AACATGGAGGGCCTGGTGATGTTCCAGGACCTGAGCATCGACTTCAGCCAGGA GGAGTGGGAGTGCCTGGACGCCGCCCAGAAGGACCTGTACAGAGATGTGATGA TGGAGAACTACAGCAGCCTGGTGAGCCTGGGCCTGAGCATCCCCAAGCCCGAC GTGATCAGCCTGCTGGAGCAGGGCAAGGAGCCCTGGATGGTGAGCAGAGATGT GCTGGGCGGCTGGTGCAGAGACAGCGAG (SEQ ID NO: 1002) | 6.85293565 |
| ZN454 | SVTFKDVAILFTQ EEWGQLSPAQR ALYRDVMLENYS NLVSL (SEQ ID NO: 215) | AVSHLPTMVQESVTFKDVAILF TQEEWGQLSPAQRALYRDVML ENYSNLVSLGLLGPKPDTFSQLE KREVWMPEDTPGGF (SEQ ID NO: 216) | GCCGTGAGCCACCTGCCCACCATGGTGCAGGAGAGCGTGACCTTCAAGGACGT GGCCATCCTGTTCACCCAGGAGGAGTGGGGCCAGCTGAGCCCCGCCCAGAGAG CCCTGTACAGAGATGTGATGCTGGAGAACTACAGCAACCTGGTGAGCCTGGGC CTGCTGGGCCCCAAGCCCGACACCTTCAGCCAGCTGGAGAAGAGAGAGGTGTG GATGCCCGAGGACACCCCCGGCGGCTTC (SEQ ID NO: 1003) | 6.8342036 |
| ZN257 | PLTIRDVTVEFSLE EWHCLDTAQQN LYRDVMLENYRN LVFL (SEQ ID NO: 217) | GPLTIRDVTVEFSLEEWHCLDTA QQNLYRDVMLENYRNLVFLGIA VSKPDLITCLEQGKEPCNMKRH EMVAKPPVMCSHI (SEQ ID NO: 218) | GGCCCCCTGACCATCAGAGATGTGACCGTGGAGTTCAGCCTGGAGGAGTGGCA CTGCCTGGACACCGCCCAGCAGAACCTGTACAGAGATGTGATGCTGGAGAACTA CAGAAACCTGGTGTTCCTGGGCATCGCCGTGAGCAAGCCCGACCTGATCACCTG CCTGGAGCAGGGCAAGGAGCCCTGCAACATGAAGAGACACGAGATGGTGGCC AAGCCCCCGTGATGTGCAGCCACATC (SEQ ID NO: 1004) | 6.83044 |
| ZN264 | SVTFDDVAVTFTK EEWGQLDLAQR TLYQEVMLENCG LLVSL (SEQ ID NO: 219) | AAAVLTDRAQVSVTFDDVAVTF TKEEWGQLDLAQRTLYQEVML ENCGLLVSLGCPVPKAELICHLE HGQEPWTRKEDLSQ (SEQ ID NO: 220) | GCCGCCGCCGTGCTGACCGACAGAGCCCAGGTGAGCGTGACCTTCGACGACGT GGCCGTGACCTTCACCAAGGAGGAGTGGGGCCAGCTGGACCTGGCCCAGAGAA CCCTGTACCAGGAGGTGATGCTGGAGAACTGCGGCCTGCTGGTGAGCCTGGGC TGCCCCGTGCCCAAGGCCGAGCTGATCTGCCACCTGGAGCACGGCCAGGAGCC CTGGACCAGAAAGGAGGACCTGAGCCAG (SEQ ID NO: 1005) | 6.82889596 |
| ZFP82 | SVMFSDVSIDFSP EEWEYLDLEQKD LYRDVMLENYSN LVSL (SEQ ID NO: 221) | ALRSVMFSDVSIDFSPEEWEYL DLEQKDLYRDVMLENYSNLVSL GCFISKPDVISSLEQGKEPWKVV RKGRRQYPDLETK (SEQ ID NO: 222) | GCCCTGAGAAGCGTGATGTTCAGCGACGTGAGCATCGACTTCAGCCCCGAGGA GTGGGAGTACCTGGACCTGGAGCAGAAGGACCTGTACAGAGATGTGATGCTGG AGAACTACAGCAACCTGGTGAGCCTGGGCTGCTTCATCAGCAAGCCCGACGTGA TCAGCAGCCTGGAGCAGGGCAAGGAGCCCTGGAAGGTGGTGAGAAAGGGCAG AAGACAGTACCCCGACCTGGAGACCAAG (SEQ ID NO: 1006) | 6.82733193 |
| ZFP14 | SVTFRDVAIDFSQ EEWEFLDPAQRD LYRDVMWENYS NFISL (SEQ ID NO: 223) | AHGSVTFRDVAIDFSQEEWEFL DPAQRDLYRDVMWENYSNFIS LGPSISKPDVITLLDEERKEPGM VVREGTRRYCPDLE (SEQ ID NO: 224) | GCCCACGGCAGCGTGACCTTCAGAGATGTGGCCATCGACTTCAGCCAGGAGGA GTGGGAGTTCCTGGACCCCGCCCAGAGAGACCTGTACAGAGATGTGATGTGGG AGAACTACAGCAACTTCATCAGCCTGGGCCCCAGCATCAGCAAGCCCGACGTGA TCACCCTGCTGGACGAGGAGAGAAAGGAGCCCGGCATGGTGGTGAGAGAGGG CACCAGAAGATACTGCCCCGACCTGGAG (SEQ ID NO: 1007) | 6.81312035 |
| ZN485 | PLTFGDVAVAFT RIEWRHLDAAQR ALYRDVMLENYG NLVSV (SEQ ID NO: 225) | APRAQIQGPLTFGDVAVAFTRI EWRHLDAAQRALYRDVMLENY GNLVSVGLLSSKPKLITQLEQGA EPWTEVREAPSGTH (SEQ ID NO: 226) | GCCCCCAGAGCCCAGATCCAGGGCCCCCTGACCTTCGGCGACGTGGCCGTGGCC TTCACCAGAATCGAGTGGAGACACCTGGACGCCGCCCAGAGAGCCCTGTACAG AGATGTGATGCTGGAGAACTACGGCAACCTGGTGAGCGTGGGCCTGCTGAGCA GCAAGCCCAAGCTGATCACCCAGCTGGAGCAGGGCGCCGAGCCCTGGACCGAG GTGAGAGAGGCCCCTAGCGGCACACAC (SEQ ID NO: 1008) | 6.81172703 |
| ZN737 | PLQFRDVAIEFSL EEWHCLDTAQR NLYRNVMLENYR NLVFL (SEQ ID NO: 227) | GPLQFRDVAIEFSLEEWHCLDT AQRNLYRNVMLENYRNLVFLGI VVSKPDLITCLEQGKKPLTMKK HEMVANPSVTCSHF (SEQ ID NO: 228) | GGCCCCCTGCAGTTCAGAGATGTGGCCATCGAGTTCAGCCTGGAGGAGTGGCA CTGCCTGGACACCGCCCAGAGAAACCTGTACAGAAACGTGATGCTGGAGAACT ACAGAAACCTGGTGTTCCTGGGCATCGTGGTGAGCAAGCCCGACCTGATCACCT GCCTGGAGCAGGGCAAGAAGCCCCTGACCATGAAGAAGCACGAGATGGTGGC CAACCCCAGCGTGACCTGCAGCCACTTC (SEQ ID NO: 1009) | 6.80882457 |
| ZNF44 | SVAFEDVAVNFT HEEWALLGPSQK NLYRDVMRETIR NLNCI (SEQ ID NO: 229) | TLPRGQPEVLEWGLPKDQDSV AFEDVAVNFTHEEWALLGPSQ KNLYRDVMRETIRNLNCIGMK WENQNIDDQHQNLRRNP (SEQ ID NO: 230) | ACCCTGCCCAGAGGCCAGCCCGAGGTGCTGGAGTGGGGCCTGCCCAAGGACCA GGACAGCGTGGCCTTCGAGGACGTGGCCGTGAACTTCACCCACGAGGAGTGGG CCCTGCTGGGCCCCAGCCAGAAGAACCTGTACAGAGATGTGATGAGAGAGACC ATCAGAAACCTGAACTGCATCGGCATGAAGTGGGAGAACCAGAACATCGACGA CCAGCACCAGAACCTGAGAAGAAACCCC (SEQ ID NO: 1010) | 6.80503304 |
| ZN596 | SMTFEDIIVDFTQ EEWALLDTSQRK LFQDVMLENISH LVSI (SEQ ID NO: 231) | PSPDSMTFEDIIVDFTQEEWALL DTSQRKLFQDVMLENISHLVSI GKQLCKSVVLSQLEQVEKLSTQ RISLLQGREVGIK (SEQ ID NO: 232) | CCCAGCCCCGACAGCATGACCTTCGAGGACATCATCGTGGACTTCACCCAGGAG GAGTGGGCCCTGCTGGACACCAGCCAGAGAAAGCTGTTCCAGGACGTGATGCT GGAGAACATCAGCCACCTGGTGAGCATCGGCAAGCAGCTGTGCAAGAGCGTGG TGCTGAGCCAGCTGGAGCAGGTGGAGAAGCTGAGCACCCAGAGAATCAGCCTG CTGCAGGGCAGAGAGGTGGGCATCAAG (SEQ ID NO: 1011) | 6.80500309 |
| ZN565 | LVTFRDVAIEFSLE EWKCLEPAQRDL YREVTLENFGHLA SL (SEQ ID NO: 233) | EESREIRAGQIVLKAMAQGLVT FRDVAIEFSLEEWKCLEPAQRDL YREVTLENFGHLASLGLSISKPD VVSLLEQGKEPW (SEQ ID NO: 234) | GAGGAGAGCAGAGAGATCAGAGCCGGCCAGATCGTGCTGAAGGCCATGGCCC AGGGCCTGGTGACCTTCAGAGATGTGGCCATCGAGTTCAGCCTGGAGGAGTGG AAGTGCCTGGAGCCCGCCCAGAGAGACCTGTACAGAGAGGTGACCCTGGAGAA CTTCGGCCACCTGGCCAGCCTGGGCCTGAGCATCAGCAAGCCCGACGTGGTGA GCCTGCTGGAGCAGGGCAAGGAGCCCTGG(SEQ ID NO: 1012) | 6.80375161 |
| ZN543 | SVTFEDVAVTFT QEEWGQLDAAQ RTLYQEVMLETC GLLMSL (SEQ ID NO: 235) | AASAQVSVTFEDVAVTFTQEE WGQLDAAQRTLYQEVMLETC GLLMSLGCPLFKPELIYQLDHRQ ELWMATKDLSQSSYPG (SEQ ID NO: 236) | GCCGCCAGCGCCCAGGTGAGCGTGACCTTCGAGGACGTGGCCGTGACCTTCACC CAGGAGGAGTGGGGCCAGCTGGACGCCGCCCAGAGAACCCTGTACCAGGAGG TGATGCTGGAGACCTGCGGCCTGCTGATGAGCCTGGGCTGCCCCCTGTTCAAGC CCGAGCTGATCTACCAGCTGGACCACAGACAGGAGCTGTGGATGGCCACCAAG GACCTGAGCCAGAGCAGCTACCCCGGC (SEQ ID NO: 1013) | 6.79786357 |
| ZFP69 | LLTFKDISIDFTQE EWGQLAPAHQN LYREVMLENYSN LVSV (SEQ ID NO: 237) | RESLEDEVTPGLPTAESQELLTF KDISIDFTQEEWGQLAPAHQNL YREVMLENYSNLVSVGYQLSKP SVISQLEKGEEPW (SEQ ID NO: 238) | AGAGAGAGCCTGGAGGACGAGGTGACCCCCGGCCTGCCCACCGCCGAGAGCCA AGAGCTGCTGACCTTCAAGGACATCAGCATCGACTTCACCCAGGAGGAGTGGG GCCAGCTGGCCCCCGCCCACCAGAACCTGTACAGAGAGGTGATGCTGGAGAAC TACAGCAACCTGGTGAGCGTGGGCTACCAGCTGAGCAAGCCCAGCGTGATCAG CCAGCTGGAGAAGGGCGAGGAGCCCTGG (SEQ ID NO: 1014) | 6.79374304 |
| SUMO1 | IKLKVIGQDSSEIH FKVKMTTHLKKL KESYCQRQGVP MNSLRFLFEGQRI ADNHTPKELGME EEDVIEVY (SEQ ID NO: 239) | EGEYIKLKVIGQDSSEIHFKVKM TTHLKKLKESYCQRQGVPMNSL RFLFEGQRIADNHTPKELGMEE EDVIEVYQEQTGG (SEQ ID NO: 240) | GAGGGCGAGTACATCAAGCTGAAGGTGATCGGCCAGGACAGCAGCGAGATCC ACTTCAAGGTGAAGATGACCACCCACCTGAAGAAGCTGAAGGAGAGCTACTGC CAGAGACAGGGCGTGCCCATGAACAGCCTGAGATTCCTGTTCGAGGGCCAGAG AATCGCCGACAACCACACCCCCAAGGAGCTGGGCATGGAGGAGGAGGACGTG ATCGAGGTGTACCAGGAGCAGACCGGCGGC (SEQ ID NO: 1015) | 6.77750481 |
| ZNF12 | PVSFKDVAVDFT QEEWQQLDPEQ KITYRDVMLENYS NLVSV (SEQ ID NO: 241) | NKSLGPVSFKDVAVDFTQEEW QQLDPEQKITYRDVMLENYSNL VSVGYHIIKPDVISKLEQGEEPW IVEGEFLLQSYPDE (SEQ ID NO: 242) | AACAAGAGCCTGGGCCCCGTGAGCTTCAAGGACGTGGCCGTGGACTTCACCCA GGAGGAGTGGCAGCAGCTGGACCCCGAGCAGAAGATCACCTACAGAGATGTG ATGCTGGAGAACTACAGCAACCTGGTGAGCGTGGGCTACCACATCATCAAGCCC GACGTGATCAGCAAGCTGGAGCAGGGCGAGGAGCCCTGGATCGTGGAGGGCG AGTTCCTGCTGCAGAGCTACCCCGACGAG (SEQ ID NO: 1016) | 6.77648818 |
| ZN169 | LMAFRDVAVAFT QKEWKLLSSAQR TLYREVMLENYS HLVSL (SEQ ID NO: 243) | SPGLLTTRKEALMAFRDVAVAF TQKEWKLLSSAQRTLYREVMLE NYSHLVSLGIAFSKPKLIEQLEQG DEPWREENEHLL (SEQ ID NO: 244) | AGCCCCGGCCTGCTGACCACCAGAAAGGAGGCCCTGATGGCCTTCAGAGATGT GGCCGTGGCCTTCACCCAGAAGGAGTGGAAGCTGCTGAGCAGCGCCCAGAGAA CCCTGTACAGAGAGGTGATGCTGGAGAACTACAGCCACCTGGTGAGCCTGGGC ATCGCCTTCAGCAAGCCCAAGCTGATCGAGCAGCTGGAGCAGGGCGACGAGCC CTGGAGAGAGGAGAACGAGCACCTGCTG (SEQ ID NO: 1017) | 6.77498642 |
| ZN433 | SVAFEDVAVTFT QEEWALLDPSQK NLCRDVMQETFR NLASI (SEQ ID NO: 245) | MFQDSVAFEDVAVTFTQEEWA LLDPSQKNLCRDVMQETFRNL ASIGKKWKPQNIYVEYENLRRN LRIVGERLFESKEGHQ (SEQ ID NO: 246) | ATGTTCCAGGACAGCGTGGCCTTCGAGGACGTGGCCGTGACCTTCACCCAGGAG GAGTGGGCCCTGCTGGACCCCAGCCAGAAGAACCTGTGCAGAGATGTGATGCA GGAGACCTTCAGAAACCTGGCCAGCATCGGCAAGAAGTGGAAGCCCCAGAACA TCTACGTGGAGTACGAGAACCTGAGAAGAAACCTGAGAATCGTGGGCGAGAGA CTGTTCGAGAGCAAGGAGGGCCACCAG (SEQ ID NO: 1018) | 6.77303438 |
| SUMO3 | INLKVAGQDGSV VQFKIKRHTPLSK LMKAYCERQGLS MRQIRFRFDGQP INETDTPAQLEM EDEDTIDVF (SEQ ID NO: 247) | ENDHINLKVAGQDGSVVQFKIK RHTPLSKLMKAYCERQGLSMR QIRFRFDGQPINETDTPAQLEM EDEDTIDVFQQQTGG (SEQ ID NO: 248) | GAGAACGACCACATCAACCTGAAGGTGGCCGGCCAGGACGGCAGCGTGGTGCA GTTCAAGATCAAGAGACACACCCCCCTGAGCAAGCTGATGAAGGCCTACTGCGA GAGACAGGGCCTGAGCATGAGACAGATCAGATTCAGATTCGACGGCCAGCCCA TCAACGAGACCGACACCCCCGCCCAGCTGGAGATGGAGGACGAGGACACCATC GACGTGTTCCAGCAGCAGACCGGCGGC (SEQ ID NO: 1019) | 6.76493545 |
| ZNF98 | VLTFRDVALEFSL EEWQCLDTAQQ NLYRNVMLENYR NLVFV (SEQ ID NO: 249) | PGPLGSLEMGVLTFRDVALEFSL EEWQCLDTAQQNLYRNVMLE NYRNLVFVGIAASKPDLITCLEQ GKEPWNVKRHEMVT (SEQ ID NO: 250) | CCCGGCCCCCTGGCAGCCTGGAGATGGGCGTGCTGACCTTCAGAGATGTGGC CCTGGAGTTCAGCCTGGAGGAGTGGCAGTGCCTGGACACCGCCCAGCAGAACC TGTACAGAAACGTGATGCTGGAGAACTACAGAAACCTGGTGTTCGTGGGCATC GCCGCCAGCAAGCCCGACCTGATCACCTGCCTGGAGCAGGGCAAGGAGCCCTG GAACGTGAAGAGACACGAGATGGTGACC (SEQ ID NO: 1020) | 6.76469777 |
| ZN175 | SVSFEDVTVDFSR EEWQQLDPAQR CLYRDVMLELYS HLFAV (SEQ ID NO: 251) | LSQKPQVLGPEKQDGSCEASVS FEDVTVDFSREEWQQLDPAQR CLYRDVMLELYSHLFAVGYHIP NPEVIFRMLKEKEPR (SEQ ID NO: 252) | CTGAGCCAGAAGCCCAGGTGCTGGGCCCCGAGAAGCAGGACGGCAGCTGCG AGGCTAGCGTGAGCTTCGAGGACGTGACCGTGGACTTCAGCAGAGAGGAGTGG CAGCAGCTGGACCCCGCCCAGAGATGCCTGTACAGAGATGTGATGCTGGAGCT GTACAGCCACCTGTTCGCCGTGGGCTACCACATCCCCAACCCCGAGGTGATCTTC AGAATGCTGAAGGAGAAGGAGCCCAGA (SEQ ID NO: 1021) | 6.76307142 |
| ZN347 | QVTFRDVAIEFSQ EEWTCLDPAQRT LYRDVMLENYRN LASL (SEQ ID NO: 253) | ALTQGQVTFRDVAIEFSQEEWT CLDPAQRTLYRDVMLENYRNL ASLGISCFDLSIISMLEQGKEPFT LESQVQIAGNPDG (SEQ ID NO: 254) | GCCCTGACCCAGGGCCAGGTGACCTTCAGAGATGTGGCCATCGAGTTCAGCCAG GAGGAGTGGACCTGCCTGGACCCCGCCCAGAGAACCCTGTACAGAGATGTGAT GCTGGAGAACTACAGAAACCTGGCCAGCCTGGGCATCAGCTGCTTCGACCTGAG CATCATCAGCATGCTGGAGCAGGGCAAGGAGCCCTTCACCCTGGAGAGCCAGG TGCAGATCGCCGGCAACCCCGACGGC (SEQ ID NO: 1022) | 6.75405678 |
| ZNF25 | PVTLKDVIVEFTK EEWKLLTPAQRT LYKDVMLENYSH LVSV (SEQ ID NO: 255) | NKFQGPVTLKDVIVEFTKEEWK LLTPAQRTLYKDVMLENYSHLV SVGYHVNKPNAVFKLKQGKEP WILEVEFPHRGFPED (SEQ ID NO: 256) | AACAAGTTCCAGGGCCCCGTGACCCTGAAGGACGTGATCGTGGAGTTCACCAA GGAGGAGTGGAAGCTGCTGACCCCCGCCCAGAGAACCCTGTACAAGGACGTGA TGCTGGAGAACTACAGCCACCTGGTGAGCGTGGGCTACCACGTGAACAAGCCC AACGCCGTGTTCAAGCTGAAGCAGGGCAAGGAGCCCTGGATCCTGGAGGTGGA GTTCCCCCACAGAGGCTTCCCCGAGGAC (SEQ ID NO: 1023) | 6.75008459 |
| ZN519 | LLTFRDVAIEFSPE EWKCLDPAQQN LYRDVMLENYRN LVS (SEQ ID NO: 257) | ELLTFRDVAIEFSPEEWKCLDPA QQNLYRDVMLENYRNLVSLAV YSYYNQGILPEQGIQDSFKKATL GRYGSCGLENICL (SEQ ID NO: 258) | GAGCTGCTGACCTTCAGAGATGTGGCCATCGAGTTCAGCCCCGAGGAGTGGAA GTGCCTGGACCCCGCCCAGCAGAACCTGTACAGAGATGTGATGCTGGAGAACT ACAGAAACCTGGTGAGCCTGGCCGTGTACAGCTACTACAACCAGGGCATCCTGC CCGAGCAGGGCATCCAGGACAGCTTCAAGAAGGCCACCCTGGGCAGATACGGC AGCTGCGGCCTGGAGAACATCTGCCTG (SEQ ID NO: 1024) | 6.74815071 |
| Z585B | SVSFRDVAIDFSR EEWRHLDLSQRN LYRDVMLETYSH LLSV(SEQ ID NO: 259) | SPQKSSALAPEDHGSSYEGSVSF RDVAIDFSREEWRHLDLSQRNL YRDVMLETYSHLLSVGYQVPKP EVVMLEQGKEPWA (SEQ ID NO: 260) | AGCCCCCAGAAGAGCAGCGCCCTGGCCCCCGAGGACCACGGCAGCAGCTACGA GGGAAGCGTGAGCTTCAGAGATGTGGCCATCGACTTCAGCAGAGAGGAGTGGA GACACCTGGACCTGAGCCAGAGAAACCTGTACAGAGATGTGATGCTGGAGACC TACAGCCACCTGCTGAGCGTGGGCTACCAGGTGCCCAAGCCCGAGGTGGTGAT GCTGGAGCAGGGCAAGGAGCCCTGGGCC (SEQ ID NO: 1025) | 6.74700322 |
| ZIM3 | RVTFEDVTVNFT QGEWQRLNPEQ RNLYRDVMLENY SNLVSV (SEQ ID NO: 261) | NNSQGRVTFEDVTVNFTQGE WQRLNPEQRNLYRDVMLENYS NLVSVGQGETTKPDVILRLEQG KEPWLEEEEVLGSGRAE (SEQ ID NO: 262) | AACAACAGCCAGGGCAGAGTGACCTTCGAGGACGTGACCGTGAACTTCACCCA GGGCGAGTGGCAGAGACTGAACCCCGAGCAGAGAAACCTGTACAGAGATGTG ATGCTGGAGAACTACAGCAACCTGGTGAGCGTGGGCCAGGGCGAGACCACCAA GCCCGACGTGATCCTGAGACTGGAGCAGGGCAAGGAGCCCTGGCTGGAGGAG GAGGAGGTGCTGGGCAGCGGCAGAGCCGAG (SEQ ID NO: 1026) | 6.74462278 |
| ZN517 | AVVFEDVAVYFT RIEWSCLAPDQQ ALYRDVMLENYG NLASL (SEQ ID NO: 263) | AMALPMPGPQEAVVFEDVAV YFTRIEWSCLAPDQQALYRDV MLENYGNLASLGFLVAKPALISL LEQGEEPGALILQVAE (SEQ ID NO: 264) | GCCATGGCCCTGCCCATGCCCGGCCCCCAGGAGGCCGTGGTGTTCGAGGACGTT GCCGTGTACTTCACCAGAATCGAGTGGAGCTGCCTGGCCCCCGACCAGCAGGCC CTGTACAGAGATGTGATGCTGGAGAACTACGGCAACCTGGCCAGCCTGGGCTTC CTGGTGGCCAAGCCCGCCCTGATCAGCCTGCTGGAGCAGGGCGAGGAGCCCGG AGCCCTGATCCTGCAGGTGGCCGAG (SEQ ID NO: 1027) | 6.71923079 |
| ZN846 | LVTFEDVAVDFT QEEWTLLDQAQ RDLYRDVMLENY KNLII (SEQ ID NO: 265) | DSSQHLVTFEDVAVDFTQEEW TLLDQAQRDLYRDVMLENYKN LIILAGSELFKRSLMSGLEQMEE LRTGVTGVLQELDLQ (SEQ ID NO: 266) | GACAGCAGCCAGCACCTGGTGACCTTCGAGGACGTGGCCGTGGACTTCACCCA GGAGGAGTGGACCCTGCTGGACCAGGCCCAGAGAGACCTGTACAGAGATGTGA TGCTGGAGAACTACAAGAACCTGATCATCCTGGCCGGCAGCGAGCTGTTCAAGA GAAGCCTGATGAGCGGCCTGGAGCAGATGGAGGAGCTGAGAACCGGCGTGAC CGGCGTGCTGCAGGAGCTGGACCTGCAG (SEQ ID NO: 1028) | 6.70970056 |
| ZN230 | AVTFKDVAVFFTE EELGLLDPAQRKL YQDVMLENFTNL LSV (SEQ ID NO: 267) | TTFKEAVTFKDVAVFFTEEELGL LDPAQRKLYQDVMLENFTNLLS VGHQPFHPFHFLREEKFWMM ETATQREGNSGGKTI (SEQ ID NO: 268) | ACCACCTTCAAGGAGGCCGTGACCTTCAAGGACGTGGCCGTGTTCTTCACCGAG GAGGAGCTGGGCCTGCTGGACCCCGCCCAGAGAAAGCTGTACCAGGACGTGAT GCTGGAGAACTTCACCAACCTGCTGAGCGTGGGCCACCAGCCCTTCCACCCCTTC CACTTCCTGAGAGAGGAGAAGTTCTGGATGATGGAGACCGCCACCCAGAGAGA GGGCAACAGCGGCGGCAAGACCATC (SEQ ID NO: 1029) | 6.70246908 |
| ZNF66 | PLQFRDVAIEFSL EEWHCLDMAQR NLYRDVMLENYR NLVFL (SEQ ID NO: 269) | GPLQFRDVAIEFSLEEWHCLDM AQRNLYRDVMLENYRNLVFLGI VVSKPDLITHLEQGKKPSTMQR HEMVANPSVLCSHF (SEQ ID NO: 270) | GGCCCCCTGCAGTTCAGAGATGTGGCCATCGAGTTCAGCCTGGAGGAGTGGCA CTGCCTGGACATGGCCCAGAGAAACCTGTACAGAGATGTGATGCTGGAGAACT ACAGAAACCTGGTGTTCCTGGGCATCGTGGTGAGCAAGCCCGACCTGATCACCC ACCTGGAGCAGGGCAAGAAGCCCAGCACCATGCAGAGACACGAGATGGTGGC CAACCCCAGCGTGCTGTGCAGCCACTTC(SEQ ID NO: 1030) | 6.69981008 |
| ZFP1 | SVSFTDVTVDFT QEEWEQLDPSQ RILYMDVMLENY SNLLSV (SEQ ID NO: 271) | NKSQGSVSFTDVTVDFTQEEW EQLDPSQRILYMDVMLENYSNL LSVEVWKADDQMERDHRNPD EQARQFLILKNQTPIEE (SEQ ID NO: 272) | AACAAGAGCCAGGGCAGCGTGAGCTTCACCGACGTGACCGTGGACTTCACCCA GGAGGAGTGGGAGCAGCTGGACCCCAGCCAGAGAATCCTGTACATGGACGTGA TGCTGGAGAACTACAGCAACCTGCTGAGCGTGGAGGTGTGGAAGGCCGACGAC CAGATGGAGAGAGACCACAGAAACCCCGACGAGCAGGCCAGACAGTTCCTGAT CCTGAAGAACCAGACCCCCATCGAGGAG (SEQ ID NO: 1031) | 6.69334133 |
| ZN713 | SLTFQDVAVDFT REEWDQLYPAQ KNLYRDVMLENY RNLVAL (SEQ ID NO: 273) | EEEEMNDGSQMVRSQESLTFQ DVAVDFTREEWDQLYPAQKNL YRDVMLENYRNLVALGYQLCKP EVIAQLELEEEWVIER (SEQ ID NO: 274) | GAGGAGGAGGAGATGAACGACGGCAGCCAGATGGTGAGAAGCCAGGAGAGC CTGACCTTCCAGGACGTGGCCGTGGACTTCACCAGAGAGGAGTGGGACCAGCT GTACCCCGCCCAGAAGAACCTGTACAGAGATGTGATGCTGGAGAACTACAGAA ACCTGGTGGCCCTGGGCTACCAGCTGTGCAAGCCCGAGGTGATCGCCCAGCTG GAGCTGGAGGAGGAGTGGGTGATCGAGAGA (SEQ ID NO: 1032) | 6.68245851 |
| ZN816 | RLTFRDVAIEFSLE EWKCLNPAQRAL YRAVMLENYRNL EFV(SEQ ID NO: 275) | EEATKKSKEKEPGMALPQGRLT FRDVAIEFSLEEWKCLNPAQRA LYRAVMLENYRNLEFVDSSLKS MMEFSSTRHSITGE (SEQ ID NO: 276) | GAGGAGGCCACCAAGAAGAGCAAGGAGAAGGAGCCCGGCATGGCCCTGCCCC AGGGCAGACTGACCTTCAGAGATGTGGCCATCGAGTTCAGCCTGGAGGAGTGG AAGTGCCTGAACCCCGCCCAGAGAGCCCTGTACAGAGCCGTGATGCTGGAGAA CTACAGAAACCTGGAGTTCGTGGACAGCAGCCTGAAGAGCATGATGGAGTTCA GCAGCACCAGACACAGCATCACCGGCGAG (SEQ ID NO: 1033) | 6.67677315 |
| ZN426 | SVTFDDVAVDFT QEEWTLLDSTQR SLYSDVMLENYK NLATV (SEQ ID NO: 277) | EKTPAGRIVADCLTDCYQDSVT FDDVAVDFTQEEWTLLDSTQRS LYSDVMLENYKNLATVGGQIIK PSLISWLEQEESRT (SEQ ID NO: 278) | GAGAAGACCCCCGCCGGCAGAATCGTGGCCGACTGCCTGACCGACTGCTACCA GGACAGCGTGACCTTCGACGACGTGGCCGTGGACTTCACCCAGGAGGAGTGGA CCCTGCTGGACAGCACCCAGAGAAGCCTGTACAGCGACGTGATGCTGGAGAAC TACAAGAACCTGGCCACCGTGGGCGGCCAGATCATCAAGCCCAGCCTGATCAGC TGGCTGGAGCAGGAGGAGAGCAGAACC (SEQ ID NO: 1034) | 6.67185066 |
| ZN701 | LLTFRDVAIEFSQ EEWKCLDPAQRT LYRDVMLENYRN LVSL (SEQ ID NO: 279) | KTRKRKRKAKESGMALLQGLLT FRDVAIEFSQEEWKCLDPAQRT LYRDVMLENYRNLVSLDTSSKC MMKMFSSTGQGNTE (SEQ ID NO: 280) | AAGACCAGAAAGAGAAAGAGAAAGGCCAAGGAGAGCGGCATGGCCCTGCTGC AGGGCCTGCTGACCTTCAGAGATGTGGCCATCGAGTTCAGCCAGGAGGAGTGG AAGTGCCTGGACCCCGCCCAGAGAACCCTGTACAGAGATGTGATGCTGGAGAA CTACAGAAACCTGGTGAGCCTGGACACCAGCAGCAAGTGCATGATGAAGATGT TCAGCAGCACCGGCCAGGGCAACACCGAG (SEQ ID NO: 1035) | 6.66820921 |
| ZN674 | SLTFKDVFVDFTL EEWQQLDSAQK NLYRDVMLENYS HLVSV (SEQ ID NO: 281) | AMSQESLTFKDVFVDFTLEEW QQLDSAQKNLYRDVMLENYSH LVSVGHLVGKPDVIFRLGPGDE SWMADGGTPVRTCAGE (SEQ ID NO: 282) | GCCATGAGCCAGGAGAGCCTGACCTTCAAGGACGTGTTCGTGGACTTCACCCTG GAGGAGTGGCAGCAGCTGGACAGCGCCCAGAAGAACCTGTACAGAGATGTGAT GCTGGAGAACTACAGCCACCTGGTGAGCGTGGGCCACCTGGTGGGCAAGCCCG ACGTGATCTTCAGACTGGGCCCCGGCGACGAGAGCTGGATGGCCGACGGCGGC ACACCTGTGAGAACCTGCGCCGGCGAG (SEQ ID NO: 1036) | 6.6636553 |
| ZN627 | SVAFEDVAVNFT LEEWALLDPSQK NLYRDVMRETFR NLASV (SEQ ID NO: 283) | DSVAFEDVAVNFTLEEWALLDP SQKNLYRDVMRETFRNLASVG KQWEDQNIEDPFKIPRRNISHIP ERLCESKEGGQGEE (SEQ ID NO: 284) | GACAGCGTGGCCTTCGAGGACGTGGCCGTGAACTTCACCCTGGAGGAGTGGGC CCTGCTGGACCCCAGCCAGAAGAACCTGTACAGAGATGTGATGAGAGAGACCT TCAGAAACCTGGCCAGCGTGGGCAAGCAGTGGGAGGACCAGAACATCGAGGA CCCCTTCAAGATCCCCAGAAGAAACATCAGCCACATCCCCGAGAGACTGTGCGA GAGCAAGGAGGGCGGCCAGGGCGAGGAG (SEQ ID NO: 1037) | 6.66232669 |
| ZNF20 | SVAFEDVAVSFT QEEWALLDPSQK NLYRDVMQETFK NLTSV (SEQ ID NO: 285) | MFQDSVAFEDVAVSFTQEEWA LLDPSQKNLYRDVMQETFKNLT SVGKTWKVQNIEDEYKNPRRN LSLMREKLCESKESHH (SEQ ID NO: 286) | ATGTTCCAGGACAGCGTGGCCTTCGAGGACGTGGCCGTGAGCTTCACCCAGGA GGAGTGGGCCCTGCTGGACCCCAGCCAGAAGAACCTGTACAGAGATGTGATGC AGGAGACCTTCAAGAACCTGACCAGCGTGGGCAAGACCTGGAAGGTGCAGAAC ATCGAGGACGAGTACAAGAACCCCAGAAGAAACCTGAGCCTGATGAGAGAGAA GCTGTGCGAGAGCAAGGAGAGCCACCAC (SEQ ID NO: 1038) | 6.65839711 |
| Z587B | TVTFEDVAVKFT QEEWNLLSEAQR CLYRDVTLENLAL MSSL (SEQ ID NO: 287) | AVVATLRLSAQGTVTFEDVAVK FTQEEWNLLSEAQRCLYRDVTL ENLALMSSLGCWCGVEDEAAP SKQSIYIQRETQVRT (SEQ ID NO: 288) | GCCGTGGTGGCCACCCTGAGACTGAGCGCCCAGGGCACCGTGACCTTCGAGGA CGTGGCCGTGAAGTTCACCCAGGAGGAGTGGAACCTGCTGAGCGAGGCCCAGA GATGCCTGTACAGAGATGTGACCCTGGAGAACCTGGCCCTGATGAGCAGCCTG GGCTGCTGGTGCGGCGTGGAGGACGAGGCCGCCCCTAGCAAGCAGAGCATCTA CATCCAGAGAGAGACCCAGGTGAGAACC (SEQ ID NO: 1039) | 6.63154785 |
| ZN316 | LVTFEDVAVYFSL EEWERLEADQRG LYQEVMQENYGI LVSL (SEQ ID NO: 289) | EEEEEDEDEDDLLTAGCQELVTF EDVAVYFSLEEWERLEADQRGL YQEVMQENYGILVSLGYPIPKP DLIFRLEQGEEPW (SEQ ID NO: 290) | GAGGAGGAGGAGGAGGACGAGGACGAGGACGACCTGCTGACCGCCGGCTGCC AGGAGCTGGTGACCTTCGAGGACGTGGCCGTGTACTTCAGCCTGGAGGAGTGG GAGAGACTGGAGGCCGACCAGAGAGGCCTGTACCAGGAGGTGATGCAGGAGA ACTACGGCATCCTGGTGAGCCTGGGCTACCCCATCCCCAAGCCCGACCTGATCTT CAGACTGGAGCAGGGCGAGGAGCCCTGG (SEQ ID NO: 1040) | 6.62746569 |
| ZN233 | MVTFKDVAVVFT REELGLLDLAQRK LYQDVMLENFRN LLSV (SEQ ID NO: 291) | TKFQEMVTFKDVAVVFTREELG LLDLAQRKLYQDVMLENFRNLL SVGYQPFKLDVILQLGKEDKLR MMETEIQGDGCSGH (SEQ ID NO: 292) | ACCAAGTTCCAGGAGATGGTGACCTTCAAGGACGTGGCCGTGGTGTTCACCAGA GAGGAGCTGGGCCTGCTGGACCTGGCCCAGAGAAAGCTGTACCAGGACGTGAT GCTGGAGAACTTCAGAAACCTGCTGAGCGTGGGCTACCAGCCCTTCAAGCTGGA CGTGATCCTGCAGCTGGGCAAGGAGGACAAGCTGAGAATGATGGAGACCGAG ATCCAGGGCGACGGCTGCAGCGGCCAC (SEQ ID NO: 1041) | 6.62252575 |
| ZN611 | RLTFRDVAIEFSLA EWKCLNPSQRAL YREVMLENYRNL EAV (SEQ ID NO: 293) | EEAAQKRKGKEPGMALPQGRL TFRDVAIEFSLAEWKCLNPSQR ALYREVMLENYRNLEAVDISSKC MMKEVLSTGQGNTE (SEQ ID NO: 294) | GAGGAGGCCGCCCAGAAGAGAAAGGGCAAGGAGCCCGGCATGGCCCTGCCCC AGGGCAGACTGACCTTCAGAGATGTGGCCATCGAGTTCAGCCTGGCCGAGTGG AAGTGCCTGAACCCCAGCCAGAGAGCCCTGTACAGAGAGGTGATGCTGGAGAA CTACAGAAACCTGGAGGCCGTGGACATCAGCAGCAAGTGCATGATGAAGGAGG TGCTGAGCACCGGCCAGGGCAACACCGAG (SEQ ID NO: 1042) | 6.61854262 |
| ZN556 | TVVFEDVVVDFTL EEWALLNPAQRK LYRDVMLETFKH LASV (SEQ ID NO: 295) | DTVVFEDVVVDFTLEEWALLNP AQRKLYRDVMLETFKHLASVD NEAQLKASGSISQQDTSGEKLSL KQKIEKFTRKNIWA (SEQ ID NO: 296) | GACACCGTGGTGTTCGAGGACGTGGTGGTGGACTTCACCCTGGAGGAGTGGGC CCTGCTGAACCCCGCCCAGAGAAAGCTGTACAGAGATGTGATGCTGGAGACCTT CAAGCACCTGGCCAGCGTGGACAACGAGGCCCAGCTGAAGGCCAGCGGCAGCA TCAGCCAGCAGGACACCAGCGGCGAGAAGCTGAGCCTGAAGCAGAAGATCGA GAAGTTCACCAGAAAGAACATCTGGGCC (SEQ ID NO: 1043) | 6.61519705 |
| ZN234 | GLTFKDVAVVFTE EELGLLDPVQRNL YQDVMLENFRNL LSV (SEQ ID NO: 297) | TTFKEGLTFKDVAVVFTEEELGL LDPVQRNLYQDVMLENFRNLL SVGHHPFKHDVFLLEKEKKLDI MKTATQRKGKSADK (SEQ ID NO: 298) | ACCACCTTCAAGGAGGGCCTGACCTTCAAGGACGTGGCCGTGGTGTTCACCGAG GAGGAGCTGGGCCTGCTGGACCCCGTGCAGAGAAACCTGTACCAGGACGTGAT GCTGGAGAACTTCAGAAACCTGCTGAGCGTGGGCCACCACCCCTTCAAGCACGA CGTGTTCCTGCTGGAGAAGGAGAAGAAGCTGGACATCATGAAGACCGCCACCC AGAGAAAGGGCAAGAGCGCCGACAAG (SEQ ID NO: 1044) | 6.60158035 |
| ZN560 | LVTFDSVAVEFTQ EEWTLLDPAQRN LYSDVMLENYKN LSSV (SEQ ID NO: 299) | SALQQEFWKIQTSNGIQMDLV TFDSVAVEFTQEEWTLLDPAQR NLYSDVMLENYKNLSSVGYQLF KPSLISWLEEEEELS (SEQ ID NO: 300) | AGCGCCCTGCAGCAGGAGTTCTGGAAGATCCAGACCAGCAACGGCATCCAGAT GGACCTGGTGACCTTCGACAGCGTGGCCGTGGAGTTCACCCAGGAGGAGTGGA CCCTGCTGGACCCCGCCCAGAGAAACCTGTACAGCGACGTGATGCTGGAGAACT ACAAGAACCTGAGCAGCGTGGGCTACCAGCTGTTCAAGCCCAGCCTGATCAGCT GGCTGGAGGAGGAGGAGGAGCTGAGC (SEQ ID NO: 1045) | 6.60066711 |
| ZNF77 | CVIFEEVAVNFTP EEWALLDHAQRS LYRDVMLETCRN LASL (SEQ ID NO: 301) | DCVIFEEVAVNFTPEEWALLDH AQRSLYRDVMLETCRNLASLDC YIYVRTSGSSSQRDVFGNGISND EEIVKFTGSDSWS (SEQ ID NO: 302) | GACTGCGTGATCTTCGAGGAGGTGGCCGTGAACTTCACCCCCGAGGAGTGGGC CCTGCTGGACCACGCCCAGAGAAGCCTGTACAGAGATGTGATGCTGGAGACCT GCAGAAACCTGGCCAGCCTGGACTGCTACATCTACGTGAGAACCAGCGGCAGC AGCAGCCAGAGAGATGTGTTCGGCAACGGCATCAGCAACGACGAGGAGATCGT GAAGTTCACCGGCAGCGACAGCTGGAGC (SEQ ID NO: 1046) | 6.58987943 |
| ZN682 | LLTFRDVTIEFSLE EWEFLNPAQQSL YRKVMLENYRNL VSL (SEQ ID NO: 303) | ELLTFRDVTIEFSLEEWEFLNPA QQSLYRKVMLENYRNLVSLGLT VSKPELISRLEQRQEPWNVKRH ETIAKPPAMSSHY (SEQ ID NO: 304) | GAGCTGCTGACCTTCAGAGATGTGACCATCGAGTTCAGCCTGGAGGAGTGGGA GTTCCTGAACCCCGCCCAGCAGAGCCTGTACAGAAAGGTGATGCTGGAGAACTA CAGAAACCTGGTGAGCCTGGGCCTGACCGTGAGCAAGCCCGAGCTGATCAGCA GACTGGAGCAGAGACAGGAGCCCTGGAACGTGAAGAGACACGAGACCATCGC CAAGCCCCCCGCCATGAGCAGCCACTAC (SEQ ID NO: 1047) | 6.58030961 |
| ZN614 | SLTLEDVAVEFS WEEWQLLDTAQ KNLYRDVMVENY NHLVSL (SEQ ID NO: 305) | IKTQESLTLEDVAVEFSWEEWQ LLDTAQKNLYRDVMVENYNHL VSLGYQTSKPDVLSKLAHGQEP WTTDAKIQNKNCPGI (SEQ ID NO: 306) | ATCAAGACCCAGGAGAGCCTGACCCTGGAGGACGTGGCCGTGGAGTTCAGCTG GGAGGAGTGGCAGCTGCTGGACACCGCCCAGAAGAACCTGTACAGAGATGTGA TGGTGGAGAACTACAACCACCTGGTGAGCCTGGGCTACCAGACCAGCAAGCCC GACGTGCTGAGCAAGCTGGCCCACGGCCAGGAGCCCTGGACCACCGACGCCAA GATCCAGAACAAGAACTGCCCCGGCATC (SEQ ID NO: 1048) | 6.57723831 |
| ZN785 | AVSFADVAVYFS PEEWECLRPAQR ALYRDVMRETFG HLGAL (SEQ ID NO: 307) | PAHVPGEAGPRRTRESRPGAVS FADVAVYFSPEEWECLRPAQRA LYRDVMRETFGHLGALGFSVPK PAFISWVEGEVEAW (SEQ ID NO: 308) | CCCGCCCACGTGCCCGGCGAGGCCGGCCCCAGAAGAACCAGAGAGAGCAGACC CGGCGCTGTGAGCTTCGCCGACGTGGCCGTGTACTTCAGCCCCGAGGAGTGGG AGTGCCTGAGACCCGCCCAGAGAGCCCTGTACAGAGATGTGATGAGAGAGACC TTCGGCCACCTGGGCGCCCTGGGCTTCAGCGTGCCCAAGCCCGCCTTCATCAGCT GGGTGGAGGGCGAGGTGGAGGCCTGG (SEQ ID NO: 1049) | 6.56301724 |
| ZN445 | TMTFKDVEVTFS QDEWGWLDSA QRNLYRDVMLE NYRNMAS (SEQ ID NO: 309) | GCPGDQVTPTRSLTAQLQETM TFKDVEVTFSQDEWGWLDSAQ RNLYRDVMLENYRNMASLVGP FTKPALISWLEAREPWG (SEQ ID NO: 310) | GGCTGCCCCGGCGACCAGGTGACCCCCACCAGAAGCCTGACCGCCCAGCTGCA GGAGACCATGACCTTCAAGGACGTGGAGGTGACCTTCAGCCAGGACGAGTGGG GCTGGCTGGACAGCGCCCAGAGAAACCTGTACAGAGATGTGATGCTGGAGAAC TACAGAAACATGGCCAGCCTGGTGGGCCCCTTCACCAAGCCCGCCCTGATCAGC TGGCTGGAGGCCAGAGAGCCCTGGGGC (SEQ ID NO: 1050) | 6.54429484 |
| ZFP30 | LVMFRDVAVDFS QEEWECLNSYQR NLYRDVILENYSN LVS (SEQ ID NO: 311) | ARDLVMFRDVAVDFSQEEWEC LNSYQRNLYRDVILENYSNLVSL AGCSISKPDVITLLEQGKEPWM VVRDEKRRWTLDLE (SEQ ID NO: 312) | GCCAGAGACCTGGTGATGTTCAGAGATGTGGCCGTGGACTTCAGCCAGGAGGA GTGGGAGTGCCTGAACAGCTACCAGAGAAACCTGTACAGAGATGTGATCCTGG AGAACTACAGCAACCTGGTGAGCCTGGCCGGCTGCAGCATCAGCAAGCCCGAC GTGATCACCCTGCTGGAGCAGGGCAAGGAGCCCTGGATGGTGGTGAGAGATGA GAAGAGAAGATGGACCCTGGACCTGGAG (SEQ ID NO: 1051) | 6.54105426 |
| ZN225 | AVTFKDVAVVFT EEELRLLDLAQRK LYREVMLENFRN LLSV (SEQ ID NO: 313) | TTLKEAVTFKDVAVVFTEEELRL LDLAQRKLYREVMLENFRNLLS VGHQSLHRDTFHFLKEEKFWM METATQREGNLGGK (SEQ ID NO: 314) | ACCACCCTGAAGGAGGCCGTGACCTTCAAGGACGTGGCCGTGGTGTTCACCGA GGAGGAGCTGAGACTGCTGGACCTGGCCCAGAGAAAGCTGTACAGAGAGGTG ATGCTGGAGAACTTCAGAAACCTGCTGAGCGTGGGCCACCAGAGCCTGCACAG AGACACCTTCCACTTCCTGAAGGAGGAGAAGTTCTGGATGATGGAGACCGCCAC CCAGAGAGAGGGCAACCTGGGCGGCAAG (SEQ ID NO: 1052) | 6.53858149 |
| ZN551 | GMTFEDVAIYFS QEEWELLDESQR FLYCDVMLENFA HVTSL (SEQ ID NO: 315) | SPPSPRSSMAAVALRDSAQGM TFEDVAIYFSQEEWELLDESQRF LYCDVMLENFAHVTSLGYCHG MENEAIASEQSVSIQ (SEQ ID NO: 316) | AGCCCCCCCAGCCCAGAAGCAGCATGGCCGCCGTGGCCCTGAGAGACAGCGC CCAGGGCATGACCTTCGAGGACGTGGCCATCTACTTCAGCCAGGAGGAGTGGG AGCTGCTGGACGAGAGCCAGAGATTCCTGTACTGCGACGTGATGCTGGAGAAC TTCGCCCACGTGACCAGCCTGGGCTACTGCCACGGCATGGAGAACGAGGCCATC GCCAGCGAGCAGAGCGTGAGCATCCAG (SEQ ID NO: 1053) | 6.53471613 |
| ZN610 | RLTFMDVAIEFSQ EEWKSLDPGQRA LYRDVMLENYRN LVFL (SEQ ID NO: 317) | DEEAQKRKAKESGMALPQGRL TFMDVAIEFSQEEWKSLDPGQ RALYRDVMLENYRNLVFLGICLP DLSIISMLKQRREPL (SEQ ID NO: 318) | GACGAGGAGGCCCAGAAGAGAAAGGCCAAGGAGAGCGGCATGGCCCTGCCCC AGGGCAGACTGACCTTCATGGACGTGGCCATCGAGTTCAGCCAGGAGGAGTGG AAGAGCCTGGACCCCGGCCAGAGAGCCCTGTACAGAGATGTGATGCTGGAGAA CTACAGAAACCTGGTGTTCCTGGGCATCTGCCTGCCCGACCTGAGCATCATCAGC ATGCTGAAGCAGAGAAGAGAGCCCCTG (SEQ ID NO: 1054) | 6.53304307 |
| ZN528 | PLKFMDVAIEFSQ EEWKCLDPAQRT LYRDVMLENYRN LVSL (SEQ ID NO: 319) | ALTQGPLKFMDVAIEFSQEEWK CLDPAQRTLYRDVMLENYRNLV SLGICLPDLSVTSMLEQKRDPW TLQSEEKIANDPDG (SEQ ID NO: 320) | GCCCTGACCCAGGGCCCCCTGAAGTTCATGGACGTGGCCATCGAGTTCAGCCAG GAGGAGTGGAAGTGCCTGGACCCCGCCCAGAGAACCCTGTACAGAGATGTGAT GCTGGAGAACTACAGAAACCTGGTGAGCCTGGGCATCTGCCTGCCCGACCTGA GCGTGACCAGCATGCTGGAGCAGAAGAGAGACCCCTGGACCCTGCAGAGCGAG GAGAAGATCGCCAACGACCCCGACGGC (SEQ ID NO: 1055) | 6.5320662 |
| ZN284 | AVTFKDVAVVFT EEELGLLDVSQRK LYRDVMLENFRN LLSV (SEQ ID NO: 321) | TMFKEAVTFKDVAVVFTEEELG LLDVSQRKLYRDVMLENFRNLL SVGHQLSHRDTFHFQREEKFWI METATQREGNSGGK (SEQ ID NO: 322) | ACCATGTTCAAGGAGGCCGTGACCTTCAAGGACGTGGCCGTGGTGTTCACCGAG GAGGAGCTGGGCCTGCTGGACGTGAGCCAGAGAAAGCTGTACAGAGATGTGAT GCTGGAGAACTTCAGAAACCTGCTGAGCGTGGGCCACCAGCTGAGCCACAGAG ACACCTTCCACTTCCAGAGAGAGGAGAAGTTCTGGATCATGGAGACCGCCACCC AGAGAGAGGGCAACAGCGGCGGCAAG (SEQ ID NO: 1056) | 6.52062588 |
| ZN418 | TVAFEDVAVNFS QEEWSLLSEVQR CLYHDVMLENW VLISSL (SEQ ID NO: 323) | QGTVAFEDVAVNFSQEEWSLLS EVQRCLYHDVMLENWVLISSLG CWCGSEDEEAPSKKSISIQRVSQ VSTPGAGVSPKKA (SEQ ID NO: 324) | CAGGGCACCGTGGCCTTCGAGGACGTGGCCGTGAACTTCAGCCAGGAGGAGTG GAGCCTGCTGAGCGAGGTGCAGAGATGCCTGTACCACGACGTGATGCTGGAGA ACTGGGTGCTGATCAGCAGCCTGGGCTGCTGGTGCGGCAGCGAGGACGAGGA GGCCCCCAGCAAGAAGAGCATCAGCATCCAGAGAGTGAGCCAGGTGAGCACCC CCGGCGCCGGCGTGAGCCCCAAGAAGGCC (SEQ ID NO: 1057) | 6.51925026 |
| MPP8 | FEVEKILDMKTEG GKVLYKVRWKGY TSDDDTWEPEIH LEDCKEVLLEFRK (SEQ ID NO: 325) | AEAFGDSEEDGEDVFEVEKILD MKTEGGKVLYKVRWKGYTSDD DTWEPEIHLEDCKEVLLEFRKKI AENKAKAVRKDIQR (SEQ ID NO: 326) | GCCGAGGCCTTCGGCGACAGCGAGGAGGACGGCGAGGACGTGTTCGAGGTGG AGAAGATCCTGGACATGAAGACCGAGGGCGGCAAGGTGCTGTACAAGGTGAG ATGGAAGGGCTACACCAGCGACGACGACACCTGGGAGCCCGAGATCCACCTGG AGGACTGCAAGGAGGTGCTGCTGGAGTTCAGAAAGAAGATCGCCGAGAACAA GGCCAAGGCCGTGAGAAAGGACATCCAGAGA (SEQ ID NO: 1058) | 6.51334634 |
| ZN490 | SISLEDVAVNFTL EEWALLDPGQR NIYRDVMRATFK NLACI (SEQ ID NO: 327) | VLQMQNSEHHGQSIKTQTDSIS LEDVAVNFTLEEWALLDPGQR NIYRDVMRATFKNLACIGEKWK DQDIEDEHKNQGRNL (SEQ ID NO: 328) | GTGCTGCAGATGCAGAACAGCGAGCACCACGGCCAGAGCATCAAGACCCAGAC CGACAGCATCAGCCTGGAGGACGTGGCCGTGAACTTCACCCTGGAGGAGTGGG CCCTGCTGGACCCCGGCCAGAGAAACATCTACAGAGATGTGATGAGAGCCACCT TCAAGAACCTGGCCTGCATCGGCGAGAAGTGGAAGGACCAGGACATCGAGGAC GAGCACAAGAACCAGGGCAGAAACCTG (SEQ ID NO: 1059) | 6.51148602 |
| ZN805 | SVTFDDVAVTFT QEEWGQLDLAQ RTLYQEVMLENC GLLVSL (SEQ ID NO: 329) | AMALTDPAQVSVTFDDVAVTF TQEEWGQLDLAQRTLYQEVML ENCGLLVSLGCPVPRPELIYHLE HGQEPWTRKEDLSQG (SEQ ID NO: 330) | GCCATGGCCCTGACCGACCCCGCCCAGGTGAGCGTGACCTTCGACGACGTGGCC GTGACCTTCACCCAGGAGGAGTGGGGCCAGCTGGACCTGGCCCAGAGAACCCT GTACCAGGAGGTGATGCTGGAGAACTGCGGCCTGCTGGTGAGCCTGGGCTGCC CCGTGCCCAGACCCGAGCTGATCTACCACCTGGAGCACGGCCAGGAGCCCTGG ACCAGAAAGGAGGACCTGAGCCAGGGC (SEQ ID NO: 1060) | 6.50974725 |
| Z780B | SVTFRDVAIDFSQ EEWECLQPDQRT LYRDVMLENYSH LISL (SEQ ID NO: 331) | VHGSVTFRDVAIDFSQEEWECL QPDQRTLYRDVMLENYSHLISL GSSISKPDVITLLEQEKEPWIVVS KETSRWYPDLES (SEQ ID NO: 332) | GTGCACGGCAGCGTGACCTTCAGAGATGTGGCCATCGACTTCAGCCAGGAGGA GTGGGAGTGCCTGCAGCCGACCAGAGAACCCTGTACAGAGATGTGATGCTGG AGAACTACAGCCACCTGATCAGCCTGGGCAGCAGCATCAGCAAGCCCGACGTG ATCACCCTGCTGGAGCAGGAGAAGGAGCCCTGGATCGTGGTGAGCAAGGAGAC CAGCAGATGGTACCCCGACCTGGAGAGC (SEQ ID NO: 1061) | 6.50607891 |
| ZN763 | PVACEDVAVNFT QEEWALLDISQR KLYREVMLETFR NLTSI (SEQ ID NO: 333) | DPVACEDVAVNFTQEEWALLDI SQRKLYREVMLETFRNLTSIGKK WKDQNIEYEYQNPRRNFRSLIE GNVNEIKEDSHCG (SEQ ID NO: 334) | GACCCCGTGGCCTGCGAGGACGTGGCCGTGAACTTCACCCAGGAGGAGTGGGC CCTGCTGGACATCAGCCAGAGAAAGCTGTACAGAGAGGTGATGCTGGAGACCT TCAGAAACCTGACCAGCATCGGCAAGAAGTGGAAGGACCAGAACATCGAGTAC GAGTACCAGAACCCCAGAAGAAACTTCAGAAGCCTGATCGAGGGCAACGTGAA CGAGATCAAGGAGGACAGCCACTGCGGC (SEQ ID NO: 1062) | 6.49330748 |
| ZN285 | RVTFKDVAVVFT KEELALLDKAQIN LYQDVMLENFRN LML (SEQ ID NO: 335) | IKFQERVTFKDVAVVFTKEELAL LDKAQINLYQDVMLENFRNLM LVRDGIKNNILNLQAKGLSYLSQ EVLHCWQIWKQRI (SEQ ID NO: 336) | ATCAAGTTCCAGGAGAGAGTGACCTTCAAGGACGTGGCCGTGGTGTTCACCAA GGAGGAGCTGGCCCTGCTGGACAAGGCCCAGATCAACCTGTACCAGGACGTGA TGCTGGAGAACTTCAGAAACCTGATGCTGGTGAGAGATGGCATCAAGAACAAC ATCCTGAACCTGCAGGCCAAGGGCCTGAGCTACCTGAGCCAGGAGGTGCTGCA CTGCTGGCAGATCTGGAAGCAGAGAATC (SEQ ID NO: 1063) | 6.48639829 |
| ZNF85 | PLTFRDVAIEFSLK EWQCLDTAQRN LYRNVMLENYRN LVFL (SEQ ID NO: 337) | GPLTFRDVAIEFSLKEWQCLDT AQRNLYRNVMLENYRNLVFLGI TVSKPDLITCLEQGKEAWSMKR HEIMVAKPTVMCSH (SEQ ID NO: 338) | GGCCCCCTGACCTTCAGAGATGTGGCCATCGAGTTCAGCCTGAAGGAGTGGCA GTGCCTGGACACCGCCCAGAGAAACCTGTACAGAAACGTGATGCTGGAGAACT ACAGAAACCTGGTGTTCCTGGGCATCACCGTGAGCAAGCCCGACCTGATCACCT GCCTGGAGCAGGGCAAGGAGGCCTGGAGCATGAAGAGACACGAGATCATGGT GGCCAAGCCCACCGTGATGTGCAGCCAC (SEQ ID NO: 1064) | 6.48512557 |
| ZN223 | AVTFKDVAVVFT EEELGLLDLAQRK LYRDVMLENFRN LLSV (SEQ ID NO: 339) | TMSKEAVTFKDVAVVFTEEELG LLDLAQRKLYRDVMLENFRNLL SVGHQPFHRDTFHFLREEKFW MMDIATQREGNSGGK (SEQ ID NO: 340) | ACCATGAGCAAGGAGGCCGTGACCTTCAAGGACGTGGCCGTGGTGTTCACCGA GGAGGAGCTGGGCCTGCTGGACCTGGCCCAGAGAAAGCTGTACAGAGATGTGA TGCTGGAGAACTTCAGAAACCTGCTGAGCGTGGGCCACCAGCCCTTCCACAGAG ACACCTTCCACTTCCTGAGAGAGGAGAAGTTCTGGATGATGGACATCGCCACCC AGAGAGAGGGCAACAGCGGCGGCAAG (SEQ ID NO: 1065) | 6.48230966 |
| ZNF90 | PLEFRDVAIEFSLE EWHCLDTAQQN LYRDVMLENYRH LVFL (SEQ ID NO: 341) | GPLEFRDVAIEFSLEEWHCLDTA QQNLYRDVMLENYRHLVFLGIV VTKPDLITCLEQGKKPFTVKRHE MIAKSPVMCFHF (SEQ ID NO: 342) | GGCCCCCTGGAGTTCAGAGATGTGGCCATCGAGTTCAGCCTGGAGGAGTGGCA CTGCCTGGACACCGCCCAGCAGAACCTGTACAGAGATGTGATGCTGGAGAACTA CAGACACCTGGTGTTCCTGGGCATCGTGGTGACCAAGCCCGACCTGATCACCTG CCTGGAGCAGGGCAAGAAGCCCTTCACCGTGAAGAGACACGAGATGATCGCCA AGAGCCCCGTGATGTGCTTCCACTTC (SEQ ID NO: 1066) | 6.47855756 |
| ZN557 | LVTFEDVAVEFTQ EEWALLDPAQRT LYRDVMLENCRN LASL (SEQ ID NO: 343) | GHTEGGELVNELLKSWLKGLVT FEDVAVEFTQEEWALLDPAQR TLYRDVMLENCRNLASLGNQV DKPRLISQLEQEDKVM (SEQ ID NO: 344) | GGCCACACCGAGGGCGGCGAGCTGGTGAACGAGCTGCTGAAGAGCTGGCTGA AGGGCCTGGTGACCTTCGAGGACGTGGCCGTGGAGTTCACCCAGGAGGAGTGG GCCCTGCTGGACCCCGCCCAGAGAACCCTGTACAGAGATGTGATGCTGGAGAA CTGCAGAAACCTGGCCAGCCTGGGCAACCAGGTGGACAAGCCCAGACTGATCA GCCAGCTGGAGCAGGAGGACAAGGTGATG (SEQ ID NO: 1067) | 6.47397343 |
| ZN425 | TVTFDDVALYFSE QEWEILEKWQK QMYKQEMKTNY ETLDSL (SEQ ID NO: 345) | AEPASVTVTFDDVALYFSEQEW EILEKWQKQMYKQEMKTNYET LDSLGYAFSKPDLITWMEQGR MLLISEQGCLDKTRRT (SEQ ID NO: 346) | GCCGAGCCCGCCAGCGTGACCGTGACCTTCGACGACGTGGCCCTGTACTTCAGC GAGCAGGAGTGGGAGATCCTGGAGAAGTGGCAGAAGCAGATGTACAAGCAGG AGATGAAGACCAACTACGAGACCCTGGACAGCCTGGGCTACGCCTTCAGCAAG CCCGACCTGATCACCTGGATGGAGCAGGGCAGAATGCTGCTGATCAGCGAGCA GGGCTGCCTGGACAAGACCAGAAGAACC (SEQ ID NO: 1068) | 6.47320582 |
| ZN229 | PLSFKDVAVVFTE EELELLDSTQRQL YQDVMQENFRN LLSV (SEQ ID NO: 347) | HSQASAISQDREEKIMSQEPLSF KDVAVVFTEEELELLDSTQRQLY QDVMQENFRNLLSVGERNPLG DKNGKDTEYIQDE (SEQ ID NO: 348) | CACAGCCAGGCCAGCGCCATCAGCCAGGACAGAGAGGAGAAGATCATGAGCCA GGAGCCCCTGAGCTTCAAGGACGTGGCCGTGGTGTTCACCGAGGAGGAGCTGG AGCTGCTGGACAGCACCCAGAGACAGCTGTACCAGGACGTGATGCAGGAGAAC TTCAGAAACCTGCTGAGCGTGGGCGAGAGAAACCCCCTGGGCGACAAGAACGG CAAGGACACCGAGTACATCCAGGACGAG(SEQ ID NO: 1069) | 6.47139743 |
| ZN606 | PVTFKDVAVDFT QEEWGQLDLVQ RTLYRDVMLETY GHLLSV (SEQ ID NO: 349) | GSLEEGRRATGLPAAQVQEPVT FKDVAVDFTQEEWGQLDLVQR TLYRDVMLETYGHLLSVGNQIA KPEVISLLEQGEEPW (SEQ ID NO: 350) | GGCAGCCTGGAGGAGGGCAGAAGAGCCACCGGCCTGCCCGCCGCCCAAGTGC AGGAGCCTGTGACCTTCAAGGACGTGGCCGTGGACTTCACCCAGGAGGAGTGG GGCCAGCTGGACCTGGTGCAGAGAACCCTGTACAGAGATGTGATGCTGGAGAC CTACGGCCACCTGCTGAGCGTGGGCAACCAGATCGCCAAGCCCGAGGTGATCA GCCTGCTGGAGCAGGGCGAGGAGCCCTGG (SEQ ID NO: 1070) | 6.46489693 |
| ZN155 | AVTFKDVAVVFT EEELGLLDPAQRK LYRDVMLENFRN LLSV (SEQ ID NO: 351) | TTFKEAVTFKDVAVVFTEEELGL LDPAQRKLYRDVMLENFRNLLS VGHQPFHQDTCHFLREEKFW MMGTATQREGNSGGK (SEQ ID NO: 352) | ACCACCTTCAAGGAGGCCGTGACCTTCAAGGACGTGGCCGTGGTGTTCACCGAG GAGGAGCTGGGCCTGCTGGACCCCGCCCAGAGAAAGCTGTACAGAGATGTGAT GCTGGAGAACTTCAGAAACCTGCTGAGCGTGGGCCACCAGCCCTTCCACCAGGA CACCTGCCACTTCCTGAGAGAGGAGAAGTTCTGGATGATGGGCACCGCCACCCA GAGAGAGGGCAACAGCGGCGGCAAG (SEQ ID NO: 1071) | 6.45744473 |
| ZN222 | AVTFKDVAVIFTE EELGLLDPAQRKL YRDVMLENFRNL LSV (SEQ ID NO: 353) | AKLYEAVTFKDVAVIFTEEELGLL DPAQRKLYRDVMLENFRNLLSV GGKIQTEMETVPEAGTHEEFSC KQIWEQIASDLT (SEQ ID NO: 354) | GCCAAGCTGTACGAGGCCGTGACCTTCAAGGACGTGGCCGTGATCTTCACCGAG GAGGAGCTGGGCCTGCTGGACCCCGCCCAGAGAAAGCTGTACAGAGATGTGAT GCTGGAGAACTTCAGAAACCTGCTGAGCGTGGGCGGCAAGATCCAGACCGAGA TGGAGACCGTGCCCGAGGCCGGCACCCACGAGGAGTTCAGCTGCAAGCAGATC TGGGAGCAGATCGCCAGCGACCTGACC (SEQ ID NO: 1072) | 6.45544011 |
| ZN442 | SVAFEDVAVNFT QEEWALLGPSQK SLYRDVMWETIR NLDCI (SEQ ID NO: 355) | RSDLFLPDSQTNEERKQYDSVA FEDVAVNFTQEEWALLGPSQK SLYRDVMWETIRNLDCIGMKW EDTNIEDQHRNPRRSL (SEQ ID NO: 356) | AGAAGCGACCTGTTCCTGCCCGACAGCCAGACCAACGAGGAGAGAAAGCAGTA CGACAGCGTGGCCTTCGAGGACGTGGCCGTGAACTTCACCCAGGAGGAGTGGG CCCTGCTGGGCCCCAGCCAGAAGAGCCTGTACAGAGATGTGATGTGGGAGACC ATCAGAAACCTGGACTGCATCGGCATGAAGTGGGAGGACACCAACATCGAGGA CCAGCACAGAAACCCCAGAAGAAGCCTG (SEQ ID NO: 1073) | 6.44268455 |
| ZNF91 | LLTFRDVAIEFSPE EWQCLDTAQQN LYRNVMLENYRN LAFL (SEQ ID NO: 357) | PGTPGSLEMGLLTFRDVAIEFSP EEWQCLDTAQQNLYRNVMLE NYRNLAFLGIALSKPDLITYLEQG KEPWNMKQHEMVD (SEQ ID NO: 358) | CCCGGCACCCCCGGCAGCCTGGAGATGGGCCTGCTGACCTTCAGAGATGTGGCC ATCGAGTTCAGCCCCGAGGAGTGGCAGTGCCTGGACACCGCCCAGCAGAACCT GTACAGAAACGTGATGCTGGAGAACTACAGAAACCTGGCCTTCCTGGGCATCGC CCTGAGCAAGCCCGACCTGATCACCTACCTGGAGCAGGGCAAGGAGCCCTGGA ACATGAAGCAGCACGAGATGGTGGAC (SEQ ID NO: 1074) | 6.44174437 |
| ZN135 | QVTFEDVVVGFS QEEWGQLKPAQ RTLYRDVMLDTF RLLVSV (SEQ ID NO: 359) | TPGVRVSTDPEQVTFEDVVVGF SQEEWGQLKPAQRTLYRDVML DTFRLLVSVGHWLPKPNVISLLE QEAELWAVESRLPQ (SEQ ID NO: 360) | ACCCCCGGCGTGAGAGTGAGCACCGACCCCGAGCAGGTGACCTTCGAGGACGT GGTGGTGGGCTTCAGCCAGGAGGAGTGGGGCCAGCTGAAGCCCGCCCAGAGA ACCCTGTACAGAGATGTGATGCTGGACACCTTCAGACTGCTGGTGAGCGTGGGC CACTGGCTGCCCAAGCCCAACGTGATCAGCCTGCTGGAGCAGGAGGCCGAGCT GTGGGCCGTGGAGAGCAGACTGCCCCAG (SEQ ID NO: 1075) | 6.44116741 |
| ZN778 | AVTFDDVAVDFT QEEWTLLDPSQR DLYRDVMLENYE NLASV (SEQ ID NO: 361) | EQTQAAGMVAGWLINCYQDA VTFDDVAVDFTQEEWTLLDPS QRDLYRDVMLENYENLASVEW RLKTKGPALRQDRSWFRA (SEQ ID NO: 362) | GAGCAGACCCAGGCCGCCGGCATGGTGGCCGGCTGGCTGATCAACTGCTACCA GGACGCCGTGACCTTCGACGACGTGGCCGTGGACTTCACCCAGGAGGAGTGGA CCCTGCTGGACCCCAGCCAGAGAGACCTGTACAGAGATGTGATGCTGGAGAAC TACGAGAACCTGGCCAGCGTGGAGTGGAGACTGAAGACCAAGGGCCCCGCCCT GAGACAGGACAGAAGCTGGTTCAGAGCC (SEQ ID NO: 1076) | 6.43548986 |
| RYBP | RPRLKNVDRSTA QQLAVTVGNVTV IITDFKEK (SEQ ID NO: 363) | PSEANSIQSANATTKTSETNHTS RPRLKNVDRSTAQQLAVTVGN VTVIITDFKEKTRSSSTSSSTVTSS AGSEQQNQSSS (SEQ ID NO: 364) | CCCAGCGAGGCCAACAGCATCCAGAGCGCCAACGCCACCACCAAGACCAGCGA GACCAACCACACCAGCAGACCCAGACTGAAGAACGTGGACAGAAGCACCGCCC AGCAGCTGGCCGTGACCGTGGGCAACGTGACCGTGATCATCACCGACTTCAAG GAGAAGACCAGAAGCAGCAGCACCAGCAGCAGCACCGTGACCAGCAGCGCCG GCAGCGAGCAGCAGAACCAGAGCAGCAGC (SEQ ID NO: 1077) | 6.42734946 |
| ZN534 | QLSFSDVAIEFSQ EEWKCLDPGQK ALYRDVMLENYR NLVSL (SEQ ID NO: 365) | ALTQGQLSFSDVAIEFSQEEWK CLDPGQKALYRDVMLENYRNL VSLGEDNVRPEACICSGICLPDL SVTSMLEQKRDPWT (SEQ ID NO: 366) | GCCCTGACCCAGGGCCAGCTGAGCTTCAGCGACGTGGCCATCGAGTTCAGCCAG GAGGAGTGGAAGTGCCTGGACCCCGGCCAGAAGGCCCTGTACAGAGATGTGAT GCTGGAGAACTACAGAAACCTGGTGAGCCTGGGCGAGGACAACGTGAGACCCG AGGCCTGCATCTGCAGCGGCATCTGCCTGCCCGACCTGAGCGTGACCAGCATGC TGGAGCAGAAGAGAGACCCCTGGACC (SEQ ID NO: 1078) | 6.42731382 |
| ZN586 | SVTFEDVAVNFSL EEWSLLNEAQRC LYRDVMLETLTLI SSL (SEQ ID NO: 367) | AAAAALRAPAQSSVTFEDVAV NFSLEEWSLLNEAQRCLYRDV MLETLTLISSLGCWHGGEDEAA PSKQSTCIHIYKDQGG (SEQ ID NO: 368) | GCCGCCGCCGCCGCCCTGAGAGCCCCCGCCCAGAGCAGCGTTACATTCGAGGAC GTGGCCGTGAACTTCAGCCTGGAGGAGTGGAGCCTGCTGAACGAGGCCCAGAG ATGCCTGTACAGAGATGTGATGCTGGAGACCCTGACCCTGATCAGCAGCCTGGG CTGCTGGCACGGCGGCGAGGACGAGGCCGCTCCTAGCAAGCAGAGCACCTGCA TCCACATCTACAAGGACCAGGGCGGC (SEQ ID NO: 1079) | 6.41123861 |
| ZN567 | SVSFNDVTVDFT QEEWQHLDHAQ KTLYMDVMLENY CHLISV (SEQ ID NO: 369) | AQGSVSFNDVTVDFTQEEWQ HLDHAQKTLYMDVMLENYCHL ISVGCHMTKPDVILKLERGEEP WTSFAGHTCLEENWKAE (SEQ ID NO: 370) | GCCCAGGGCAGCGTGAGCTTCAACGACGTGACCGTGGACTTCACCCAGGAGGA GTGGCAGCACCTGGACCACGCCCAGAAGACCCTGTACATGGACGTGATGCTGG AGAACTACTGCCACCTGATCAGCGTGGGCTGCCACATGACCAAGCCCGACGTGA TCCTGAAGCTGGAGAGAGGCGAGGAGCCCTGGACCAGCTTCGCCGGCCACACC TGCCTGGAGGAGAACTGGAAGGCCGAG (SEQ ID NO: 1080) | 6.40288995 |
| ZN440 | PVAFKDVAVNFT QEEWALLDISQR KLYREVMLETFR NLTSL (SEQ ID NO: 371) | DPVAFKDVAVNFTQEEWALLDI SQRKLYREVMLETFRNLTSLGKR WKDQNIEYEHQNPRRNFRSLIE EKVNEIKDDSHCG (SEQ ID NO: 372) | GACCCCGTGGCCTTCAAGGACGTGGCCGTGAACTTCACCCAGGAGGAGTGGGC CCTGCTGGACATCAGCCAGAGAAAGCTGTACAGAGAGGTGATGCTGGAGACCT TCAGAAACCTGACCAGCCTGGGCAAGAGATGGAAGGACCAGAACATCGAGTAC GAGCACCAGAACCCCAGAAGAAACTTCAGAAGCCTGATCGAGGAGAAGGTGAA CGAGATCAAGGACGACAGCCACTGCGGC (SEQ ID NO: 1081) | 6.40187146 |
| ZN583 | LVTFGDVAVNFS QEEWEWLNPAQ RNLYRKVMLENY RSLVSL (SEQ ID NO: 373) | SKDLVTFGDVAVNFSQEEWEW LNPAQRNLYRKVMLENYRSLVS LGVSVSKPDVISLLEQGKEPWM VKKEGTRGPCPDWEY (SEQ ID NO: 374) | AGCAAGGACCTGGTGACCTTCGGCGACGTGGCCGTGAACTTCAGCCAGGAGGA GTGGGAGTGGCTGAACCCCGCCCAGAGAAACCTGTACAGAAAGGTGATGCTGG AGAACTACAGAAGCCTGGTGAGCCTGGGCGTGAGCGTGAGCAAGCCCGACGTG ATCAGCCTGCTGGAGCAGGGCAAGGAGCCCTGGATGGTGAAGAAGGAGGGCA CCAGAGGCCCCTGCCCCGACTGGGAGTAC (SEQ ID NO: 1082) | 6.39776145 |
| ZN441 | SVAFEDVAINFTC EEWALLGPSQKS LYRDVMQETIRN LDCI (SEQ ID NO: 375) | DSVAFEDVAINFTCEEWALLGP SQKSLYRDVMQETIRNLDCIGM IWQNHDIEEDQYKDLRRNLRC HMVERACEIKDNSQC (SEQ ID NO: 376) | GACAGCGTGGCCTTCGAGGACGTGGCCATCAACTTCACCTGCGAGGAGTGGGC CCTGCTGGGCCCAGCCAGAAGAGCCTGTACAGAGATGTGATGCAGGAGACCA TCAGAAACCTGGACTGCATCGGCATGATCTGGCAGAACCACGACATCGAGGAG GACCAGTACAAGGACCTGAGAAGAAACCTGAGATGCCACATGGTGGAGAGAGC CTGCGAGATCAAGGACAACAGCCAGTGC (SEQ ID NO: 1083) | 6.38715626 |
| ZNF43 | PLTFMDVAIEFCL EEWQCLDIAQQ NLYRNVMLENYR NLVFL (SEQ ID NO: 377) | GPLTFMDVAIEFCLEEWQCLDI AQQNLYRNVMLENYRNLVFLGI AVSKPDLITCLEQEKEPWEPMR RHEMVAKPPVMCSH (SEQ ID NO: 378) | GGCCCCCTGACCTTCATGGACGTGGCCATCGAGTTCTGCCTGGAGGAGTGGCAG TGCCTGGACATCGCCCAGCAGAACCTGTACAGAAACGTGATGCTGGAGAACTAC AGAAACCTGGTGTTCCTGGGCATCGCCGTGAGCAAGCCCGACCTGATCACCTGC CTGGAGCAGGAGAAGGAGCCCTGGGAGCCCATGAGAAGACACGAGATGGTGG CCAAGCCCCCCGTGATGTGCAGCCAC (SEQ ID NO: 1084) | 6.38246564 |
| CBX5 | EPEKIIGATDSCG DLMFLMKWKDT DEADLVLAKEAN VKCPQIVIAFYEE RLT (SEQ ID NO: 379) | QSNDIARGFERGLEPEKIIGATD SCGDLMFLMKWKDTDEADLVL AKEANVKCPQIVIAFYEERLTW HAYPEDAENKEKET (SEQ ID NO: 380) | CAGAGCAACGACATCGCCAGAGGCTTCGAGAGAGGCCTGGAGCCCGAGAAGAT CATCGGCGCCACCGACAGCTGCGGCGACCTGATGTTCCTGATGAAGTGGAAGG ACACCGACGAGGCCGACCTGGTGCTGGCCAAGGAGGCCAACGTGAAGTGCCCC CAGATCGTGATCGCCTTCTACGAGGAGAGACTGACCTGGCACGCCTACCCCGAG GACGCCGAGAACAAGGAGAAGGAGACC (SEQ ID NO: 1085) | 6.36905016 |
| ZN589 | PVTFEDVAVLFTE AEWKRLSLEQRN LYKEVMLENLRN LVS (SEQ ID NO: 381) | ALPAKDSAWPWEEKPRYLGPV TFEDVAVLFTEAEWKRLSLEQR NLYKEVMLENLRNLVSLAESKPE VHTCPSCPLAFGSQ (SEQ ID NO: 382) | GCCCTGCCCGCCAAGGACAGCGCCTGGCCCTGGAGGAGAAGCCCAGATACCT GGGCCCCGTGACCTTCGAGGACGTGGCCGTGCTGTTCACCGAGGCCGAGTGGA AGAGACTGAGCCTGGAGCAGAGAAACCTGTACAAGGAGGTGATGCTGGAGAA CCTGAGAAACCTGGTGAGCCTGGCCGAGAGCAAGCCCGAGGTGCACACCTGCC CCAGCTGCCCCCTGGCCTTCGGCAGCCAG (SEQ ID NO: 1086) | 6.36425087 |
| ZNF10 | LVTFKDVFVDFTR EEWKLLDTAQQI VYRNVMLENYKN LVSL (SEQ ID NO: 383) | DAKSLTAWSRTLVTFKDVFVDF TREEWKLLDTAQQIVYRNVML ENYKNLVSLGYQLTKPDVILRLE KGEEPWLVEREIHQ (SEQ ID NO: 384) | GACGCCAAGAGCCTGACCGCCTGGAGCAGAACCCTGGTGACCTTCAAGGACGT GTTCGTGGACTTCACCAGAGAGGAGTGGAAGCTGCTGGACACCGCCCAGCAGA TCGTGTACAGAAACGTGATGCTGGAGAACTACAAGAACCTGGTGAGCCTGGGC TACCAGCTGACCAAGCCCGACGTGATCCTGAGACTGGAGAAGGGCGAGGAGCC CTGGCTGGTGGAGAGAGAGATCCACCAG (SEQ ID NO: 1087) | 6.36134473 |
| ZN563 | AVAFEDVAVNFT QEEWALLGPSQK NLYRYVMQETIR NLDC (SEQ ID NO: 385) | DAVAFEDVAVNFTQEEWALLG PSQKNLYRYVMQETIRNLDCIR MIWEEQNTEDQYKNPRRNLRC HMVERFSESKDSSQCG (SEQ ID NO: 386) | GACGCCGTGGCCTTCGAGGACGTGGCCGTGAACTTCACCCAGGAGGAGTGGGC CCTGCTGGGCCCCAGCCAGAAGAACCTGTACAGATACGTGATGCAGGAGACCA TCAGAAACCTGGACTGCATCAGAATGATCTGGGAGGAGCAGAACACCGAGGAC CAGTACAAGAACCCCAGAAGAAACCTGAGATGCCACATGGTGGAGAGATTCAG CGAGAGCAAGGACAGCAGCCAGTGCGGC (SEQ ID NO: 1088) | 6.3562145 |
| ZN561 | SVTFDDVAVDFT PEEWALLDTTEK YLYRDVMLENYM NLASV (SEQ ID NO: 387) | EKTKVERMVEDYLASGYQDSVT FDDVAVDFTPEEWALLDTTEKY LYRDVMLENYMNLASVEWEIQ PRTKRSSLQQGFLKN (SEQ ID NO: 388) | GAGAAGACCAAGGTGGAGAGAATGGTGGAGGACTACCTGGCCAGCGGCTACC AGGACAGCGTGACCTTCGACGACGTGGCCGTGGACTTCACCCCCGAGGAGTGG GCCCTGCTGGACACCACCGAGAAGTACCTGTACAGAGATGTGATGCTGGAGAA CTACATGAACCTGGCCAGCGTGGAGTGGGAGATCCAGCCCAGAACCAAGAGAA GCAGCCTGCAGCAGGGCTTCCTGAAGAAC (SEQ ID NO: 1089) | 6.3525504 |
| ZN136 | SVAFEDVDVNFT QEEWALLDPSQK NLYRDVMWETM RNLASI (SEQ ID NO: 389) | DSVAFEDVDVNFTQEEWALLD PSQKNLYRDVMWETMRNLASI GKKWKDQNIKDHYKHRGRNLR SHMLERLYQTKDGSQRG (SEQ ID NO: 390) | GACAGCGTGGCCTTCGAGGACGTGGACGTGAACTTCACCCAGGAGGAGTGGGC CCTGCTGGACCCCAGCCAGAAGAACCTGTACAGAGATGTGATGTGGGAGACCA TGAGAAACCTGGCCAGCATCGGCAAGAAGTGGAAGGACCAGAACATCAAGGAC CACTACAAGCACAGAGGCAGAAACCTGAGAAGCCACATGCTGGAGAGACTGTA CCAGACCAAGGACGGCAGCCAGAGAGGC (SEQ ID NO: 1090) | 6.35103846 |
| ZN630 | PVTFEDVAVDFT QEEWQQLNPAQ KTLHRDVMLETY NHLVSV (SEQ ID NO: 391) | IESQEPVTFEDVAVDFTQEEWQ QLNPAQKTLHRDVMLETYNHL VSVGCSGIKPDVIFKLEHGKDP WIIESELSRWIYPDR (SEQ ID NO: 392) | ATCGAGAGCCAGGAGCCCGTGACCTTCGAGGACGTGGCCGTGGACTTCACCCA GGAGGAGTGGCAGCAGCTGAACCCCGCCCAGAAGACCCTGCACAGAGATGTGA TGCTGGAGACCTACAACCACCTGGTGAGCGTGGGCTGCAGCGGCATCAAGCCC GACGTGATCTTCAAGCTGGAGCACGGCAAGGACCCCTGGATCATCGAGAGCGA GCTGAGCAGATGGATCTACCCCGACAGA (SEQ ID NO: 1091) | 6.34648094 |
| ZN527 | LVTFRDVALDFSQ EEWEWLKPSQK DLYRDVMLENYR NLVWL (SEQ ID NO: 393) | AVGLCKAMSQGLVTFRDVALD FSQEEWEWLKPSQKDLYRDVM LENYRNLVWLGLSISKPNMISLL EQGKEPWMVERKMSQ (SEQ ID NO: 394) | GCCGTGGGCCTGTGCAAGGCCATGAGCCAGGGCCTGGTGACCTTCAGAGATGT GGCCCTGGACTTCAGCCAGGAGGAGTGGGAGTGGCTGAAGCCCAGCCAGAAG GACCTGTACAGAGATGTGATGCTGGAGAACTACAGAAACCTGGTGTGGCTGGG CCTGAGCATCAGCAAGCCCAACATGATCAGCCTGCTGGAGCAGGGCAAGGAGC CCTGGATGGTGGAGAGAAAGATGAGCCAG (SEQ ID NO: 1092) | 6.34024936 |
| ZN333 | PVTFADVAVVFT PEEWVFLDSTQR SLYRDVMLENYR NLAS (SEQ ID NO: 395) | DKVEEEAMAPGLPTACSQEPVT FADVAVVFTPEEWVFLDSTQRS LYRDVMLENYRNLASVADQLC KPNALSYLEERGEQW (SEQ ID NO: 396) | GACAAGGTGGAGGAGGAGGCCATGGCCCCGGCCTGCCCACCGCTTGCAGCCA AGAGCCTGTGACCTTCGCCGACGTGGCCGTGGTGTTCACCCCCGAGGAGTGGGT GTTCCTGGACAGCACCCAGAGAAGCCTGTACAGAGATGTGATGCTGGAGAACT ACAGAAACCTGGCCAGCGTGGCCGACCAGCTGTGCAAGCCCAACGCCCTGAGC TACCTGGAGGAGAGAGGCGAGCAGTGG (SEQ ID NO: 1093) | 6.33883721 |
| Z324B | MTFEDVAVYFSQ EEWGLLDTAQRA LYRHVMLENFTL VTSL (SEQ ID NO: 397) | TFEDVAVYFSQEEWGLLDTAQR ALYRHVMLENFTLVTSLGLSTSR PRVVIQLERGEEPWVPSGKDM TLARNTYGRLNSGS (SEQ ID NO: 398) | ACCTTCGAGGACGTGGCCGTGTACTTCAGCCAGGAGGAGTGGGGCCTGCTGGA CACCGCCCAGAGAGCCCTGTACAGACACGTGATGCTGGAGAACTTCACCCTGGT GACCAGCCTGGGCCTGAGCACCAGCAGACCCAGAGTGGTGATCCAGCTGGAGA GAGGCGAGGAGCCCTGGGTGCCCAGCGGCAAGGACATGACCCTGGCCAGAAA CACCTACGGCAGACTGAACAGCGGCAGC (SEQ ID NO: 1094) | 6.33798774 |
| ZN786 | PLTFEDVAIYFSE QEWQDLEAWQ KELYKHVMRSNY ETLVSL (SEQ ID NO: 399) | AEPPRLPLTFEDVAIYFSEQEWQ DLEAWQKELYKHVMRSNYETL VSLDDGLPKPELISWIEHGGEPF RKWRESQKSGNII (SEQ ID NO: 400) | GCCGAGCCCCCAGACTGCCCCTGACCTTCGAGGACGTGGCCATCTACTTCAGC GAGCAGGAGTGGCAGGACCTGGAGGCCTGGCAGAAGGAGCTGTACAAGCACG TGATGAGAAGCAACTACGAGACCCTGGTGAGCCTGGACGACGGCCTGCCCAAG CCCGAGCTGATCAGCTGGATCGAGCACGGCGGCGAGCCCTTCAGAAAGTGGAG AGAGAGCCAGAAGAGCGGCAACATCATC (SEQ ID NO: 1095) | 6.31659272 |
| ZN709 | SVVFEDVAVNFT QEEWALLGPSQK KLYRDVMQETFV NLASI (SEQ ID NO: 401) | DSVVFEDVAVNFTQEEWALLG PSQKKLYRDVMQETFVNLASIG ENWEEKNIEDHKNQGRKLRSH MVERLCERKEGSQFGE (SEQ ID NO: 402) | GACAGCGTGGTGTTCGAGGACGTGGCCGTGAACTTCACCCAGGAGGAGTGGGC CCTGCTGGGCCCCAGCCAGAAGAAGCTGTACAGAGATGTGATGCAGGAGACCT TCGTGAACCTGGCCAGCATCGGCGAGAACTGGGAGGAGAAGAACATCGAGGA CCACAAGAACCAGGGCAGAAAGCTGAGAAGCCACATGGTGGAGAGACTGTGC GAGAGAAAGGAGGGCAGCCAGTTCGGCGAG (SEQ ID NO: 1096) | 6.31480293 |
| ZN792 | CVTFEDVTIYFSQ EEWVLLDEAQRL LYCDVMLENFALI ASL (SEQ ID NO: 403) | AAAALRDPAQGCVTFEDVTIYF SQEEWVLLDEAQRLLYCDVML ENFALIASLGLISFRSHIVSQLEM GKEPWVPDSVDMT(SEQ ID NO: 404) | GCCGCCGCCGCCCTGAGAGACCCCGCCCAGGGCTGCGTGACCTTCGAAGACGT GACCATCTACTTCAGCCAGGAGGAGTGGGTGCTGCTGGACGAGGCCCAGAGAC TGCTGTACTGCGACGTGATGCTGGAGAACTTCGCCCTGATCGCCAGCCTGGGCC TGATCAGCTTCAGAAGCCACATCGTGAGCCAGCTGGAGATGGGCAAGGAGCCC TGGGTGCCCGACAGCGTGGACATGACC (SEQ ID NO: 1097) | 6.29907418 |
| ZN599 | LVSFEDVVVTFTG EEWGHLDLAQRT LYQEVMLETCRLL VSL (SEQ ID NO: 405) | AAPALALVSFEDVVVTFTGEEW GHLDLAQRTLYQEVMLETCRLL VSLGHPVPKPELIYLLEHGQELW TVKRGLSQSTCAG (SEQ ID NO: 406) | GCCGCCCCCGCCCTGGCCCTGGTGAGCTTCGAGGACGTGGTGGTGACCTTCACC GGCGAGGAGTGGGGCCACCTGGACCTGGCCCAGAGAACCCTGTACCAGGAGGT GATGCTGGAGACCTGCAGACTGCTGGTGAGCCTGGGCCACCCCGTGCCCAAGC CCGAGCTGATCTACCTGCTGGAGCACGGCCAGGAGCTGTGGACCGTGAAGAGA GGCCTGAGCCAGAGCACCTGCGCCGGC (SEQ ID NO: 1098) | 6.29676005 |
| ZN613 | SLTLEDVAVEFT WEEWQLLGPAQ KDLYRDVMLENY SNLVSV (SEQ ID NO: 407) | IKSQESLTLEDVAVEFTWEEWQ LLGPAQKDLYRDVMLENYSNLV SVGYQASKPDALFKLEQGEPWT VENEIHSQICPEIK (SEQ ID NO: 408) | ATCAAGAGCCAGGAGAGCCTGACCCTGGAGGACGTGGCCGTGGAGTTCACCTG GGAGGAGTGGCAGCTGCTGGGCCCCGCCCAGAAGGACCTGTACAGAGATGTGA TGCTGGAGAACTACAGCAACCTGGTGAGCGTGGGCTACCAGGCCAGCAAGCCC GACGCCCTGTTCAAGCTGGAGCAGGGCGAGCCCTGGACCGTGGAGAACGAGAT CCACAGCCAGATCTGCCCCGAGATCAAG (SEQ ID NO: 1099) | 6.28970926 |
| ZF69B | LLTFKDVSVDFTQ EEWGQLAPAHR NLYREVMLENYG NLVSV (SEQ ID NO: 409) | GESLESRVTLGSLTAESQELLTFK DVSVDFTQEEWGQLAPAHRNL YREVMLENYGNLVSVGCQLSKP GVISQLEKGEEPW (SEQ ID NO: 410) | GGCGAGAGCCTGGAGAGCAGAGTGACCCTGGGCAGCCTGACCGCCGAGAGCC AGGAGCTGCTGACCTTCAAGGACGTGAGCGTGGACTTCACCCAGGAGGAGTGG GGCCAGCTGGCCCCCGCCCACAGAAACCTGTACAGAGAGGTGATGCTGGAGAA CTACGGCAACCTGGTGAGCGTGGGCTGCCAGCTGAGCAAGCCCGGCGTGATCA GCCAGCTGGAGAAGGGCGAGGAGCCCTGG (SEQ ID NO: 1100) | 6.28648867 |
| ZN799 | SVALEDVAVNFT REEWALLGPCQK NLYKDVMQETIR NLDCV (SEQ ID NO: 411) | ASVALEDVAVNFTREEWALLGP CQKNLYKDVMQETIRNLDCVG MKWKDQNIEDQYRYPRKNLRC RMLERFVESKDGTQCG (SEQ ID NO: 412) | GCCAGCGTGGCCCTGGAGGACGTGGCCGTGAACTTCACCAGAGAGGAGTGGGC CCTGCTGGGCCCCTGCCAGAAGAACCTGTACAAGGACGTGATGCAGGAGACCA TCAGAAACCTGGACTGCGTGGGCATGAAGTGGAAGGACCAGAACATCGAGGAC CAGTACAGATACCCCAGAAAGAACCTGAGATGCAGAATGCTGGAGAGATTCGT GGAGAGCAAGGACGGCACCCAGTGCGGC (SEQ ID NO: 1101) | 6.28580406 |
| ZN569 | TVTFKDVAIDFTQ EEWKRLDPAQRK LYRNVMLENYNN LITV (SEQ ID NO: 413) | TESQGTVTFKDVAIDFTQEEWK RLDPAQRKLYRNVMLENYNNLI TVGYPFTKPDVIFKLEQEEEPW VMEEEVLRRHWQGE (SEQ ID NO: 414) | ACCGAGAGCCAGGGCACCGTGACCTTCAAGGACGTGGCCATCGACTTCACCCAG GAGGAGTGGAAGAGACTGGACCCCGCCCAGAGAAAGCTGTACAGAAACGTGA TGCTGGAGAACTACAACAACCTGATCACCGTGGGCTACCCCTTCACCAAGCCCG ACGTGATCTTCAAGCTGGAGCAGGAGGAGGAGCCCTGGGTGATGGAGGAGGA GGTGCTGAGAAGACACTGGCAGGGCGAG (SEQ ID NO: 1102) | 6.28572758 |
| ZN564 | SVASEDVAVNFT LEEWALLDPSQK KLYRDVMRETFR NLACV (SEQ ID NO: 415) | DSVASEDVAVNFTLEEWALLDP SQKKLYRDVMRETFRNLACVGK KWEDQSIEDWYKNQGRILRNH MEEGLSESKEYDQCG (SEQ ID NO: 416) | GACAGCGTGGCCAGCGAGGACGTGGCCGTGAACTTCACCCTGGAGGAGTGGGC CCTGCTGGACCCCAGCCAGAAGAAGCTGTACAGAGATGTGATGAGAGAGACCT TCAGAAACCTGGCCTGCGTGGGCAAGAAGTGGGAGGACCAGAGCATCGAGGA CTGGTACAAGAACCAGGGCAGAATCCTGAGAAACCACATGGAGGAGGGCCTGA GCGAGAGCAAGGAGTACGACCAGTGCGGC (SEQ ID NO: 1103) | 6.28268424 |
| ZN546 | SLAFRDVSIDLSQ EEWECLDAVQR DLYKDVMLENYS NLVSL (SEQ ID NO: 417) | EETQGELTSSCGSKTMANVSLA FRDVSIDLSQEEWECLDAVQRD LYKDVMLENYSNLVSLGYTIPKP DVITLLEQEKEPW (SEQ ID NO: 418) | GAGGAGACCCAGGGCGAGCTGACCAGCAGCTGCGGCAGCAAGACCATGGCCA ACGTGAGCCTGGCCTTCAGAGATGTGAGCATCGACCTGAGCCAGGAGGAGTGG GAGTGCCTGGACGCCGTGCAGAGAGACCTGTACAAGGACGTGATGCTGGAGAA CTACAGCAACCTGGTGAGCCTGGGCTACACCATCCCCAAGCCCGACGTGATCAC CCTGCTGGAGCAGGAGAAGGAGCCCTGG (SEQ ID NO: 1104) | 6.27774396 |
| ZFP92 | PVSFEDVSVYFTK TEWKLLDLRQKV LYKRVMLENYSH LVSL (SEQ ID NO: 419) | AAILLTTRPKVPVSFEDVSVYFTK TEWKLLDLRQKVLYKRVMLENY SHLVSLGFSFSKPHLISQLERGE GPWVADIPRTW (SEQ ID NO: 420) | GCCGCCATCCTGCTGACCACCAGACCCAAGGTGCCCGTGAGCTTCGAGGACGTG AGCGTGTACTTCACCAAGACCGAGTGGAAGCTGCTGGACCTGAGACAGAAGGT GCTGTACAAGAGAGTGATGCTGGAGAACTACAGCCACCTGGTGAGCCTGGGCT TCAGCTTCAGCAAGCCCCACCTGATCAGCCAGCTGGAGAGAGGCGAGGGCCCC TGGGTGGCCGACATCCCCAGAACCTGG (SEQ ID NO: 1105) | 6.273403 |
| YAF2 | RPRLKNVDRSSA QHLEVTVGDLTVI ITDFKEK (SEQ ID NO: 421) | KDKVEKEKSEKETTSKKNSHKKT RPRLKNVDRSSAQHLEVTVGDL TVIITDFKEKTKSPPASSAASAD QHSQSGSSSDNT (SEQ ID NO: 422) | AAGGACAAGGTGGAGAAGGAGAAGAGCGAGAAGGAGACCACCAGCAAGAAG AACAGCCACAAGAAGACCAGACCCAGACTGAAGAACGTGGACAGAAGCAGCGC CCAGCACCTGGAGGTGACCGTGGGCGACCTGACCGTGATCATCACCGACTTCAA GGAGAAGACCAAGAGCCCCCCGCCAGCAGCGCCGCCAGCGCTGACCAACACA GCCAAAGCGGAAGCAGCAGCGACAACACC(SEQ ID NO: 1106) | 6.25768891 |
| ZN723 | PLTFTDVAIKFSLE EWQFLDTAQQN LYRDVMLENYRN LVFL (SEQ ID NO: 423) | GPLTFTDVAIKFSLEEWQFLDTA QQNLYRDVMLENYRNLVFLGV GVSKPDLITCLEQGKEPWNMK RHKMVAKPPVVCSHF (SEQ ID NO: 424) | GGCCCCCTGACCTTCACCGACGTGGCCATCAAGTTCAGCCTGGAGGAGTGGCAG TTCCTGGACACCGCCCAGCAGAACCTGTACAGAGATGTGATGCTGGAGAACTAC AGAAACCTGGTGTTCCTGGGCGTGGGCGTGAGCAAGCCCGACCTGATCACCTGC CTGGAGCAGGGCAAGGAGCCCTGGAACATGAAGAGACACAAGATGGTGGCCA AGCCCCCCGTGGTGTGCAGCCACTTC (SEQ ID NO: 1107) | 6.25047465 |
| ZNF34 | EVTFEDVAVYLSR EEWGRLGPAQR GLYRDVMLETYG NLVSL (SEQ ID NO: 425) | RKPNPQAMAALFLSAPPQAEV TFEDVAVYLSREEWGRLGPAQR GLYRDVMLETYGNLVSLGVGPA GPKPGVISQLERGDE (SEQ ID NO: 426) | AGAAAGCCAACCCCCAGGCCATGGCCGCCCTGTTCCTGAGCGCCCCCCCTCAA GCCGAAGTGACCTTCGAGGACGTGGCCGTGTACCTGAGCAGAGAGGAGTGGG GCAGACTGGGCCCCGCCCAGAGAGGCCTGTACAGAGATGTGATGCTGGAGACC TACGGCAACCTGGTGAGCCTGGGCGTGGGCCCCGCCGGACCCAAACCTGGAGT GATCAGCCAGCTGGAGAGAGGCGACGAG (SEQ ID NO: 1108) | 6.23513709 |
| ZN439 | PVAFKDVAVNFT QEEWALLDISQK NLYREVMLETFW NLTSI (SEQ ID NO: 427) | LSLSPILLYTCEMFQDPVAFKDV AVNFTQEEWALLDISQKNLYRE VMLETFWNLTSIGKKWKDQNI EYEYQNPRRNFRSV (SEQ ID NO: 428) | CTGAGCCTGAGCCCCATCCTGCTGTACACCTGCGAGATGTTCCAGGACCCCGTG GCCTTCAAGGACGTGGCCGTGAACTTCACCCAGGAGGAGTGGGCCCTGCTGGA CATCAGCCAGAAGAACCTGTACAGAGAGGTGATGCTGGAGACCTTCTGGAACCT GACCAGCATCGGCAAGAAGTGGAAGGACCAGAACATCGAGTACGAGTACCAGA ACCCCAGAAGAAACTTCAGAAGCGTG (SEQ ID NO: 1109) | 6.22934428 |
| ZFP57 | PVTFEDVAVNFT QEEWDCLDASQ RVLYQDVMSETF KNLTS (SEQ ID NO: 429) | AAGEPRSLLFFQKPVTFEDVAV NFTQEEWDCLDASQRVLYQDV MSETFKNLTSVARIFLHKPELITK LEQEEEQWRETRV (SEQ ID NO: 430) | GCCGCCGGCGAGCCCAGAAGCCTGCTGTTCTTCCAGAAGCCCGTGACCTTCGAG GACGTGGCCGTGAACTTCACCCAGGAGGAGTGGGACTGCCTGGACGCCAGCCA GAGAGTGCTGTACCAGGACGTGATGAGCGAGACCTTCAAGAACCTGACCAGCG TGGCCAGAATCTTCCTGCACAAGCCCGAGCTGATCACCAAGCTGGAGCAGGAG GAGGAGCAGTGGAGAGAGACCAGAGTG (SEQ ID NO: 1110) | 6.2234497 |
| ZNF19 | MVTFEDVAVHFT KTEWTGLSPAQR ALYRSVMLENFG NLTAL (SEQ ID NO: 431) | AAMPLKAQYQEMVTFEDVAV HFTKTEWTGLSPAQRALYRSV MLENFGNLTALGYPVPKPALISL LERGDMAWGLEAQDDP (SEQ ID NO: 432) | GCCGCCATGCCCCTGAAGGCCCAGTACCAGGAGATGGTGACCTTCGAGGACGT GGCCGTGCACTTCACCAAGACCGAGTGGACCGGCCTGAGCCCCGCCCAGAGAG CCCTGTACAGAAGCGTGATGCTGGAGAACTTCGGCAACCTGACCGCCCTGGGCT ACCCCGTGCCCAAGCCCGCCCTGATCAGCCTGCTGGAGAGAGGCGACATGGCCT GGGGCCTGGAGGCCCAGGACGACCCC (SEQ ID NO: 1111) | 6.21632085 |
| ZN404 | PLTFSDVAIDFSQ EEWEYLNSDQRD LYRDVMLENYTN LVSL (SEQ ID NO: 433) | ARVPLTFSDVAIDFSQEEWEYL NSDQRDLYRDVMLENYTNLVSL DFNFTTESNKLSSEKRNYEVNAY HQETWKRNKTFNL (SEQ ID NO: 434) | GCCAGAGTGCCCCTGACCTTCAGCGACGTGGCCATCGACTTCAGCCAGGAGGA GTGGGAGTACCTGAACAGCGACCAGAGAGACCTGTACAGAGATGTGATGCTGG AGAACTACACCAACCTGGTGAGCCTGGACTTCAACTTCACCACCGAGAGCAACA AGCTGAGCAGCGAGAAGAGAAACTACGAGGTGAACGCCTACCACCAGGAGACC TGGAAGAGAAACAAGACCTTCAACCTG (SEQ ID NO: 1112) | 6.20126205 |
| ZN274 | PVTFEDVTLGFTP EEWGLLDLKQKS LYREVMLENYRN LVSV (SEQ ID NO: 435) | ASRLPTAWSCEPVTFEDVTLGF TPEEWGLLDLKQKSLYREVMLE NYRNLVSVEHQLSKPDVVSQLE EAEDFWPVERGIPQ (SEQ ID NO: 436) | GCCAGCAGACTGCCCACCGCCTGGAGCTGCGAGCCCGTGACCTTCGAGGACGT GACCCTGGGCTTCACCCCCGAGGAGTGGGGCCTGCTGGACCTGAAGCAGAAGA GCCTGTACAGAGAGGTGATGCTGGAGAACTACAGAAACCTGGTGAGCGTGGAG CACCAGCTGAGCAAGCCCGACGTGGTGAGCCAGCTGGAGGAGGCCGAGGACTT CTGGCCCGTGGAGAGAGGCATCCCCCAG (SEQ ID NO: 1113) | 6.19652061 |
| CBX3 | DPERIIGATDSSG ELMFLMKWKDS DEADLVLAKEAN MKCPQIVIAFYEE RLT (SEQ ID NO: 437) | SKKKRDAADKPRGFARGLDPER IIGATDSSGELMFLMKWKDSDE ADLVLAKEANMKCPQIVIAFYE ERLTWHSCPEDEAQ (SEQ ID NO: 438) | AGCAAGAAGAAGAGAGATGCCGCCGACAAGCCCAGAGGCTTCGCCAGAGGCCT GGACCCCGAGAGAATCATCGGCGCCACCGACAGCAGCGGCGAGCTGATGTTCC TGATGAAGTGGAAGGACAGCGACGAGGCCGACCTGGTGCTGGCCAAGGAGGC CAACATGAAGTGCCCCCAGATCGTGATCGCCTTCTACGAGGAGAGACTGACCTG GCACAGCTGCCCCGAGGACGAGGCCCAG (SEQ ID NO: 1114) | 6.19641648 |
| ZNF30 | SVTFEDVAIAFSQ QEWESLDSSQRG LYRDVMLENYRN LVSM (SEQ ID NO: 439) | AHKYVGLQYHGSVTFEDVAIAF SQQEWESLDSSQRGLYRDVML ENYRNLVSMGHSRSKPHVIALL EQWKEPEVTVRKDGR (SEQ ID NO: 440) | GCCCACAAGTACGTGGGCCTGCAGTACCACGGCAGCGTGACCTTCGAGGACGT GGCCATCGCCTTCAGCCAGCAGGAGTGGGAGAGCCTGGACAGCAGCCAGAGA GGCCTGTACAGAGATGTGATGCTGGAGAACTACAGAAACCTGGTGAGCATGGG CCACAGCAGAAGCAAGCCCCACGTGATCGCCCTGCTGGAGCAGTGGAAGGAGC CCGAGGTGACCGTGAGAAAGGACGGCAGA(SEQ ID NO: 1115) | 6.19503476 |
| ZN250 | KLTFEDVAVLLSQ DEWDRLCPAQR GLYRNVMMETY GNVVSL (SEQ ID NO: 441) | AAARLLPVPAGPQPLSFQAKLT FEDVAVLLSQDEWDRLCPAQR GLYRNVMMETYGNVVSLGLPG SKPDIISQLERGEDPW (SEQ ID NO: 442) | GCCGCCGCCAGACTGCTGCCCGTGCCCGCCGGCCCCAACCTCTTAGCTTCCAG GCCAAGCTGACCTTCGAGGACGTGGCCGTGCTGCTGAGCCAGGACGAGTGGGA CAGACTGTGCCCCGCCCAGAGAGGCCTGTACAGAAACGTGATGATGGAGACCT ACGGCAACGTGGTGAGCCTGGGCCTGCCCGGCAGCAAGCCCGACATCATCAGC CAGCTGGAGAGAGGCGAGGACCCCTGG (SEQ ID NO: 1116) | 6.17058573 |
| ZN570 | LVTFRDVAVDFS QEEWDCLDSSQR HLYSNVMLENYR ILVSL (SEQ ID NO: 443) | AVGLLKAMYQELVTFRDVAVD FSQEEWDCLDSSQRHLYSNVM LENYRILVSLGLCFSKPSVILLLEQ GKAPWMVKRELTK (SEQ ID NO: 444) | GCCGTGGGCCTGCTGAAGGCCATGTACCAGGAGCTGGTGACCTTCAGAGATGT GGCCGTGGACTTCAGCCAGGAGGAGTGGGACTGCCTGGACAGCAGCCAGAGA CACCTGTACAGCAACGTGATGCTGGAGAACTACAGAATCCTGGTGAGCCTGGGC CTGTGCTTCAGCAAGCCCAGCGTGATCCTGCTGCTGGAGCAGGGCAAGGCCCCC TGGATGGTGAAGAGAGAGCTGACCAAG (SEQ ID NO: 1117) | 6.16932644 |
| ZN675 | LLTFRDVAIEFSLE EWQCLDTAQRN LYKNVILENYRNL VFL (SEQ ID NO: 445) | GLLTFRDVAIEFSLEEWQCLDTA QRNLYKNVILENYRNLVFLGIAV SKQDLITCLEQEKEPLTVKRHEM VNEPPVMCSHF (SEQ ID NO: 446) | GGCCTGCTGACCTTCAGAGATGTGGCCATCGAGTTCAGCCTGGAGGAGTGGCA GTGCCTGGACACCGCCCAGAGAAACCTGTACAAGAACGTGATCCTGGAGAACT ACAGAAACCTGGTGTTCCTGGGCATCGCCGTGAGCAAGCAGGACCTGATCACCT GCCTGGAGCAGGAGAAGGAGCCCCTGACCGTGAAGAGACACGAGATGGTGAA CGAGCCCCCCGTGATGTGCAGCCACTTC (SEQ ID NO: 1118) | 6.15995772 |
| ZN695 | LLAFRDVALEFSP EEWECLDPAQRS LYRDVMLENYRN LISL (SEQ ID NO: 447) | GLLAFRDVALEFSPEEWECLDP AQRSLYRDVMLENYRNLISLGE DSFNMQFLFHSLAMSKPELIICL EARKEPWNVNTEK (SEQ ID NO: 448) | GGCCTGCTGGCCTTCAGAGATGTGGCCCTGGAGTTCAGCCCCGAGGAGTGGGA GTGCCTGGACCCCGCCCAGAGAAGCCTGTACAGAGATGTGATGCTGGAGAACT ACAGAAACCTGATCAGCCTGGGCGAGGACAGCTTCAACATGCAGTTCCTGTTCC ACAGCCTGGCCATGAGCAAGCCCGAGCTGATCATCTGCCTGGAGGCCAGAAAG GAGCCCTGGAACGTGAACACCGAGAAG (SEQ ID NO: 1119) | 6.15609798 |
| ZN548 | RVVFEDVAIYFSQ EEWGHLDEAQRL LYRDVMLENLAL LSSL (SEQ ID NO: 449) | NLTEGRVVFEDVAIYFSQEEWG HLDEAQRLLYRDVMLENLALLS SLGSWHGAEDEEAPSQQGFSV GVSEVTASKPCLSSQ (SEQ ID NO: 450) | AACCTGACCGAGGGCAGAGTGGTGTTCGAGGACGTGGCCATCTACTTCAGCCA GGAGGAGTGGGGCCACCTGGACGAGGCCCAGAGACTGCTGTACAGAGATGTG ATGCTGGAGAACCTGGCCCTGCTGAGCAGCCTGGGCAGCTGGCACGGCGCCGA GGACGAAGAGGCCCCTAGCCAGCAGGGCTTCAGCGTGGGCGTGAGCGAGGTG ACCGCCAGCAAGCCCTGCCTGAGCAGCCAG (SEQ ID NO: 1120) | 6.14238152 |
| ZN227 | AVTFKDVAVVFS REELRLLDLTQRK LYRDVMVENFKN LVAV (SEQ ID NO: 451) | SQNYDLPQKKQEKMTKFQEAV TFKDVAVVFSREELRLLDLTQRK LYRDVMVENFKNLVAVGHLPF QPDMVSQLEAEEKLW (SEQ ID NO: 452) | AGCCAGAACTACGACCTGCCCCAGAAGAAGCAGGAGAAGATGACCAAGTTCCA GGAGGCCGTGACCTTCAAGGACGTGGCCGTGGTGTTCAGCAGAGAGGAGCTGA GACTGCTGGACCTGACCCAGAGAAAGCTGTACAGAGATGTGATGGTGGAGAAC TTCAAGAACCTGGTGGCCGTGGGCCACCTGCCCTTCCAGCCCGACATGGTGAGC CAGCTGGAGGCCGAGGAGAAGCTGTGG (SEQ ID NO: 1121) | 6.13508917 |
| ZN132 | MVTFEDVAVYFS QEEWELLDAAQR HLYHSVMLENLE LVTSL (SEQ ID NO: 453) | GPAQHTSWPCGSAVPTLKSMV TFEDVAVYFSQEEWELLDAAQR HLYHSVMLENLELVTSLGSWH GVEGEGAHPKQNVSVE (SEQ ID NO: 454) | GGCCCCGCCCAGCACACCAGCTGGCCCTGCGGCAGCGCCGTTCCTACACTGAAG AGCATGGTGACCTTCGAGGACGTGGCCGTGTACTTCAGCCAGGAGGAGTGGGA GCTGCTGGACGCCGCCCAGAGACACCTGTACCACAGCGTGATGCTGGAGAACCT GGAGCTGGTGACCAGCCTGGGCAGCTGGCACGGCGTGGAGGGCGAGGGAGCT CACCCCAAGCAGAACGTGAGCGTGGAG (SEQ ID NO: 1122) | 6.13316124 |
| ZN738 | PLTFRDVVIEFSQ EEWQCLDTAQQ DLYRKVMLENFR NLVFL (SEQ ID NO: 455) | SGYPGAERNLLEYSYFEKGPLTF RDVVIEFSQEEWQCLDTAQQD LYRKVMLENFRNLVFLGIDVSKP DLITCLEQGKDPW (SEQ ID NO: 456) | AGCGGCTACCCCGGCGCCGAGAGAAACCTGCTGGAGTACAGCTACTTCGAGAA GGGCCCCCTGACCTTCAGAGATGTGGTGATCGAGTTCAGCCAGGAGGAGTGGC AGTGCCTGGACACCGCCCAGCAGGACCTGTACAGAAAGGTGATGCTGGAGAAC TTCAGAAACCTGGTGTTCCTGGGCATCGACGTGAGCAAGCCCGACCTGATCACC TGCCTGGAGCAGGGCAAGGACCCCTGG (SEQ ID NO: 1123) | 6.12742065 |
| ZN420 | LVMFRDVAIDFS QEEWECLDSAQR DLYRDVMLENYS NLVSL (SEQ ID NO: 457) | ARKLVMFRDVAIDFSQEEWECL DSAQRDLYRDVMLENYSNLVSL DLPSRCASKDLSPEKNTYETELS QWEMSDRLENCDL (SEQ ID NO: 458) | GCCAGAAAGCTGGTGATGTTCAGAGATGTGGCCATCGACTTCAGCCAGGAGGA GTGGGAGTGCCTGGACAGCGCCCAGAGAGACCTGTACAGAGATGTGATGCTGG AGAACTACAGCAACCTGGTGAGCCTGGACCTGCCCAGCAGATGCGCCAGCAAG GACCTGAGCCCCGAGAAGAACACCTACGAGACCGAGCTGAGCCAGTGGGAGAT GAGCGACAGACTGGAGAACTGCGACCTG (SEQ ID NO: 1124) | 6.1074573 |
| ZN514 | MTFEDVAVEFSQ WEWGQLNPAQ KDLYREVMLENF RNLAIL (SEQ ID NO: 459) | TFEDVAVEFSQWEWGQLNPA QKDLYREVMLENFRNLAILGLLV SKPYVICQLEEGGEPFMVEREIS TGAHSDWKRRSKSK (SEQ ID NO: 460) | ACCTTCGAGGACGTGGCCGTGGAGTTCAGCCAGTGGGAGTGGGGCCAGCTGAA CCCCGCCCAGAAGGACCTGTACAGAGAGGTGATGCTGGAGAACTTCAGAAACC TGGCCATCCTGGGCCTGCTGGTGAGCAAGCCCTACGTGATCTGCCAGCTGGAGG AGGGCGGCGAGCCCTTCATGGTGGAGAGAGAGATCAGCACCGGCGCCCACAGC GACTGGAAGAGAAGAAGCAAGAGCAAG (SEQ ID NO: 1125) | 6.10685195 |
| ZN626 | PLQFRDVAIEFSL EEWHCLDTAQR NLYRNVMLENYS NLVFL (SEQ ID NO: 461) | GPLQFRDVAIEFSLEEWHCLDT AQRNLYRNVMLENYSNLVFLGI TVSKPDLITCLEQGRKPLTMKR NEMIAKPSVMCSHF (SEQ ID NO: 462) | GGCCCCCTGCAGTTCAGAGATGTGGCCATCGAGTTCAGCCTGGAGGAGTGGCA CTGCCTGGACACCGCCCAGAGAAACCTGTACAGAAACGTGATGCTGGAGAACT ACAGCAACCTGGTGTTCCTGGGCATCACCGTGAGCAAGCCCGACCTGATCACCT GCCTGGAGCAGGGCAGAAAGCCCCTGACCATGAAGAGAAACGAGATGATCGCC AAGCCCAGCGTGATGTGCAGCCACTTC (SEQ ID NO: 1126) | 6.10541852 |
| ZN806 | RVTFKDIAVIFTKE ELAVLDKAQINLY QDVMLENFRNFI SV (SEQ ID NO: 463) | IKFQERVTFKDIAVIFTKEELAVL DKAQINLYQDVMLENFRNFISV DGIKNNILNLQGKGLIYLSQEEL HCWKIWKQRIR (SEQ ID NO: 464) | ATCAAGTTCCAGGAGAGAGTGACCTTCAAGGACATCGCCGTGATCTTCACCAAG GAGGAGCTGGCCGTGCTGGACAAGGCCCAGATCAACCTGTACCAGGACGTGAT GCTGGAGAACTTCAGAAACTTCATCAGCGTGGACGGCATCAAGAACAACATCCT GAACCTGCAGGGCAAGGGCCTGATCTACCTGAGCCAGGAGGAGCTGCACTGCT GGAAGATCTGGAAGCAGAGAATCAGA (SEQ ID NO: 1127) | 6.09805184 |
| ZN559 | SVTFEDVAVDFT QEEWTLLDQTQR NLYRDVMLENYK NLVAV (SEQ ID NO: 465) | VAGWLTNYSQDSVTFEDVAVD FTQEEWTLLDQTQRNLYRDVM LENYKNLVAVDWESHINTKWS APQQNFLQGKTSSVVEM (SEQ ID NO: 466) | GTGGCCGGCTGGCTGACCAACTACAGCCAGGACAGCGTGACCTTCGAGGACGT GGCCGTGGACTTCACCCAGGAGGAGTGGACCCTGCTGGACCAGACCCAGAGAA ACCTGTACAGAGATGTGATGCTGGAGAACTACAAGAACCTGGTGGCCGTGGAC TGGGAGAGCCACATCAACACCAAGTGGAGCGCCCCCCAGCAGAACTTCCTGCA GGGCAAGACCAGCAGCGTGGTGGAGATG (SEQ ID NO: 1128) | 6.09618421 |
| ZN460 | SVTFEDVAVTFT QEEWGQLDVTQ RALYVEVMLETC GLLVAL (SEQ ID NO: 467) | AAAWMAPAQESVTFEDVAVTF TQEEWGQLDVTQRALYVEVML ETCGLLVALGDSTKPETVEPIPS HLALPEEVSLQEQLA (SEQ ID NO: 468) | GCCGCCGCCTGGATGGCCCCGCCCAGGAGAGCGTGACCTTCGAGGACGTGGC CGTGACCTTCACCCAGGAGGAGTGGGGCCAGCTGGACGTGACCCAGAGAGCCC TGTACGTGGAGGTGATGCTGGAGACCTGCGGCCTGCTGGTGGCCCTGGGCGAC AGCACCAAGCCCGAGACCGTGGAGCCCATCCCCAGCCACCTGGCCCTGCCCGAG GAAGTGAGCCTGCAGGAGCAGCTGGCC (SEQ ID NO: 1129) | 6.08494207 |
| ZN268 | PLSFMDVFVDFT WEEWQLLDPAQ KCLYRSVMLENY SNLVSL (SEQ ID NO: 469) | VLEWLFISQEQPKITKSWGPLSF MDVFVDFTWEEWQLLDPAQK CLYRSVMLENYSNLVSLGYQHT KPDIIFKLEQGEELC (SEQ ID NO: 470) | GTGCTGGAGTGGCTGTTCATCAGCCAGGAGCAGCCCAAGATCACCAAGAGCTG GGGCCCCCTGAGCTTCATGGACGTGTTCGTGGACTTCACCTGGGAGGAGTGGCA GCTGCTGGACCCCGCCCAGAAGTGCCTGTACAGAAGCGTGATGCTGGAGAACT ACAGCAACCTGGTGAGCCTGGGCTACCAGCACACCAAGCCCGACATCATCTTCA AGCTGGAGCAGGGCGAGGAGCTGTGC (SEQ ID NO: 1130) | 6.040812 |
| ZN304 | CVTFEDVFVYFSR EEWELLEEAQRFL YRDVMLENFALV ATL (SEQ ID NO: 471) | AAAVLMDRVQSCVTFEDVFVY FSREEWELLEEAQRFLYRDVML ENFALVATLGFWCEAEHEAPSE QSVSVEGVSQVRTAE (SEQ ID NO: 472) | GCCGCCGCCGTGCTGATGGACAGAGTGCAGAGCTGCGTGACCTTCGAGGACGT GTTCGTGTACTTCAGCAGAGAGGAGTGGGAGCTGCTGGAGGAGGCCCAGAGAT TCCTGTACAGAGATGTGATGCTGGAGAACTTCGCCCTGGTGGCCACCCTGGGCT TCTGGTGCGAGGCCGAGCACGAGGCTCCCAGCGAGCAGAGCGTGAGCGTGGA GGGCGTGAGCCAGGTGAGAACCGCCGAG (SEQ ID NO: 1131) | 6.03800144 |
| ZIM2 | LVTFEDVLVDFSP EELSSLSAAQRNL YREVMLENYRNL VSL (SEQ ID NO: 473) | AGSQFPDFKHLGTFLVFEELVTF EDVLVDFSPEELSSLSAAQRNLY REVMLENYRNLVSLGHQFSKPD IISRLEEEESYA (SEQ ID NO: 474) | GCCGGCAGCCAGTTCCCCGACTTCAAGCACCTGGGCACCTTCCTGGTGTTCGAG GAGCTGGTGACCTTCGAGGACGTGCTGGTGGACTTCAGCCCCGAGGAGCTGAG CAGCCTGAGCGCCGCCCAGAGAAACCTGTACAGAGAGGTGATGCTGGAGAACT ACAGAAACCTGGTGAGCCTGGGCCACCAGTTCAGCAAGCCCGACATCATCAGCA GACTGGAGGAGGAGGAGAGCTACGCC (SEQ ID NO: 1132) | 6.03746453 |
| ZN605 | QISFEDVAVDFTL EEWQLLNPTQKN LYRDVMLENYSN LVFL (SEQ ID NO: 475) | IQSQISFEDVAVDFTLEEWQLLN PTQKNLYRDVMLENYSNLVFLE VWLDNPKMWLRDNQDNLKS MERGHKYDVFGKIFNS (SEQ ID NO: 476) | ATCCAGAGCCAGATCAGCTTCGAGGACGTGGCCGTGGACTTCACCCTGGAGGA GTGGCAGCTGCTGAACCCCACCCAGAAGAACCTGTACAGAGATGTGATGCTGG AGAACTACAGCAACCTGGTGTTCCTGGAGGTGTGGCTGGACAACCCCAAGATGT GGCTGAGAGACAACCAGGACAACCTGAAGAGCATGGAGAGAGGCCACAAGTA CGACGTGTTCGGCAAGATCTTCAACAGC (SEQ ID NO: 1133) | 6.01346476 |
| ZN844 | LVAFEDVAVNFT QEEWSLLDPSQK NLYREVMQETLR NLASI (SEQ ID NO: 477) | DLVAFEDVAVNFTQEEWSLLDP SQKNLYREVMQETLRNLASIGE KWKDQNIEDQYKNPRNNLRSL LGERVDENTEENHCG (SEQ ID NO: 478) | GACCTGGTGGCCTTCGAGGACGTGGCCGTGAACTTCACCCAGGAGGAGTGGAG CCTGCTGGACCCCAGCCAGAAGAACCTGTACAGAGAGGTGATGCAGGAGACCC TGAGAAACCTGGCCAGCATCGGCGAGAAGTGGAAGGACCAGAACATCGAGGA CCAGTACAAGAACCCCAGAAACAACCTGAGAAGCCTGCTGGGCGAGAGAGTGG ACGAGAACACCGAGGAGAACCACTGCGGC (SEQ ID NO: 1134) | 5.98806163 |
| SUMO5 | IKLRVIGQDSSEIH FKVKMTTPLKKLK KSYCQRQGVPVN SLRFLFEGQRIAD NHTPEELGMEEE DVIEVY (SEQ ID NO: 479) | KDEDIKLRVIGQDSSEIHFKVKM TTPLKKLKKSYCQRQGVPVNSL RFLFEGQRIADNHTPEELGMEE EDVIEVYQEQIGG (SEQ ID NO: 480) | AAGGACGAGGACATCAAGCTGAGAGTGATCGGCCAGGACAGCAGCGAGATCC ACTTCAAGGTGAAGATGACCACCCCCCTGAAGAAGCTGAAGAAGAGCTACTGCC AGAGACAGGGCGTGCCCGTGAACAGCCTGAGATTCCTGTTCGAGGGCCAGAGA ATCGCCGACAACCACACCCCCGAGGAGCTGGGCATGGAGGAGGAGGACGTGAT CGAGGTGTACCAGGAGCAGATCGGCGGC (SEQ ID NO: 1135) | 5.96583945 |
| ZN101 | SVAFEDVAVNFT QEEWALLSPSQK NLYRDVTLETFRN LASV (SEQ ID NO: 481) | DSVAFEDVAVNFTQEEWALLSP SQKNLYRDVTLETFRNLASVGIQ WKDQDIENLYQNLGIKLRSLVE RLCGRKEGNEHRE (SEQ ID NO: 482) | GACAGCGTGGCCTTCGAGGACGTGGCCGTGAACTTCACCCAGGAGGAGTGGGC CCTGCTGAGCCCCAGCCAGAAGAACCTGTACAGAGATGTGACCCTGGAGACCTT CAGAAACCTGGCCAGCGTGGGCATCCAGTGGAAGGACCAGGACATCGAGAACC TGTACCAGAACCTGGGCATCAAGCTGAGAAGCCTGGTGGAGAGACTGTGCGGC AGAAAGGAGGGCAACGAGCACAGAGAG (SEQ ID NO: 1136) | 5.90648424 |
| ZN783 | PVTFDDVAVYFSE LEWGKLEDWQK ELYKHVMRGNYE TLVSL (SEQ ID NO: 483) | RNFWILRLPPGSKGEAPKVPVT FDDVAVYFSELEWGKLEDWQK ELYKHVMRGNYETLVSLDYAISK PDILTRIERGEEPC (SEQ ID NO: 484) | AGAAACTTCTGGATCCTGAGACTGCCCCCGGCAGCAAGGGCGAGGCCCCAA GGTGCCCGTGACATTCGACGACGTGGCCGTGTACTTCAGCGAGCTGGAGTGGG GCAAGCTGGAGGACTGGCAGAAGGAGCTGTACAAGCACGTGATGAGAGGCAA CTACGAGACCCTGGTGAGCCTGGACTACGCCATCAGCAAGCCCGACATCCTGAC CAGAATCGAGAGAGGCGAGGAGCCCTGC (SEQ ID NO: 1137) | 5.87160607 |
| ZN417 | TVTFEDVAVNFS QEEWCLLSEAQR CLYRDVMLENLA LISSL (SEQ ID NO: 485) | AAAAPRRPTQQGTVTFEDVAV NFSQEEWCLLSEAQRCLYRDV MLENLALISSLGCWCGSKDEEA PCKQRISVQRESQSRT (SEQ ID NO: 486) | GCCGCCGCCGCCCCAGAAGACCCACCCAGCAGGGCACCGTGACCTTCGAGGA CGTGGCCGTGAACTTCAGCCAGGAGGAGTGGTGCCTGCTGAGCGAGGCCCAGA GATGCCTGTACAGAGATGTGATGCTGGAGAACCTGGCCCTGATCAGCAGCCTG GGCTGCTGGTGCGGCAGCAAGGACGAGGAGGCCCCCTGCAAGCAGAGAATCA GCGTGCAGAGAGAGAGCCAGAGCAGAACC (SEQ ID NO: 1138) | 5.85910987 |
| ZN182 | LVTFEDVAVDFT QEEWQYLNPPQ RTLYRDVMLETYS NLVFV (SEQ ID NO: 487) | SGEDSGSFYSWQKAKREQGLV TFEDVAVDFTQEEWQYLNPPQ RTLYRDVMLETYSNLVFVGQQV TKPNLILKLEVEECPA (SEQ ID NO: 488) | AGCGGCGAGGACAGCGGCAGCTTCTACAGCTGGCAGAAGGCCAAGAGAGAGC AGGGCCTGGTGACCTTCGAGGACGTGGCCGTGGACTTCACCCAGGAGGAGTGG CAGTACCTGAACCCCCCCCAGAGAACCCTGTACAGAGATGTGATGCTGGAGACC TACAGCAACCTGGTGTTCGTGGGCCAGCAGGTGACCAAGCCCAACCTGATCCTG AAGCTGGAGGTGGAGGAGTGCCCCGCC (SEQ ID NO: 1139) | 5.80251318 |
| ZN823 | SVAFEDVAVNFT QEEWALLGPSQK SLYRNVMQETIR NLDCI (SEQ ID NO: 489) | DSVAFEDVAVNFTQEEWALLG PSQKSLYRNVMQETIRNLDCIE MKWEDQNIGDQCQNAKRNLR SHTCEIKDDSQCGETFG (SEQ ID NO: 490) | GACAGCGTGGCCTTCGAGGACGTGGCCGTGAACTTCACCCAGGAGGAGTGGGC CCTGCTGGGCCCCAGCCAGAAGAGCCTGTACAGAAACGTGATGCAGGAGACCA TCAGAAACCTGGACTGCATCGAGATGAAGTGGGAGGACCAGAACATCGGCGAC CAGTGCCAGAACGCCAAGAGAAACCTGAGAAGCCACACCTGCGAGATCAAGGA CGACAGCCAGTGCGGCGAGACCTTCGGC (SEQ ID NO: 1140) | 5.75436578 |
| ZN177 | SVTFQEVAVDFS QEEWALLDPAQK NLYKDVMLENFR NLASV (SEQ ID NO: 491) | AAGWLTTWSQNSVTFQEVAV DFSQEEWALLDPAQKNLYKDV MLENFRNLASVGYQLCRHSLIS KVDQEQLKTDERGILQG (SEQ ID NO: 492) | GCCGCCGGCTGGCTGACCACCTGGAGCCAGAACAGCGTGACCTTCCAGGAGGT GGCCGTGGACTTCAGCCAGGAGGAGTGGGCCCTGCTGGACCCCGCCCAGAAGA ACCTGTACAAGGACGTGATGCTGGAGAACTTCAGAAACCTGGCCAGCGTGGGC TACCAGCTGTGCAGACACAGCCTGATCAGCAAGGTGGACCAGGAGCAGCTGAA GACCGACGAGAGAGGCATCCTGCAGGGC (SEQ ID NO: 1141) | 5.66150299 |
| ZN197 | LVMFEEVSVCFTS EEWACLGPIQRA LYWDVMLENYG NVTSL (SEQ ID NO: 493) | ENPRNQLMALMLLTAQPQELV MFEEVSVCFTSEEWACLGPIQR ALYWDVMLENYGNVTSLEWET MTENEEVTSKPSSSQR (SEQ ID NO: 494) | GAGAACCCCAGAAACCAGCTGATGGCCCTGATGCTGCTGACCGCCCAGCCCCAG GAGCTGGTGATGTTCGAGGAGGTGAGCGTGTGCTTCACCAGCGAGGAGTGGGC CTGCCTGGGCCCCATCCAGAGAGCCCTGTACTGGGACGTGATGCTGGAGAACTA CGGCAACGTGACCAGCCTGGAGTGGGAGACCATGACCGAGAACGAGGAGGTG ACCAGCAAGCCCAGCAGCAGCCAGAGA (SEQ ID NO: 1142) | 5.65816459 |
| ZN717 | LVSFEEVAVHFT WEEWQDLDDA QRTLYRDVMLET YSSLVSL (SEQ ID NO: 495) | LETYNSLVSLQELVSFEEVAVHF TWEEWQDLDDAQRTLYRDVM LETYSSLVSLGHCITKPEMIFKLE QGAEPWIVEETPN (SEQ ID NO: 496) | CTGGAGACCTACAACAGCCTGGTGAGCCTGCAGGAGCTGGTGAGCTTCGAGGA GGTGGCCGTGCACTTCACCTGGGAGGAGTGGCAGGACCTGGACGACGCCCAGA GAACCCTGTACAGAGATGTGATGCTGGAGACCTACAGCAGCCTGGTGAGCCTG GGCCACTGCATCACCAAGCCCGAGATGATCTTCAAGCTGGAGCAGGGCGCCGA GCCCTGGATCGTGGAGGAGACCCCCAAC (SEQ ID NO: 1143) | 5.64802359 |
| ZN669 | SVAFEDVAVNFT QEEWALLDSSQK NLYREVMQETCR NLASV (SEQ ID NO: 497) | RHFRRPEPCREPLASPIQDSVAF EDVAVNFTQEEWALLDSSQKN LYREVMQETCRNLASVGSQWK DQNIEDHFEKPGKDI (SEQ ID NO: 498) | AGACACTTCAGAAGACCCGAGCCCTGCAGAGAGCCCCTGGCCAGCCCCATCCAG GACAGCGTGGCCTTCGAGGACGTGGCCGTGAACTTCACCCAGGAGGAGTGGGC CCTGCTGGACAGCAGCCAGAAGAACCTGTACAGAGAGGTGATGCAGGAGACCT GCAGAAACCTGGCCAGCGTGGGCAGCCAGTGGAAGGACCAGAACATCGAGGA CCACTTCGAGAAGCCCGGCAAGGACATC (SEQ ID NO: 1144) | 5.58623836 |
| ZN256 | IVTFEDVAVYFS WKEWGLLDEAQ KCLYHDVMLENL TLTTSL (SEQ ID NO: 499) | AAAELTAPAQGIVTFEDVAVYF SWKEWGLLDEAQKCLYHDVM LENLTLTTSLGGSGAGDEEAPY QQSTSPQRVSQVRIPK (SEQ ID NO: 500) | GCCGCCGCCGAGCTGACCGCCCCCGCCCAGGGCATCGTGACCTTCGAAGACGTG GCCGTGTACTTCAGCTGGAAGGAGTGGGGCCTGCTGGACGAGGCCCAGAAGTG CCTGTACCACGACGTGATGCTGGAGAACCTGACCCTGACCACCAGCCTGGGCGG CAGCGGCGCCGGAGATGAGGAGGCTCCTTACCAGCAGAGCACCAGCCCCCAGA GAGTGAGCCAGGTGAGAATCCCCAAG (SEQ ID NO: 1145) | 5.57864488 |
| ZN251 | PLTFQDVAVYFS QAEGRQLGPQQ RALYRDVMLENY GNVASL (SEQ ID NO: 501) | AATFQLPGHQEMPLTFQDVAV YFSQAEGRQLGPQQRALYRDV MLENYGNVASLGFPVPKPELIS QLEQGKELWVLNLLGA (SEQ ID NO: 502) | GCCGCCACCTTCCAGCTGCCCGGCCACCAGGAGATGCCCCTGACCTTCCAGGAC GTGGCCGTGTACTTCAGCCAGGCCGAGGGCAGACAGCTGGGCCCCCAGCAGAG AGCCCTGTACAGAGATGTGATGCTGGAGAACTACGGCAACGTGGCCAGCCTGG GCTTCCCCGTGCCCAAGCCCGAGCTGATCAGCCAGCTGGAGCAGGGCAAGGAG CTGTGGGTGCTGAACCTGCTGGGCGCC (SEQ ID NO: 1146) | 5.54680119 |
| CBX4 | SEFKPFFGNIIITD VTANCLTVTFKEY VT (SEQ ID NO: 503) | RSEAGEPPSSLQVKPETPASAA VAVAAAAAPTTTAEKPPAEAQ DEPAESLSEFKPFFGNIIIITDVTA NCLTVTFKEYVTV (SEQ ID NO: 504) | AGAAGCGAGGCCGGCGAGCCCCCAGCAGCCTGCAGGTGAAGCCCGAGACACC TGCCAGCGCCGCTGTGGCCGTGGCTGCTGCTGCTCCCACCACCACAGCCGA GAAGCCCCCCGCCGAGGCCCAGGACGAACCTGCCGAGAGCCTGAGCGAGTTCA AGCCCTTCTTCGGCAACATCATCATCACCGACGTGACCGCCAACTGCCTGACCGT GACCTTCAAGGAGTACGTGACCGTG (SEQ ID NO: 1147) | 5.47206529 |
| PCGF2 | MCALCGGYFIDA TTIVECLHSFCKTC IVRYLETNKYCPM (SEQ ID NO: 505) | HRTTRIKITELNPHLMCALCGGY FIDATTIVECLHSFCKTCIVRYLET NKYCPMCDVQVHKTRPLLSIRS DKTLQDIVYK (SEQ ID NO: 506) | CACAGAACCACCAGAATCAAGATCACCGAGCTGAACCCCCACCTGATGTGCGCC CTGTGCGGCGGCTACTTCATCGACGCCACCACCATCGTGGAGTGCCTGCACAGC TTCTGCAAGACCTGCATCGTGAGATACCTGGAGACCAACAAGTACTGCCCCATG TGCGACGTGCAGGTGCACAAGACCAGACCCCTGCTGAGCATCAGAAGCGACAA GACCCTGCAGGACATCGTGTACAAG (SEQ ID NO: 1148) | 5.41711547 |
| CDY2 | FEVEAIVDKRQD KNGNTQYLVRW KGYDKQDDTWE PEQHLMNCEKCV HDFNR (SEQ ID NO: 507) | ASQEFEVEAIVDKRQDKNGNT QYLVRWKGYDKQDDTWEPEQ HLMNCEKCVHDFNRRQTEKQK KLTWTTTSRIFSNNARRR (SEQ ID NO: 508) | GCCAGCCAGGAGTTCGAGGTGGAGGCCATCGTGGACAAGAGACAGGACAAGA ACGGCAACACCCAGTACCTGGTGAGATGGAAGGGCTACGACAAGCAGGACGAC ACCTGGGAGCCCGAGCAGCACCTGATGAACTGCGAGAAGTGCGTGCACGACTT CAACAGAAGACAGACCGAGAAGCAGAAGAAGCTGACCTGGACCACCACCAGCA GAATCTTCAGCAACAACGCCAGAAGAAGA (SEQ ID NO: 1149) | 5.20865573 |
| CDYL2 | YEVERIVDKRKNK KGKWEYLIRWKG YGSTEDTWEPEH HLLHCEEFIDEFN (SEQ ID NO: 509) | ASGDLYEVERIVDKRKNKKGKW EYLIRWKGYGSTEDTWEPEHHL LHCEEFIDEFNGLHMSKDKRIKS GKQSSTSKLLRDS (SEQ ID NO: 510) | GCCAGCGGCGACCTGTACGAGGTGGAGAGAATCGTGGACAAGAGAAAGAACA AGAAGGGCAAGTGGGAGTACCTGATCAGATGGAAGGGCTACGGCAGCACCGA GGACACCTGGGAGCCCGAGCACCACCTGCTGCACTGCGAGGAGTTCATCGACG AGTTCAACGGCCTGCACATGAGCAAGGACAAGAGAATCAAGAGCGGCAAGCAG AGCAGCACCAGCAAGCTGCTGAGAGACAGC (SEQ ID NO: 1150) | 5.17777542 |
| ZN287 | SMTFKDVAVDIT QEDWELMRPVQ KELYKTVTLQNY WNMVSL (SEQ ID NO: 511) | PRGKHAFQTGWLNDLVTKESM TFKDVAVDITQEDWELMRPVQ KELYKTVTLQNYWNMVSLGLT VYRPTVIPILEEPWMVI (SEQ ID NO: 512) | CCCAGAGGCAAGCACGCCTTCCAGACCGGCTGGCTGAACGACCTGGTGACCAA GGAGAGCATGACCTTCAAGGACGTGGCCGTGGACATCACCCAGGAGGACTGGG AGCTGATGAGACCCGTGCAGAAGGAGCTGTACAAGACCGTGACCCTGCAGAAC TACTGGAACATGGTGAGCCTGGGCCTGACCGTGTACAGACCCACCGTGATCCCC ATCCTGGAGGAGCCCTGGATGGTGATC (SEQ ID NO: 1151) | 5.15786106 |
| HERC2 | RKADLENHNKDG GFWTVIDGKVYD IKDFQTQSLTGNS ILAQFAGEDPVV ALEAALQFEDTRE SMHAFCVGQY (SEQ ID NO: 513) | TLIRKADLENHNKDGGFWTVID GKVYDIKDFQTQSLTGNSILAQF AGEDPVVALEAALQFEDTRES MHAFCVGQYLEPDQ (SEQ ID NO: 514) | ACCCTGATCAGAAAGGCCGACCTGGAGAACCACAACAAGGACGGCGGCTTCTG GACCGTGATCGACGGCAAGGTGTACGACATCAAGGACTTCCAGACCCAGAGCC TGACCGGCAACAGCATCCTGGCCCAGTTCGCCGGCGAGGACCCCGTGGTGGCC CTGGAAGCTGCTCTGCAGTTCGAGGACACCAGAGAGAGCATGCACGCCTTCTGC GTGGGCCAGTACCTGGAGCCCGACCAG (SEQ ID NO: 1152) | 5.12990133 |
| ZN562 | SVTFDDVAVEFTP EEWALLDTTQKY LYRDVMLENYM NLASV (SEQ ID NO: 515) | EKTKIGTMVEDHRSNSYQDSVT FDDVAVEFTPEEWALLDTTQKY LYRDVMLENYMNLASVDFFFCL TSEWEIQPRTKRSS (SEQ ID NO: 516) | GAGAAGACCAAGATCGGCACCATGGTGGAGGACCACAGAAGCAACAGCTACCA GGACAGCGTGACCTTCGACGACGTGGCCGTGGAGTTCACCCCCGAGGAGTGGG CCCTGCTGGACACCACCCAGAAGTACCTGTACAGAGATGTGATGCTGGAGAACT ACATGAACCTGGCCAGCGTGGACTTCTTCTGCCTGACCAGCGAGTGGGAGA TCCAGCCCAGAACCAAGAGAAGCAGC (SEQ ID NO: 1153) | 5.08331004 |
| ZN461 | LVMFRDVAIDVS QEEWECLNPAQ RNLYKEVMLENY SNLVSL (SEQ ID NO: 517) | AHELVMFRDVAIDVSQEEWEC LNPAQRNLYKEVMLENYSNLVS LGLSVSKPAVISSLEQGKEPWM VVREETGRWCPGTWK (SEQ ID NO: 518) | GCCCACGAGCTGGTGATGTTCAGAGATGTGGCCATCGACGTGAGCCAGGAGGA GTGGGAGTGCCTGAACCCCGCCCAGAGAAACCTGTACAAGGAGGTGATGCTGG AGAACTACAGCAACCTGGTGAGCCTGGGCCTGAGCGTGAGCAAGCCCGCCGTG ATCAGCAGCCTGGAGCAGGGCAAGGAGCCCTGGATGGTGGTGAGAGAGGAGA CCGGCAGATGGTGCCCCGGCACCTGGAAG (SEQ ID NO: 1154) | 5.05101639 |
| Z324A | MAFEDVAVYFSQ EEWGLLDTAQRA LYRRVMLDNFAL VASL (SEQ ID NO: 519) | AFEDVAVYFSQEEWGLLDTAQ RALYRRVMLDNFALVASLGLST SRPRVVIQLERGEEPWVPSGTD TTLSRTTYRRRNPGS (SEQ ID NO: 520) | GCCTTCGAGGACGTGGCCGTGTACTTCAGCCAGGAGGAGTGGGGCCTGCTGGA CACCGCCCAGAGAGCCCTGTACAGAAGAGTGATGCTGGACAACTTCGCCCTGGT GGCCAGCCTGGGCCTGAGCACCAGCAGACCCAGAGTGGTGATCCAGCTGGAGA GAGGCGAGGAGCCCTGGGTGCCCAGCGGCACCGACACCACCCTGAGCAGAACC ACCTACAGAAGAAGAAACCCCGGCAGC (SEQ ID NO: 1155) | 5.01043067 |
| ZN766 | HLTFRDVAIEFSQ EEWKCLDPVQKA LYRDVMLENYRN LVSL (SEQ ID NO: 521) | AQLRRGHLTFRDVAIEFSQEEW KCLDPVQKALYRDVMLENYRN LVSLGICLPDLSIISMMKQRTEP WTVENEMKVAKNPD (SEQ ID NO: 522) | GCCCAGCTGAGAAGAGGCCACCTGACCTTCAGAGATGTGGCCATCGAGTTCAGC CAGGAGGAGTGGAAGTGCCTGGACCCCGTGCAGAAGGCCCTGTACAGAGATGT GATGCTGGAGAACTACAGAAACCTGGTGAGCCTGGGCATCTGCCTGCCCGACCT GAGCATCATCAGCATGATGAAGCAGAGAACCGAGCCCTGGACCGTGGAGAACG AGATGAAGGTGGCCAAGAACCCCGAC (SEQ ID NO: 1156) | 4.9926318 |
| ID2 | LLYNMNDCYSKL KELVPSIPQNKKV SKMEILQHVIDYI LDL (SEQ ID NO: 523) | SDHSLGISRSKTPVDDPMSLLYN MNDCYSKLKELVPSIPQNKKVS KMEILQHVIDYILDLQIALDSHP TIVSLHHQRPGQ (SEQ ID NO: 524) | AGCGACCACAGCCTGGGCATCAGCAGAAGCAAGACCCCCGTGGACGACCCCAT GAGCCTGCTGTACAACATGAACGACTGCTACAGCAAGCTGAAGGAGCTGGTGC CCAGCATCCCCCAGAACAAGAAGGTGAGCAAGATGGAGATCCTGCAGCACGTG ATCGACTACATCCTGGACCTGCAGATCGCCCTGGACAGCCACCCCACCATCGTG AGCCTGCACCACCAGAGACCCGGCCAG (SEQ ID NO: 1157) | 4.86972562 |
| TOX | PQKPVSAYALFFR DTQAAIKGQNPN ATFGEVSKIVAS MWDGLGEEQK QVYKKKTEAAKK EYLKQLAAY (SEQ ID NO: 525) | KDPNEPQKPVSAYALFFRDTQA AIKGQNPNATFGEVSKIVASM WDGLGEEQKQVYKKKTEAAKK EYLKQLAAYRASLVSK (SEQ ID NO: 526) | AAGGACCCCAACGAGCCCAGAAGCCGTGAGCGCCTACGCCCTGTTCTTCAGA GACACCCAGGCCGCCATCAAGGGCCAGAACCCCAACGCCACCTTCGGCGAGGT GAGCAAGATCGTGGCCAGCATGTGGGACGGCCTGGGCGAGGAGCAGAAGCAG GTGTACAAGAAGAAGACCGAGGCCGCCAAGAAGGAGTACCTGAAGCAGCTGG CCGCCTACAGAGCCAGCCTGGTGAGCAAG (SEQ ID NO: 1158) | 4.84737013 |
| ZN274 | PVTFQDVAVDFS REEWGLLGPTQR TEYRDVMLETFG HLVSV (SEQ ID NO: 527) | QEEKQEDAAICPVTVLPEEPVTF QDVAVDFSREEWGLLGPTQRT EYRDVMLETFGHLVSVGWETT LENKELAPNSDIPEE (SEQ ID NO: 528) | CAGGAGGAGAAGCAGGAGGACGCCGCCATCTGCCCCGTGACCGTGCTGCCCGA GGAGCCTGTGACCTTCCAGGACGTGGCCGTGGACTTCAGCAGAGAGGAGTGGG GCCTGCTGGGCCCCACCCAGAGAACCGAGTACAGAGATGTGATGCTGGAGACC TTCGGCCACCTGGTGAGCGTGGGCTGGGAGACCACCCTGGAGAACAAGGAGCT GGCCCCCAACAGCGACATCCCCGAGGAG (SEQ ID NO: 1159) | 4.82395142 |
| ZN75C | LLTFEDMAVYFSE EEWQLLGPLEKTL YNDVMQDIYETA ISL (SEQ ID NO: 529) | KSTKDWKMAPKLIWPESQSLLT FEDMAVYFSEEEWQLLGPLEKT LYNDVMQDIYETAISLGKQRTG KIMGIEMASSFSKE (SEQ ID NO: 530) | AAGAGCACCAAGGACTGGAAGATGGCCCCCAAGCTGATCTGGCCCGAGAGCCA GAGCCTGCTGACCTTCGAGGACATGGCCGTGTACTTCAGCGAGGAGGAGTGGC AGCTGCTGGGCCCCCTGGAGAAGACCCTGTACAACGACGTGATGCAGGACATCT ACGAGACCGCCATCAGCCTGGGCAAGCAGAGAACCGGCAAGATCATGGGCATC GAGATGGCCAGCAGCTTCAGCAAGGAG (SEQ ID NO: 1160) | 4.81809368 |
| SCMH1 | SSWTVEDVMQF VREADPQLGPHA DLFRKHEIDGKAL LLLRSDMMMKY MGLKLGPALKLS YHIDRL (SEQ ID NO: 531) | DASRLSGRDPSSWTVEDVMQF VREADPQLGPHADLFRKHEIDG KALLLLRSDMMMKYMGLKLGP ALKLSYHIDRLKQGKF (SEQ ID NO: 532) | GACGCCAGCAGACTGAGCGGCAGAGACCCCAGCAGCTGGACCGTGGAGGACG TGATGCAGTTCGTGAGAGAGGCCGACCCCCAGCTGGGCCCCCACGCCGACCTGT TCAGAAAGCACGAGATCGACGGCAAGGCCCTGCTGCTGCTGAGAAGCGACATG ATGATGAAGTACATGGGCCTGAAGCTGGGCCCCGCCCTGAAGCTGAGCTACCAC ATCGACAGACTGAAGCAGGGCAAGTTC (SEQ ID NO: 1161) | 4.79639316 |
| ZN560 | SVTFEDTAVDFT QEEWILLDPVQR NLYRDVMLENYE NVAKV (SEQ ID NO: 533) | AYCLTNCYQYSVTFEDTAVDFT QEEWILLDPVQRNLYRDVMLE NYENVAKVGFQLFKPSVISWLE EEELRTLQQGVLQDW (SEQ ID NO: 534) | GCCTACTGCCTGACCAACTGCTACCAGTACAGCGTGACCTTCGAGGACACCGCC GTGGACTTCACCCAGGAGGAGTGGATCCTGCTGGACCCCGTGCAGAGAAACCT GTACAGAGATGTGATGCTGGAGAACTACGAGAACGTGGCCAAGGTGGGCTTCC AGCTGTTCAAGCCCAGCGTGATCAGCTGGCTGGAGGAGGAGGAGCTGAGAACC CTGCAGCAGGGCGTGCTGCAGGACTGG (SEQ ID NO: 1162) | 4.77465441 |
| SCML4 | SAWTVEDVVWF VKDADPQALGPH VELFRKHEIDGNA LLLLKSDMVMKY LGLKLGPALKLCY HIDKL (SEQ ID NO: 535) | ARRPRSRNPSAWTVEDVVWFV KDADPQALGPHVELFRKHEIDG NALLLLKSDMVMKYLGLKLGPA LKLCYHIDKLKQAKF (SEQ ID NO: 536) | GCCAGAAGACCCAGAAGCAGAAACCCCAGCGCCTGGACCGTGGAGGACGTGGT GTGGTTCGTGAAGGACGCCGACCCCCAGGCCCTGGGCCCCCACGTGGAGCTGTT CAGAAAGCACGAGATCGACGGCAACGCCCTGCTGCTGCTGAAGAGCGACATGG TGATGAAGTACCTGGGCCTGAAGCTGGGCCCCGCCCTGAAGCTGTGCTACCACA TCGACAAGCTGAAGCAGGCCAAGTTC (SEQ ID NO: 1163) | 4.74079704 |
| ZN214 | AVTFEDVTIIFTW EEWKFLDSSQKR LYREVMWENYT NVMSV (SEQ ID NO: 537) | AVTFEDVTIIFTWEEWKFLDSSQ KRLYREVMWENYTNVMSVEN WNESYKSQEEKFRYLEYENFSY WQGWWNAGAQMYENQ (SEQ ID NO: 538) | GCCGTGACCTTCGAGGACGTGACCATCATCTTCACCTGGGAGGAGTGGAAGTTC CTGGACAGCAGCCAGAAGAGACTGTACAGAGAGGTGATGTGGGAGAACTACAC CAACGTGATGAGCGTGGAGAACTGGAACGAGAGCTACAAGAGCCAGGAGGAG AAGTTCAGATACCTGGAGTACGAGAACTTCAGCTACTGGCAGGGCTGGTGGAA CGCCGGCGCCCAGATGTACGAGAACCAG (SEQ ID NO: 1164) | 4.72989473 |
| CBX7 |  (SEQ ID NO: 539) | ELSAIGEQVFAVESIRKKRVRKG KVEYLVKWKGWPPKYSTWEPE EHILDPRLVMAYEEKEERDRAS GYRKRGPKPKRLLL(SEQ ID NO: 540) | GAGCTGAGCGCCATCGGCGAGCAGGTGTTCGCCGTGGAGAGCATCAGAAAGAA GAGAGTGAGAAAGGGCAAGGTGGAGTACCTGGTGAAGTGGAAGGGCTGGCCC CCCAAGTACAGCACCTGGGAGCCCGAGGAGCACATCCTGGACCCCAGACTGGT GATGGCCTACGAGGAGAAGGAGGAGAGAGACAGAGCCAGCGGCTACAGAAAG AGAGGCCCCAAGCCCAAGAGACTGCTGCTG (SEQ ID NO: 1165) | 4.70199486 |
| ID1 | LYDMNGCYSRLK ELVPTLPQNRKVS KVEILQHVIDYIR DL (SEQ ID NO: 541) | GGAGARLPALLDEQQVNVLLY DMNGCYSRLKELVPTLPQNRKV SKVEILQHVIDYIRDLQLELNSES EVGTPGGRGLPVR (SEQ ID NO: 542) | GGCGGCGCCGGCGCCAGACTGCCCGCCCTGCTGGACGAGCAGCAGGTTAACGT GCTGCTGTACGACATGAACGGCTGCTACAGCAGACTGAAGGAGCTGGTGCCCA CCCTGCCCCAGAACAGAAAGGTGAGCAAGGTGGAGATCCTGCAGCACGTGATC GACTACATCAGAGACCTGCAGCTGGAGCTGAACAGCGAGAGCGAGGTGGGCAC CCCCGGCGGCAGAGGCCTGCCTGTGAGA (SEQ ID NO: 1166) | 4.66128008 |
| CREM | AKECRRRKKEYVK CLESRVAVLEVQ NKKLIEELETLKDI CSPKTD (SEQ ID NO: 543) | VVMAASPGSLHSPQQLAEEAT RKRELRLMKNREAAKECRRRKK EYVKCLESRVAVLEVQNKKLIEE LETLKDICSPKTDY (SEQ ID NO: 544) | GTGGTGATGGCCGCCAGCCCCGGCAGCCTGCACAGCCCCCAGCAGCTTGCTGA GGAAGCCACCAGAAAGAGAGAGCTGAGACTGATGAAGAACAGAGAGGCCGCC AAGGAGTGCAGAAGAAGAAAGAAGGAGTACGTGAAGTGCCTGGAGAGCAGAG TGGCCGTGCTGGAGGTGCAGAACAAGAAGCTGATCGAGGAGCTGGAGACCCTG AAGGACATCTGCAGCCCCAAGACCGACTAC (SEQ ID NO: 1167) | 4.58757659 |
| FER3L | QRQAANIRERKR MFNLNEAFDQLR RKVPTFAYEKRLS RIETLRLAIVYISF M (SEQ ID NO: 545) | SLLGRPKRKRVITYAQRQAANIR ERKRMFNLNEAFDQLRRKVPTF AYEKRLSRIETLRLAIVYISFMTEL LESCEKKESG (SEQ ID NO: 546) | AGCCTGCTGGGCAGACCCAAGAGAAAGAGAGTGATCACCTACGCCCAGAGACA GGCCGCCAACATCAGAGAGAGAAAGAGAATGTTCAACCTGAACGAGGCCTTCG ACCAGCTGAGAAGAAAGGTGCCCACCTTCGCCTACGAGAAGAGACTGAGCAGA ATCGAGACCCTGAGACTGGCCATCGTGTACATCAGCTTCATGACCGAGCTGCTG GAGAGCTGCGAGAAGAAGGAGAGCGGC (SEQ ID NO: 1168) | 4.55608825 |
| SCX | QRHTANARERDR TNSVNTAFTALRT LIPTEPADRKLSKI ETLRLASSYISHL (SEQ ID NO: 547) | GGGPGGRPGREPRQRHTANA RERDRTNSVNTAFTALRTLIPTE PADRKLSKIETLRLASSYISHLGN VLLAGEACGDGQP (SEQ ID NO: 548) | GGCGGCGGCCCCGGCGGCAGACCCGGCAGAGAGCCAGACAAAGACACACCG CCAACGCCAGAGAGAGAGACAGAACCAACAGCGTGAACACCGCCTTCACCGCC CTGAGAACCCTGATCCCCACCGAGCCCGCCGACAGAAAGCTGAGCAAGATCGA GACCCTGAGACTGGCCAGCAGCTACATCAGCCACCTGGGCAACGTGCTGCTGGC CGGCGAGGCCTGCGGCGATGGACAGCCC (SEQ ID NO: 1169) | 4.38664628 |
| ASCL1 | AVARRNERERNR VKLVNLGFATLRE HVPNGAANKKM SKVETLRSAVEYIR AL (SEQ ID NO: 549) | SGFGYSLPQQQPAAVARRNER ERNRVKLVNLGFATLREHVPNG AANKKMSKVETLRSAVEYIRAL QQLLDEHDAVSAAFQ (SEQ ID NO: 550) | AGCGGCTTCGGCTACAGCCTGCCCCAGCAGCAGCCCGCCGCCGTGGCTAGAAG AAACGAGAGAGAGAGAAACAGAGTGAAGCTGGTGAACCTGGGCTTCGCCACCC TGAGAGAGCACGTGCCCAACGGCGCCGCCAACAAGAAGATGAGCAAGGTGGA GACCCTGAGAAGCGCCGTGGAGTACATCAGAGCCCTGCAGCAGCTGCTGGACG AGCACGACGCCGTGAGCGCCGCCTTCCAG (SEQ ID NO: 1170) | 4.23952129 |
| ZN764 | AVSFADVAVYFC REEWGCLRPAQR ALYRDVMRETYG HLSAL (SEQ ID NO: 551) | APLPPRDPNGAGPEWREPGAV SFADVAVYFCREEWGCLRPAQ RALYRDVMRETYGHLSALGIGG NKPALISWVEEEAELW (SEQ ID NO: 552) | GCCCCCCTGCCCCCCAGAGACCCCAACGGCGCCGGCCCTGAATGGAGAGAGCC CGGAGCCGTGAGCTTCGCCGACGTGGCCGTGTACTTCTGCAGAGAGGAGTGGG GCTGCCTGAGACCCGCCCAGAGAGCCCTGTACAGAGATGTGATGAGAGAGACC TACGGCCACCTGAGCGCCCTGGGCATCGGCGGCAACAAGCCCGCCCTGATCAGC TGGGTGGAGGAGGAGGCCGAGCTGTGG (SEQ ID NO: 1171) | 4.16413141 |
| SCML2 | STWSVDEVIQFM KHTDPQISGPLA DLFRQHEIDGKAL FLLKSDVMMKY MGLKLGPALKLC YYIEKL (SEQ ID NO: 553) | KQGFSKDPSTWSVDEVIQFMK HTDPQISGPLADLFRQHEIDGK ALFLLKSDVMMKYMGLKLGPA LKLCYYIEKLKEGKYS (SEQ ID NO: 554) | AAGCAGGGCTTCAGCAAGGACCCCAGCACCTGGAGCGTGGACGAGGTGATCCA GTTCATGAAGCACACCGACCCCCAGATCAGCGGCCCCCTGGCCGACCTGTTCAG ACAGCACGAGATCGACGGCAAGGCCCTGTTCCTGCTGAAGAGCGACGTGATGA TGAAGTACATGGGCCTGAAGCTGGGCCCCGCCCTGAAGCTGTGCTACTACATCG AGAAGCTGAAGGAGGGCAAGTACAGC (SEQ ID NO: 1172) | 4.16119992 |
| ASCL5 | IQKRNERERQRV KCVNEGYARLRG HLPGALAEKRLSK VETLRAAIRYIKYL (SEQ ID NO: 555) | GAFGVYEYPFEPAFIQKRNERER QRVKCVNEGYARLRGHLPGAL AEKRLSKVETLRAAIRYIKYLQEL LSSAPDGSTPPA (SEQ ID NO: 556) | GGCGCCTTCGGCGTGTACGAGTACCCCTTCGAGCCCGCCTTCATCCAGAAGAGA AACGAGAGAGAGAGACAGAGAGTGAAGTGCGTGAACGAGGGCTACGCCAGAC TGAGAGGCCACCTGCCCGGCGCCCTGGCCGAGAAGAGACTGAGCAAGGTGGA GACCCTGAGAGCCGCCATCAGATACATCAAGTACCTGCAGGAGCTGCTGAGCA GCGCCCCCGACGGCAGCACCCCCCTGCT (SEQ ID NO: 1173) | 4.14708139 |
| TWST1 | QRVMANVRERQ RTQSLNEAFAAL RKIIPTLPSDKLSKI QTLKLAARYIDFL (SEQ ID NO: 557) | SGGGSPQSYEELQTQRVMANV RERQRTQSLNEAFAALRKIIPTL PSDKLSKIQTLKLAARYIDFLYQV LQSDELDSKMAS (SEQ ID NO: 558) | AGCGGCGGCGGCAGCCCCCAGAGCTACGAGGAGCTGCAGACCCAGAGAGTGA TGGCCAACGTGAGAGAGAGACAGAGAACCCAGAGCCTGAACGAGGCCTTCGCC GCCCTGAGAAAGATCATCCCCACCCTGCCCAGCGACAAGCTGAGCAAGATCCAG ACCCTGAAGCTGGCCGCCAGATACATCGACTTCCTGTACCAGGTGCTGCAGAGC GACGAGCTGGACAGCAAGATGGCCAGC (SEQ ID NO: 1174) | 4.09571741 |
| ZN319 | DLQRHRRVHTGE KPYKCPNCDKAF K (SEQ ID NO: 559) | FVQHRCDPAREKPLKCPDCEKR FKYASDLQRHRRVHTGEKPYKC PNCDKAFKQREHLNKHQGVHA REQQFKCVWCGERFL (SEQ ID NO: 560) | TTCGTGCAGCACAGATGCGACCCCGCCAGAGAGAAGCCCCTGAAGTGCCCCGA CTGCGAGAAGAGATTCAAGTACGCCAGCGACCTGCAGAGACACAGAAGAGTGC ACACCGGCGAGAAGCCCTACAAGTGCCCCAACTGCGACAAGGCCTTCAAGCAG AGAGAGCACCTGAACAAGCACCAGGGCGTGCACGCCAGAGAGCAGCAGTTCAA GTGCGTGTGGTGCGGCGAGAGATTCCTG (SEQ ID NO: 1175) | 4.08013835 |
| ZN749 | CMVFEDVAIYFS QEEWGILNDAQ RHLHSNVMLENF ALLSSV (SEQ ID NO: 561) | NLTEDCMVFEDVAIYFSQEEW GILNDAQRHLHSNVMLENFALL SSVGCWHGAKDEEVPSKQCVS VRVLQVTIPKPALSTL (SEQ ID NO: 562) | AACCTGACCGAGGACTGCATGGTGTTCGAGGACGTGGCCATCTACTTCAGCCAG GAGGAGTGGGGCATCCTGAACGACGCCCAGAGACACCTGCACAGCAACGTGAT GCTGGAGAACTTCGCCCTGCTGAGCAGCGTGGGCTGCTGGCACGGCGCCAAGG ACGAGGAGGTGCCCAGCAAGCAGTGCGTGAGCGTGAGAGTGCTGCAGGTGAC CATCCCCAAGCCCGCCCTGAGCACCCTG (SEQ ID NO: 1176) | 4.06508464 |

**Table 2: Pfam Activators**

| **Gene** | **Pfam Domain sequence** | **Extended Domain sequence** | **Extended Domain DNA sequence** | **Avg Activation** |
|---|---|---|---|---|
| ZN473 | FVTLKDVGMDFTL GDWEQLGLEQGD TFWDTALDNCQD LFLL (SEQ ID NO: 563) | AEEFVTLKDVGMDFTLGDWEQ LGLEQGDTFWDTALDNCQDLF LLDPPRPNLTSHPDGSEDLEPLA GGSPEATSPDVTETK (SEQ ID NO: 564) | GCCGAGGAGTTCGTGACCCTGAAGGACGTGGGCATGGACTTCACCCTGGGC GACTGGGAGCAGCTGGGCCTGGAGCAGGGCGACACCTTCTGGGACACCGCC CTGGACAACTGCCAGGACCTGTTCCTGCTGGACCCCCCCAGACCCAACCTGAC CAGCCACCCCGACGGCAGCGAGGACCTGGAGCCCCTGGCTGGAGGAAGCCC TGAGGCCACCAGCCCCGACGTGACCGAGACCAAG (SEQ ID NO: 1178) | -8.6232004 |
| FOXO3 | HEKFPSDLDLDMF NGSLECDMESIIRS ELMDADGLDFNFD S (SEQ ID NO: 565) | DSLSGSSLYSTSANLPVMGHEK FPSDLDLDMFNGSLECDMESII RSELMDADGLDFNFDSLISTQN VVGLNVGNFTGAKQ (SEQ ID NO: 566) | GACAGCCTGAGCGGCAGCAGCCTGTACAGCACCAGCGCCAACCTGCCCGTGA TGGGCCACGAGAAGTTCCCCAGCGACCTGGACCTGGACATGTTCAACGGCAG CCTGGAGTGCGACATGGAGAGCATCATCAGAAGCGAGCTGATGGACGCCGA CGGCCTGGACTTCAACTTCGACAGCCTGATCAGCACCCAGAACGTGGTGGGC CTGAACGTGGGCAACTTCACCGGCGCCAAGCAG (SEQ ID NO: 1179 ) | -8.3891724 |
| FOXO1 | QEKLPSDLDGMFIE RLDCDMESIIRNDL MDGDTLDFNFDN (SEQ ID NO: 567) | GGYSSVSSCNGYGRMGLLHQE KLPSDLDGMFIERLDCDMESIIR NDLMDGDTLDFNFDNVLPNQ SFPHSVKTTTHSWVSG (SEQ ID NO: 568) | GGCGGCTACAGCAGCGTGAGCAGCTGCAACGGCTACGGCAGAATGGGCCTG CTGCACCAGGAGAAGCTGCCCAGCGACCTGGACGGCATGTTCATCGAGAC TGGACTGCGACATGGAGAGCATCATCAGAAACGACCTGATGGACGGCGACA CCCTGGACTTCAACTTCGACAACGTGCTGCCCAACCAGAGCTTCCCCCACAGC GTGAAGACCACCACCCACAGCTGGGTGAGCGGC (SEQ ID NO: 1180) | -8.3703632 |
| MYBA | GNCIEHVQPTSAFI QQPFIDEDPDKEK KIKELEMLLMSAEN EVRRK (SEQ ID NO: 569) | FYIPVQIPGYQYVSPEGNCIEHV QPTSAFIQQPFIDEDPDKEKKIK ELEMLLMSAENEVRRKRIPSQP GSFSSWSGSFLM (SEQ ID NO: 570 ) | TTCTACATCCCCGTGCAGATCCCCGGCTACCAGTACGTGAGCCCCGAGGGCA ACTGCATCGAGCACGTGCAGCCCACCAGCGCCTTCATCCAGCAGCCCTTCATC GACGAGGACCCCGACAAGGAGAAGAAGATCAAGGAGCTGGAGATGCTGCT GATGAGCGCCGAGAACGAGGTGAGAAGAAAGAGAATCCCCAGCCAGCCCG GCAGCTTCAGCAGCTGGAGCGGCAGCTTCCTGATG (SEQ ID NO: 1181) | -8.2096102 |
| MYB | VNIVNVPQPAAAA IQRHYNDEDPEKE KRIKELELLLMSTE NELKGQ(SEQ ID NO: 571) | EAQNVSSHVPYPVALHVNIVNV PQPAAAAIQRHYNDEDPEKEK RIKELELLLMSTENELKGQQVLP TQNHTCSYPGWHST (SEQ ID NO: 572) | GAGGCCCAGAACGTGAGCAGCCACGTGCCCTACCCCGTGGCCCTGCACGTGA ACATCGTGAACGTGCCCCAGCCCGCCGCCGCCGCCATCCAGAGACACTACAA CGACGAGGACCCCGAGAAGGAGAAGAGAATCAAGGAGCTGGAGCTGCTGCT GATGAGCACCGAGAACGAGCTGAAGGGCCAGCAGGTGCTGCCCACCCAGAA CCACACCTGCAGCTACCCCGGCTGGCACAGCACC (SEQ ID NO: 1182) | -7.2112528 |
| NCOA2 | ESPSDEGALLDQLY LALRNFDGLEEIDR ALGIPELVSQSQAV DPEQ (SEQ ID NO: 573) | PFGSSPDDLLCPHPAAESPSDE GALLDQLYLALRNFDGLEEIDRA LGIPELVSQSQAVDPEQFSSQD SNIMLEQKAPVFP (SEQ ID NO: 574) | CCCTTCGGCAGCAGCCCCGACGACCTGCTGTGCCCCCACCCCGCTGCTGAAA GCCCCAGCGACGAGGGCGCTCTGCTGGACCAGCTGTACCTGGCCCTGAGAAA CTTCGACGGCCTGGAGGAGATCGACAGAGCCCTGGGCATCCCCGAGCTGGT GAGCCAGAGCCAGGCCGTGGACCCCGAGCAGTTCAGCAGCCAGGACAGCAA CATCATGCTGGAGCAGAAGGCCCCCGTGTTCCCC (SEQ ID NO: 1183) | -7.1119077 |
| SMCA2 | PFSPVQLHQLRAQI LAYKMLARGQPLP ETLQLAV (SEQ ID NO: 575) | SQPGALIPGDPQAMSQPNRGP SPFSPVQLHQLRAQILAYKMLA RGQPLPETLQLAVQGKRTLPGL QQQQQQQQQQQQQQ (SEQ ID NO: 576) | AGCCAGCCCGGCGCCCTGATCCCCGGCGACCCCCAGGCTATGAGCCAACCTA ACAGAGGCCCCAGCCCCTTCAGCCCCGTGCAGCTGCACCAGCTGAGAGCCCA GATCCTGGCCTACAAGATGCTGGCCAGAGGCCAGCCCCTGCCCGAGACCCTG CAGCTGGCCGTGCAAGGCAAGAGAACCCTGCCCGGCCTGCAGCAGCAGCAG CAGCAGCAGCAGCAGCAGCAGCAGCAGCAGCAG (SEQ ID NO: 1184) | -6.7916451 |
| KIBRA | LPEGWEEARDFDG KVYYIDHTNRTTS WID (SEQ ID NO: 577) | PRPELPLPEGWEEARDFDGKVY YIDHTNRTTSWIDPRDRYTKPLT FADCISDELPLGWEEAYDPQVG DYFIDHNTKTTQI (SEQ ID NO: 578) | CCCAGACCCGAGCTGCCCCTGCCCGAGGGCTGGGAGGAGGCCAGAGACTTC GACGGCAAGGTGTACTACATCGACCACACCAACAGAACCACCAGCTGGATCG ACCCCAGAGACAGATACACCAAGCCCCTGACCTTCGCCGACTGCATCAGCGA CGAGCTGCCCCTGGGCTGGGAGGAGGCCTACGACCCCCAGGTGGGAGACTA CTTCATCGACCACAACACCAAGACCACCCAGATC (SEQ ID NO: 1185) | -6.707792 |
| NCOA3 | EGQSDERALLDQL HTLLSNTDATGLEE IDRALGIPELVNQG QALEPKQ (SEQ ID NO: 579) | LRNSLDDLVGPPSNLEGQSDER ALLDQLHTLLSNTDATGLEEIDR ALGIPELVNQGQALEPKQDAF QGQEAAVMMDQKAG (SEQ ID NO: 580) | CTGAGAAACAGCCTGGACGACCTGGTGGGCCCCCCAGCAACCTGGAGGGC CAGAGCGACGAGAGAGCCCTGCTGGACCAGCTGCACACCCTGCTGAGCAAC ACCGACGCCACCGGCCTGGAGGAGATCGACAGAGCCCTGGGCATCCCCGAG CTGGTGAACCAGGGCCAGGCCCTGGAGCCCAAGCAGGACGCCTTCCAGGGC CAGGAGGCTGCCGTGATGATGGACCAGAAGGCCGGC (SEQ ID NO: 1186) | -6.4149356 |
| FOXO6 | PDRFPADLDLDMF SGSLECDVESIILND FMDSDEMDFNFD S (SEQ ID NO: 581 ) | LDALPGPYAAAAAGPLGAAPD RFPADLDLDMFSGSLECDVESII LNDFMDSDEMDFNFDSALPPP PPGLAGAPPPNQSWV (SEQ ID NO: 582) | CTGGACGCCCTGCCCGGCCCCTACGCCGCTGCTGCTGGACCTCTTGGAGC TGCCCCTGACAGATTCCCCGCCGACCTGGACCTGGACATGTTCAGCGGCAGC CTGGAGTGCGACGTGGAGAGCATCATCCTGAACGACTTCATGGACAGCGAC GAGATGGACTTCAACTTCGACAGCGCCCTGCCCCCCCCCCTCCTGGACTTGC TGGAGCTCCTCCTCCCAACCAGAGCTGGGTG (SEQ ID NO: 1187) | -6.0518896 |
| ZN597 | PILFEDLAVYFSQEE CVTLHPAQRSLSKD GTKESLEDAALM (SEQ ID NO: 583) | ASMPPTPEAQGPILFEDLAVYF SQEECVTLHPAQRSLSKDGTKE SLEDAALMGEEGKPEINQQLSL ESMELDELALEKYP (SEQ ID NO: 584) | GCCAGCATGCCCCCCACCCCCGAGGCCCAGGGCCCCATCCTGTTCGAGGACC TTGCTGTGTACTTCAGCCAGGAGGAGTGCGTGACCCTGCACCCCGCCCAGAG AAGCCTGAGCAAGGACGGCACCAAGGAGAGCCTGGAGGACGCCGCCCTGAT GGGCGAGGAGGGCAAGCCCGAGATCAACCAGCAGCTGAGCCTGGAGAGCA TGGAGCTGGACGAGCTGGCCCTGGAGAAGTACCCC (SEQ ID NO: 1188) | -5.9555177 |
| APBB1 | LPAGWMRVQDTS GTYYWHIPTGTTQ WEP (SEQ ID NO: 585) | GSPSYGSPEDTDSFWNPNAFET DSDLPAGWMRVQDTSGTYYW HIPTGTTQWEPPGRASPSQGSS PQEESQLTWTGFAHGE (SEQ ID NO: 586 ) | GGCAGCCCCAGCTACGGCAGCCCCGAGGACACCGACAGCTTCTGGAACCCCA ACGCCTTCGAGACCGACAGCGACCTGCCCGCCGGCTGGATGAGAGTGCAGG ACACCAGCGGCACCTACTACTGGCACATCCCCACCGGCACCACCCAGTGGGA GCCCCCCGGAAGAGCTAGCCCTAGCCAGGGCAGCAGCCCCCAGGAGGAGAG CCAGCTGACCTGGACCGGCTTCGCCCACGGCGAG (SEQ ID NO: 1189) | -5.8338079 |
| ANM2 | VAIADYAATDETQL SFLRGEKILILRQTT ADWWWGERAGC CGYIP  (SEQ ID NO: 587) | ECSEAGLLQEGVQPEEFVAIAD YAATDETQLSFLRGEKILILRQTT ADWWWGERAGCCGYIPANH VGKHVDEYDPEDTWQ (SEQ ID NO: 588 ) | GAGTGCAGCGAGGCCGGCCTGCTGCAGGAGGGCGTGCAGCCCGAGGAGTTC GTGGCTATCGCCGACTACGCCGCCACCGACGAGACCCAGCTGAGCTTCCTGA GAGGCGAGAAGATCCTGATCCTGAGACAGACCACCGCCGACTGGTGGTGGG GCGAGAGAGCCGGCTGCTGCGGCTACATCCCCGCCAACCACGTGGGCAAGC ACGTGGACGAGTACGACCCCGAGGACACCTGGCAG (SEQ ID NO: 1190) | -5.6456716 |
| MED9 | SFLPLVHNIIKCMD KDSPEVHQDLNAL KSKFQEMRKLIST MPGIHLSPEQQQ QQLQSLREQVRTK NELLQKYKSL(SEQ ID NO: 589) | NYSFLPLVHNIIKCMDKDSPEV HQDLNALKSKFQEMRKLISTMP GIHLSPEQQQQQLQSLREQVR TKNELLQKYKSLCMF (SEQ ID NO: 590) | AACTACAGCTTCCTGCCCCTGGTGCACAACATCATCAAGTGCATGGACAAGG ACAGCCCCGAGGTGCACCAGGACCTGAACGCCCTGAAGAGCAAGTTCCAGG AGATGAGAAAGCTGATCAGCACCATGCCCGGCATCCACCTGAGCCCCGAGCA GCAGCAGCAGCAGCTGCAGAGCCTGAGAGAGCAGGTGAGAACCAAGAACG AGCTGCTGCAGAAGTACAAGAGCCTGTGCATGTTC (SEQ ID NO: 1191) | -5.5377024 |
| CXXC1 | YCICRKPDINCFMI GCDNCNEWFHGD CIRITEKMAKAIRE WYCRECRE (SEQ ID NO: 591) | AGEDSKSENGENAPIYCICRKPD INCFMIGCDNCNEWFHGDCIRI TEKMAKAIREWYCRECREKDPK LEIRYRHKKSRER (SEQ ID NO: 592) | GCCGGCGAGGACAGCAAGAGCGAGAACGGCGAGAACGCCCCCATCTACTGC ATCTGCAGAAAGCCCGACATCAACTGCTTCATGATCGGCTGCGACAACTGCA ACGAGTGGTTCCACGGCGACTGCATCAGAATCACCGAGAAGATGGCCAAGG CCATCAGAGAGTGGTACTGCAGAGAGTGCAGAGAGAAGGACCCCAAGCTGG AGATCAGATACAGACACAAGAAGAGCAGAGAGAGA (SEQ ID NO: 1192) | -5.4566266 |
| CRTC2 | PNIILTGDSSPGFSK EIAAALAGVPGFEV SAAGLELGLGLEDE LRMEPLGLEGLNM LSDPCALLPDPAVE ESFRSDR (SEQ ID NO: 593 ) | GPNIILTGDSSPGFSKEIAAALA GVPGFEVSAAGLELGLGLEDEL RMEPLGLEGLNMLSDPCALLPD PAVEESFRSDRLQ (SEQ ID NO: 594) | GGCCCCAACATCATCCTGACCGGCGACAGCAGCCCCGGCTTCAGCAAGGAGA TCGCCGCCGCCCTGGCTGGCGTGCCTGGATTCGAGGTGAGCGCTGCTGGACT GGAGCTGGGCCTGGGCCTGGAGGACGAGCTGAGAATGGAGCCCCTGGGCCT GGAGGGCCTGAACATGCTGAGCGACCCCTGCGCCCTGCTGCCCGACCCCGCT GTGGAGGAGAGCTTCAGAAGCGACAGACTGCAG (SEQ ID NO: 1193) | -5.293256 |
| NOTC2 | WANCSSPLPCWD YINNQCDELCNTV ECLFDNFEC (SEQ ID NO: 595) | EACNSHACQWDGGDCSLTME NPWANCSSPLPCWDYINNQC DELCNTVECLFDNFECQGNSKT CKYDKYCADHFKDNHCDQ (SEQ ID NO: 596) | GAGGCCTGCAACAGCCACGCCTGCCAGTGGACGGCGGCGACTGCAGCCTG ACCATGGAGAACCCCTGGGCCAACTGCAGCAGCCCCCTGCCCTGCTGGGACT ACATCAACAACCAGTGCGACGAGCTGTGCAACACCGTGGAGTGCCTGTTCGA CAACTTCGAGTGCCAGGGCAACAGCAAGACCTGCAAGTACGACAAGTACTGC GCCGACCACTTCAAGGACAACCACTGCGACCAG (SEQ ID NO: 1194) | -5.2584004 |
| CACO1 | KECPICKERFPAES DKDALEDHMDGH (SEQ ID NO: 597) | SGGEEANLLLPELGSAFYDMAS GFTVGTLSETSTGGPATPTWKE CPICKERFPAESDKDALEDHMD GHFFFSTQDPFTFE (SEQ ID NO: 598) | AGCGGCGGCGAGGAGGCCAACCTGCTGCTGCCCGAGCTGGGCAGCGCTTTC TACGACATGGCCAGCGGCTTCACCGTGGGCACCCTGAGCGAGACCAGCACCG GCGGACCCGCTACCCCCACATGGAAGGAGTGCCCCATCTGCAAGGAGAGATT CCCCGCCGAGAGCGACAAGGACGCCCTGGAGGACCACATGGACGGCCACTT CTTCTTCAGCACCCAGGACCCCTTCACCTTCGAG (SEQ ID NO: 1195) | -4.6832738 |
| PYGO1 | PCGICTNEVNDDQ DAILCEASCQKWF HRICTGMTETAYG LLTAEASAVWGCD TCMA (SEQ ID NO: 599) | RHGHSSSDPVYPCGICTNEVND DQDAILCEASCQKWFHRICTG MTETAYGLLTAEASAVWGCDT CMADKDVQLMRTRETF (SEQ ID NO: 600) | AGACACGGCCACAGCAGCAGCGACCCCGTGTACCCCTGCGGCATCTGCACCA ACGAGGTGAACGACGACCAGGACGCCATCCTGTGCGAGGCCAGCTGCCAGA AGTGGTTCCACAGAATCTGCACCGGCATGACCGAGACCGCCTACGGCCTGCT GACCGCCGAGGCCAGCGCTGTGTGGGGATGCGACACCTGCATGGCCGACAA GGACGTGCAGCTGATGAGAACCAGAGAGACCTTC (SEQ ID NO: 1196) | -4.3430928 |
| IKKA | AQMIEENLNCLGH LSTIIHEANEEQGN SMMNLDWSW (SEQ ID NO: 601) | LVGSSLEGAVTPQTSAWLPPTS AEHDHSLSCVVTPQDGETSAQ MIEENLNCLGHLSTIIHEANEEQ GNSMMNLDWSWLTE (SEQ ID NO: 602) | CTGGTGGGCAGCAGCCTGGAGGGCGCCGTGACCCCCAGACAAGCGCTTGG CTGCCCCCTACCAGCGCTGAGCACGACCACAGCCTGAGCTGCGTGGTGACCC CCCAGGACGGCGAGACCAGCGCCCAGATGATCGAGGAGAACCTGAACTGCC TGGGCCACCTGAGCACCATCATCCACGAGGCCAACGAGGAGCAGGGCAACA GCATGATGAACCTGGACTGGAGCTGGCTGACCGAG (SEQ ID NO: 1197) | -4.3328612 |
| APC16 | DLAPPRKALFTYPK GAGEMLEDGSERF LCESVFSYQVASTL KQVKHDQQVARM EKLAGLVEELEADE WRFKPIEQLLGF (SEQ ID NO: 603 ) | DLAPPRKALFTYPKGAGEMLED GSERFLCESVFSYQVASTLKQVK HDQQVARMEKLAGLVEELEAD EWRFKPIEQLLGFT (SEQ ID NO: 604) | GACCTGGCCCCCCCAGAAAGGCCCTGTTCACCTACCCCAAGGGCGCCGGCG AGATGCTGGAGGACGGCAGCGAGAGATTCCTGTGCGAGAGCGTGTTCAGCT ACCAGGTGGCCAGCACCCTGAAGCAGGTGAAGCACGACCAGCAGGTGGCCA GAATGGAGAAGCTGGCCGGCCTGGTGGAGGAGCTGGAGGCCGACGAGTGG AGATTCAAGCCCATCGAGCAGCTGCTGGGCTTCACC (SEQ ID NO: 1198) | -4.1227423 |
| WWP2 | LPPGWEKRQDNG RVYYVNHNTRTTQ WED (SEQ ID NO: 605) | AMQHFSQRFLYQSSSASTDHD PLGPLPPGWEKRQDNGRVYYV NHNTRTTQWEDPRTQGMIQE PALPPGWEMKYTSEGVRY (SEQ ID NO: 606) | GCCATGCAGCACTTCAGCCAGAGATTCCTGTACCAGAGCAGCAGCGCCAGCA CCGACCACGACCCCCTGGGCCCCTTCCTCCTGGATGGGAGAAGAGACAGGA CAACGGCAGAGTGTACTACGTGAACCACAACACCAGAACCACCCAGTGGGA GGACCCCAGAACCCAGGGCATGATCCAGGAGCCCGCCCTGCCCCCCGGATGG GAGATGAAGTACACCAGCGAGGGCGTGAGATAC (SEQ ID NO: 1199) | -4.0489585 |
| RIP | FLVQEVMEEEWN ALQSVENCPEDLA QLEELIDMAVLEEI QQELINQEQSIISEY EKSLQFDEKCLSIM (SEQ ID NO: 607) | NSQNSFLVQEVMEEEWNALQ SVENCPEDLAQLEELIDMAVLE EIQQELINQEQSIISEYEKSLQFD EKCLSIMLAEWEAN (SEQ ID NO: 608) | AACAGCCAGAACAGCTTCCTGGTGCAGGAGGTGATGGAGGAGGAGTGGAAC GCCCTGCAGAGCGTGGAGAACTGCCCCGAGGACCTGGCCCAGCTGGAGGAG CTGATCGACATGGCCGTGCTGGAGGAGATCCAGCAGGAGCTGATCAACCAG GAGCAGAGCATCATCAGCGAGTACGAGAAGAGCCTGCAGTTCGACGAGAAG TGCCTGAGCATCATGCTGGCCGAGTGGAGGCCAAC (SEQ ID NO: 1200 ) | -3.97129 |
| AF9 | AYLDELVELHRRL MTLRERHILQQIV NLIEETGHFHITNT TFDFDLCSLDKTTV RKLQSYL (SEQ ID NO: 609) | KSDKQIKNGECDKAYLDELVEL HRRLMTLRERHILQQIVNLIEET GHFHITNTTFDFDLCSLDKTTVR KLQSYLETSGTS (SEQ ID NO: 610) | AAGAGCGACAAGCAGATCAAGAACGGCGAGTGCGACAAGGCCTACCTGGAC GAGCTGGTGGAGCTGCACAGAAGACTGATGACCCTGAGAGAGACACATC CTGCAGCAGATCGTGAACCTGATCGAGGAGACCGGCCACTTCCACATCACCA ACACCACCTTCGACTTCGACCTGTGCAGCCTGGACAAGACCACCGTGAGAAA GCTGCAGAGCTACCTGGAGACCAGCGGCACCAGC (SEQ ID NO: 1201) | -3.7419986 |
| ZFP28 | LVTIKNLAVDFRQQ LHPAQKNFCKNGI WENNSDLGSA (SEQ ID NO: 610) | LEYSLLGEHWDYDALFETQPGL VTIKNLAVDFRQQLHPAQKNFC KNGIWENNSDLGSAGHCVAKP DLVSLLEQEKEPWMV (SEQ ID NO: 612) | CTGGAGTACAGCCTGCTGGGCGAGCACTGGGACTACGACGCCCTGTTCGAGA CCCAGCCCGGCCTGGTGACCATCAAGAACCTGGCCGTGGACTTCAGACAGCA GCTGCACCCCGCCCAGAAGAACTTCTGCAAGAACGGCATCTGGGAGAACAAC AGCGACCTGGGCAGCGCCGGCCACTGCGTGGCCAAGCCCGACCTGGTTAGC CTGCTGGAGCAGGAGAAGGAGCCCTGGATGGTG (SEQ ID NO: 1202) | -3.7291024 |
| WWP1 | LPPGWEKRVDSTD RVYFVNHNTKTTQ WED (SEQ ID NO: 613) | AMQQFNQRYLYSASMLAAEN DPYGPLPPGWEKRVDSTDRVY FVNHNTKTTQWEDPRTQGLQ NEEPLPEGWEIRYTREGVR (SEQ ID NO: 614 ) | GCCATGCAGCAGTTCAACCAGAGATACCTGTACAGCGCCAGCATGCTGGCCG CCGAGAACGACCCCTACGGCCCCCTGCCTCCTGGCTGGGAGAAGAGAGTGG ACAGCACCGACAGAGTGTACTTCGTGAACCACAACACCAAGACCACCCAGTG GGAGGACCCCAGAACCCAGGGCCTGCAGAACGAGGAGCCCCTGCCCGAGGG CTGGGAGATCAGATACACCAGAGAGGGCGTGAGA(SEQ ID NO: 1203) | -3.728405 |
| DPY30 | PTRAYLDQTVVPIL LQGLAVLAKERPP NPIEFLASYLLKNK( SEQ ID NO: 615) | EYGLTDNVERIVENEKINAEKSS KQKVDLQSLPTRAYLDQTVVPIL LQGLAVLAKERPPNPIEFLASYL LKNKAQFEDRN (SEQ ID NO: 616) | GAGTACGGCCTGACCGACAACGTGGAGAGAATCGTGGAGAACGAGAAGATC AACGCCGAGAAGAGCAGCAAGCAGAAGGTGGACCTGCAGAGCCTGCCCACC AGAGCCTACCTGGACCAGACCGTGGTGCCCATCCTGCTGCAGGGCCTGGCCG TGCTGGCCAAGGAGAGACCCCCAACCCCATCGAGTTCCTGGCCAGCTACCT GCTGAAGAACAAGGCCCAGTTCGAGGACAGAAAC (SEQ ID NO: 1204) | -3.696281 |
| KS6B2 | LQSEEDVSQFDTRF TRQTPVDSPDDTA LSESANQAFLGFT (SEQ ID NO: 617 ) | HMNWDDLLAWRVDPPFRPCL QSEEDVSQFDTRFTRQTPVDSP DDTALSESANQAFLGFTYVAPS VLDSIKEGFSFQPKLR (SEQ ID NO: 618) | CACATGAACTGGGACGACCTGCTGGCCTGGAGAGTGGACCCCCCTTCAGAC CCTGCCTGCAGAGCGAGGAGGACGTGAGCCAGTTCGACACCAGATTCACCAG ACAGACCCCCGTGGACAGCCCCGACGACACCGCCCTGAGCGAGAGCGCCAA CCAGGCCTTCCTGGGCTTCACCTACGTGGCCCCCAGCGTGCTGGACAGCATCA AGGAGGGCTTCAGCTTCCAGCCCAAGCTGAGA (SEQ ID NO: 1205) | -3.4939583 |
| PYGO2 | PCGACRSEVNDDQ DAILCEASCQKWF HRECTGMTESAYG LLTTEASAVWACD LCLK (SEQ ID NO: 619) | SGPQPPPGLVYPCGACRSEVN DDQDAILCEASCQKWFHRECT GMTESAYGLLTTEASAVWACD LCLKTKEIQSVYIREGM (SEQ ID NO: 620) | AGCGGCCCCCAGCCCCCCCCGGCCTGGTTTACCCTTGCGGAGCTTGCAGAA GCGAGGTGAACGACGACCAGGACGCCATCCTGTGCGAGGCCAGCTGCCAGA AGTGGTTCCACAGAGAGTGCACCGGCATGACCGAGAGCGCCTACGGCCTGCT GACCACCGAGGCCAGCGCCGTGTGGGCTTGCGACCTGTGCCTGAAGACCAA GGAGATCCAGAGCGTGTACATCAGAGAGGGCATG (SEQ ID NO: 1206) | -3.4423787 |
| U2AF4 | FEEVFTELQEKYGEI EEMNVCDNLGDH LVGNVYVKFRREE DGERAVAELSNR WFNGQAV (SEQ ID NO: 621) | VEVQEHYDSFFEEVFTELQEKY GEIEEMNVCDNLGDHLVGNVY VKFRREEDGERAVAELSNRWF NGQAVHGELSPVTDFR (SEQ ID NO: 622) | GTGGAGGTGCAGGAGCACTACGACAGCTTCTTCGAGGAGGTGTTCACCGAG CTGCAGGAGAAGTACGGCGAGATCGAGGAGATGAACGTGTGCGACAACCTG GGCGACCACCTGGTGGGCAACGTGTACGTGAAGTTCAGAAGAGAGGAGGAC GGCGAGAGAGCCGTGGCCGAGCTGAGCAACAGATGGTTCAACGGCCAGGCC GTGCACGGCGAGCTGAGCCCCGTGACCGACTTCAGA (SEQ ID NO: 1207) | -3.3553928 |
| ITCH | LPPGWEQRVDQH GRVYYVDHVEKRT TWDR (SEQ ID NO: 623) | SGLIIPLTISGGSGPRPLNPVTQA PLPPGWEQRVDQHGRVYYVD HVEKRTTWDRPEPLPPGWERR VDNMGRIYYVDHFTR (SEQ ID NO: 624) | AGCGGCCTGATCATCCCCCTGACCATCAGCGGCGGCAGCGGCCCCAGACCCC TGAACCCCGTGACCCAAGCTCCTCTGCCCCCCGGATGGAGCAGAGAGTGGA CCAGCACGGCAGAGTGTACTACGTGGACCACGTGGAGAAGAGAACCACCTG GGACAGACCCGAGCCCCTGCCCCCCGGCTGGGAGAGAAGAGTGGACAACAT GGGCAGAATCTACTACGTGGACCACTTCACCAGA (SEQ ID NO: 1208) | -3.3366968 |
| ENL | AYTDELVELHRRL MALRERNVLQQIV NLIEETGHFNVTNT TFDFDLFSLDETTV RKLQSCL (SEQ ID NO: 625) | SKPEKILKKGTYDKAYTDELVEL HRRLMALRERNVLQQIVNLIEE TGHFNVTNTTFDFDLFSLDETT VRKLQSCLEAVAT (SEQ ID NO: 626) | AGCAAGCCCGAGAAGATCCTGAAGAAGGGCACCTACGACAAGGCCTACACC GACGAGCTGGTGGAGCTGCACAGAAGACTGATGGCCCTGAGAGAGAGAAAC GTGCTGCAGCAGATCGTGAACCTGATCGAGGAGACCGGCCACTTCAACGTGA CCAACACCACCTTCGACTTCGACCTGTTCAGCCTGGACGAGACCACCGTGAGA AAGCTGCAGAGCTGCCTGGAGGCCGTGGCCACC (SEQ ID NO: 1209 ) | -3.3117985 |
| STAT2 | SQPVPEPDLPCDLR HLNTEPMEIFRNC VKIEEIMPNGDPLL AGQNTVDEVYVSR P (SEQ ID NO: 627) | SQTVPEPDQGPVSQPVPEPDLP CDLRHLNTEPMEIFRNCVKIEEI MPNGDPLLAGQNTVDEVYVSR PSHFYTDGPLMPSD (SEQ ID NO: 628) | AGCCAGACCGTGCCCGAGCCCGACCAGGGCCCCGTGAGCCAGCCTGTTCCTG AGCCTGACCTGCCCTGCGACCTGAGACACCTGAACACCGAGCCCATGGAGAT CTTCAGAAACTGCGTGAAGATCGAGGAGATCATGCCCAACGGCGACCCCCTG CTGGCCGGCCAGAACACCGTGGACGAGGTGTACGTGAGCAGACCCAGCCAC TTCTACACCGACGGCCCCCTGATGCCCAGCGAC (SEQ ID NO: 1210) | -3.1207026 |
| NOTC1 | EEACELPECQEDA GNKVCSLQCNNH ACGWDGGDC (SEQ ID NO: 629) | LCHILDYSFGGGAGRDIPPPLIEE ACELPECQEDAGNKVCSLQCN NHACGWDGGDCSLNFNDPW KNCTQSLQCWKYFSDG (SEQ ID NO: 630) | CTGTGCCACATCCTGGACTACAGCTTCGGCGGCGCCGGCAGAGACATCC CCCCCCCTCTGATCGAGGAGGCTTGCGAGCTGCCCGAGTGCCAGGAGGACGC CGGCAACAAGGTGTGCAGCCTGCAGTGCAACAACCACGCCTGCGGCTGGGA CGGCGGCGACTGCAGCCTGAACTTCAACGACCCCTGGAAGAACTGCACCCAG AGCCTGCAGTGCTGGAAGTACTTCAGCGACGGC (SEQ ID NO: 1211) | -3.1201108 |
| CRTC3 | PNTILPEDSSTSLFK DLNSALAGLPEVSL NVDTPFPLEEELQI EPLSLDGLNMLSDS SMGLLDPSVEETFR ADR (SEQ ID NO: 631) | NCGSLPNTILPEDSSTSLFKDLN SALAGLPEVSLNVDTPFPLEEEL QIEPLSLDGLNMLSDSSMGLLD PSVEETFRADRL (SEQ ID NO: 632 ) | AACTGCGGCAGCCTGCCCAACACCATCCTGCCCGAGGACAGCAGCACCAGCC TGTTCAAGGACCTGAACAGCGCCCTGGCCGGCCTGCCCGAGGTGAGCCTGAA CGTGGACACCCCCTTCCCCCTGGAGGAGGAGCTGCAGATCGAGCCCCTGAGC CTGGACGGCCTGAACATGCTGAGCGACAGCAGCATGGGCCTGCTGGACCCC AGCGTGGAGGAGACCTTCAGAGCCGACAGACTG (SEQ ID NO: 1212) | -3.0736492 |
| SAV1 | LPPGWSVDWTMR GRKYYIDHNTNTT HWSH (SEQ ID NO: 633) | HASGIGRVAATSLGNLTNHGSE DLPLPPGWSVDWTMRGRKYYI DHNTNTTHWSHPLEREGLPPG WERVESSEFGTYYVDH (SEQ ID NO: 634) | CACGCCAGCGGCATCGGCAGAGTGGCCGCCACCAGCCTGGGCAACCTGACC AACCACGGCAGCGAGGACCTGCCCCTGCCCCCGGATGGAGCGTGGACTGG ACCATGAGAGGCAGAAAGTACTACATCGACCACAACACCAACACCACCCACT GGAGCCACCCCCTGGAGAGAGAGGGCCTGCCCCCGGATGGAGAGAGTG GAGAGCAGCGAGTTCGGCACCTACTACGTGGACCAC (SEQ ID NO: 1213) | -2.9035402 |
| DPF1 | GEDFYREAIEHCRS YNARLCAERSLRLP FLDSQTGVAQNNC YIWMEKTHRGPGL APGQIYTYPARCW RKKR (SEQ ID NO: 635) | PLSLGEDFYREAIEHCRSYNARL CAERSLRLPFLDSQTGVAQNNC YIWMEKTHRGPGLAPGQIYTYP ARCWRKKRRLNIL (SEQ ID NO: 636) | CCCCTGAGCCTGGGCGAGGACTTCTACAGAGAGGCCATCGAGCACTGCAGAA GCTACAACGCCAGACTGTGCGCCGAGAGAAGCCTGAGACTGCCCTTCCTGGA CAGCCAGACCGGCGTGGCCCAGAACAACTGCTACATCTGGATGGAGAAGAC CCACAGAGGCCCCGGCCTGGCCCCCGGCCAGATCTACACCTACCCCGCTAGA TGCTGGAGAAAGAAGAGAAGACTGAACATCCTG (SEQ ID NO: 1214) | -2.7433919 |
| ABL1 | VALYDFVASGDNT LSITKGEKLRVLGY NHNGEWCEAQTK NGQGWVP (SEQ ID NO: 637) | KENLLAGPSENDPNLFVALYDF VASGDNTLSITKGEKLRVLGYN HNGEWCEAQTKNGQGWVPS NYITPVNSLEKHSWYHG (SEQ ID NO: 638) | AAGGAGAACCTGCTGGCCGGCCCCAGCGAGAACGACCCCAACCTGTTCGTGG CCCTGTACGACTTCGTGGCCAGCGGCGACAACACCCTGAGCATCACCAAGGG CGAGAAGCTGAGAGTGCTGGGCTACAACCACAACGGCGAGTGGTGCGAGGC CCAGACCAAGAACGGCCAGGGCTGGGTGCCCAGCAACTACATCACCCCCGTG AACAGCCTGGAGAAGCACAGCTGGTACCACGGC (SEQ ID NO: 1215) | -2.6728209 |
| WBP4 | VKTVWVEGLSEDG FTYYYNTETGESR WEK (SEQ ID NO: 639) | YYDLISGASQWEKPEGFQGDLK KTAVKTVWVEGLSEDGFTYYYN TETGESRWEKPDDFIPHTSDLP SSKVNENSLGTLDE (SEQ ID NO: 64 0) | TACTACGACCTGATCAGCGGCGCCAGCCAGTGGGAGAAGCCCGAGGGCTTC CAGGGCGACCTGAAGAAGACCGCCGTGAAGACCGTGTGGGTGGAGGGCCT GAGCGAGGACGGCTTCACCTACTACTACAACACCGAGACCGGCGAGAGCAG ATGGGAGAAGCCCGACGACTTCATCCCCCACACCAGCGACCTGCCCAGCAGC AAGGTGAACGAGAACAGCCTGGGCACCCTGGACGAG (SEQ ID NO: 1216) | -2.6121807 |
| BTK | VALYDYMPMNAN DLQLRKGDEYFILE ESNLPWWRARDK NGQEGYIP (SEQ ID NO: 641) | PEPAAAPVSTSELKKVVALYDY MPMNANDLQLRKGDEYFILEE SNLPWWRARDKNGQEGYIPS NYVTEAEDSIEMYEWYS (SEQ ID NO: 642) | CCCGAGCCCGCCGCCGCCCCCGTGAGCACCAGCGAGCTGAAGAAGGTGGTG GCTCTGTACGACTACATGCCCATGAACGCCAACGACCTGCAGCTGAGAAAGG GCGACGAGTACTTCATCCTGGAGGAGAGCAACCTGCCCTGGTGGAGAGCCA GAGACAAGAACGGCCAGGAGGGCTACATCCCCAGCAACTACGTGACCGAGG CCGAGGACAGCATCGAGATGTACGAGTGGTACAGC (SEQ ID NO: 1217) | -2.5651252 |
| SMRC2 | TREWTEQETLLLLE ALEMYKDDWNKV SEHVGSRTQDECIL HFLRL (SEQ ID NO: 643) | MYTKKNVPSKSKAAASATREW TEQETLLLLEALEMYKDDWNKV SEHVGSRTQDECILHFLRLPIED PYLEDSEASLGPLA (SEQ ID NO: 644 ) | ATGTACACCAAGAAGAACGTGCCCAGCAAGAGCAAGGCCGCCGCCAGCGCC ACCAGAGAGTGGACCGAGCAGGAGACCCTGCTGCTGCTGGAGGCCCTGGAG ATGTACAAGGACGACTGGAACAAGGTGAGCGAGCACGTGGGCAGCAGAACC CAGGACGAGTGCATCCTGCACTTCCTGAGACTGCCCATCGAGGACCCCTACCT GGAGGACAGCGAGGCCAGCCTGGGCCCCCTGGCC (SEQ ID NO: 1218) | -2.4978538 |
| MTA3 | CESCYATQSHQWY SWGPPNMQCRLC AICWLYWKKYGG( SEQ ID NO: 645) | GAVNGAVGTTFQPQNPLLGRA CESCYATQSHQWYSWGPPNM QCRLCAICWLYWKKYGGLKMP TQSEEEKLSPSPTTEDPR (SEQ ID NO: 646) | GGCGCCGTGAACGGCGCCGTGGGCACCACCTTCCAGCCCAGAACCCCCTGC TGGGCAGAGCCTGCGAGAGCTGCTACGCCACCCAGAGCCACCAGTGGTACA GCTGGGGCCCCCCAACATGCAGTGCAGACTGTGCGCCATCTGCTGGCTGTA CTGGAAGAAGTACGGCGGCCTGAAGATGCCCACCCAGAGCGAGGAGGAGA AGCTGAGCCCCAGCCCCACCACCGAGGACCCCAGA (SEQ ID NO: 1219) | -2.4098352 |
| WWTR1 | LPPGWEMTFTAT GQRYFLNHIEKITT WQD (SEQ ID NO: 647) | GAAGSPAQQHAHLRQQSYDV TDELPLPPGWEMTFTATGQRY FLNHIEKITTWQDPRKAMNQP LNHMNLHPAVSSTPVPQR (SEQ ID NO: 648) | GGCGCCGCCGGCAGCCCGCCCAGCAGCACGCCCACCTGAGACAACAAAGCT ACGACGTGACCGACGAGCTGCCCCTGCCCCCGGCTGGGAGATGACCTTCAC CGCCACCGGACAGAGATACTTCCTGAACCACATCGAGAAGATCACCACCTGG CAGGACCCCAGAAAGGCCATGAACCAGCCCCTGAACCACATGAACCTGCACC CCGCCGTGAGCAGCACCCCCGTGCCCCAGAGA (SEQ ID NO: 1220) | -2.3989581 |
| EGR3 | GKFAFDSPSNWC QDNIISLMSAGILG VPPASGALSTQTST ASMVQPPQGDVE AMYPALPPYSNCG DLYS (SEQ ID NO: 649 ) | TVTYLGKFAFDSPSNWCQDNII SLMSAGILGVPPASGALSTQTS TASMVQPPQGDVEAMYPALP PYSNCGDLYSEPVSFH(SEQ ID NO: 650) | ACCGTGACCTACCTGGGCAAGTTCGCCTTCGACAGCCCCAGCAACTGGTGCC AGGACAACATCATCAGCCTGATGAGCGCCGGCATCCTGGGCGTGCCCCCCGC TAGCGGAGCTCTGAGCACACAGACCAGCACCGCCAGCATGGTGCAGCCCCCC CAGGGCGACGTGGAGGCCATGTACCCCGCCCTTCCTCCCTACAGCAACTGCG GCGACCTGTACAGCGAGCCCGTGAGCTTCCAC (SEQ ID NO: 1221) | -2.337045 |
| NFIX | LTQDEFHPFIEALLP HVRAFSYTWFNLQ ARKRKYFKKHE (SEQ ID NO: 651) | YSPYCLTQDEFHPFIEALLPHVR AFSYTWFNLQARKRKYFKKHEK RMSKDEERAVKDELLGEKPEIK QKWASRLLAKLRK (SEQ ID NO: 652) | TACAGCCCCTACTGCCTGACCCAGGACGAGTTCCACCCCTTCATCGAGGCCCT GCTGCCCCACGTGAGAGCCTTCAGCTACACCTGGTTCAACCTGCAGGCCAGA AAGAGAAAGTACTTCAAGAAGCACGAGAAGAGAATGAGCAAGGACGAGGA GAGAGCCGTGAAGGACGAGCTGCTGGGCGAGAAGCCCGAGATCAAGCAGA AGTGGGCCAGCAGACTGCTGGCCAAGCTGAGAAAG (SEQ ID NO: 1222) | -2.289111 |
| KPCI | SGEFGLDNFDSQF TNEPVQLTPDDDD IVRKIDQSEFEGFE (SEQ ID NO: 653) | QGHPFFRNVDWDMMEQKQV VPPFKPNISGEFGLDNFDSQFT NEPVQLTPDDDDIVRKIDQSEF EGFEYINPLLMSAEECV (SEQ ID NO: 654) | CAGGGCCACCCCTTCTTCAGAAACGTGGACTGGGACATGATGGAGCAGAAGC AGGTGGTGCCCCCCTTCAAGCCCAACATCAGCGGCGAGTTCGGCCTGGACAA CTTCGACAGCCAGTTCACCAACGAGCCCGTGCAGCTGACCCCCGACGACGAC GACATCGTGAGAAAGATCGACCAGAGCGAGTTCGAGGGCTTCGAGTACATC AACCCCCTGCTGATGAGCGCCGAGGAGTGCGTG (SEQ ID NO: 1223) | -2.2334296 |
| LMBL1 | MKLEAVDRRNPAL IRVASVEDVEDHRI KIHFDGWSHGYDF WIDADHPDIHPAG WCSKTGHPLQPPL GP (SEQ ID NO: 655) | SFLVNMKLEAVDRRNPALIRVA SVEDVEDHRIKIHFDGWSHGY DFWIDADHPDIHPAGWCSKTG HPLQPPLGPREPSSAS (SEQ ID NO: 656) | AGCTTCCTGGTGAACATGAAGCTGGAGGCCGTGGACAGAAGAAACCCCGCC CTGATCAGAGTGGCCAGCGTGGAGGACGTGGAGGACCACAGAATCAAGATC CACTTCGACGGCTGGAGCCACGGCTACGACTTCTGGATCGACGCCGACCACC CCGACATCCACCCCGCCGGCTGGTGCAGCAAGACCGGACACCCCCTGCAGCC TCCTCTGGGCCCCAGAGAGCCCAGCAGCGCTAGC (SEQ ID NO: 1224) | -2.2075416 |
| NOTC1 | WKNCTQSLQCWK YFSDGHCDSQCNS AGCLFDGFDC (SEQ ID NO: 657) | LQCNNHACGWDGGDCSLNFN DPWKNCTQSLQCWKYFSDGH CDSQCNSAGCLFDGFDCQRAE GQCNPLYDQYCKDHFSDGH (SEQ ID NO: 658) | CTGCAGTGCAACAACCACGCCTGCGGCTGGGACGGCGGCGACTGCAGCCTG AACTTCAACGACCCCTGGAAGAACTGCACCCAGAGCCTGCAGTGCTGGAAGT ACTTCAGCGACGGCCACTGCGACAGCCAGTGCAACAGCGCCGGCTGCCTGTT CGACGGCTTCGACTGCCAGAGAGCCGAGGGCCAGTGCAACCCCCTGTACGAC CAGTACTGCAAGGACCACTTCAGCGACGGCCAC (SEQ ID NO: 1225) | -2.1840238 |
| FIGN | TDLSAIMPSQLRPV TYQDFENAFCKIQP SISQKELDMYVEW NKMF (SEQ ID NO: 659) | LLVQRTEGFSGLDVAHLCQEAV VGPLHAMPATDLSAIMPSQLR PVTYQDFENAFCKIQPSISQKEL DMYVEWNKMFGCSQ (SEQ ID NO: 660) | CTGCTGGTGCAGAGAACCGAGGGCTTCAGCGGCCTGGACGTGGCCCACCTGT GCCAGGAGGCCGTGGTGGACCTCTGCACGCCATGCCCGCCACCGACCTGA GCGCCATCATGCCCAGCCAGCTGAGACCCGTGACCTACCAGGACTTCGAGAA CGCCTTCTGCAAGATCCAGCCCAGCATCAGCCAGAAGGAGCTGGACATGTAC GTGGAGTGGAACAAGATGTTCGGCTGCAGCCAG (SEQ ID NO: 1226) | -2.1805996 |
| IMA5 | NPYCALIEEAYGLD KIEFLQSHENQEIY QKAFDLIEHYFGTE DEDSSIAPQ (SEQ ID NO: 661) | RLGEQEAKRNGTGINPYCALIEE AYGLDKIEFLQSHENQEIYQKAF DLIEHYFGTEDEDSSIAPQVDLN QQQYIFQQCEA (SEQ ID NO: 662) | AGACTGGGCGAGCAGGAGGCCAAGAGAAACGGCACCGGCATCAACCCCTAC TGCGCCCTGATCGAGGAGGCCTACGGCCTGGACAAGATCGAGTTCCTGCAGA GCCACGAGAACCAGGAGATCTACCAGAAGGCCTTCGACCTGATCGAGCACTA CTTCGGCACCGAGGACGAGGACAGCAGCATCGCCCCCCAGGTGGACCTGAA CCAGCAGCAGTACATCTTCCAGCAGTGCGAGGCC (SEQ ID NO: 1227) | -2.15155 |
| ZN496 | PFKDMILCFSEED WSLLDPAQTGFYG EFIIGEDY (SEQ ID NO: 663) | QEENVRDTQQVTTLQLPPSRVS PFKDMILCFSEEDWSLLDPAQT GFYGEFIIGEDYGVSMPPNDLA AQPDLSQGEENEPR (SEQ ID NO: 664) | CAGGAGGAGAACGTGAGAGACACCCAGCAGGTGACCACCCTGCAGCTGCCC CCCAGCAGAGTGAGCCCCTTCAAGGACATGATCCTGTGCTTCAGCGAGGAGG ACTGGAGCCTGCTGGACCCCGCCCAGACCGGCTTCTACGGCGAGTTCATCAT CGGCGAGGACTACGGCGTGAGCATGCCCCCCAACGACCTGGCCGCCCAGCCC GACCTTAGCCAGGGCGAGGAGAACGAGCCCAGA (SEQ ID NO: 1228) | -2.1412028 |

**Table 3: KRAB Repressor Mutants**

| **Variant** | **Start of Mutation** | **Amino Acid sequence** | **DNA sequence** | **Norm Avg D13 ( 0 = Wild Type score)** |
|---|---|---|---|---|
| GlutamicAcid ;3;5 | 5 | DAKSLEEESRTLVTFKDV FVDFTREEWKLLDTAQQ IVYRNVMLENYKNLVSL GYQLTKPDVILRLEKGEE P (SEQ ID NO: 665) | GATGCCAAGTCCCTTGAGGAGGAGTCTCGGACCCTGGTTACCTTTAAGGATG TGTTCGTCGACTTCACTAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1229) | 1.28823665 |
| GlutamicAcid ;3;7 | 7 | DAKSLTAEEETLVTFKDV FVDFTREEWKLLDTAQQ IVYRNVMLENYKNLVSL GYQLTKPDVILRLEKGEE P (SEQ ID NO: 666) | GATGCCAAGTCCTTGACCGCTGAGGAGGAGACCCTGGTCACTTTCAAGGACG TTTTTGTGGATTTCACGAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1230) | 1.14468005 |
| AsparticAcid; 3;7 | 7 | DAKSLTADDDTLVTFKD VFVDFTREEWKLLDTAQ QIVYRNVMLENYKNLVS LGYQLTKPDVILRLEKGE EP (SEQ ID NO: 667) | GATGCTAAGTCCCTGACCGCCGATGATGACACCCTTGTGACTTTTAAGGATGT CTTCGTTGACTTCACGAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1231) | 1.10622079 |
| Isoleucine;3; 67 | 67 | DAKSLTAWSRTLVTFKD VFVDFTREEWKLLDTAQ QIVYRNVMLENYKNLVS LGYQLTKPDVILRLEKIIIP (SEQ ID NO: 668) | GATGCTAAGTCTCTGACCGCCTGGTCCCGGACTCTTGTTACGTTTAAGGACGT GTTCGTCGATTTCACCAGGGAAGAATGGAAACTACTAGACACAGCACAACAA ATAGTATACAGAAACGTAATGCTAGAGAACTACAAAAACCTAGTAAGCCTAG GATACCAACTCACAAAACCAGACGTAATACTAAGACTAGAAAAAATAATAAT ACCA (SEQ ID NO: 1232) | 1.08345235 |
| Proline;3;6 | 6 | DAKSLTPPPRTLVTFKDV FVDFTREEWKLLDTAQQ IVYRNVMLENYKNLVSL GYQLTKPDVILRLEKGEE P (SEQ ID NO: 669) | GATGCCAAGTCCCTTACCCCCCCTCCCCGGACTCTGGTGACGTTTAAGGATGT CTTCGTTGACTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1233) | 1.06556067 |
| Asparagine;1 ;1 | 1 | DNKSLTAWSRTLVTFKD VFVDFTREEWKLLDTAQ QIVYRNVMLENYKNLVS LGYQLTKPDVILRLEKGE EP (SEQ ID NO: 670) | GATAATAAGTCCCTTACCGCCTGGTCTCGGACGCTGGTTACTTTCAAGGACGT CTTCGTGGATTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1234) | 1.05496491 |
| Proline;3;9 | 9 | DAKSLTAWSPPPVTFKD VFVDFTREEWKLLDTAQ QIVYRNVMLENYKNLVS LGYQLTKPDVILRLEKGE EP (SEQ ID NO: 671) | GATGCTAAGTCTTTGACTGCCTGGTCCCCTCCCCCCGTTACCTTCAAGGATGT GTTTGTCGACTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1235) | 1.05426168 |
| GlutamicAcid ;2;6 | 6 | DAKSLTEESRTLVTFKDV FVDFTREEWKLLDTAQQ IVYRNVMLENYKNLVSL GYQLTKPDVILRLEKGEE P (SEQ ID NO: 672) | GACGCCAAGTCCCTGACGGAGGAGTCTCGGACCCTTGTGACTTTTAAGGATG TCTTCGTTGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1236) | 1.04111335 |
| Alanine;2;7 | 7 | DAKSLTAAARTLVTFKDV FVDFTREEWKLLDTAQQ IVYRNVMLENYKNLVSL GYQLTKPDVILRLEKGEE P (SEQ ID NO: 673) | GACGCTAAGTCCCTTACCGCCGCCGCGAGGACCCTGGTTACTTTTAAGGATG TCTTCGTGGATTTCACGCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1237) | 1.04035858 |
| Valine;1;4 | 4 | DAKSVTAWSRTLVTFKD VFVDFTREEWKLLDTAQ QIVYRNVMLENYKNLVS LGYQLTKPDVILRLEKGE EP (SEQ ID NO: 674) | GATGCCAAGTCTGTGACCGCTTGGTCCCGGACGCTTGTCACCTTCAAGGACG TTTTGTGGATTTCACTAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1238) | 0.96430263 |
| Proline;1;7 | 7 | DAKSLTAPSRTLVTFKDV FVDFTREEWKLLDTAQQ IVYRNVMLENYKNLVSL GYQLTKPDVILRLEKGEE P(SEQ ID NO: 675) | GATGCTAAGTCTCTTACCGCCCCCTCCCGGACTCTGGTCACCTTCAAGGACGT GTTCGTTGATTTACGAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1239) | 0.96424497 |
| Glycine;2;7 | 7 | DAKSLTAGGRTLVTFKD VFVDFTREEWKLLDTAQ QIVYRNVMLENYKNLVS LGYQLTKPDVILRLEKGE EP (SEQ ID NO: 676) | GACGCCAAGTCCCTTACCGCTGGCGGGCGGACCCTGGTGACTTTCAAGGATG TTTTCGTCGATTTTACGAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1240) | 0.94837463 |
| Asparagine;3 ;5 | 5 | DAKSLNNNSRTLVTFKD VFVDFTREEWKLLDTAQ QIVYRNVMLENYKNLVS LGYQLTKPDVILRLEKGE EP (SEQ ID NO: 677) | GATGCCAAGTCTCTTAATAATAATTCCCGGACTCTGGTGACCTTTAAGGACGT TTTCGTCGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1241) | 0.92066978 |
| Glutamine;3; 7 | 7 | DAKSLTAQQQTLVTFKD VFVDFTREEWKLLDTAQ QIVYRNVMLENYKNLVS LGYQLTKPDVILRLEKGE EP (SEQ ID NO: 678) | GATGCCAAGTCCCTTACCGCTCAGCAGCAGACTCTGGTCACGTTCAAGGACG TTTTTGTGGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1242) | 0.91183545 |
| Proline;2;10 | 10 | DAKSLTAWSRPPVTFKD VFVDFTREEWKLLDTAQ QIVYRNVMLENYKNLVS LGYQLTKPDVILRLEKGE EP (SEQ ID NO: 679) | GATGCTAAGTCTCTTACTGCCTGGTCCCGGCCTCCCGTGACCTTCAAGGATGT CTTCGTTGACTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1243) | 0.89572995 |
| Threonine;2; 7 | 7 | DAKSLTATTRTLVTFKDV FVDFTREEWKLLDTAQQ IVYRNVMLENYKNLVSL GYQLTKPDVILRLEKGEE P (SEQ ID NO: 680) | GACGCTAAGTCCCTGACCGCCACGACCCGGACTCTTGTCACTTTCAAGGATGT GTTCGTTGATTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1244) | 0.88884291 |
| GlutamicAcid ;2;10 | 10 | DAKSLTAWSREEVTFKD VFVDFTREEWKLLDTAQ QIVYRNVMLENYKNLVS LGYQLTKPDVILRLEKGE EP (SEQ ID NO: 681) | GATGCCAAGTCTCTTACCGCTTGGTCCAGGGAGGAGGTCACTTTCAAGGACG TGTTCGTTGATTTACCCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1245) | 0.86791044 |
| AsparticAcid; 2;7 | 7 | DAKSLTADDRTLVTFKD VFVDFTREEWKLLDTAQ QIVYRNVMLENYKNLVS LGYQLTKPDVILRLEKGE EP (SEQ ID NO: 682) | GATGCCAAGTCCCTTACCGCTGATGACCGGACCCTGGTCACGTTCAAGGATG TGTTTGTTGACTTCACTAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1246) | 0.86480685 |
| Glutamine;3; 6 | 6 | DAKSLTQQQRTLVTFKD VFVDFTREEWKLLDTAQ QIVYRNVMLENYKNLVS LGYQLTKPDVILRLEKGE EP (SEQ ID NO: 683) | GATGCCAAGTCCCTTACGCAGCAGCAGCGGACTCTGGTTACCTTCAAGGACG TGTTTGTCGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1247) | 0.86314843 |
| GlutamicAcid ;3;4 | 4 | DAKSEEEWSRTLVTFKD VFVDFTREEWKLLDTAQ QIVYRNVMLENYKNLVS LGYQLTKPDVILRLEKGE EP (SEQ ID NO: 684) | GACGCCAAGTCCGAGGAGGAGTGGTCTAGGACCCTTGTCACCTTTAAGGATG TTTTCGTGGATTTCACTCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1248) | 0.84553985 |
| AsparticAcid; 2;6 | 6 | DAKSLTDDSRTLVTFKDV FVDFTREEWKLLDTAQQ IVYRNVMLENYKNLVSL GYQLTKPDVILRLEKGEE P (SEQ ID NO: 685) | GATGCCAAGTCCCTTACCGACGACTCTAGGACGCTGGTTACTTTTAAGGATGT GTTCGTCGATTTCACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1249) | 0.84522896 |
| Asparagine;2 ;7 | 7 | DAKSLTANNRTLVTFKD VFVDFTREEWKLLDTAQ QIVYRNVMLENYKNLVS LGYQLTKPDVILRLEKGE EP (SEQ ID NO: 686) | GACGCTAAGTCCCTGACGGCCAATAATCGGACCCTTGTTACCTTTAAGGATGT GTTCGTCGATTTCACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1250) | 0.84228978 |
| Asparagine;3 ;0 | 0 | NNNSLTAWSRTLVTFKD VFVDFTREEWKLLDTAQ QIVYRNVMLENYKNLVS LGYQLTKPDVILRLEKGE EP (SEQ ID NO: 687) | AATAATAATTCTCTTACCGCCTGGTCCAGGACGCTGGTGACCTTCAAGGATGT TTTTGTCGATTTCACTCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1251) | 0.83772353 |
| Glycine;3;5 | 5 | DAKSLGGGSRTLVTFKD VFVDFTREEWKLLDTAQ QIVYRNVMLENYKNLVS LGYQLTKPDVILRLEKGE EP (SEQ ID NO: 688) | GATGCCAAGTCCCTGGGCGGTGGGTCTCGGACCCTTGTTACTTTTAAGGACG TGTTCGTCGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1252) | 0.83010312 |
| AsparticAcid; 3;6 | 6 | DAKSLTDDDRTLVTFKD VFVDFTREEWKLLDTAQ QIVYRNVMLENYKNLVS LGYQLTKPDVILRLEKGE EP (SEQ ID NO: 689) | GATGCCAAGTCCCTTACCGACGACGATAGGACTCTGGTTACCTTCAAGGATG TGTTCGTCGATTTTACGCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1253) | 0.82122205 |
| AsparticAcid; 3;5 | 5 | DAKSLDDDSRTLVTFKD VFVDFTREEWKLLDTAQ QIVYRNVMLENYKNLVS LGYQLTKPDVILRLEKGE EP (SEQ ID NO: 690) | GATGCCAAGTCTCTGGACGATGATTCCCGGACCCTTGTCACCTTTAAGGATGT GTTCGTTGACTTCACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1254 ) | 0.81720761 |
| Lysine;2;62 | 62 | DAKSLTAWSRTLVTFKD VFVDFTREEWKLLDTAQ QIVYRNVMLENYKNLVS LGYQLTKPDVIKKLEKGE EP (SEQ ID NO: 691 ) | GACGCCAAGTCTCTGACTGCTTGGTCCCGGACCCTGGTGACGTTTAAGGATG TCTTCGTTGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATAAAAAAACTAGAAAAAGGAGAA GAACCA(SEQ ID NO: 1255) | 0.79372667 |
| AsparticAcid; 1;7 | 7 | DAKSLTADSRTLVTFKDV FVDFTREEWKLLDTAQQ IVYRNVMLENYKNLVSL GYQLTKPDVILRLEKGEE P(SEQ ID NO: 692) | GATGCTAAGTCCCTTACCGCCGATTCTAGGACGCTGGTGACCTTCAAGGATG TCTTTGTTGACTTCACTCGGGAAGAATGGAAACTACTAGACACAGCACAACA AATAGTATACAGAAACGTAATGCTAG AAAACTACAAAAACCTAGTAAGCCTA GGATACCAACTAACAAAACCAGACGTAATACTAAGACTAGAAAAAGGAGAA GAACCA (SEQ ID NO: 1256) | 0.77854784 |
| Lysine;3;5 | 5 | DAKSLKKKSRTLVTFKDV FVDFTREEWKLLDTAQQ IVYRNVMLENYKNLVSL GYQLTKPDVILRLEKGEE P (SEQ ID NO: 693) | GATGCCAAGTCTCTGAAGAAGAAGTCCCGGACCCTTGTCACTTTCAAGGACG TTTTTGTGGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1257) | 0.76670536 |
| GlutamicAcid ;1;7 | 7 | DAKSLTAESRTLVTFKDV FVDFTREEWKLLDTAQQ IVYRNVMLENYKNLVSL GYQLTKPDVILRLEKGEE P (SEQ ID NO: 694) | GATGCCAAGTCTCTGACCGCTGAGTCCAGGACTCTTGTTACGTTCAAGGATGT CTTTGTGGACTTCACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1258) | 0.76316547 |
| Proline;2;7 | 7 | DAKSLTAPPRTLVTFKDV FVDFTREEWKLLDTAQQ IVYRNVMLENYKNLVSL GYQLTKPDVILRLEKGEE P (SEQ ID NO: 695) | GACGCCAAGTCCCTTACGGCTCCCCCTCGGACCCTGGTCACTTTCAAGGATGT TTTCGTGGATTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1259) | 0.7537889 |
| Proline;3;7 | 7 | DAKSLTAPPPTLVTFKDV FVDFTREEWKLLDTAQQ IVYRNVMLENYKNLVSL GYQLTKPDVILRLEKGEE P (SEQ ID NO: 696 ) | GATGCCAAGTCCCTTACTGCTCCTCCCCCACGCTGGTTACCTTTAAGGACGT GTTCGTCGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1260) | 0.74610592 |
| AsparticAcid; 2;8 | 8 | DAKSLTAWDDTLVTFKD VFVDFTREEWKLLDTAQ QIVYRNVMLENYKNLVS LGYQLTKPDVILRLEKGE EP(SEQ ID NO: 697 ) | GATGCTAAGTCCCTGACGGCCTGGGACGATACTTTGGTGACCTTTAAGGACG TCTTCGTTGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1261) | 0.74252065 |
| Asparagine;3 ;7 | 7 | DAKSLTANNNTLVTFKD VFVDFTREEWKLLDTAQ QIVYRNVMLENYKNLVS LGYQLTKPDVILRLEKGE EP (SEQ ID NO: 698 ) | GACGCTAAGTCCCTTACGGCCAATAATAATACTCTGGTTACCTTTAAGGATGT CTTCGTGGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1262) | 0.72618378 |
| Methionine;1 ;7 | 7 | DAKSLTAMSRTLVTFKD VFVDFTREEWKLLDTAQ QIVYRNVMLENYKNLVS LGYQLTKPDVILRLEKGE EP  (SEQ ID NO: 699) | GATGCCAAGTCTCTGACTGCTATGTCCAGGACCCTTGTCACCTTCAAGGACGT GTTTGTTGATTTCACGCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1263) | 0.71061025 |
| Asparagine;3 ;8 | 8 | DAKSLTAWNNNLVTFK DVFVDFTREEWKLLDTA QQIVYRNVMLENYKNLV SLGYQLTKPDVILRLEKG EEP (SEQ ID NO: 700) | GATGCCAAGTCCCTTACCGCTTGGAATAATAATCTGGTGACTTTCAAGGATGT TTTTGTCGACTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1264) | 0.70490419 |
| Asparagine;2 ;9 | 9 | DAKSLTAWSNNLVTFKD VFVDFTREEWKLLDTAQ QIVYRNVMLENYKNLVS LGYQLTKPDVILRLEKGE EP (SEQ ID NO: 701) | GATGCTAAGTCCCTGACCGCCTGGTCTAATAATTTGGTTACCTTCAAGGATGT GTTTGTCGACTTCACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1265) | 0.7021223 |
| Proline;2;6 | 6 | DAKSLTPPSRTLVTFKDV FVDFTREEWKLLDTAQQ IVYRNVMLENYKNLVSL GYQLTKPDVILRLEKGEE P (SEQ ID NO: 702) | GACGCCAAGTCCCTTACGCCCCCTTCTCGGACTCTGGTGACCTTTAAGGATGT CTTCGTTGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1266) | 0.70094498 |
| GlutamicAcid ;3;6 | 6 | DAKSLTEEERTLVTFKDV FVDFTREEWKLLDTAQQ IVYRNVMLENYKNLVSL GYQLTKPDVILRLEKGEE P (SEQ ID NO: 703) | GACGCCAAGTCCCTGACGGAGGAGGAGCGGACTCTTGTGACCTTTAAGGAT GTTTTCGTCGATTTCACCAGGGAAGAATGGAAACTACTAGACACAGCACAAC AAATAGTATACAGAAACGTAATGCTAGAAAACTACAAAAACCTAGTAAGCCT AGGATACCAACTAACAAAACCAGACGTAATACTAAGACTAGAAAAAGGAGA AGAACCA (SEQ ID NO: 1267) | 0.69641957 |
| Alanine;3;5 | 5 | DAKSLAAASRTLVTFKDV FVDFTREEWKLLDTAQQ IVYRNVMLENYKNLVSL GYQLTKPDVILRLEKGEE P (SEQ ID NO: 704 ) | GATGCTAAGTCTTTGGCCGCGGCCTCCAGGACCCTTGTGACTTTCAAGGACG TTTTTGTCGATTTCACCCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1268) | 0.69263933 |
| Glutamine;2; 7 | 7 | DAKSLTAQQRTLVTFKD VFVDFTREEWKLLDTAQ QIVYRNVMLENYKNLVS LGYQLTKPDVILRLEKGE EP (SEQ ID NO: 705) | GATGCTAAGTCCCTGACCGCCCAGCAGCGGACGCTTGTTACTTTCAAGGATG TCTTTGTGGACTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1269) | 0.67752105 |
| GlutamicAcid ;3;9 | 9 | DAKSLTAWSEEEVTFKD VFVDFTREEWKLLDTAQ QIVYRNVMLENYKNLVS LGYQLTKPDVILRLEKGE EP (SEQ ID NO: 706) | GATGCCAAGTCCCTTACCGCTTGGTCTGAGGAGGAGGTTACTTTCAAGGATG TGTTTGTCGACTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1270) | 0.66806615 |
| Glutamine;2; 6 | 6 | DAKSLTQQSRTLVTFKD VFVDFTREEWKLLDTAQ QIVYRNVMLENYKNLVS LGYQLTKPDVILRLEKGE EP (SEQ ID NO: 707) | GATGCCAAGTCTCTGACTCAGCAGTCCCGGACCCTTGTGACGTTCAAGGACG TTTTCGTCGATTTTACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1271) | 0.65472821 |
| GlutamicAcid ;1;39 | 39 | DAKSLTAWSRTLVTFKD VFVDFTREEWKLLDTAQ QIVYREVMLENYKNLVS LGYQLTKPDVILRLEKGE EP (SEQ ID NO: 708) | GACGCTAAGTCCCTTACGGCCTGGTCTAGGACCCTGGTCACCTTTAAGGATGT TTTCGTGGATTTCACTCGGGAAGAATGGAAACTACTAGACACAGCACAACAA ATAGTATACAGAGAAGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1272) | 0.65227203 |
| Proline;1;10 | 10 | DAKSLTAWSRPLVTFKD VFVDFTREEWKLLDTAQ QIVYRNVMLENYKNLVS LGYQLTKPDVILRLEKGE EP (SEQ ID NO: 709) | GATGCCAAGTCTCTTACCGCTTGGTCCAGGCCCCTGGTGACTTTCAAGGATGT CTTTGTTGACTTCACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1273) | 0.64837531 |
| Threonine;1; 4 | 4 | DAKSTTAWSRTLVTFKD VFVDFTREEWKLLDTAQ QIVYRNVMLENYKNLVS LGYQLTKPDVILRLEKGE EP (SEQ ID NO: 710) | GACGCTAAGTCCACCACCGCCTGGTCTAGGACTCTTGTTACGTTCAAGGATGT GTTTGTCGATTTCACTCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1274) | 0.64360588 |
| Asparagine;3 ;9 | 9 | DAKSLTAWSNNNVTFK DVFVDFTREEWKLLDTA QQIVYRNVMLENYKNLV SLGYQLTKPDVILRLEKG EEP (SEQ ID NO: 711) | GACGCCAAGTCTCTTACCGCTTGGTCCAATAATAATGTTACTTTCAAGGATGT GTTTGTCGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1275) | 0.63949165 |
| AsparticAcid; 3;8 | 8 | DAKSLTAWDDDLVTFKD VFVDFTREEWKLLDTAQ QIVYRNVMLENYKNLVS LGYQLTKPDVILRLEKGE EP (SEQ ID NO: 712) | GATGCTAAGTCCCTTACTGCCTGGGACGATGATCTGGTCACCTTCAAGGACG TTTTTGTGGATTTCACCAGGGAAGAATGGAAACTACTAGACACAGCACAACA AATAGTATACAGAAACGTAATGCTAG AAAACTACAAAAACCTAGTAAGCCTA GGATACCAACTAACAAAACCAGACGTAATACTAAGACTAGAAAAAGGAGAA GAACCA (SEQ ID NO: 1276) | 0.63564982 |
| Alanine;2;6 | 6 | DAKSLTAASRTLVTFKDV FVDFTREEWKLLDTAQQ IVYRNVMLENYKNLVSL GYQLTKPDVILRLEKGEE P (SEQ ID NO: 713) | GATGCTAAGTCCCTTACTGCCGCCTCTCGGACGCTGGTGACCTTCAAGGACGT TTTCGTCGATTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1277) | 0.63459967 |
| Glycine;1;7 | 7 | DAKSLTAGSRTLVTFKDV FVDFTREEWKLLDTAQQ IVYRNVMLENYKNLVSL GYQLTKPDVILRLEKGEE P (SEQ ID NO: 714) | GACGCCAAGTCTTTGACGGCTGGCTCCCGGACCCTTGTGACTTTCAAGGATG TCTTCGTTGATTTACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1278) | 0.6338712 |
| Serine;1;7 | 7 | DAKSLTASSRTLVTFKDV FVDFTREEWKLLDTAQQ IVYRNVMLENYKNLVSL GYQLTKPDVILRLEKGEE P (SEQ ID NO: 715) | GACGCTAAGAGTCTGACTGCCTCTTCCCGGACCCTTGTCACGTTCAAGGATGT TTTCGTGGATTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1279) | 0.6299573 |
| Serine;2;6 | 6 | DAKSLTSSSRTLVTFKDV FVDFTREEWKLLDTAQQ IVYRNVMLENYKNLVSL GYQLTKPDVILRLEKGEE P (SEQ ID NO: 716) | GATGCCAAGTCCCTGACGTCTAGTTCAAGGACTCTTGTGACCTTTAAGGACGT CTTCGTTGATTTCACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1280) | 0.61708486 |
| AsparticAcid; 2;3 | 3 | DAKDDTAWSRTLVTFKD VFVDFTREEWKLLDTAQ QIVYRNVMLENYKNLVS LGYQLTKPDVILRLEKGE EP (SEQ ID NO: 717) | GATGCTAAGGATGACACTGCCTGGTCCCGGACGCTTGTGACCTTTAAGGATG TCTTCGTTGACTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1281) | 0.61493283 |
| Histidine;1;5 5 | 55 | DAKSLTAWSRTLVTFKD VFVDFTREEWKLLDTAQ QIVYRNVMLENYKNLVS LGYQHTKPDVILRLEKGE EP (SEQ ID NO: 718) | GATGCCAAGTCTCTGACCGCTTGGTCCAGGACGCTTGTGACTTTCAAGGATG TCTTCGTTGACTTTACCCGGGAAGAATGGAAACTACTAGACACAGCACAACA AATAGTATACAGAAACGTAATGCTAGAAAACTACAAAAACCTAGTAAGCCTA GGATACCAACACACAAAACCAGACGTAATACTAAGACTAGAAAAAGGAGAA GAACCA (SEQ ID NO: 1282) | 0.59443523 |
| Lysine;2;6 | 6 | DAKSLTKKSRTLVTFKDV FVDFTREEWKLLDTAQQ IVYRNVMLENYKNLVSL GYQLTKPDVILRLEKGEE P (SEQ ID NO: 719) | GATGCCAAGTCCCTTACCAAGAAGTCTAGGACTCTGGTTACCTTCAAGGACG TCTTTGTGGATTTCACGCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1283) | 0.59389075 |
| GlutamicAcid ;2;9 | 9 | DAKSLTAWSEELVTFKD VFVDFTREEWKLLDTAQ QIVYRNVMLENYKNLVS LGYQLTKPDVILRLEKGE EP (SEQ ID NO: 720) | GATGCCAAGTCCCTTACTGCTTGGTCTGAGGAGCTGGTGACCTTCAAGGATG TCTTTGTTGACTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1284) | 0.58357862 |
| Glutamine;3; 8 | 8 | DAKSLTAWQQQLVTFK DVFVDFTREEWKLLDTA QQIVYRNVMLENYKNLV SLGYQLTKPDVILRLEKG EEP (SEQ ID NO: 721) | GATGCCAAGTCCCTTACCGCTTGGCAGCAGCAGCTGGTCACTTTCAAGGACG TGTTCGTTGATTTTACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1285) | 0.58274443 |
| Threonine;1; 11 | 11 | DAKSLTAWSRTTVTFKD VFVDFTREEWKLLDTAQ QIVYRNVMLENYKNLVS LGYQLTKPDVILRLEKGE EP (SEQ ID NO: 722) | GATGCCAAGTCCCTTACGGCTTGGTCTAGGACCACCGTGACTTTCAAGGACG TTTTCGTCGATTTTACTCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1286) | 0.57209112 |
| Asparagine;1 ;7 | 7 | DAKSLTANSRTLVTFKDV FVDFTREEWKLLDTAQQ IVYRNVMLENYKNLVSL GYQLTKPDVILRLEKGEE P (SEQ ID NO: 723) | GATGCCAAGTCTCTTACCGCTAATTCCAGGACGCTGGTCACCTTCAAGGATGT TTTTGTGGACTTCACTCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1287) | 0.57143202 |
| Glutamine;3; 5 | 5 | DAKSLQQQSRTLVTFKD VFVDFTREEWKLLDTAQ QIVYRNVMLENYKNLVS LGYQLTKPDVILRLEKGE EP (SEQ ID NO: 724) | GACGCCAAGTCTCTGCAGCAGCAGTCCCGGACTCTTGTTACCTTCAAGGATGT GTTTGTCGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1288) | 0.57133084 |
| Proline;2;8 | 8 | DAKSLTAWPPTLVTFKD VFVDFTREEWKLLDTAQ QIVYRNVMLENYKNLVS LGYQLTKPDVILRLEKGE EP (SEQ ID NO: 725) | GATGCTAAGTCCCTTACCGCCTGGCCTCCCACCCTGGTTACTTTTAAGGATGT GTTCGTCGACTTCACGAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1289) | 0.56714292 |
| GlutamicAcid ;3;3 | 3 | DAKEEEAWSRTLVTFKD VFVDFTREEWKLLDTAQ QIVYRNVMLENYKNLVS LGYQLTKPDVILRLEKGE EP (SEQ ID NO: 726) | GACGCTAAGGAGGAGGAGGCCTGGTCCAGGACCCTTGTTACCTTTAAGGAT GTGTTCGTCGATTTCACTCGGGAAGAATGGAAACTACTAGACACAGCACAAC AAATAGTATACAGAAACGTAATGCTAGAAAACTACAAAAACCTAGTAAGCCT AGGATACCAACTAACAAAACCAGACGTAATACTAAGACTAGAAAAAGGAGA AGAACCA (SEQ ID NO: 1290) | 0.55531398 |
| Asparagine;2 ;6 | 6 | DAKSLTNNSRTLVTFKD VFVDFTREEWKLLDTAQ QIVYRNVMLENYKNLVS LGYQLTKPDVILRLEKGE EP (SEQ ID NO: 727) | GATGCCAAGTCCCTGACCAATAATTCTCGGACCCTTGTCACTTTTAAGGACGT GTTCGTTGATTTCACGAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1291) | 0.55430182 |
| AsparticAcid; 1;11 | 11 | DAKSLTAWSRTDVTFKD VFVDFTREEWKLLDTAQ QIVYRNVMLENYKNLVS LGYQLTKPDVILRLEKGE EP (SEQ ID NO: 728) | GATGCTAAGTCTCTTACGGCCTGGTCCAGGACCGATGTGACTTTCAAGGACG TTTTCGTCGATTTTACCCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1292) | 0.55416963 |
| Threonine;3; 7 | 7 | DAKSLTATTTTLVTFKDV FVDFTREEWKLLDTAQQ IVYRNVMLENYKNLVSL GYQLTKPDVILRLEKGEE P (SEQ ID NO: 729) | GACGCCAAGTCCCTTACTGCTACTACCACCACGCTGGTTACCTTTAAGGATGT CTTCGTGGATTTCACAAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1293) | 0.54915125 |
| AsparticAcid; 2;9 | 9 | DAKSLTAWSDDLVTFKD VFVDFTREEWKLLDTAQ QIVYRNVMLENYKNLVS LGYQLTKPDVILRLEKGE EP (SEQ ID NO: 730) | GATGCCAAGTCCCTTACCGCTTGGTCTGATGACCTGGTTACCTTCAAGGACGT CTTCGTGGATTTTACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1294) | 0.54802537 |
| Lysine;1;7 | 7 | DAKSLTAKSRTLVTFKDV FVDFTREEWKLLDTAQQ IVYRNVMLENYKNLVSL GYQLTKPDVILRLEKGEE P (SEQ ID NO: 731) | GATGCTAAGTCCCTCACCGCCAAGTCTAGGACCTTGGTCACGTTCAAGGACG TTTTCGTGGATTTTACTCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1295) | 0.54460274 |
| GlutamicAcid ;1;15 | 15 | DAKSLTAWSRTLVTFED VFVDFTREEWKLLDTAQ QIVYRNVMLENYKNLVS LGYQLTKPDVILRLEKGE EP (SEQ ID NO: 732) | GATGCCAAGTCTCTGACCGCTTGGTCCCGGACCCTTGTCACGTTCGAGGATGT TTTCGTGGACTTTACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1296) | 0.54206866 |
| AsparticAcid; 2;15 | 15 | DAKSLTAWSRTLVTFDD VFVDFTREEWKLLDTAQ QIVYRNVMLENYKNLVS LGYQLTKPDVILRLEKGE EP (SEQ ID NO: 733) | GATGCCAAGTCCCTGACTGCTTGGTCTAGGACCCTTGTGACCTTTGATGATGT TTTCGTCGACTTCACGCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1297) | 0.526979 |
| AsparticAcid; 1;15 | 15 | DAKSLTAWSRTLVTFDD VFVDFTREEWKLLDTAQ QIVYRNVMLENYKNLVS LGYQLTKPDVILRLEKGE EP (SEQ ID NO: 734) | GACGCTAAGTCCCTTACCGCCTGGTCTCGGACGCTGGTGACCTTTGATGATGT CTTCGTTGATTTCACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1298) | 0.52579234 |
| Glutamine;3; 9 | 9 | DAKSLTAWSQQQVTFK DVFVDFTREEWKLLDTA QQIVYRNVMLENYKNLV SLGYQLTKPDVILRLEKG EEP (SEQ ID NO: 735) | GACGCTAAGTCCCTTACCGCCTGGTCTCAGCAGCAGGTTACTTTCAAGGATGT CTTTGTGGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1299) | 0.52493949 |
| GlutamicAcid ;1;50 | 50 | DAKSLTAWSRTLVTFKD VFVDFTREEWKLLDTAQ QIVYRNVMLENYKNLVE LGYQLTKPDVILRLEKGE EP (SEQ ID NO: 736) | GATGCTAAGAGCCTTACCGCCTGGTCCCGGACCCTGGTGACTTTCAAGGACG TCTTCGTTGATTTACGAGGGAAGAATGGAAACTACTAGACACAGCACAACA AATAGTATACAGAAACGTAATGCTAGAAAACTACAAAAACCTAGTAGAACTA GGATACCAACTAACAAAACCAGACGTAATACTAAGACTAGAAAAAGGAGAA GAACCA (SEQ ID NO: 1300) | 0.52368253 |
| GlutamicAcid ;3;8 | 8 | DAKSLTAWEEELVTFKD VFVDFTREEWKLLDTAQ QIVYRNVMLENYKNLVS LGYQLTKPDVILRLEKGE EP (SEQ ID NO: 737) | GACGCCAAGTCCCTTACCGCTTGGGAGGAGGAGCTGGTCACTTTCAAGGATG TTTTTGTGGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1301) | 0.51955266 |
| GlutamicAcid ;1;35 | 35 | DAKSLTAWSRTLVTFKD VFVDFTREEWKLLDTAQ QEVYRNVMLENYKNLVS LGYQLTKPDVILRLEKGE EP (SEQ ID NO: 738) | GATGCTAAGTCCCTGACCGCCTGGTCTCGGACCCTTGTGACGTTCAAGGACG TCTTTGTTGATTTCACTAGGGAAGAATGGAAACTACTAGACACAGCACAACA AGAAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1302) | 0.5179103 |
| Proline;1;11 | 11 | DAKSLTAWSRTPVTFKD VFVDFTREEWKLLDTAQ QIVYRNVMLENYKNLVS LGYQLTKPDVILRLEKGE EP (SEQ ID NO: 739) | GATGCCAAGTCTCTTACGGCTTGGTCCCGGACCCCCGTTACCTTTAAGGACGT CTTCGTGGATTTCACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1303) | 0.51721922 |
| GlutamicAcid ;3;54 | 54 | DAKSLTAWSRTLVTFKD VFVDFTREEWKLLDTAQ QIVYRNVMLENYKNLVS LGYEEEKPDVILRLEKGEE P (SEQ ID NO: 740) | GACGCCAAGTCCCTTACGGCTTGGTCTCGGACTCTGGTCACCTTTAAGGATGT GTTCGTTGATTTCACCAGGGAAGAATGGAAACTACTAGACACAGCACAACAA ATAGTATACAGAAACGTAATGCTAGAAAACTACAAAAACCTAGTAAGCCTAG GATACGAAGAAGAAAAACCAGACGTAATACTAAGACTAGAAAAAGGAGAAG AACCA (SEQ ID NO: 1304) | 0.51510696 |
| AsparticAcid; 2;35 | 35 | DAKSLTAWSRTLVTFKD VFVDFTREEWKLLDTAQ QDDYRNVMLENYKNLV SLGYQLTKPDVILRLEKG EEP (SEQ ID NO: 741) | GATGCTAAGTCCCTGACTGCCTGGTCTAGGACCCTTGTCACCTTTAAGGATGT GTTCGTTGATTTCACGCGGGAAGAATGGAAACTACTAGACACAGCACAACAA GACGACTACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1305) | 0.51046316 |
| Alanine;3;6 | 6 | DAKSLTAAARTLVTFKDV FVDFTREEWKLLDTAQQ IVYRNVMLENYKNLVSL GYQLTKPDVILRLEKGEE P (SEQ ID NO: 742) | GACGCGAAGTCCCTGACGGCCGCTGCCCGGACTCTTGTTACCTTCAAGGATG TGTTCGTCGATTTTACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1306) | 0.50902755 |
| Glutamine;1; 7 | 7 | DAKSLTAQSRTLVTFKDV FVDFTREEWKLLDTAQQ IVYRNVMLENYKNLVSL GYQLTKPDVILRLEKGEE P (SEQ ID NO: 743) | GATGCTAAGTCCCTTACGGCCCAGTCTCGGACTCTGGTTACCTTCAAGGATGT CTTCGTGGACTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1307) | 0.50677669 |
| Alanine;3;7 | 7 | DAKSLTAAAATLVTFKD VFVDFTREEWKLLDTAQ QIVYRNVMLENYKNLVS LGYQLTKPDVILRLEKGE EP (SEQ ID NO: 744) | GATGCTAAGTCCCTCACGGCGGCCGCTGCCACCCTTGTCACCTTCAAGGACGT TTTCGTGGATTTTACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1308) | 0.50591148 |
| Lysine;2;5 | 5 | DAKSLKKWSRTLVTFKD VFVDFTREEWKLLDTAQ QIVYRNVMLENYKNLVS LGYQLTKPDVILRLEKGE EP (SEQ ID NO: 745) | GATGCCAAGTCCCTGAAGAAGTGGTCTCGGACCCTTGTGACTTTTAAGGATG TTTTCGTCGACTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1309) | 0.50454904 |
| Glycine;3;7 | 7 | DAKSLTAGGGTLVTFKD VFVDFTREEWKLLDTAQ QIVYRNVMLENYKNLVS LGYQLTKPDVILRLEKGE EP (SEQ ID NO: 746) | GATGCTAAGTCCCTGACGGCCGGGGGTGGCACCCTTGTTACCTTCAAGGACG TCTTTGTGGATTTCACTAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1310) | 0.49495873 |
| AsparticAcid; 3;0 | 0 | DDDSLTAWSRTLVTFKD VFVDFTREEWKLLDTAQ QIVYRNVMLENYKNLVS LGYQLTKPDVILRLEKGE EP (SEQ ID NO: 747) | GATGATGATTCTCTGACGGCCTGGTCCCGGACTCTTGTCACCTTTAAGGACGT TTTCGTGGACTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1311) | 0.48978651 |
| Glutamine;1; 32 | 32 | DAKSLTAWSRTLVTFKD VFVDFTREEWKLLDTQQ QIVYRNVMLENYKNLVS LGYQLTKPDVILRLEKGE EP (SEQ ID NO: 748) | GACGCCAAGTCCCTGACCGCTTGGTCTCGGACGCTTGTGACCTTTAAGGATG TTTTCGTCGATTTCACTAGGGAAGAATGGAAACTACTAGACACACAACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1312) | 0.48972298 |
| Tyrosine;2;69 | 69 | DAKSLTAWSRTLVTFKD VFVDFTREEWKLLDTAQ QIVYRNVMLENYKNLVS LGYQLTKPDVILRLEKGE YY (SEQ ID NO: 749) | GATGCTAAGTCCCTTACCGCCTGGTCTCGGACGCTGGTCACCTTCAAGGACGT GTTCGTTGATTTACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAT ACTAC (SEQ ID NO: 1313) | 0.48765802 |
| GlutamicAcid ;2;7 | 7 | DAKSLTAEERTLVTFKDV FVDFTREEWKLLDTAQQ IVYRNVMLENYKNLVSL GYQLTKPDVILRLEKGEE P (SEQ ID NO: 750) | GATGCTAAGTCCCTTACGGCCGAGGAGAGGACTCTGGTCACCTTCAAGGACG TGTTCGTTGATTTACCCGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1314) | 0.48459406 |
| Histidine;1;7 | 7 | DAKSLTAHSRTLVTFKDV FVDFTREEWKLLDTAQQ IVYRNVMLENYKNLVSL GYQLTKPDVILRLEKGEE P (SEQ ID NO: 751) | GATGCCAAGTCTCTGACGGCTCACTCCAGGACTCTTGTCACCTTCAAGGATGT GTTCGTTGACTTTACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1315) | 0.48125191 |
| Arginine;2;5 | 5 | DAKSLRRWSRTLVTFKD VFVDFTREEWKLLDTAQ QIVYRNVMLENYKNLVS LGYQLTKPDVILRLEKGE EP (SEQ ID NO: 752) | GATGCCAAGTCCCTTCGCCGGTGGTCTCGAACTCTGGTCACCTTTAAGGATGT GTTCGTTGACTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1316) | 0.4801544 |
| Serine;3;9 | 9 | DAKSLTAWSSSSVTFKD VFVDFTREEWKLLDTAQ QIVYRNVMLENYKNLVS LGYQLTKPDVILRLEKGE EP (SEQ ID NO: 753) | GATGCTAAGTCTCTTACTGCCTGGTCCAGCTCAAGTGTGACCTTTAAGGACGT TTTCGTCGATTTCACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1317) | 0.47920968 |
| Tryptophan;1 ;53 | 53 | DAKSLTAWSRTLVTFKD VFVDFTREEWKLLDTAQ QIVYRNVMLENYKNLVS LGWQLTKPDVILRLEKG EEP (SEQ ID NO: 754) | GATGCCAAGTCTCTTACCGCTTGGTCCCGGACTCTGGTCACGTTTAAGGACGT TTTCGTGGATTTCACCAGGGAAGAATGGAAACTACTAGACACAGCACAACAA ATAGTATACAGAAACGTAATGCTAGAAAACTACAAAAACCTAGTAAGCCTAG GATGGCAACTAACAAAACCAGACGTAATACTAAGACTAGAAAAAGGAGAAG AACCA (SEQ ID NO: 1318) | 0.47320791 |
| Serine;3;1 | 1 | DSSSLTAWSRTLVTFKD VFVDFTREEWKLLDTAQ QIVYRNVMLENYKNLVS LGYQLTKPDVILRLEKGE EP (SEQ ID NO: 755) | GATTCATCTTCCCTGACGGCCTGGAGTAGGACCCTTGTTACCTTCAAGGACGT CTTTGTGGATTTCACTCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1319) | 0.47295365 |
| Lysine;2;34 | 34 | DAKSLTAWSRTLVTFKD VFVDFTREEWKLLDTAQ KKVYRNVMLENYKNLVS LGYQLTKPDVILRLEKGE EP (SEQ ID NO: 756) | GATGCCAAGTCTCTGACCGCTTGGTCCCGGACTCTTGTGACGTTCAAGGACG TCTTTGTTGATTTCACCAGGGAAGAATGGAAACTACTAGACACAGCACAAAA AAAAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1320) | 0.47168537 |
| Lysine;3;7 | 7 | DAKSLTAKKKTLVTFKDV FVDFTREEWKLLDTAQQ IVYRNVMLENYKNLVSL GYQLTKPDVILRLEKGEE P (SEQ ID NO: 757) | GACGCTAAGTCCCTGACCGCCAAGAAGAAGACTCTTGTCACCTTCAAGGATG TTTTCGTGGATTTTACGAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1321) | 0.47075243 |
| Glycine;3;9 | 9 | DAKSLTAWSGGGVTFK DVFVDFTREEWKLLDTA QQIVYRNVMLENYKNLV SLGYQLTKPDVILRLEKG EEP (SEQ ID NO: 758) | GATGCTAAGTCCCTTACCGCCTGGTCTGGGGGTGGCGTGACCTTCAAGGATG TTTTCGTCGACTTTACTAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1322) | 0.46950237 |
| AsparticAcid; 3;9 | 9 | DAKSLTAWSDDDVTFK DVFVDFTREEWKLLDTA QQIVYRNVMLENYKNLV SLGYQLTKPDVILRLEKG EEP (SEQ ID NO: 759) | GATGCCAAGTCCCTTACTGCTTGGTCTGACGATGATGTGACCTTCAAGGATGT TTTCGTCGACTTTACCAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1323) | 0.4685333 |
| Threonine;2; 3 | 3 | DAKTTTAWSRTLVTFKD VFVDFTREEWKLLDTAQ QIVYRNVMLENYKNLVS LGYQLTKPDVILRLEKGE EP (SEQ ID NO: 760) | GATGCTAAGACTACCACGGCCTGGTCCAGGACTCTTGTTACCTTTAAGGATGT CTTCGTGGACTTCACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1324) | 0.46756614 |
| Glycine;3;8 | 8 | DAKSLTAWGGGLVTFK DVFVDFTREEWKLLDTA QQIVYRNVMLENYKNLV SLGYQLTKPDVILRLEKG EEP (SEQ ID NO: 761) | GATGCCAAGTCCCTTACTGCTTGGGGCGGTGGCTGGTGACCTTCAAGGACG TTTTTGTCGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1325) | 0.46569305 |
| GlutamicAcid ;1;10 | 10 | DAKSLTAWSRELVTFKD VFVDFTREEWKLLDTAQ QIVYRNVMLENYKNLVS LGYQLTKPDVILRLEKGE EP (SEQ ID NO: 762) | GACGCCAAGTCTCTGACTGCTTGGTCCCGGGAGCTTGTGACCTTTAAGGATG TCTTCGTTGATTTCACCAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1326) | 0.46430578 |
| AsparticAcid; 3;4 | 4 | DAKSDDDWSRTLVTFKD VFVDFTREEWKLLDTAQ QIVYRNVMLENYKNLVS LGYQLTKPDVILRLEKGE EP (SEQ ID NO: 763) | GACGCCAAGTCTGATGATGACTGGTCCCGGACCCTTGTCACCTTCAAGGATG TTTTCGTGGATTTTACTAGGGAAGAATGGAAACTACTAGACACAGCACAACA AATAGTATACAGAAACGTAATGCTAGAAAACTACAAAAACCTAGTAAGCCTA GGATACCAACTAACAAAACCAGACGTAATACTAAGACTAGAAAAAGGAGAA GAACCA (SEQ ID NO: 1327) | 0.4639914 |
| Serine;3;5 | 5 | DAKSLSSSSRTLVTFKDV FVDFTREEWKLLDTAQQ IVYRNVMLENYKNLVSL GYQLTKPDVILRLEKGEE P (SEQ ID NO: 764) | GACGCCAAGTCACTTAGCAGTTCCTCTAGGACTCTGGTGACCTTTAAGGATGT CTTCGTTGATTTCACCCGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1328) | 0.46366844 |
| Serine;3;7 | 7 | DAKSLTASSSTLVTFKDV FVDFTREEWKLLDTAQQ IVYRNVMLENYKNLVSL GYQLTKPDVILRLEKGEE P (SEQ ID NO: 765) | GACGCTAAGTCCCTTACGGCCTCTAGTTCAACCCTGGTGACCTTCAAGGATGT CTTCGTTGATTTACTAGGGAAGAATGGAAACTACTAGACACAGCACAACAA ATAGTATACAGAAACGTAATGCTAGAAAACTACAAAAACCTAGTAAGCCTAG GATACCAACTAACAAAACCAGACGTAATACTAAGACTAGAAAAAGGAGAAG AACCA (SEQ ID NO: 1329) | 0.45082917 |

**Table 4: Tiling Repressors**

| **Gene** | **Sequence** | **Avg Day 5 Score** |
|---|---|---|
| KRBOX1 | MMTAVSLTTRPQESVAFEDVAVYFTTKEWAIMVPAERALYRDVMLENYEAVAFVVPPTSKPALVSHLEQGKESCFTQPQG (SEQ ID NO: 766) | 7.8278029 |
| ZNF461 | MAHELVMFRDVAIDVSQEEWECLNPAQRNLYKEVMLENYSNLVSLGLSVSKPAVISSLEQGKEPWMVVREETGRWCPGTW (SEQ ID NO: 767) | 7.7007641 |
| ZNF875 | MSCSGAGGITAFVAFRDVAVYFTQEEWRLLSPAQRTLHREVMLETYNHLVSLEIPSSKPKLIAQLERGEAPWREERKCPL (SEQ ID NO: 768) | 7.41648619 |
| ZNF57 | MDSVVFEDVAVDFTLEEWALLDSAQRDLYRDVMLETFRNLASVDDGTQFKANGSVSLQDMYGQEKSKEQTIPNFTGNNSC (SEQ ID NO: 769) | 7.33652783 |
| CBX5 | NDIARGFERGLEPEKIIGATDSCGDLMFLMKWKDTDEADLVLAKEANVKCPQIVIAFYEERLTWHAYPEDAENKEKETAK (SEQ ID NO: 770) | 6.80089192 |
| CBX3 | SKKKRDAADKPRGFARGLDPERIIGATDSSGELMFLMKWKDSDEADLVLAKEANMKCPQIVIAFYEERLTWHSCPEDEAQ (SEQ ID NO: 771) | 6.7879261 |
| CBX1 | KKKKEESEKPRGFARGLEPERIIGATDSSGELMFLMKWKNSDEADLVPAKEANVKCPQVVISFYEERLTWHSYPSEDDDK (SEQ ID NO: 772) | 6.68230107 |
| CTCF | PVTVPVATTSVEELQGAYENEVSKEGLAESEPMICHTLPLPEGFQVVKVGANGEVETLEQGELPPQEDPSWQKDPDYQPP (SEQ ID NO: 773) | 6.25722327 |
| RYBP | PPSEANSIQSANATTKTSETNHTSRPRLKNVDRSTAQQLAVTVGNVTVIITDFKEKTRSSSTSSSTVTSSAGSEQQNQSS (SEQ ID NO: 774) | 6.18591064 |
| IRF2BP1 | MASVQASRRQWCYLCDLPKMPWAMVWDFSEAVCRGCVNFEGADRIELLIDAARQLKRSHVLPEGRSPGPPALKHPATKDL (SEQ ID NO: 775) | 5.6588422 |
| MGA | PHTSANLVMTPQGQLLTLKGPLFSGPVVAVSPDLLESDLKPQVAGSAVALPENDDLFMMPRIVNVTSLATEGGLVDMGGS (SEQ ID NO: 776) | 5.6156366 |
| CBX7 | ELFLQEPPAPDVLQAAGEWEPAAQPPEEEADADLAEGPPPWTPALPSSEVTVTDITANSITVTFREAQAAEGFFRDRSGK (SEQ ID NO: 777) | 5.00371758 |
| IKZF5 | TPSIGNSQPSTPAPALPVQDPQLLHHCQHCDMYFADNILYTIHMGCHGYENPFQCNICGCKCKNKYDFACHFARGQHNQH (SEQ ID NO: 778) | 4.98433253 |
| REST | IHSHEGSDLSDNMSEGSDDSGLHGARPVPQESSRKNAKEALAVKAAKGDFVCIFCDRSFRKGKDYSKHLNRHLVNVYYLE (SEQ ID NO: 779) | 4.52776703 |
| CBX4 | RSEAGEPPSSLQVKPETPASAAVAVAAAAAPTTTAEKPPAEAQDEPAESLSEFKPFFGNIIITDVTANCLTVTFKEYVTV (SEQ ID NO: 780) | 4.52247518 |
| KLF10 | APFKEEEKSPVSAPKLPKAQATSVIRHTADAQLCNHQTCPMKAASILNYQNNSFRRRTHLNVEAARKNIPCAAVSPNRSK (SEQ ID NO: 781) | 4.49441205 |
| SCMH1 | DASRLSGRDPSSWTVEDVMQFVREADPQLGPHADLFRKHEIDGKALLLLRSDMMMKYMGLKLGPALKLSYHIDRLKQGKF (SEQ ID NO: 782) | 4.38546257 |
| SCML2 | KQGFSKDPSTWSVDEVIQFMKHTDPQISGPLADLFRQHEIDGKALFLLKSDVMMKYMGLKLGPALKLCYYIEKLKEGKYS (SEQ ID NO: 783) | 4.3468225 |
| HIVEP3 | GSGSESG KERRTTSKEISVIQHTSSFEKSDSLEQPSG LEG EDKPLAQFPSPPPAPHG RSAHSLQPKL VRQPN IQVPEI LV (SEQ ID NO: 784) | 4.34368367 |
| HSF2 | SSAQKVQIKQETIESRLSELKSENESLWKEVSELRAKHAQQQQVIRKIVQFIVTLVQNNQLVSLKRKRPLLLNTNGAQKK (SEQ ID NO: 785) | 4.31444003 |
| MBD1 | TSPVLVPGCPSKAVDPGLPSVKQEPPDPEEDKEENKDDSASKLAPEEEAGGAGTPVITEIFSLGGTRFRDTAVWLPRSKD (SEQ ID NO: 786) | 4.25873478 |
| BAZ2A | GQDSEQPQAQLQPEAQLHAPAQPQPQLQLQLQSHKGFLEQEGSPLSLGQSQHDLSQSAFLSWLSQTQSHSSLLSSSVLTP (SEQ ID NO: 787) | 4.17482075 |
| CHD4 | EIILCDTCPRAYHMVCLDPDMEKAPEGKWSCPHCEKEGIQWEAKEDNSEGEEILEEVGGDLEEEDDHHMEFCRVCKDGGE (SEQ ID NO: 788) | 4.15410157 |
| ATF7IP | DSSGVIDLTMDDEESGASQDPKKLNHTPVSTMSSSQPVSRPLQPIQPAPPLQPSGVPTSGPSQTTIHLLPTAPTTVNVTH (SEQ ID NO: 789) | 4.13988404 |
| PCGF2 | MHRTTRIKITELNPHLMCALCGGYFIDATTIVECLHSFCKTCIVRYLETNKYCPMCDVQVHKTRPLLSIRSDKTLQDIVY (SEQ ID NO: 790) | 4.08140835 |
| HSF1 | KIRQDSVTKLLTDVQLMKGKQECMDSKLLAMKHENEALWREVASLRQKHAQQQKVVNKLIQFLISLVQSNRILGVKRKIP (SEQ ID NO: 791) | 3.89748786 |
| WIZ | LQQKLEEVRQPPPRVRPVPSLVPRPPQTSLVKFVGNIYTLKCRFCEVEFQGPLSIQEEWVRHLQRHILEMNFSKADPPPE (SEQ ID NO: 792) | 3.88045025 |
| CBX7 | MELSAIGEQVFAVESIRKKRVRKGKVEYLVKWKGWPPKYSTWEPEEHILDPRLVMAYEEKEERDRASGYRKRGPKPKRLL (SEQ ID NO: 793) | 3.86396312 |
| UHRF1 | CGGRQDPDKQLMCDECDMAFHIYCLDPPLSSVPSEDEWYCPECRNDASEVVLAGERLRESKKKAKMASATSSSQRDWGKG (SEQ ID NO: 794) | 3.85377779 |
| MGA | ALSEVPQLKQEISECLIASSFEDDSRVASPLDQNGSFNVVIKEEPLDDYDYELGECPEGVTVKQEETDEETDVYSNSDDD (SEQ ID NO: 795) | 3.82825762 |
| SIN3B | SAGHEKLPVHVEDALTYLDQVKIRFGSDPATYNGFLEIMKEFKSQSIDTPGVIRRVSQLFHEHPDLIVGFNAFLPLGYRI (SEQ ID NO: 796) | 3.67861396 |
| MECP2 | TSTQVMVIKRPGRKRKAEADPQAIPKKRGRKPGSVVAAAAAEAKKKAVKESSIRSVQETVLPIKKRKTRETVSIEVKEVV (SEQ ID NO: 797) | 3.54786112 |
| ATF7IP | MDSLEEPQKKVFKARKTMRVSDRQQLEAVYKVKEELLKTDVKLLNGNHENGDLDPTSPLENMDYIKDKEEVNGIEEICFD (SEQ ID NO: 798) | 3.46711815 |
| INSM2 | CHKKFRRQAYLRKHLSTHEAGSARALAPGFGSERGAPLAFACPLCGAHFPTADIREKHRLWHAVREELLLPALAGAPPET (SEQ ID NO: 799) | 3.41894392 |
| KDM5B | LLDSSNSSASASQAMNIKIEPEETTEARTHNLRRRMGCPTPKCENEKEMKSSIKQEPIERKDYIVENEKEKPKSRSKKAT (SEQ ID NO: 800) | 3.41515767 |
| TRIM24 | PVSGSSPFATQVGVIRCPVCSQECAERHIIDNFFVKDTTEVPSSTVEKSNQVCTSCEDNAEANGFCVECVEWLCKTCIRA (SEQ ID NO: 801) | 3.34747947 |
| MBD1 | MAEDWLDCPALGPGWKRREVFRKSGATCGRSDTYYQSPTGDRIRSKVELTRYLGPACDLTLFDFKQGILCYPAPKAHPVA (SEQ ID NO: 802) | 3.31964355 |
| SIN3A | PVQGQQQFQRLKVEDALSYLDQVKLQFGSQPQVYNDFLDIMKEFKSQSIDTPGVISRVSQLFKGHPDLIMGFNTFLPPGY (SEQ ID NO: 803) | 3.30578618 |
| DMD | RAKQPDLAPGLTTIGASPTQTVTLVTQPVVTKETAISKLEMPSSLMLEVPALADFNRAWTELTDWLSLLDQVIKSQRVMV (SEQ ID NO: 804) | 3.29433336 |
| ZNF827 | HMRCHQHFLRTEAKVKEEIPDPDVKGSPHLSDSACLGQQREGGGTELVGTMMTSNTPERTSQGGAGVSPLLVKEEPKEDN (SEQ ID NO: 805) | 3.26257067 |
| HIPK2 | QRQTIVIPDTPSPTVSVITISSDTDEEEEQKHAPTSTVSKQRKNVISCVTVHDSPYSDSSSNTSPYSVQQRAGHNNANAF (SEQ ID NO: 806) | 3.2515196 |
| MBD1 | SGDGTQRQRLKTLCKDCRAQRIAFNREQRMFKRVGCGECAACQVTEDCGACSTCLLQLPHDVASGLFCKCERRRCLRIVE (SEQ ID NO: 807) | 3.18634467 |
| TET2 | KMAEKAREKEEECEKYGPDYVPQKSHGKKVKREPAEPHETSEPTYLRFIKSLAERTMSVTTDSTVTTSPYAFTRVTGPYN (SEQ ID NO: 808) | 3.17106197 |
| CBX8 | GSGPPSSGGGLYRDMGAQGGRPSLIARIPVARILGDPEEESWSPSLTNLEKVVVTDVTSNFLTVTIKESNTDQGFFKEKR (SEQ ID NO: 809) | 3.14200153 |
| HES3 | GSTMDSAGLGQEAPALFRPCTPAVWAPAPAAGGPRSPPPLLLLPESLPGSSASVPPPQPASSRCAESPGLGLRVWRPWGS (SEQ ID NO: 810) | 3.13473999 |
| TRPS1 | EDGHAISTIKEEPKIDFRVYNLLTPDSKMGEPVSESVVKREKLEEKDGLKEKVWTESSSDDLRNVTWRGADILRGSPSYT (SEQ ID NO: 811) | 3.11964253 |
| TRIM24 | VGSRGSSGSSSKPAGADSTHKVPVVMLEPIRIKQENSGPPENYDFPVVIVKQESDEESRPQNANYPRSILTSLLLNSSQS (SEQ ID NO: 812 ) | 3.10950731 |
| TET1 | NEGDQPKTPENIPSKEPKDGSPVQPSLLSLMKDRRLTLEQVVAIEALTQLSEAPSENSSPSKSEKDEESEQRTASLLNSC (SEQ ID NO: 813) | 3.01568183 |
| BAZ2B | CRKGDNEELLLLCDGCDKGCHTYCHRPKITTIPDGDWFCPACIAKASGQTLKIKKLHVKGKKTNESKKGKKVTLTGDTED (SEQ ID NO: 814) | 2.99449946 |
| ATRX | SDEQKIKPVTENLVLSSHTGFCQSSGDEALSKSVPVTVDDDDDDNDPENRIAKKMLLEEIKANLSSDEDGSSDDEPEEGK (SEQ ID NO: 815) | 2.99299407 |
| DMD | QQHKVLQEDLEQEQVRVNSLTHMVVVVDESSGDHATAALEEQLKVLGDRWANICRWTEDRWVLLQDILLKWQRLTEEQCL (SEQ ID NO: 816) | 2.95216006 |
| TRIM24 | QVCTSCEDNAEANGFCVECVEWLCKTCIRAHQRVKFTKDHTVRQKEEVSPEAVGVTSQRPVFCPFHKKEQLKLYCETCDK (SEQ ID NO: 817) | 2.94621718 |
| AHRR | LDVSIKMEKDSGCEGAADGCVPSQVWLGASDRSHPATFPTRMHLKTEPDSRQQVYISHLGHGVRGAQPHGRATAGRSREL (SEQ ID NO: 818) | 2.91519283 |
| CBX4 | MELPAVGEHVFAVESIEKKRIRKGRVEYLVKWRGWSPKYNTWEPEENILDPRLLIAFQNRERQEQLMGYRKRGPKPKPLV (SEQ ID NO: 819) | 2.8451435 |
| AUTS2 | GSLQGAFQPKTSNPIDVAARPGTVPHTLLQKDPRLTDPFRPMLRKPGKWCAMHVHIAWQIYHHQQKVKKQMQSDPHKLD F (SEQ ID NO: 820) | 2.80116399 |
| PLAG1 | LKVKTEPVDFLDPFTCNVSVPIKDELLPVMSLPSSELLSKPFTNTLQLNLYNTPFQSMQSSGSAHQMITTLPLGMTCPID (SEQ ID NO: 821) | 2.78382024 |
| ZNF827 | LVKEEPKEDNGLPTSFTLNAADRPANHTKLKDPSEYVANSASALFSQDISVKMASDFLMKLSAANQKEPMNLNFKVKEEP (SEQ ID NO: 822) | 2.76221951 |
| TET3 | EPQNHFSSFKYSGNAVVESYSVLGNCRPSDPYSMNSVYSYHSYYAQPSLTSVNGFHSKYALPSFSYYGFPSSNPVFPSQF (SEQ ID NO: 823) | 2.67090027 |
| RCOR3 | IPEFDPGATKYTDKDNGGMLVWSPYHSIPDAKLDEYIAIAKEKHGYNVEQALGMLFWHKHNIEKSLADLPNFTPFPDEWT (SEQ ID NO: 824) | 2.6595258 |
| DNMT3B | KNPVSFHPLFEGGLCQTCRDRFLELFYMYDDDGYQSYCTVCCEGRELLLCSNTSCCRCFCVECLEVLVGTGTAAEAKLQE (SEQ ID NO: 825) | 2.65664419 |
| IKZF2 | DTTKAPKGSLKDIYKVFNGEGEQIRAFKCEHCRVLFLDHVMYTIHMGCHGYRDPLECNICGYRSQDRYEFSSHIVRGEHT (SEQ ID NO: 826) | 2.65607702 |
| DNMT3B | PAVRTRNNNSVSSRERHRPSPRSTRGRQGRNHVDESPVEFPATRSLRRRATASAGTPWPSPPSSYLTIDLTDDTEDTHGT (SEQ ID NO: 827) | 2.64875126 |
| ZNF827 | AESYKETQMVKIKEEPMEVDIQDSHVSISPSRNVGYSTLIGREKTEPLQKMPEGRVPPERNLFSQDISVKMASELLFQLS (SEQ ID NO: 828) | 2.6349517 |
| ATF7IP | NQKLQKVIQWLLEEKLCALQCAVFDKTLAELKTRVEKIECNKRHKTVLTELQAKIARLTKRFEAAKEDLKKRHEHPPNPP (SEQ ID NO: 829) | 2.62333283 |
| KDM5C | VVVKEELGGDVKVESTSPKTFLESKEELSHSPEPCTKMTMRLRRNHSNAQFIESYVCRMCSRGDEDDKLLLCDGCDDNYH (SEQ ID NO: 830) | 2.62226329 |
| SUV39H1 | MAENLKGCSVCCKSSWNQLQDLCRLAKLSCPALGISKRNLYDFEVEYLCDYKKIREQEYYLVKWRGYPDSESTWEPRQNL (SEQ ID NO: 831) | 2.61048387 |
| HES1 | APCKLGSQAGEAAKVFGGFQVVPAPDGQFAFLIPNGAFAHSGPVIPVYTSNSGTSVGPNAVSPSSGPSLTADSMWRPWRN (SEQ ID NO: 832) | 2.54719284 |
| HIVEP3 | VKKEDSKEQPDLPSLAPPSSLPLSETSSRPAKSQEGTDSKKVLQFPSLHTTTNVSWCYLNYIKPNHIQHADRRSSVYAGW (SEQ ID NO: 833) | 2.54212172 |
| ZNF446 | QLSCSVKEEPNVDGQEVAPSSPPLAAQSPEGNHGHQEPASTSFHPPRIQEEWGLLDRSQKELYWDAMLEKYGTVVSLGLP (SEQ ID NO: 834) | 2.53378337 |
| HEY2 | TSSVIRLNSPTTTSQIMARKKRRGIIEKRRRDRINNSLSELRRLVPTAFEKQGSAKLEKAEILQMTVDHLKMLQATGGKG (SEQ ID NO: 835) | 2.52262483 |
| PRDM11 | TVVKTEVCSPLRDQEYGQPCSRRPDSSAMEVEPKKLKGKRDLIVPKSFQQVDFWFCESCQEYFVDECPNHGPPVFVSDTP (SEQ ID NO: 836) | 2.50622782 |
| PHF19 | EGQYVLCRWTDGLYYLGKIKRVSSSKQSCLVTFEDNSKYWVLWKDIQHAGVPGEEPKCNICLGKTSGPLNEILICGKCGL (SEQ ID NO: 837) | 2.50329194 |
| CBX3 | KVEEAEPEEFVVEKVLDRRVVNGKVEYFLKWKGFTDADNTWEPEENLDCPELIEAFLNSQKAGKEKDGTKRKSLSDSESD (SEQ ID NO: 838) | 2.46766682 |
| FBRS | NPELPPRLGPVPSGLSQKGTQIPDHFRPPLRKPGKWCAMHVRVAYMILRHQEKMKGDSHKLDFRNDLLPCLPGPYGALPP (SEQ ID NO: 839) | 2.4558407 |
| CIC | FPSKVCLQLKIREVRQKIMQAATPTEQPPGAEAPLPVPPPTGTAAAPAPTPSPAGGPDPTSPSSDSGTAQAAPPLPPPPE (SEQ ID NO: 840) | 2.42893202 |
| PCGF6 | EEEEEEEEEDMSHFSLRLEGGRQDSEDEEERLINLSELTPYILCSICKGYLIDATTITECLHTFCKSCIVRHFYYSNRCP (SEQ ID NO: 841) | 2.4085226 |
| MNT | DDKKTSNLSVLRTALRYIQSLKRKEKEYEHEMERLAREKIATQQRLAELKHELSQWMDVLEIDRVLRQTGQPEDDQASTS (SEQ ID NO: 842) | 2.39586222 |
| HSF1 | LEHVHGSGPYSAPSPAYSSSSLYAPDAVASSGPIISDITELAPASPMASPGGSIDERPLSSSPLVRVKEEPPSPPQSPRV (SEQ ID NO: 843) | 2.39135328 |
| SERTAD2 | ITTSTGFLTDLTLDDILFADIDTSMYDFDPCTSSSGTASKMAPVSADDLLKTLAPYSSQPVTPSQPFKMDLTELDHIMEV (SEQ ID NO: 844) | 2.35712893 |
| BMI1 | MHRTTRIKITELNPHLMCVLCGGYFIDATTIIECLHSFCKTCIVRYLETSKYCPICDVQVHKTRPLLNIRSDKTLQDIVY (SEQ ID NO: 845) | 2.34528179 |
| TET3 | RPRLPGPLPPGEAGLPAPSTRPLLSSEVPQISPQEGLPLSQSALSIAKEKNISLQTAIAIEALTQLSSALPQPSHSTPQA (SEQ ID NO: 846) | 2.33879669 |
| USP7 | MYDPKTRSLNYCGHIYTPISCKIRDLLPVMCDRAGFIQDTSLILYEEVKPNLTERIQDYDVSLDKALDELMDGDIIVFQK (SEQ ID NO: 847) | 2.32632143 |
| TRIM28 | SQPPVFKVFPGSTTEDYNLIVIERGAAAAATGQPGTAPAGTPGAPPLAGMAIVKEEETEAAIGAPPTATEGPETKPVLMA (SEQ ID NO: 848) | 2.30375349 |
| L3MBTL3 | TVAGIPASKVSKWSTDEVSEFIQSLPGCEEHGKVFKDEQIDGEAFLLMTQTDIVKIMSIKLGPALKIFNSILMFKAAEKN (SEQ ID NO: 849) | 2.28373726 |
| IKZF4 | PQEGLLRGTPGPSKEVLRVVGESGEPVKAFKCEHCRILFLDHVMFTIHMGCHGFRDPFECNICGYHSQDRYEFSSHIVRG (SEQ ID NO: 850) | 2.28172595 |
| KDM5B | CGSGNDEDRLLLCDGCDDSYHTFCLIPPLHDVPKGDWRCPKCLAQECSKPQEAFGFEQAARDYTLRTFGEMADAFKSDYF (SEQ ID NO: 851) | 2.26045013 |
| GATAD2A | QVVMPPLVRGAQQIHSIRQHSSTGPPPLLLAPRASVPSVQIQGQRIIQQGLIRVANVPNTSLLVNIPQPTPASLKGTTAT (SEQ ID NO: 852) | 2.24549867 |
| BRMS1 | QERIQRLEEDRQSLDLSSEWWDDKLHARGSSRSWDSLPPSKRKKAPLVSGPYIVYMLQEIDILEDWTAIKKARAAVSPQK (SEQ ID NO: 853) | 2.23400084 |
| DNMT1 | PLSKPRTPRRSKSDGEAKPEPSPSPRITRKSTRQTTITSHFAKGPAKRKPQEESERAKSDESIKEEDKDQDEKRRRVTSR (SEQ ID NO: 854) | 2.18589467 |
| ZNF366 | MG H M H LHSDSKPFKCLYCPSKFTLKG N LTRH M KVKHGVM ERG LHSQG LG RG RIALAQTAGVLRSLEQEEPFDLSQKRRAK (SEQ ID NO: 855) | 2.17275454 |
| NAB2 | GELQLYRVLQRANLLSYYETFIQQGGDDVQQLCEAGEEEFLEIMALVGMATKPLHVRRLQKALREWATNPGLFSQPVPAV (SEQ ID NO: 856) | 2.17138822 |
| KDM1A | QLQEKHVKDEQIEHWKKIVKTQEELKELLNKMVNLKEKIKELHQQYKEASEVKPPRDITAEFLVKSKHRDLTALCKEYDE(SEQ ID NO: 857) | 2.13679618 |
| TGS1 | LQSKKDTETENPPVENTLSPKLEITEKWEKYWNEYGGGLLWQSWQEKHPGQALSSEPWNFPDTKEEWEQHYSQLYWYVLE (SEQ ID NO: 858) | 2.11925487 |
| PRDM11 | VSKEPGQLEDEEEEPSSFKADSPAEASLASDPHELPTTSFCPNCIRLKKKVRELQAELDMLKSGKLPEPPVLPPQVLELP (SEQ ID NO: 859) | 2.07885469 |
| HDAC6 | PGEENLLGEAAGGQDMADSMLMQGSRGLTDQAIFYAVTPLPWCPHLVAVCPIPAAGLDVTQPCGDCGTIQENWVCLSCYQ (SEQ ID NO: 860) | 2.03482535 |
| CBX1 | MGKKQNKKKVEEVLEEEEEEYVVEKVLDRRVVKGKVEYLLKWKGFSDEDNTWEPEENLDCPDLIAEFLQSQKTAHETDKS (SEQ ID NO: 861) | 2.01737869 |
| TRIM8 | VEEKVNQLKEEVRLQYEKLHQLLDEDLRQTVEVLDKAQAKFCSENAAQALHLGERMQEAKKLLGSLQLLFDKTEDVSFMK (SEQ ID NO: 862) | 1.99485782 |
| MDFI | QTHPSLASQGSKKSKSSSKSTTSQIPLQAQEDCCVHCILSCLFCEFLTLCNIVLDCATCGSCSSEDSCLCCCCCGSGECA (SEQ ID NO: 863) | 1.98953136 |
| NCOR1 | HRGSTAGEVYRSHLPTHLDPAMPFHRALDPAAAAYLFQRQLSPTPGYPSQYQLYAMENTRQTILNDYITSQQMQVNLRPD (SEQ ID NO: 864) | 1.98515002 |
| HIVEP3 | KKERKPQKPGKYICQYCSRPCAKPSVLQKHIRSHTGERPYPCGPCGFSFKTKSNLYKHRKSHAHRIKAGLASGMGGEMYP (SEQ ID NO: 865) | 1.97820999 |
| IKZF1 | AASENSQDALRVVSTSGEQMKVYKCEHCRVLFLDHVMYTIHMGCHGFRDPFECNMCGYHSQDRYEFSSHITRGEHRFHMS (SEQ ID NO: 866) | 1.97447058 |
| CDK2AP1 | TSSQYRQLLSDYGPPSLGYTQGTGNSQVPQSKYAELLAIIEELGKEIRPTYAGSKSAMERLKRGIIHARGLVRECLAETE (SEQ ID NO: 867) | 1.96228244 |
| ERF | SEGESEEVEVTDISDEDEEDGEVFKTPRAPPAPPKPEPGEAPGASQCMPLKLRFKRRWSEDCRLEGGGGPAGGFEDEGED (SEQ ID NO: 868) | 1.94839175 |
| KDM5C | IPKGVWRCPKCVMAECKRPPEAFGFEQATREYTLQSFGEMADSFKADYFNMPVHMVPTELVEKEFWRLVNSIEEDVTVEY (SEQ ID NO: 869) | 1.94183352 |
| ZNF446 | LLGWITAHVLKQEVLPAAQKTEEPLGSPHPSGTVESPGEGPQDTRIEGSVQLSCSVKEEPNVDGQEVAPSSPPLAAQSPE (SEQ ID NO: 870) | 1.92348688 |
| CBFA2T3 | DPRELRERHRPLVVPGSRQEEVIDHKLTEREWAEEWKHLNNLLNCIMDMVEKTRRSLTVLRRCQEADREELNHWARRYSD (SEQ ID NO: 871) | 1.92105376 |
| USP7 | SDRREDYYDIQLSIKGKKNIFESFVDYVAVEQLDGDNKYDAGEHGLQEAEKGVKFLTLPPVLHLQLMRFMYDPQTDQNIK (SEQ ID NO: 872) | 1.90807263 |
| SETDB1 | EIAELQQA VVEE LG ISM EELRH FI DEELEKM DCVQQRKKQLAELETWVIQKESEV AHVDQLFDDASRA VTNCESL VKDFY (SEQ ID NO: 873) | 1.89374023 |
| PHF1 | FPTDIPKSAPHSMTASSSSVSSPSPGLPRRSAPPSPLCRSLSPGTGGGVRGGVGYLSRGDPVRVLARRVRPDGSVQYLVE (SEQ ID NO: 874) | 1.88511381 |
| IKZF3 | LKPPPICPRDSVKVINKEGEVMDVYRCDHCRVLFLDYVMFTIHMGCHGFRDPFECNMCGYRSHDRYEFSSHIARGEHRAL (SEQ ID NO: 875) | 1.87983916 |
| HDAC4 | EHQALLDEPYLDRLPGQKEAHAQAGVQVKQEPIESDEEEAEPPREVEPGQRQPSEQELLFRQQALLLEQQRIHQLRNYQA (SEQ ID NO: 876) | 1.86554151 |
| DNMT3L | AEPSMDVILVGSSELSSSVSPGTGRDLIAYEVKANQRNIEDICICCGSLQVHTQHPLFEGGICAPCKDKFLDALFLYDDD (SEQ ID NO: 877) | 1.85705086 |
| HDAC9 | SSACVDDTLGQVGAVKVKEEPVDSDEDAQIQEMESGEQAAFMQQPFLEPTHTRALSVRQAPLAAVGMDGLEKHRLVSRTH (SEQ ID NO: 878) | 1.85605728 |
| ZSCAN22 | VKVEEEEEASLSQGGESSHDHIAHSEAARLRFRHFRYEEASGPHEALAHLRALCCQWLQPEAHSKEQILELLVLEQFLGA (SEQ ID NO: 879) | 1.83277386 |
| E2F6 | QDIHSIQAFHEQIVIAVKAPAETRLDVPAPREDSITVHIRSTNGPIDVYLCEVEQGQTSNKRSEGVGTSSSESTHPEGPE (SEQ ID NO: 880) | 1.82276829 |
| KDM5A | AAQNCQRPCKDKVDWVQCDGGCDEWFHQVCVGVSPEMAENEDYICINCAKKQGPVSPGPAPPPSFIMSYKLPMEDLKETS (SEQ ID NO: 881) | 1.78690918 |
| RBBP7 | KEGKIVDAKAIFTGHSAVVEDVAWHLLHESLFGSVADDQKLMIWDTRSNTTSKPSHLVDAHTAEVNCLSFNPYSEFILAT (SEQ ID NO: 882) | 1.77403489 |
| ATRX | EREREKLREVIEIEDASPTKCPITTKLVLDEDEETKEPLVQVHRNMVIKLKPHQVDGVQFMWDCCCESVKKTKKSPGSGC (SEQ ID NO: 883) | 1.77379044 |
| ZNF446 | EPETARLRFRGFCYQEVAGPREALARLRELCCQWLQPEAHSKEQMLEMLVLEQFLGTLPPEIQAWVRGQRPGSPEEAAAL (SEQ ID NO: 884) | 1.7514371 |
| GATAD2B | PVVQNAASIVQPSPAHVGQQGLSKLPSRPGAQGVEPQNLRTLQGHSVIRSATNTTLPHMLMSQRVIAPNPAQLQGQRGPP (SEQ ID NO: 885) | 1.71132166 |
| TET2 | TESCHSQMHRPIKVEPGCKPHACMHTAPPENKTWKKVTKQENPPASCDNVQQKSIIETMEQHLKQFHAKSLFDHKALTLK (SEQ ID NO: 886) | 1.67625849 |
| NCOR2 | VVPKEEKEEETAAAPPVEEGEEQKPPAAEELAVDTGKAEEPVKSECTEEAEEGPAKGKDAEAAEATAEGALKAEKKEGGS (SEQ ID NO: 887) | 1.65967202 |
| BCOR | PPMKSLSSTSAGGKKQAQPSCAPASRPPAKQQKIKENQKTDVLCADEEEDCQAASLLQKYTDNSEKPSGKRLCKTKHLIP (SEQ ID NO: 889) | 1.65199942 |
| RBL2 | TSRDSSPVMRSSSTLPVPQPSSAPPTPTRLTGANSDMEEEERGDLIQFYNNIYIKQIKTFAMKYSQANMDAPPLSPYPFV (SEQ ID NO: 890) | 1.65141032 |
| KDM5D | IPRGIWRCPKCILAECKQPPEAFGFEQATQEYSLQSFGEMADSFKSDYFNMPVHMVPTELVEKEFWRLVSSIEEDVTVEY (SEQ ID NO: 891) | 1.59348979 |
| TRIM8 | STQHLVALPGGAQPVHSSPVFPPSQYPNGSAAQQPMLPQYGGRKILVCSVDNCYCSSVANHGGHQPYPRSGHFPWTVPSQ (SEQ ID NO: 892) | 1.56673764 |
| KLF3 | ILPVIQPVVVQPVPFMYTSHLQQPLMVSLSEEMENSSSSMQVPVIESYEKPISQKKIKIEPGIEPQRTDYYPEEMSPPLM (SEQ ID NO: 893) | 1.56352551 |
| MTA2 | YGGLKTPTQLEGATRGTTEPHSRGHLSRPEAQSLSPYTTSANRAKLLAKNRQTFLLQTTKLTRLARRMCRDLLQPRRAAR (SEQ ID NO: 894) | 1.53904229 |
| PHF1 | MAQPPRLSRSGASSLWDPASPAPTSGPRPRLWEGQDVLARWTDGLLYLGTIKKVDSAREVCLVQFEDDSQFLVLWKDISP (SEQ ID NO: 895) | 1.53480472 |
| RCOR1 | AKLARRSQERDNLGMLVWSPNQNLSEAKLDEYIAIAKEKHGYNMEQALGMLFWHKHNIEKSLADLPNFTPFPDEWTVEDK (SEQ ID NO: 896) | 1.39637997 |

### References

Al Chiblak, M., Steinbeck, F., Thiesen, H.-J., and Lorenz, P. (2019). DUF3669, a "domain of unknown function" within ZNF746 and ZNF777, oligomerizes and contributes to transcriptional repression. BMC Mol Cell Biol 20, 60.
Amabile, A., Migliara, A., Capasso, P., Biffi, M., Cittaro, D., Naldini, L., and Lombardo, A. (2016). Inheritable Silencing of Endogenous Genes by Hit-and-Run Targeted Epigenetic Editing. Cell 167, 219-232.e14.
Arnold, C.D., Nemčko, F., Woodfin, A.R., Wienerroither, S., Vlasova, A., Schleiffer, A., Pagani, M., Rath, M., and Stark, A. (2018). A high-throughput method to identify transactivation domains within transcription factor sequences. EMBO J. e98896.
Ashkenazy, H., Erez, E., Martz, E., Pupko, T., and Ben-Tal, N. (2010). ConSurf 2010: calculating evolutionary conservation in sequence and structure of proteins and nucleic acids. Nucleic Acids Res. 38, W529-W533.
Bakan, A., Meireles, L.M., and Bahar, I. (2011). ProDy: protein dynamics inferred from theory and experiments. Bioinformatics 27, 1575-1577.
Ballas, N., Battaglioli, E., Atouf, F., Andres, M.E., Chenoweth, J., Anderson, M.E., Burger, C., Moniwa, M., Davie, J.R., Bowers, W.J., et al. (2001). Regulation of neuronal traits by a novel transcriptional complex. Neuron 31, 353-365.
Berezin, C., Glaser, F., Rosenberg, J., Paz, I., Pupko, T., Fariselli, P., Casadio, R., and Ben-Tal, N. (2004). ConSeq: the identification of functionally and structurally important residues in protein sequences. Bioinformatics 20, 1322-1324.
Bersaglieri, C., and Santoro, R. (2019). Genome Organization in and around the Nucleolus. Cells 8.
Bintu, L., Yong, J., Antebi, Y.E., McCue, K., Kazuki, Y., Uno, N., Oshimura, M., and Elowitz, M.B. (2016). Dynamics of epigenetic regulation at the single-cell level. Science 351, 720-724.
Birbach, A., Bailey, S.T., Ghosh, S., and Schmid, J.A. (2004). Cytosolic, nuclear and nucleolar localization signals determine subcellular distribution and activity of the NF-kappaB inducing kinase NIK. J. Cell Sci. 117, 3615-3624.
Birtle, Z., and Ponting, C.P. (2006). Meisetz and the birth of the KRAB motif. Bioinformatics 22, 2841-2845.
Blackledge, N.P., Farcas, A.M., Kondo, T., King, H.W., McGouran, J.F., Hanssen, L.L.P., Ito, S., Cooper, S., Kondo, K., Koseki, Y., et al. (2014). Variant PRC1 complex-dependent H2A ubiquitylation drives PRC2 recruitment and polycomb domain formation. Cell 157, 1445-1459.
Chang, Y., Sun, L., Kokura, K., Horton, J.R., Fukuda, M., Espejo, A., Izumi, V., Koomen, J.M., Bedford, M.T., Zhang, X., et al. (2011). MPP8 mediates the interactions between DNA methyltransferase Dnmt3a and H3K9 methyltransferase GLP/G9a. Nat. Commun. 2, 533.
Cheng, C.-T., Kuo, C.-Y., and Ann, D.K. (2014). KAPtain in charge of multiple missions: Emerging roles of KAP1. World J. Biol. Chem. 5, 308-320.
Chevron, M.P., Girard, F., Claustres, M., and Demaille, J. (1994). Expression and subcellular localization of dystrophin in skeletal, cardiac and smooth muscles during the human development. Neuromuscul. Disord. 4, 419-432.
Childs, K.S., and Goodbourn, S. (2003). Identification of novel co-repressor molecules for Interferon Regulatory Factor-2. Nucleic Acids Res. 31, 3016-3026.
Chittock, E.C., Latwiel, S., Miller, T.C.R., and Miller, C.W. (2017). Molecular architecture of polycomb repressive complexes. Biochem. Soc. Trans. 45, 193-205.
Chupreta, S., Holmstrom, S., Subramanian, L., and Iñiguez-Lluhí, J.A. (2005). A small conserved surface in SUMO is the critical structural determinant of its transcriptional inhibitory properties. Mol. Cell. Biol. 25, 4272-4282.
Copley, R.R. (2005). The EH1 motif in metazoan transcription factors. BMC Genomics 6, 169.
Corsetti, M.T., Levi, G., Lancia, F., Sanseverino, L., Ferrini, S., Boncinelli, E., and Corte, G. (1995). Nucleolar localisation of three Hox homeoproteins. J. Cell Sci. 108 (Pt 1), 187-193.
Dorris, D.R., and Struhl, K. (2000). Artificial recruitment of TFIID, but not RNA polymerase II holoenzyme, activates transcription in mammalian cells. Mol. Cell. Biol. 20, 4350-4358.
Drueppel, L., Pfleiderer, K., Schmidt, A., Hillen, W., and Berens, C. (2004). A short autonomous repression motif is located within the N-terminal domain of CTCF. FEBS Lett. 572, 154-158.
Duboule, D., and Morata, G. (1994). Colinearity and functional hierarchy among genes of the homeotic complexes. Trends in Genetics 10, 358-364.
El-Gebali, S., Mistry, J., Bateman, A., Eddy, S.R., Luciani, A., Potter, S.C., Qureshi, M., Richardson, L.J., Salazar, G.A., Smart, A., et al. (2019). The Pfam protein families database in 2019. Nucleic Acids Res. 47, D427-D432.
Elliott, K., Sakamuro, D., Basu, A., Du, W., Wunner, W., Staller, P., Gaubatz, S., Zhang, H., Prochownik, E., Eilers, M., et al. (1999). Bin1 functionally interacts with Myc and inhibits cell proliferation via multiple mechanisms. Oncogene 18, 3564-3573.
ENCODE Project Consortium, Moore, J.E., Purcaro, M.J., Pratt, H.E., Epstein, C.B., Shoresh, N., Adrian, J., Kawli, T., Davis, C.A., Dobin, A., et al. (2020). Expanded encyclopaedias of DNA elements in the human and mouse genomes. Nature 583, 699-710.
Erijman, A., Kozlowski, L., Sohrabi-Jahromi, S., Fishburn, J., Warfield, L., Schreiber, J., Noble, W.S., Söding, J., and Hahn, S. (2020). A High-Throughput Screen for Transcription Activation Domains Reveals Their Sequence Features and Permits Prediction by Deep Learning. Mol. Cell 78, 890-902.e6.
Feng, J., Liu, T., Qin, B., Zhang, Y., and Liu, X.S. (2012). Identifying ChIP-seq enrichment using MACS. Nat. Protoc. 7, 1728-1740.
Fowler, D.M., and Fields, S. (2014). Deep mutational scanning: a new style of protein science. Nat. Methods 11, 801-807.
Frey, F., Sheahan, T., Finkl, K., Stoehr, G., Mann, M., Benda, C., and Miiller, J. (2016). Molecular basis of PRC1 targeting to Polycomb response elements by PhoRC. Genes Dev. 30, 1116-1127.
Garcia, E., Marcos-Gutiérrez, C., del Mar Lorente, M., Moreno, J.C., and Vidal, M. (1999). RYBP, a new repressor protein that interacts with components of the mammalian Polycomb complex, and with the transcription factor YY1. EMBO J. 18, 3404-3418.
Gaudreau, L., Keaveney, M., Nevado, J., Zaman, Z., Bryant, G.O., Struhl, K., and Ptashne, M. (1999). Transcriptional activation by artificial recruitment in yeast is influenced by promoter architecture and downstream sequences. Proc. Natl. Acad. Sci. U. S. A. 96, 2668-2673.
Gilbert, S.F. (1971). Developmental Biology (Sinauer Associates).
Gilbert, L.A., Horlbeck, M.A., Adamson, B., Villalta, J.E., Chen, Y., Whitehead, E.H., Guimaraes, C., Panning, B., Ploegh, H.L., Bassik, M.C., et al. (2014). Genome-Scale CRISPR-Mediated Control of Gene Repression and Activation. Cell 159, 647-661.
Guetg, C., Lienemann, P., Sirri, V., Grummt, I., Hernandez-Verdun, D., Hottiger, M.O., Fussenegger, M., and Santoro, R. (2010). The NoRC complex mediates the heterochromatin formation and stability of silent rRNA genes and centromeric repeats. EMBO J. 29, 2135-2146.
Haney, M.S., Bohlen, C.J., Morgens, D.W., Ousey, J.A., Barkal, A.A., Tsui, C.K., Ego, B.K., Levin, R., Kamber, R.A., Collins, H., et al. (2018). Identification of phagocytosis regulators using magnetic genome-wide CRISPR screens. Nat. Genet. 50, 1716-1727.
Helleboid, P.-Y., Heusel, M., Duc, J., Piot, C., Thorball, C.W., Coluccio, A., Pontis, J., Imbeault, M., Turelli, P., Aebersold, R., et al. (2019). The interactome of KRAB zinc finger proteins reveals the evolutionary history of their functional diversification. EMBO J. 38, e101220.
Heredia, J.D., Park, J., Brubaker, R.J., Szymanski, S.K., Gill, K.S., and Procko, E. (2018). Mapping Interaction Sites on Human Chemokine Receptors by Deep Mutational Scanning. J. Immunol. 200, 3825-3839.
Holland, P.W.H., Booth, H.A.F., and Bruford, E.A. (2007). Classification and nomenclature of all human homeobox genes. BMC Biol. 5, 47.
Hueber, S.D., Weiller, G.F., Djordjevic, M.A., and Frickey, T. (2010). Improving Hox protein classification across the major model organisms. PLoS One 5, e10820.
Hyun, K., Jeon, J., Park, K., and Kim, J. (2017). Writing, erasing and reading histone lysine methylations. Exp. Mol. Med. 49, e324.
Imbeault, M., Helleboid, P.-Y., and Trono, D. (2017). KRAB zinc-finger proteins contribute to the evolution of gene regulatory networks. Nature 543, 550-554.
Johnson, M., Zaretskaya, I., Raytselis, Y., Merezhuk, Y., McGinnis, S., and Madden, T.L. (2008). NCBI BLAST: a better web interface. Nucleic Acids Res. 36, W5-W9.
Jolma, A., Yan, J., Whitington, T., Toivonen, J., Nitta, K.R., Rastas, P., Morgunova, E., Enge, M., Taipale, M., Wei, G., et al. (2013). DNA-binding specificities of human transcription factors. Cell 152, 327-339.
Keung, A.J., Bashor, C.J., Kiriakov, S., Collins, J.J., and Khalil, A.S. (2014). Using targeted chromatin regulators to engineer combinatorial and spatial transcriptional regulation. Cell 158, 110-120.
Kim, G.-D., Ni, J., Kelesoglu, N., Roberts, R.J., and Pradhan, S. (2002). Co-operation and communication between the human maintenance and de novo DNA (cytosine-5) methyltransferases. EMBO J. 21, 4183-4195.
Kinsella, R.J., Kähäri, A., Haider, S., Zamora, J., Proctor, G., Spudich, G., Almeida-King, J., Staines, D., Derwent, P., Kerhornou, A., et al. (2011). Ensembl BioMarts: a hub for data retrieval across taxonomic space. Database 2011, bar030.
Konermann, S., Brigham, M.D., Trevino, A.E., Joung, J., Abudayyeh, O.O., Barcena, C., Hsu, P.D., Habib, N., Gootenberg, J.S., Nishimasu, H., et al. (2014). Genome-scale transcriptional activation by an engineered CRISPR-Cas9 complex. Nature 517, 583-588.
Kotler, E., Shani, O., Goldfeld, G., Lotan-Pompan, M., Tarcic, O., Gershoni, A., Hopf, T.A., Marks, D.S., Oren, M., and Segal, E. (2018). A Systematic p53 Mutation Library Links Differential Functional Impact to Cancer Mutation Pattern and Evolutionary Conservation. Mol. Cell 71, 873.
Lambert, S.A, Jolma, A., Campitelli, L.F., Das, P.K., Yin, Y., Albu, M., Chen, X., Taipale, J., Hughes, T.R., and Weirauch, M.T. (2018). The Human Transcription Factors. Cell 175, 598-599.
Langmead, B., Trapnell, C., Pop, M., and Salzberg, S.L. (2009). Ultrafast and memory-efficient alignment of short DNA sequences to the human genome. Genome Biol. 10, R25.
Lechner, M.S., Begg, G.E., Speicher, D.W., and Rauscher, F.J. (2000). Molecular Determinants for Targeting Heterochromatin Protein 1-Mediated Gene Silencing: Direct Chromoshadow Domain-KAP-1 Corepressor Interaction Is Essential. Mol. Cell. Biol. 20, 6449-6465.
Li, Q., Wang, X., Lu, Z., Zhang, B., Guan, Z., Liu, Z., Zhong, Q., Gu, L., Zhou, J., Zhu, B., et al. (2010). Polycomb CBX7 directly controls trimethylation of histone H3 at lysine 9 at the p16 locus. PLoS One 5, e13732.
Liu, J., Perumal, N.B., Oldfield, C.J., Su, E.W., Uversky, V.N., and Dunker, A.K. (2006). Intrinsic disorder in transcription factors. Biochemistry 45, 6873-6888.
Losson, R., and Nielsen, A.L. (2010). The NIZP1 KRAB and C2HR domains cross-talk for transcriptional regulation. Biochim. Biophys. Acta 1799, 463-468.
Lynch, V.J., Roth, J.J., and Wagner, G.P. (2006). Adaptive evolution of Hox-gene homeodomains after cluster duplications. BMC Evol. Biol. 6, 86.
Mallo, M., and Alonso, C.R. (2013). The regulation of Hox gene expression during animal development. Development 140, 3951-3963.
Mann, R.S., and Hogness, D.S. (1990). Functional dissection of Ultrabithorax proteins in D. melanogaster. Cell 60, 597-610.
Margolin, J.F., Friedman, J.R., Meyer, W.K., Vissing, H., Thiesen, H.J., and Rauscher, F.J. (1994). Krüppel-associated boxes are potent transcriptional repression domains. Proc. Natl. Acad. Sci. U. S. A. 91, 4509-4513.
Martin, R.M., Ter-Avetisyan, G., Herce, H.D., Ludwig, A.K., Lättig-Tünnemann, G., and Cardoso, M.C. (2015). Principles of protein targeting to the nucleolus. Nucleus 6, 314-325.
McWilliam, H., Li, W., Uludag, M., Squizzato, S., Park, Y.M., Buso, N., Cowley, A.P., and Lopez, R. (2013). Analysis Tool Web Services from the EMBL-EBI. Nucleic Acids Res. 41, W597- W600.
Mifsud, W., and Bateman, A. (2002). Membrane-bound progesterone receptors contain a cytochrome b5-like ligand-binding domain. Genome Biol. 3, RESEARCH0068.
Mitchell, P.J., and Tjian, R. (1989). Transcriptional regulation in mammalian cells by sequence-specific DNA binding proteins. Science 245, 371-378.
Mitrea, D.M., Cika, J.A., Guy, C.S., Ban, D., Banerjee, P.R., Stanley, C.B., Nourse, A., Deniz, A.A., and Kriwacki, R.W. (2016). Nucleophosmin integrates within the nucleolus via multi-modal interactions with proteins displaying R-rich linear motifs and rRNA. Elife 5.
Moussa, H.F., Bsteh, D., Yelagandula, R., Pribitzer, C., Stecher, K., Bartalska, K., Michetti, L., Wang, J., Zepeda-Martinez, J.A., Elling, U., et al. (2019). Canonical PRC1 controls sequence-independent propagation of Polycomb-mediated gene silencing. Nat. Commun. 10, 1931.
Najafabadi, H.S., Mnaimneh, S., Schmitges, F.W., Garton, M., Lam, K.N., Yang, A., Albu, M., Weirauch, M.T., Radovani, E., Kim, P.M., et al. (2015). C2H2 zinc finger proteins greatly expand the human regulatory lexicon. Nat. Biotechnol. 33, 555-562.
Nevado, J., Gaudreau, L., Adam, M., and Ptashne, M. (1999). Transcriptional activation by artificial recruitment in mammalian cells. Proceedings of the National Academy of Sciences 96, 2674-2677.
Nott, A., Holtman, I.R., Coufal, N.G., Schlachetzki, J.C.M., Yu, M., Hu, R., Han, C.Z., Pena, M., Xiao, J., Wu, Y., et al. (2019). Brain cell type-specific enhancer-promoter interactome maps and disease-risk association. Science 366, 1134-1139.
Partridge, E.C., Christopher Partridge, E., Chhetri, S.B., Prokop, J.W., Ramaker, R.C., Jansen, C.S., Goh, S.-T., Mackiewicz, M., Newberry, K.M., Brandsmeier, L.A., et al. (2020). Occupancy maps of 208 chromatin-associated proteins in one human cell type. Nature 583, 720-728.
Peng, H., Gibson, L.C., Capili, A.D., Borden, K.L.B., Osborne, M.J., Harper, S.L., Speicher, D.W., Zhao, K., Marmorstein, R., Rock, T.A., et al. (2007). The Structurally Disordered KRAB Repression Domain Is Incorporated into a Protease Resistant Core upon Binding to KAP-1-RBCC Domain. Journal of Molecular Biology 370, 269-289.
Peng, H., Ivanov, A.V., Oh, H.J., Lau, Y.-F.C., and Rauscher, F.J., 3rd (2009). Epigenetic gene silencing by the SRY protein is mediated by a KRAB-O protein that recruits the KAP1 corepressor machinery. J. Biol. Chem. 284, 35670-35680.
Pupko, T., Bell, R.E., Mayrose, I., Glaser, F., and Ben-Tal, N. (2002). Rate4Site: an algorithmic tool for the identification of functional regions in proteins by surface mapping of evolutionary determinants within their homologues. Bioinformatics 18 Suppl 1, S71-S77.
Ramalingam, A., and Prendergast, G.C. (2007). Bin1 homolog hob1 supports a Rad6-Set1 pathway of transcriptional repression in fission yeast. Cell Cycle 6, 1655-1662.
Ravarani, C.N.J., Erkina, T.Y., De Baets, G., Dudman, D.C., Erkine, A.M., and Babu, M.M. (2018). High-throughput discovery of functional disordered regions: investigation of transactivation domains. Mol. Syst. Biol. 14, e8190.
Roney, I.J., Rudner, A.D., Couture, J.-F., and Kærn, M. (2016). Improvement of the reverse tetracycline transactivator by single amino acid substitutions that reduce leaky target gene expression to undetectable levels. Sci. Rep. 6, 27697.
Rueden, C.T., Schindelin, J., Hiner, M.C., DeZonia, B.E., Walter, A.E., Arena, E.T., and Eliceiri, K.W. (2017). ImageJ2: ImageJ for the next generation of scientific image data. BMC Bioinformatics 18, 529.
Sadowski, I., Ma, J., Triezenberg, S., and Ptashne, M. (1988). GAL4-VP16 is an unusually potent transcriptional activator. Nature 335, 563-564.
Schmitges, F.W., Radovani, E., Najafabadi, H.S., Barazandeh, M., Campitelli, L.F., Yin, Y., Jolma, A., Zhong, G., Guo, H., Kanagalingam, T., et al. (2016). Multiparameter functional diversity of human C2H2 zinc finger proteins. Genome Res. 26, 1742-1752.
Schnabel, C.A., and Abate-Shen, C. (1996). Repression by HoxA7 is mediated by the homeodomain and the modulatory action of its N-terminal-arm residues. Mol. Cell. Biol. 16, 2678-2688.
Sidore, A.M., Plesa, C., Samson, J.A., Lubock, N.B., and Kosuri, S. (2020). DropSynth 2.0: high-fidelity multiplexed gene synthesis in emulsions. Nucleic Acids Res.
Sievers, F., Wilm, A., Dineen, D., Gibson, T.J., Karplus, K., Li, W., Lopez, R., McWilliam, H., Remmert, M., Söding, J., et al. (2011). Fast, scalable generation of high-quality protein multiple sequence alignments using Clustal Omega. Mol. Syst. Biol. 7, 539.
Sievers, Q.L., Petzold, G., Bunker, R.D., Renneville, A., S 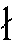 abicki, M., Liddicoat, B.J., Abdulrahman, W., Mikkelsen, T., Ebert, B.L., and Thomä, N.H. (2018). Defining the human C2H2 zinc finger degrome targeted by thalidomide analogs through CRBN. Science *362.*
Sims, R.J., 3rd, Nishioka, K., and Reinberg, D. (2003). Histone lysine methylation: a signature for chromatin function. Trends Genet. 19, 629-639.
Sirri, V., Urcuqui-Inchima, S., Roussel, P., and Hernandez-Verdun, D. (2008). Nucleolus: the fascinating nuclear body. Histochem. Cell Biol. 129, 13-31.
Staller, M.V., Holehouse, A.S., Swain-Lenz, D., Das, R.K., Pappu, R.V., and Cohen, B.A. (2018). A High-Throughput Mutational Scan of an Intrinsically Disordered Acidic Transcriptional Activation Domain. Cell Syst 6, 444-455.e6.
Stielow, B., Finkernagel, F., Stiewe, T., Nist, A., and Suske, G. (2018). MGA, L3MBTL2 and E2F6 determine genomic binding of the non-canonical Polycomb repressive complex PRC1.6. PLoS Genet. 14, e1007193.
Takahashi, H., Kasahara, K., and Kokubo, T. (2009). Saccharomyces cerevisiae Med9 comprises two functionally distinct domains that play different roles in transcriptional regulation. Genes Cells 14, 53-67.
UniProt Consortium (2015). UniProt: a hub for protein information. Nucleic Acids Res. 43, D204-D212.
Wang, R., Taylor, A.B., and Kim, C.A. (2010). Ring1B C-terminal domain/RYBP C-terminal domain Complex.
Waterhouse, A.M., Procter, J.B., Martin, D.M.A., Clamp, M., and Barton, G.J. (2009). Jalview Version 2--a multiple sequence alignment editor and analysis workbench. Bioinformatics 25, 1189-1191.
Wellik, D.M., and Capecchi, M.R. (2003). Hox10 and Hox11 genes are required to globally pattern the mammalian skeleton. Science 301, 363-367.
Witzgall, R., O'Leary, E., Leaf, A., Onaldi, D., and Bonventre, J.V. (1994). The Kriippel-associated box-A (KRAB-A) domain of zinc finger proteins mediates transcriptional repression. Proc. Natl. Acad. Sci. U. S. A. 91, 4514-4518.
Zhao, Y., and Potter, S.S. (2001). Functional specificity of the Hoxa13 homeobox. Development 128, 3197-3207.
Zhao, J., Wang, M., Chang, L., Yu, J., Song, A., Liu, C., Huang, W., Zhang, T., Wu, X., Shen, X., et al. (2020). RYBP/YAF2-PRC1 complexes and histone H1-dependent chromatin compaction mediate propagation of H2AK119ub1 during cell division. Nat. Cell Biol. 22, 439-452.
Zulkower, V., and Rosser, S. (2020). DNA Chisel, a versatile sequence optimizer. Bioinformatics.

## Claims

1. A synthetic transcription factor comprising one or more transcriptional activator domains fused to a heterologous DNA binding domain,
wherein at least one of the one or more transcriptional activator domains comprises an activator domain having an amino acid sequence having at least 95% identity to SEQ ID NO: 563

2. The synthetic transcription factor of claim 1, wherein the at least one activator domain comprises an amino acid sequence of SEQ ID NO: 563 or SEQ ID NO: 564.

3. The synthetic transcription factor of claim 1 or 2, wherein the activator domain is 80 amino acids in length or less.

4. The synthetic transcription factor of any of claims 1-3, wherein the one or more transcriptional activator domains further comprise: an activator domain having an amino acid sequence having at least 90% identity to SEQ ID NO: 579; an activator domain having an amino acid sequence having at least 90% identity to SEQ ID NO: 565; or a combination thereof.

5. The synthetic transcription factor of claim 4, wherein the one or more transcriptional activator domains comprise an activator domain having an amino acid sequence having at least 90% identity to SEQ ID NO: 580; an activator domain having an amino acid sequence having at least 90% identity to SEQ ID NO: 566; or a combination thereof.

6. The synthetic transcription factor of any of claims 1-5, wherein the heterologous DNA binding domain is a programmable DNA binding domain or a DNA binding domain derived from a Clustered Regularly Interspaced Short Palindromic Repeats associated (Cas) protein.

7. A nucleic acid encoding a synthetic transcription factor of any of claims 1-6.

8. The nucleic acid of claim 7, wherein the nucleic acid is under control of an inducible promoter or a tissue specific promoter.

9. A cell comprising at least one synthetic transcription factor of any of claims 1-6, or a nucleic acid of claims 7 or 8.

10. A system comprising at least one synthetic transcription factor of any of claims 1-6 and, optionally, a guide RNA or a nucleic acid encoding a guide RNA.

11. A method of modulating the expression of at least one target gene in a cell in vitro, the method comprising introducing into the cell at least one synthetic transcription factor of any of claims 1-6 or a nucleic acid encoding thereof.

12. The method of claim 11, wherein the gene expression of the at least one target gene is increased as compared to normal gene expression levels for the at least one target gene.

13. The method of claim 11 or 12, wherein the synthetic transcription factor comprises a Cas protein DNA binding domain and the method further comprises contacting the cell with at least one guide RNA or a nucleic acid encoding thereof.

14. A composition comprising a synthetic transcription factor of any of claims 1-6 or a nucleic acid of encoding thereof for use in modulating the expression of at least one target gene in a subject.

15. The composition of claim 14, wherein the composition increases gene expression of the at least one target gene as compared to normal gene expression levels for the at least one target gene.

## Patentansprüche

1. Synthetischer Transkriptionsfaktor, der eine oder mehrere Transkriptionsaktivatordomänen umfasst, die an eine heterologe DNA-Bindungsdomäne fusioniert sind,
wobei mindestens eine der einen oder mehreren Transkriptionsaktivatordomänen eine Aktivatordomäne umfasst, deren Aminosäuresequenz eine Identität von mindestens 95 % zu SEQ ID NO: 563 aufweist.

2. Synthetische Transkriptionsfaktor nach Anspruch 1, wobei die mindestens eine Aktivatordomäne eine Aminosäuresequenz von SEQ ID NO: 563 oder SEQ ID NO: 564 umfasst.

3. Synthetische Transkriptionsfaktor nach Anspruch 1 oder 2, wobei die Aktivatordomäne eine Länge von 80 Aminosäuren oder weniger aufweist.

4. Synthetische Transkriptionsfaktor nach einem der Ansprüche 1 bis 3, wobei die eine oder mehreren Transkriptionsaktivatordomänen ferner umfassen: eine Aktivatordomäne mit einer Aminosäuresequenz, die mindestens 90 % Identität zu SEQ ID NO: 579 aufweist; eine Aktivatordomäne mit einer Aminosäuresequenz, die mindestens 90 % Identität zu SEQ ID NO: 565 aufweist; oder eine Kombination davon.

5. Synthetische Transkriptionsfaktor nach Anspruch 4, wobei die eine oder mehreren Transkriptionsaktivatordomänen umfassen eine Aktivatordomäne mit einer Aminosäuresequenz, die mindestens 90 % Identität zu SEQ ID NO: 580 aufweist; eine Aktivatordomäne mit einer Aminosäuresequenz, die mindestens 90 % Identität zu SEQ ID NO: 566 aufweist; oder eine Kombination davon.

6. Synthetische Transkriptionsfaktor nach einem der Ansprüche 1 bis 5, wobei die heterologe DNA-Bindungsdomäne eine programmierbare DNA-Bindungsdomäne oder eine DNA-Bindungsdomäne ist, die von einem Cas- (Clustered Regularly Interspaced Short Palindromic Repeats associated) Protein abgeleitet ist.

7. Nukleinsäure, die einen synthetischen Transkriptionsfaktor nach einem der Ansprüche 1 bis 6 kodiert.

8. Nukleinsäure nach Anspruch 7, wobei die Nukleinsäure unter der Kontrolle eines induzierbaren Promotors oder eines gewebespezifischen Promotors steht.

9. Zelle, die mindestens einen synthetischen Transkriptionsfaktor gemäß einem der Ansprüche 1 bis 6 oder eine Nukleinsäure gemäß Anspruch 7 oder 8 umfasst.

10. System, das mindestens einen synthetischen Transkriptionsfaktor gemäß einem der Ansprüche 1 bis 6 und optional eine Leit-RNA oder eine für eine Leit-RNA kodierende Nukleinsäure umfasst.

11. Verfahren zur Modulation der Expression mindestens eines Zielgens in einer Zelle in vitro, wobei das Verfahren das Einbringen mindestens eines synthetischen Transkriptionsfaktors gemäß einem der Ansprüche 1 bis 6 oder einer dafür kodierenden Nukleinsäure in die Zelle umfasst.

12. Verfahren nach Anspruch 11, wobei die Genexpression des mindestens einen Zielgens im Vergleich zu normalen Genexpressionsniveaus für das mindestens eine Zielgen erhöht ist.

13. Verfahren nach Anspruch 11 oder 12, wobei der synthetische Transkriptionsfaktor eine Cas-Protein-DNA-Bindungsdomäne umfasst und das Verfahren ferner das Inkontaktbringen der Zelle mit mindestens einer Leit-RNA oder einer dafür kodierenden Nukleinsäure umfasst.

14. Zusammensetzung, umfassend einen synthetischen Transkriptionsfaktor gemäß einem der Ansprüche 1 bis 6 oder eine Nukleinsäure, die dafür kodiert, zur Verwendung bei der Modulation der Expression mindestens eines Zielgens in einem Subjekt.

15. Zusammensetzung nach Anspruch 14, wobei die Zusammensetzung die Genexpression des mindestens einen Zielgens im Vergleich zu normalen Genexpressionsniveaus für das mindestens eine Zielgen erhöht.

## Revendications

1. Facteur de transcription synthétique comprenant un ou plusieurs domaines activateurs transcriptionnels fusionnés à un domaine de liaison d'ADN hétérologue, dans lequel au moins un du ou des domaines activateurs transcriptionnels comprend un domaine activateur ayant une séquence d'acides aminés ayant au moins 95 % d'identité avec SEQ ID NO: 563.

2. Facteur de transcription synthétique selon la revendication 1, dans lequel l'au moins un domaine activateur comprend une séquence d'acides aminés de SEQ ID NO: 563 ou SEQ ID NO: 564.

3. Facteur de transcription synthétique selon la revendication 1 ou 2, dans lequel le domaine activateur a une longueur de 80 acides aminés ou moins.

4. Facteur de transcription synthétique selon l'une quelconque des revendications 1 à 3, dans lequel le ou les domaines activateurs transcriptionnels comprennent en outre : un domaine activateur ayant une séquence d'acides aminés ayant au moins 90 % d'identité avec SEQ ID NO: 579 ; un domaine activateur ayant une séquence d'acides aminés ayant au moins 90 % d'identité avec SEQ ID NO: 565 ; ou une combinaison correspondante.

5. Facteur de transcription synthétique selon la revendication 4, dans lequel le ou les domaines activateurs transcriptionnels comprennent un domaine activateur ayant une séquence d'acides aminés ayant au moins 90 % d'identité avec SEQ ID NO: 580 ; un domaine activateur ayant une séquence d'acides aminés ayant au moins 90 % d'identité avec SEQ ID NO: 566 ; ou une combinaison correspondante.

6. Facteur de transcription synthétique selon l'une quelconque des revendications 1 à 5, dans lequel le domaine de liaison d'ADN hétérologue est un domaine de liaison d'ADN programmable ou un domaine de liaison d'ADN dérivé d'une protéine associée à de courtes répétitions palindromiques groupées et régulièrement espacées (Cas).

7. Acide nucléique codant pour un facteur de transcription synthétique selon l'une quelconque des revendications 1 à 6.

8. Acide nucléique selon la revendication 7, dans lequel l'acide nucléique est sous le contrôle d'un promoteur inductible ou d'un promoteur spécifique de tissu.

9. Cellule comprenant au moins un facteur de transcription synthétique selon l'une quelconque des revendications 1 à 6, ou un acide nucléique selon la revendication 7 ou 8.

10. Système comprenant au moins un facteur de transcription synthétique selon l'une quelconque des revendications 1 à 6 et, éventuellement, un ARN guide ou un acide nucléique codant pour un ARN guide.

11. Procédé de modulation de l'expression d'au moins un gène cible dans une cellule *in vitro*, le procédé comprenant l'introduction dans la cellule d'au moins un facteur de transcription synthétique selon l'une quelconque des revendications 1 à 6 ou d'un acide nucléique codant pour celui-ci.

12. Procédé selon la revendication 11, dans lequel l'expression génique de l'au moins un gène cible est augmentée par rapport à des niveaux normaux d'expression génique pour l'au moins un gène cible.

13. Procédé selon la revendication 11 ou 12, dans lequel le facteur de transcription synthétique comprend un domaine de liaison d'ADN de protéine Cas et le procédé comprend en outre la mise en contact de la cellule avec au moins un ARN guide ou un acide nucléique codant pour celui-ci.

14. Composition comprenant un facteur de transcription synthétique selon l'une quelconque des revendications 1 à 6 ou un acide nucléique codant pour celui-ci pour une utilisation dans la modulation de l'expression d'au moins un gène cible chez un sujet.

15. Composition selon la revendication 14, dans laquelle la composition augmente l'expression génique de l'au moins un gène cible par rapport à des niveaux normaux d'expression génique pour l'au moins un gène cible.
